# EUROPEAN PATENT SPECIFICATION

(11) **EP 0 934 065 B1**
(45) Date of publication and mention of the grant of the patent: **07.06.2006**
(21) Application number: 97939667.8
(22) Date of filing: 29.08.1997
(51) Int. Cl.: A61K 31/395, C07D 291/02, C07D 281/00, C07D 273/08, A61P 35/00

(54) **NOVEL CRYPTOPHYCIN DERIVATIVES AS ANTI-NEOPLASTIC AGENTS**
NEUE CRYPTOPHYCIN-DERIVATE ALS ANTINEOPLASTIKA
NOUVELLES AGENTS ANTINEOPLASTIQUES A BASE DES DERIVES DE LA CRYPTOPHYCINE

(30) Priority: 30.08.1996 US 25816 P; 26.02.1997 US 39113 P; 03.03.1997 US 39530 P; 04.03.1997 US 40029 P
(43) Date of publication of application: 11.08.1999
(73) Proprietor: UNIVERSITY OF HAWAII, Honolulu, HI 96822 (US); WAYNE STATE UNIVERSITY, Detroit MI 48202 (US)
(72) Inventor: AL-AWAR, Rima, S., Indianapolis, IN 46214 (US); EHLHARDT, William, J., Indianapolis, IN 46256 (US); GOTTUMUKKALA, Subbaraju, V., Tirupati, 517 502 (A.P.) (IN); MARTINELLI, Michael, J., Zionsville, IN 46007 (US); MOHER, Eric, D., Indianapolis, IN 46237 (US); MOORE, Richard, E., Honolulu, HI 96816 (US); MUNROE, John, E., Indianapolis, IN 46220 (US); NORMAN, Bryan, H., Indianapolis, IN 46236 (US); PATEL, Vinod, F., Carmel, IN 46032 (US); SHIH, Chuan, Carmel, IN 46033 (US); TOTH, John, E., Indianapolis, IN 46278 (US); VASUDEVAN, Venkatraghavan, Indianapolis, IN 46280 (US); RAY, James, E., Indianapolis, IN 46268 (US)
(74) Representative: Srinivasan, Ravi Chandran
(86) International application number: PCT/US1997/015240
(87) International publication number: WO 1998/008505

(56) References cited:
- WO-A-95/17093
- WO-A-96/39829
- WO-A-96/40184
- WO-A-97/07798
- WO-A-97/08334
- WO-A-97/23211
- WO-A-97/31632
- US-A- 4 845 085
- US-A- 4 845 086
- US-A- 4 946 835
- US-A- 5 194 605

## Description

This invention relates to the fields of pharmaceutical and organic chemistry and provides novel cryptophycin compounds useful as anti-microtubule agents.

Neoplastic diseases, characterized by the proliferation of cells not subject to the normal control of cell growth, are a major cause of death in humans and other mammals. Clinical experience in cancer chemotherapy has demonstrated that new and more effective drugs are desirable to treat these diseases.

The microtubule system of eucaryotic cells is a major component of the cytoskeleton and is a dynamic assembly and disassembly; this is heterodimers of tubulin are polymerized and form microtubule. Microtubules play a key role in the regulation of cell architecture, metabolism, and division. The dynamic state of microtubules is critical to their normal function. With respect to cell division, tubulin is polymerized into microtubules that form the mitotic spindle.

The microtubules are then depolymerized when the mitotic spindle's use has been fulfilled. Accordingly, agents which disrupt the polymerization or depolymerization of microtubules, and thereby inhibit mitosis, comprise some of the most effective cancer chemotherapeutic agents in clinical use.

Additionally, the compounds claimed herein possess fungicidal properties as well. Further, such agents having the ability to disrupt the microtubule system can be useful for research purposes.

Certain cryptophycin compounds are known in the literature; however, cryptophycin compounds having even greater solubility and stability are desired for most pharmaceutical uses. Further, a broader library of cryptophycin compounds could provide additional treatment options for the patient suffering from cancer. Applicants have now discovered novel compounds which can provide greater aqueous solubility as well as compounds having the ability to disrupt the microtubule system. Compounds of this invention can be useful for the treatment of neoplasms. Such compounds can be prepared using total synthetic methods and are therefore well suited for development as pharmaceutically useful agents.

The presently claimed invention provides compounds according to formula (I) 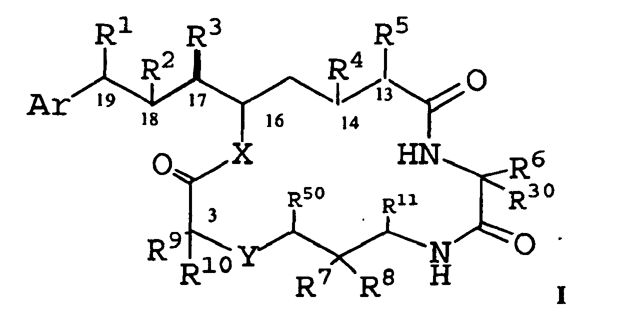 wherein:
Ar is selected from phenyl, an unsubstituted aromatic group, an aromatic group substituted with a halogen atom or a C₁-C₇ alkyl group, an unsubstituted 3- to 8-membered heteroaromatic group, C₁-C₁₂ alkyl, C₂-C₁₂ alkenyl, C₂-C₁₂ alkynyl,
NR⁵¹R⁵², OR⁵³, and a group of formula Ar'; 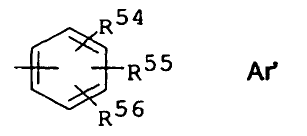
R¹ is selected from halogen and OH;
R² is halogen or OR³¹, provided that one, but not both, of R¹ and R² is halogen;
R³ is a C₁-C₆ alkyl group;
R⁴ is H or OH;
R⁵ is H or OH; or R⁴ and R⁵ may be taken together to form a second bond between C₁₃ and C₁₄;
R⁶ is selected from benzyl, hydroxybenzyl, (C₁-C₃ alkoxy)benzyl, halohydroxybenzyl, dihalohydroxybenzyl, halo(C₁-C₃ alkoxy)benzyl and dihalo(C₁₋C₃ alkoxy)benzyl groups;
R⁷ is H or a C₁-C₆ lower alkyl group;
R⁸ is H or a C₁-C₆ lower alkyl group;
R⁹ is H or a C₁-C₆ lower alkyl group;
R¹⁰ is H or a C₁-C₆ lower alkyl group;
R¹¹ is hydrogen;
R³¹ is R³²;
R³² is selected from naturally occurring amino acids, C₁₂H₂₂O₁₁ and C₆H₁₀O₅ carbohydrates, C₁₂H₂₂O₁₁ and C₆H₁₀O₅ carbohydrates which carry from 1 to 3 amino substituents, (saccharide)_{q} wherein saccharide is lactose, maltose, sucrose, fructose or starch and C(O)R³³;
R³³ is R³⁷R³⁸;
R³⁷ is (C₁-C₆)alkyl;
R³⁸ is COOR³⁹, 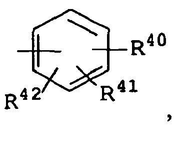 NH₂ or a naturally occurring amino acid;
R³⁹ is H or (C₁-C₆)alkyl;
R⁴⁰, R⁴¹, and R⁴² are each independently selected from hydrogen, OR⁴³, halo, NH₂, NO₂, OPO(OR⁴⁶)₂, OR⁴⁴phenyl, and R⁴⁵;
R⁴³ is C₁-C₆ alkyl;
R⁴⁴ is C₁-C₆ alkylene;
R⁴⁵ is selected from an aromatic group and an aromatic group substituted with a hydrogen atom or a C₁-C₇ alkyl group;
R⁴⁶ is selected from H, Na, and -C(CH₃)₃;
R⁵¹ is selected from hydrogen and C₁-C₃ alkyl;
R⁵² is selected from hydrogen and C₁-C₃ alkyl;
R⁵³ is selected from C₁-C₁₂ alkyl;
R⁵⁴ is selected from hydrogen, C₁-C₆ alkyl, an aromatic group, phenyl, COOR⁵⁷, PO₃H, SO₃H, SO₂R⁵⁸, NR⁵⁹R⁶⁰, NHOR⁶¹, NHCH₂R^{61'}, CN, NO₂, halogen, OR⁶², and SR⁶³;
R⁵⁵ is selected from hydrogen, C₁-C₆ alkyl, an aromatic group, phenyl, COOR⁵⁷, PO₃H, SO₃H, SO₂R⁵⁸, NR⁵⁹R⁶⁰, NHOR⁶¹, NHCH₂R^{61'}, CN, NO₂, halogen, OR⁶², and SR⁶³;
R⁵⁶ is selected from hydrogen, C₁-C₆ alkyl, an aromatic group, phenyl, COOR⁵⁷, PO₃H, SO₃H, SO₂R⁵⁸, NR⁵⁹R⁶⁰, NHOR⁶¹, NHCH₂R^{61'}, CN, NO₂, halogen, OR⁶², and SR⁶³;
R⁵⁷ is selected from hydrogen and C₁-C₁₂ alkyl;
R⁵⁸ is selected from hydrogen and C₁-C₁₂ alkyl;
R⁵⁹ is selected from hydrogen, (C₁-C₆) alkyl and fluorenylmethoxycarbonyl (FMOC);
R⁶⁰ is selected from hydrogen and (C₁-C₆) alkyl;
R⁶¹ is selected from hydrogen, OR⁶⁴, CH₂NHR⁶⁵, NHR⁶⁵ and fluorenylmethoxycarbonyl (FMOC);
R^{61'} is selected from hydrogen, OR⁶⁴, CH₂NHR⁶⁵, NHR⁶⁵ and fluorenylmethoxycarbonyl (FMOC);
R⁶² is selected from hydrogen and C₁-C₆ alkyl;
R⁶³ is selected from hydrogen and C₁-C₆ alkyl;
R⁶⁴ is selected from hydrogen, (C₁-C₆) alkyl, and CH₂NR⁶⁶R⁶⁷;
R⁶⁵ is selected from hydrogen, C₁-C₆ alkyl, NH₂, and fluorenylmethoxycarbonyl (FMOC);
R⁶⁶ is selected from hydrogen, C₁-C₆ alkyl and fluorenylmethoxycarbonyl (FMOC);
R⁶⁷ is selected from hydrogen and C₁-C₆ alkyl;
q is 2, 3, or 4;
X is O, NH or (C₁-C₇ alkyl)amino; and
Y is C, O, NH, S, SO, SO₂ or (C₁-C₇ alkyl)amino;
or a pharmaceutically acceptable salt or solvate thereof.

Typically Y is O. Typically X is O.

Typically, R⁶ is benzyl, hydroxybenzyl, halohydroxybenzyl, dihalohydroxybenzyl, haloalkoxybenzyl or dihaloalkoxybenzyl, R⁹ is isobutyl and R¹⁰ is hydrogen.

Typically R⁷ and R⁸ are each methyl.

Typically, R² is a glycinate.

The present invention also provides a pharmaceutical formulation comprising a compound of the invention and one or more pharmaceutically acceptable carrier or diluent. Also provided is a compound according to the invention, for use in treating the human or animal body, in particular for use in treating a neoplasm in a mammal or a fungal infection in a mammal.

The present invention also provides the use of a compound of the invention in the manufacture of a medicament for use in treating a neoplasm in a mammal or a fungal infection in a mammal.

As used herein, a said C₁-C₇ alkyl group may be saturated, unsaturated, branched, or straight chain. Examples include methyl, ethyl, n-propyl, iso-propyl, n-butyl, propenyl, ethenyl, sec-butyl, n-pentyl, isobutyl, tert-butyl, sec-butyl, methylated butyl groups, pentyl, tert pentyl, sec-pentyl and methylated pentyl groups. The term "alkenyl" refers to an alkyl group, as defined above, having from one to three double bonds. The term "alkynyl" refers to an alkyl group, as defined above, having at least one triple bond. It is especially preferred that alkynyl has only one triple bond. The term C₁-C_{n'} alkyl; wherein n' is an integer from 2 to 12 means an alkyl group having from one to the indicated number of carbon atoms. The C₁-C_{n'} alkyl can be straight or branched chain.

As used herein, the term "alkoxybenzyl" refers to a benzyl group having an alkoxy substituent at any available position on the benzyl ring. Methoxy is especially preferred. Accordingly, the term "haloalkoxybenzyl" refers to a benzyl group having a halo substituent in addition to an alkoxy substituent. Each halo or alkoxy group is substituted at any available carbon. Similarly, "halohydroxybenzyl" refers to a hydroxy substituted benzyl group that also has a halo substituent at any available carbon on the benzyl ring. Finally, the term "dihaloalkoxybenzyl" refers to an alkoxy substituted benzyl which additionally has two halo substituents each independently substituted at any available carbon on the benzyl ring.

"Substituted aromatic" refers to a group substituted with from one to three substituents selected from the group consisting of C₁-C₇ alkyl and halo.

The term "lower alkyl" shall refer to both saturated and unsaturated lower alkyl groups. (For example a saturated group has no double or triple bonds).

As used herein, unsubstituted aromatic group refers to common aromatic rings having 4n+2 electrons in a moncyclic or bicyclic conjugated system, for example furyl, pyrrolyl, thienyl, pyridyl, and the like, or a bicyclic conjugated system, for example indolyl or naphthyl.

As used herein "substituted aromatic group" refers to a said aromatic group substituted with a single group selected from the group consisting of halogen and C₁-C₆ alkyl group.

As used herein, "heteroaromatic" refers to aromatic rings which contain one or more non-carbon atoms selected from the group consisting of oxygen, nitrogen, and sulfur. An especially preferred group of heteroaromatic rings have from three to six members. It is particularly preferred that the heteroaromatic group will have from one to three non-carbon atoms in the ring. A five member ring containing one oxygen atom is one preferred heteroaromatic group.

As used herein, "halogen" or "halo" refers to those members of the group on the periodic table historically known as halogens. Methods of halogenation include the addition of hydrogen halides, substitution at high temperature, photohalogenation, and such methods are known to the skilled artisan. Especially preferred halogens include chloro, fluoro, and bromo.

As used herein, the term "mammal" shall refer to the Mammalia class of higher vertebrates. The term "animal" shall include mammals, reptiles, amphibians, and fish. The term "mammal" includes a human. The term "treating" as used herein includes prophylaxis of the named condition or amelioration or elimination of the condition once it has been established. The cryptophycin compounds claimed herein can be useful for veterinary health purposes as well as for the treatment of a human patient.

As used herein, the term "derivatizing" refers to standard chemical transformations known to the artisan which give access to desired compounds of this invention.

The presently claimed processes provide a means for preparing totally synthetic cryptophycin compounds. Conveniently, commercially available amino acids can be cyclized into the cryptophycin molecule.

Many of the known cryptophycin compounds have promising antitumor activity; however, poor aqueous solubility can present issues during intravenous administration of drug. Such issues are related to the use of solubilizing sufactants, such a Cremophor, which may possess inherent toxicity. The present invention provides new cryptophycin compounds having antitumor activity as well as greater aqueous solubility properties. Such compounds have desired solubility characteristics as well as acceptable potency.

The processes to prepare the compounds of this invention most preferably are completed in the presence of a solvent. The skilled artisan can select appropriate solvents using standard methodologies. Suitable inert organic solvents include those known to the skilled artisan, for example tetrahydrofuran (THF) and dimethylformamide (DHF). DMF is especially preferred. Aqueous based solvents may be appropriate for some of the processes utilized herein. The pH of such aqueous solvent s may be adjusted as desired to facilitate the process.

Some typical compounds of this invention are provided in tabular form.

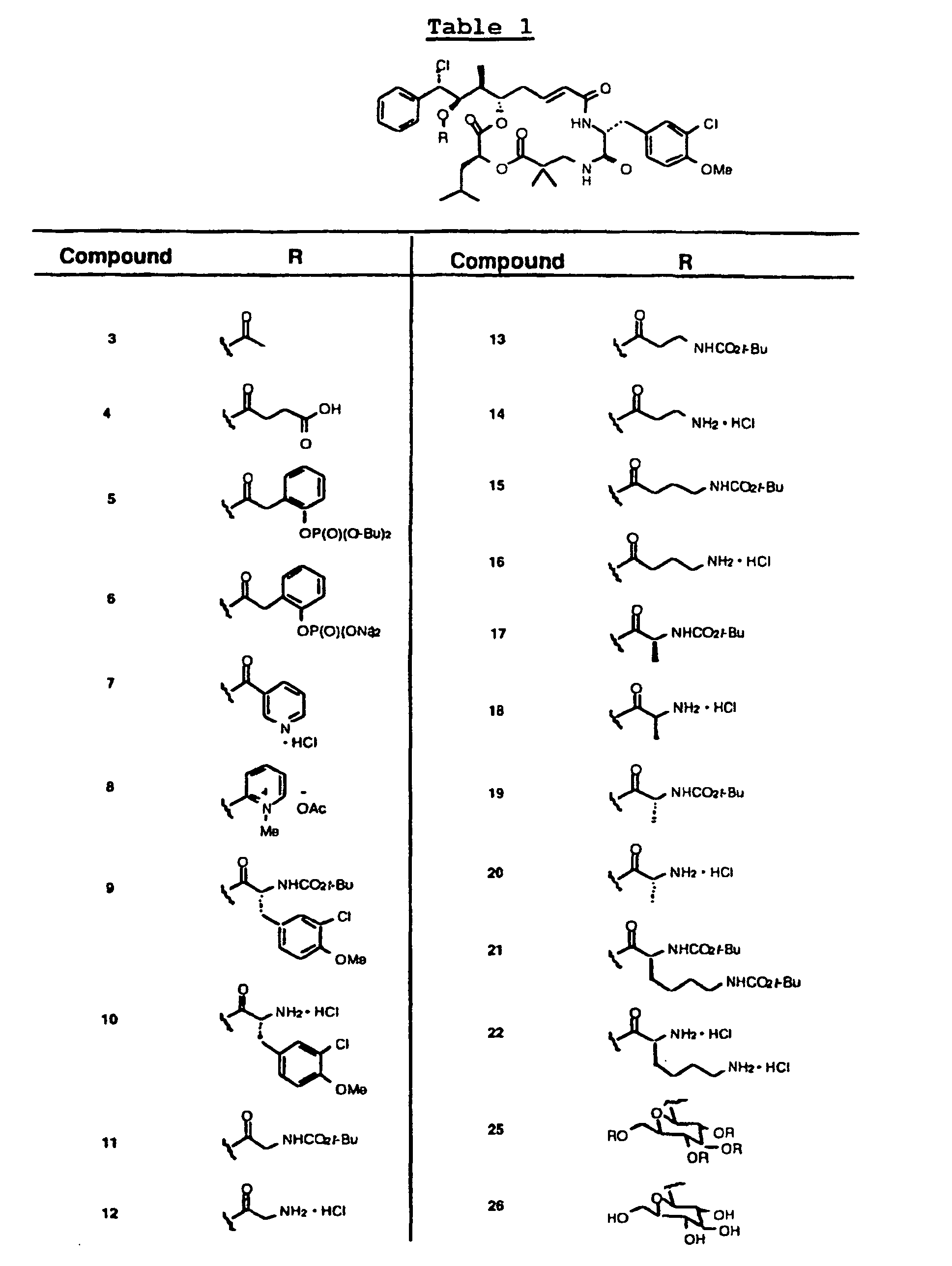

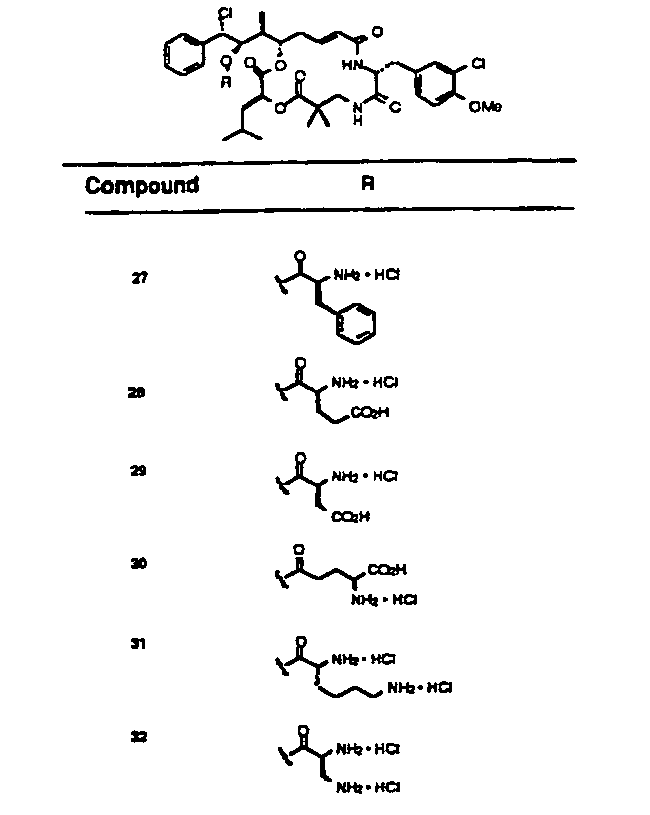

Additional compounds of interest are as named by Table I; however, Ar is Ar' instead of phenyl and R⁵⁴ is OCH₃ at the para position of Ar', while R⁵⁵ and R⁵⁶ are each hydrogen.

Further compounds of interest are as named by Table I; however, an NH group replaces O at the Y position of the molecule. For example, one compound in this series is:

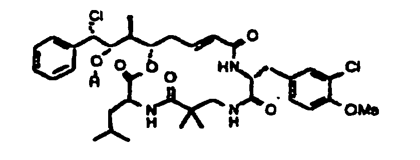

Further preferred compounds are, for example, but not limited to, those named above in Table I, wherein each named compound has a single methyl group in place of the gem dimethyl group at position 6 of the cryptophycin ring. See, for example, 23 below.

Especially preferred compounds of this invention have a dimethyl group at position 6 of the cryptophycin ring.

A preferred compound of this invention is as presented in Table I wherein Ar is Ar'; only one of R⁵⁴, R⁵⁵, R⁵⁶ is OCH₃; R⁹ and R¹⁰ are each methyl.

Generally known silylating agents are employed in the processes for making compounds of this invention. See for example, Calvin, E.W., "Silicon Reagents in Organic Synthesis", Academic Press, (London, 1988). Particularly useful silylating agents include "tri-lower alkyl silyl" agents, the term of which contemplates triisopropylsilyl, trimethylysilyl and triethylsilyl, trimethylsilyl halides, silylated ureas such as bis(trimethylsilyl)urea (BSU) and silylated amides such as N,O-bis(trimethylsilyl)acetamide (BSA). Of these, BSA is preferred.

Some preferred characteristics of this invention are set forth in the following tabular form wherein the features may be independently selected to provide preferred embodiments of this invention:
A) R⁸ is ethyl, propyl, isopropyl, butyl, isobutyl or isopentyl;
B) R⁷ is ethyl, propyl, isopropyl, butyl, isobutyl, pentyl, or isopentyl;
C) R⁷ is H, R⁸ is methyl, R³ is methyl, and X and Y are not both O;
D) R³ is ethyl, propyl, isopropyl, butyl, isobutyl, pentyl or isopentyl;
E) R⁹ is methyl, ethyl, propyl, butyl, isobutyl, pentyl, or isopentyl;
F) R¹⁰ is methyl, ethyl, propyl, butyl, isobutyl, pentyl, or isopentyl;
G) a cryptophycin compound wherein at least one of the groups selected from the group consisting of C-3, C-6, C-7, C-10, C-16, C-17, andC-18 has R stereochemistry (numbering as set forth in Formula I supra.);
H) a cryptophycin compound wherein at least one of the groups selected from the group consisting of C-3, C-6, C-7, C-10, C-16, C-17, and C-18 has S stereochemistry (numbering as set forth in Formula I supra.);
I) Ar' is phenyl with substituent selected from the group consisting of NR⁵⁹R⁶⁰, NHOR⁶¹ and NHCHR^{61'} ;
J) a compound wherein Y is selected from the group consisting of alkylamino, NH, and O;
K) a compound wherein Y is O, R⁷ and R¹⁰ are each hydrogen; R⁹ is C₁-C₆ alkyl; and R¹ is halo;
L) R⁷, R⁸ are each methyl;
M) R⁷ is hydrogen;
N) R² is a glycinate;
O) R² is an acylate;
R) R⁴ and R⁵ form a double bond;
T) a compound of Formula I is used for the preparation of a medicament for use disruption of a microtubulin system;
U) a compound of Formula I is used the preparation of a medicament for use as a antineoplastic agent;
V) a compound of Formula I is used the preparation of a medicament for use for the treatment of cancer in a mammal;
W) a compound of Formula I is used the preparation of a medicament for use as an antifungal agent;
   R⁶ is selected from the group consisting

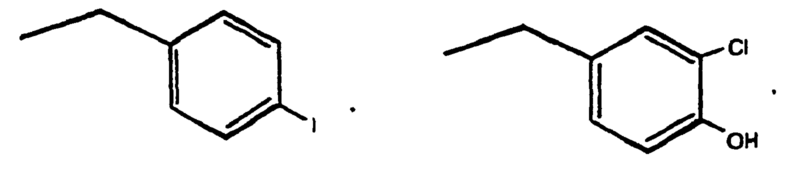

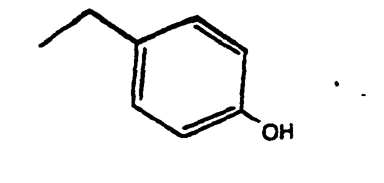

DD) Ar is phenyl;
EE) Ar is phenyl substituted with one or two from the group consisting of halo and methyl;
   a compound of Formula I is used for the preparation of a medicament for use in the treatment of fungal infection;
KK) R⁴ and R⁵ are each hydrogen;
LL) Ar is para ethyl substituted phenyl;
MM) Ar is para methyl substituted phenyl:
NN) Y is NH;
OO) R³ is methyl;

The compounds of the present invention can be used in a method of alleviating a pathological condition caused by hyperproliferating mammalian cells comprising administering to a subject an effective amount of a pharmaceutical or veterinary composition disclosed herein to inhibit proliferation of the cells. Said method can further comprise administering to the subject at least one additional therapy directed to alleviating the pathological condition. Said pathological condition is characterized by the formation of neoplasms. Typically, the neoplasms are selected from the group consisting of mammary, small-cell lung, non-small-cell lung, colorectal, leukemia, melanoma, pancreatic adenocarcinoma, central nervous system (CNS), ovarian, prostate, sarcoma of soft tissue or bone, head and neck, gastric which includes pancreatic and esophageal, stomach, myeloma, bladder, renal, neuroendocrine which includes thyroid and non-Hodgkins disease and Hodgkin's disease neoplasms.

As used herein "neoplastic" refers to a neoplasm, which is an abnormal growth, such growth occurring because of a proliferation of cells not subject to the usual limitations of growth. As used herein, "anti-neoplastic agent" is any compound, composition, admixture, co-mixture, or blend which inhibits, eliminates, retards, or reverses the neoplastic phenotype of a cell.

Anti-mitotic agents may be classified into three groups on the basis of their molecular mechanism of action. The first group consists of agents, including colchicine and colcemid, which inhibit the formation of microtubules by sequestering tubulin. The second group consists of agents, including vinblastine and vincristine, which induce the formation of paracrystalline aggregates fo tubulin. Vinblastine and vincristine are well known anticancer drugs: their action of disrupting mitotic spindle microtubules preferentially inhibits hyperproliferative cells.' The third group consist of agents, including taxol, which promote the polymerization of tubuline and thus stabilizes microtubules.

The exhibition of drug resistance and multiple-drug resistance phenotype by many tumor cells and the clinically proven mode of action of anti-microtubile agents against neoplastic cells necessitates the development of anti-microtubile agents cytotoxic to non-drug resistance neoplastic cells as well as cytotoxic to neoplastic cells with a drug resistant phenotype.

Chemotherapy, surgery, radiation therapy, therapy with biological response modifiers, and immunotherapy are currently used in the treatment of cancer. Each mode of therapy has specific indications which are known to those of ordinary skill in the art, and one or all may be employed in an attempt to achieve total destruction of neoplastic cells. Moreover, combination chemotherapy, chemotherapy utilizing compounds of Formula I in combination with other neoplastic agents, is also provided by the subject invention as combination therapy is generally more effective than the use of a single anti-neoplastic agent. Thus, a further aspect of the present invention provides compositions containing a therapeutically effective amount of at least one compound of Formula I, including the non-toxic addition salts thereof, which serve to provide the above recited benefits. Such compositions can also be provided together with physiologically tolerable liquid, gel, or solid carriers, diluents, adjuvants and excipients. Such carriers, adjuvants, and excipients may be found in the U.S. *Pharmacopoeia,* Vol. XXII and National Formulary Vol. XVII, U.S. *Pharmacopoeia* Convention, Inc., Rockville, MD (1989). Additional modes of treatment are provided in AHFS Drug Information, 1993 e. By the American Hospital Formulary Service, pp. 522-660. Each of these references are well known and readily available to the skilled artisan.

The present invention further provides a pharmaceutical composition used to treat neoplastic disease containing at least one compound of Formula I and at least one additional anti-neoplastic agent. Anti-neoplastic agents which may be utilized in combination with Formula I compounds include those provided in the Merck Index 11, pp 16-17, Merck & Co., Inc. (1989). The Merck Index is widely recognized and readily available to the skilled artisan.

In a further embodiment of this invention, antineoplastic agents may be antimetabolite which may include but are in no way limited to those selected from the group consisting of methotrexate, 5-fluorouracil, 6-mercaptopurine, cytosine, arabinoside, hydroxyurea, and 2-chlorodeoxyadenosine. In another embodiment of the present invention, the anti-neoplastic agents contemplated are alkylating agents which may include but are in no way limited to those selected from the group consisting of cyclophosphamide, mephalan, busulfan, paraplatin, chlorambucil, and nitrogen mustard. In a further embodiment, the anti-neoplastic agents are plant alkaloids which may include but are in no way limited to those selected fromt he group consisting of vincristine, vinblastine, taxol, and etoposide. In a further embodiment, the anti-neoplastic agents contemplated are antibiotics which may include, but ae in no way limited to those selected from the group consisting of doxorubicin, daunorubicin, mitomycin C, and bleomycin. In a further embodiment, the anti-neoplastic agents contemplated are hormone which may include, but are in no way limited to those selected fromt he group consisting of calusterone, diomostavolone, propionate, epitiostanol, mepitiostane, testolactone, tamoxifen, polyestradiol phosphate, megesterol acetate, flutamide, nilutamide, and trilotane.

In a further embodiment, the anti-neoplastic agents contemplated include enzymes which may include those selected from the group consisting of L-Asparginase and aminoacridine derivatives such as, but not limited to, amsacrine. Additional anti-neoplastic agents include those provided by Skeel, Roland T., "Antineoplastic Drugs and Biologic Response Modifier: Classification, Use and Toxicity of Clinically Useful Agents' Handbook of Cancer Chemotherapy (3^{rd} ed.), Little Brown & Co. (1991).

These compounds and compositions can be administered to mammals for veterinary use. For example, domestic animals can be treated in much the same way as a human clinical patient. In general, the dosage required for therapeutic effect will vary according to the type of use, mode of administration, as well as the particularized requirements of the individual hosts. Typically, dosages will range from about 0.001 to 1000 mg/kg, and more usually 0.01 to 10 mg/kg of the host body weight. Alternatively, dosages within these ranges can be administered by constant infusion over an extended period of time, usually exceeding 24 hours, until the desired therapeutic benefits are obtained. Indeed, drug dosage, as well as route of administration, must be selected on the basis of relative effectiveness, relative toxicity, growth characteristics of tumor and effect of Formula I compound on cell cycle, drug pharmacokinetics, age, sex, physical condition of the patient and prior treatment, which can be determined by the skilled artisan.

The compound of Formula I, with or without additional anti-neoplastic agents, may be formulated into therapeutic compositions as natural or salt forms. Pharmaceutically acceptable non-toxic salts include base addition salts which may be derived from inorganic bases such as for example, sodium, potassium, ammonium, calcium, or ferric hydroxides, and such organic bases as isopropylamine, trimethylamine, 2-ethylamino ethanol, histidine and procaine. Such salts may also be formed as acid addition salts with any free cationic groups and will generally be formed with inorganic acids such as for example, hydrochloric or phosphoric acids or organic acids such as acetic, oxalic, tartaric and mandelic. Additional excipients which further the invention are provided to the skilled artisan for example in the U.S. Pharmacopoeia.

The suitability of particular carriers for inclusion in a given therapeutic composition depends on the preferred route of administration. For example, anti-neoplastic compositions may be formulated for oral administration. Such compositions are typically prepared as liquid solution or suspensions or in solid forms. Oral formulation usually include such additives as binders, fillers, carriers, preservatives, stabilizing agents, emulsifiers, buffers, mannitol, lactose, starch, magnesium stearate, sodium saccharin, cellulose and magnesium carbonate. These compositions may take the form of solutions, suspensions, tablets, pills, capsules, sustained release formulations, or powders, and typically contain 1% to 95% of active ingredient. More preferably, the composition contains from about 2% to about 70% active ingredient.

Compositions of the present invention may be prepared as injectables, either as liquid solutions, suspensions, or emulsions; solid forms suitable for solution in or suspension in liquid prior to injection. Such injectables may be administered subcutaneously, intravenously, intraperitoneally, intramuscularly, intrathecally, or intrapleurally. The active ingredient or ingredients are often mixed with diluents, carriers, or excipients which are physiologically tolerable and compatible with the active ingredient(s). Suitable diluents and excipients are for example, water, saline, dextrose and glycerol and combinations thereof. In addition, if desired, the compositions may contain minor amounts of auxiliary substances such as wetting or emulsifying agents, stabilizing or pH buffering agents.

The compounds of the invention can be used in methods for using Formula I compounds to inhibit the proliferation of mammalian cells by contacting these cells with a Formula I compound in an amount sufficient to inhibit the proliferation of the mammalian cell. A preferred embodiment is a method to inhibit the proliferation of hyperproliferative mammalian cells. For purposes of this invention "hyperproliferative mammalian cells" are mammalian cells which are not subject to the characteristic limitations of growth (programmed cell death for example). A further preferred embodiment is when the mammalian cell is human. The compounds of the invention can also be used to contact the mammalian cell with at least one Formula I compound and at least one anti-neoplastic agent. The types of anti-neoplastic agents contemplated are discussed *supra.*

The compounds of the invention can also be used in methods in which a compound of Formula I inhibits the proliferation of hyperproliferative cells with drug-resistant phenotypes, including those with multiple drug-resistant phenotypes, by contacting said cell with a compound of Formula I in an amount sufficient to inhibit the proliferation of a hyperproliferative mammalian cell. A preferred embodiment is when the mammalian cell is human. The Formula I compound can be used with at least one additional anti-neoplastic agent, discussed *supra*.

The compounds of the invention can also be used in a method for alleviating pathological conditions caused by hyperproliferating mammalian cells for example, neoplasia, by administering to a subject an effective amount of a pharmaceutical composition containing Formula I compound to inhibit the proliferation of the hyperproliferating cells. As used herein "pathological condition" refers to any pathology arising from the proliferation of mammalian cells that are not subject to the normal limitations of growth. Such proliferation of cells may be due to neoplasms as discussed *supra.*

In a further preferred embodiment the neoplastic cells are human. Such pathological conditions can be alleviated by utilizing a compound of Formula I in combination with other therapies, as well as other anti-neoplastic agents.

The effectiveness of the claimed compound can be assessed using standard methods know to the skilled artisan.

Examples of such methods are as follows:
Compounds of this invention have been found to be useful against pathogenic fungi. For example, the usefulness for treating *Cryptococcus neoformans* can be illustrated with test results against *Cryptococcus neoformans* employing yeast nitrogen base dextrose agar medium. In carrying out the assay, a compound of this invention is solubiized in dimethyl sulfoxide supplemented with Tween 20. Twofold dilutions are made with sterile distilled water/10 percent DMSO to obtain final drug concentrations in the agar dilution assay plates ranging from 0.008 µg/ml to 16.0 µg/ml against an expanded panel of **84** *Cryptococcus neoformans* strains. The minimum inhibitory concentration against the panel of **84** *Cryptococcus neoformans* isolates is determined to illustrate the desired antifungal activity.

The compounds are screened for minimum inhibitory concentrations against KB, a human nasopharyngeal carcinoma cell line, LoVo, a human colorectal adenocarcinoma cell line using The Corbett assay, see Corbett, T.H. et al. Cytotoxic Anticancer Drugs: Models and Concepts for Drug Discovery and Development, pp 35-87, Kluwer Academic Publishers: Norwell, 1992. See also, Valeriote, et al. Discovery and Development of Anticancer Agents; Kluwer Academic Publisher, Norwell, 1993 is used for the evaluation of compounds.

The most active compounds are further evaluated for cytotoxicity against four different cell types, for example a murine leukemia, a murine solid tumor, a human solid tumor, and a low malignancy fibroblast using the Corbett assay.

The compounds are further evaluated against a broad spectrum of murine and human tumors implanted in mice, including drug resistant tumors.

Tumor burden (T/C) (mean tumor burden in treated animals versus mean tumor burden in untreated animals) are used as a further assessment. T/C values that are less than 42% are considered to be active by National Cancer Institute Standards; T/C values less than 10% are considered to have excellent activity and potential clincial activity by National Cancer Institute standard.

### Material

Vinblastine, cytochalasin B, tetramethylrhodamine isothiocyanate (TRITC)-phalloidin, sulforhodamine B (SRB) and antibodies against β-tubulin and vimentin are commercially available from recognized commercial vendors. Basal Medium Eagle containing Earle's salts (BME) and Fetal Bovine Serum (FBS) are also commercially available.

### Cell Lines

The Jurkat T cell leukemia line and A-10 rat aortic smooth muscle cells are obtained from the American Type Culture Collection and are cultured in BME containing 10% FBS and 50µg/ml gentamycin sulfate. Human ovarian carcinoma cells (SKOV3) and a sub-line which has been selected for resistance to vinblastine (SKVLBL) were a generous gift from Dr. Victor Ling of the Ontario Cancer Institute. Both cell lines are maintained in BME containing 10% FBS and 50µg/ml gentamycin sulfate. Vinblastine is added to a final concentration of 1µg/ml to SKVLB1 cells 24 hours after passage to maintain selection pressure for P-glycoprotein-overexpressing cells.

### Cell Proliferation and Cycle Arrest Assays

Cell proliferation assays are performed as described by Skehan et al. For Jurkat cells, cultures are treated with the indicated drugs as described in Skehan and total cell numbers are determined by counting the cells in a hemacytometer. The percentage of cells in mitosis are determined by staining with 0.4% Giemsa in PBS followed by rapid washes with PBS. At least 1000 cells per treatment are scored for the presence of mitotic figures and the mitotic index is calculated as the ration of the cells with mitotic figures to the total number of cells counted.

### Immunofluorescence Assays

A-10 cells are grown to near-confluency on glass coverslips in BME/10% FBS. Compounds in PBS are added to the indicated final concentrations and cells are incubated for an additional 24 hours. For the staining of microtubules and intermediate filaments, the cells are fixed with cold methanol and incubated with PBS containing 10% calf serum to block nonspecific binding sites. Cells are then incubated at 37°C for 60 min. With either monoclonal anti-β-tubulin or with monoclonal anti-vimentin at dilutions recommended by the manufacturer. Bound primary antibodies are subsequently visualized by a 45 minute incubation with fluorescein-conjugated rabbit antimouse IgG. The coverslips are mounted on microscope slides and the fluorescence patterns are examined and photographed using a Zeiss Photomicroscope I11 equipped with epifluorescence optics for fluorescein. For staining of microfilaments, cells are fixed with 3% paraformaldehyde, permeabilized with 0.2% Triton X-100 and chemically reduced with sodium borohydride (1mg/ML). PBS containing 100nM TRITC-phalloidin is then added and the mixture is allowed to incubate for 45 min. At 37°C. The cells are washed rapidly with PBS before the coverslips are mounted and immediately photographed as described above.

### Effects of cryptophycins and vinblastine on Jurkat cell proliferation and cell cycle

Dose-response curves for the effects of cryptophycin compounds and vinblastine on cell proliferation and the percentage of cells in mitosis are determined.

### Effects of cytochalasin B, vinblastine and cryptophycins on the cytoskeleton

Aortic smooth muscle (A-10) cells are grown on glass coverslips and treated with PBS, 2µM cytochalasin B, 100nM vinblastine or 10nM cryptophycin compounds. After 24 hours, microtubules and vimentin intermediate filaments are visualized by indirect immunofluorescence and microfilaments are stained using TRITC-phalloidin. The morphological effects of each drug is examined. Untreated cells displayed extensive microtubule networks complete with perinuclear microtubule organizing centers. Vimentin intermediate filaments were also evenly distributed throughout the cytoplasm, while bundles of microfilaments were concentrated along the major axis of the cell. Cytochalasin B caused complete depolymerization of microfilaments along with the accumulation of paracrystalline remnants. This compound did not affect the distribution of either microtubules or intermediate filaments. The cryptophycin treated microtubules and vimentin intermediates are observed for depletion of microtubules, and collapse of rimentin intermediate filaments.

### Effects of cryptophycins and vinblastine on taxol-stabilized microtubules

A-10 cells are treated for 3 hours with 0 or 10µM taxol before the addition of PBS, 100nM vinblastine or 10nM cryptophycin compound. After 24 hours, microtubule organization is examined by immunofluorescence as described above. Compared with those in control cells, microtubules in taxol-treated cells were extensively bundled, especially in the cell polar regions. As before, vinblastine caused complete depolymerization of microtubules non-pretreated cells. However, pretreatment with taxol prevented microtubule depolymerization in response to vinblastine. Similarly, microtubules pretreated with taxol are observed with cryptophycin treatment.

### Reversibility of microtubule depolymerization by vinblastine and cryptophycin

A-10 cells are treated with either 100nM vinblastine or 10nM cryptophycins for 24 hr., resulting in complete microtubule depolymerization. The cells are then washed and incubated in drug-free medium for periods of 1 hour or 24 hours. Microtubules repolymerized rapidly after the removal of vinblastine, showing significant levels of microtubules after 1 hour and complete morphological recovery by 24 hour. Cells are visualized for microtubule state after treatment with a cryptophycin compound of this invention at either 1 hour or 24 hours after removal of the cryptophycin compounds.

### Effects of combinations of vinblastine and cryptophycins on cell proliferation

SKOV3 cells are treated with combinations of cryptophycins and vinblastine for 48 hours. The percentages of surviving cells are then determined and the IC₅₀s for each combination is calculated.

### Toxicity of cryptophycins, vinblastine and taxol toward SKOV3 and SKVLB1 cells

SKVLB1 cells are resistant to natural product anticancer drugs because o their over expression of P-glycoprotein. The abilities of taxol, vinblastine and cryptophycin compounds to inhibit the growth of SKOV3 and SKVLB1 cells are observed. Taxol caused dose-dependent inhibition of the proliferation of both cell lines with IC₅₀s for SKOV3 and SKVLB1 cells of 1 and 8000nM, respectively. Vinblastine also inhibited the growth of both cell lines, with IC₅₀S of 0.35 and 4200nM for SKOV3 and SKVLB1 cells, respectively. Cryptophycins compounds of this invention demonstrate activity with an IC₅₀S of from about 1 to about 1000pM for SKOV3 and SKVLB1 cells.

Thus, it can be demonstrated that the present invention provides novel cryptophycin compounds which are potent inhibitors of cell proliferation, acting by disruption of the microtubule network and inhibition of mitosis. These studies can illustrate that cryptophycin compounds disrupt microtubule organization and thus normal cellular functions, including those of mitosis.

Classic anti-microtubule agents, such as colchicine and *Vinca* alkaloids, arrest cell division at mitosis. It seems appropriate to compare the effect of one of these agents on cell proliferation with the cryptophycin compounds. For this purpose, the *Vinca* alkaloid vinblastine was selected as representative of the classic anti-microtubule agents. Accordingly, the effect of cryptophycin compounds and vinblastine on the proliferation and cell cycle progression of the Jurkat T-cell leukemia cell line is compared.

Since antimitotic effects are commonly mediated by disruption of microtubules in the mitotic spindles, the effects of cryptophycin compounds on cytoskeletal structures are characterized by fluorescence microscopy. Immunofluorescence staining of cells treated with either a cryptophycin compound or vinblastine demonstrate that both compounds cause the complete loss of microtubules. Similar studies with SKOV 3 cells can show that the anti-microtubule effects of cryptophycin compounds are not unique to the smooth muscle cell line.

### GC3 human Colon Carcinoma Screen

Selected wells of a 96 well plate were seeded with GC3 human colon carcinoma cells (1x10 cells in 100µl assay medium/well) twenty four hours prior to test compound addition. Cell free assay medium was added to other select wells of the 96 well plate. The assay medium (RPMI-1640 was the medium used; however, any medium that will allow the cells to survive would be acceptable) was supplemented with 10% dialyzed fetal bovine serum and 25 mM HEPES buffer.

The test compound was stored in an amber bottle prior to testing. Fresh dimethylsulfoxide stock solution (200µg/ml) was prepared immediately prior to preparation of test sample dilutions in phosphate-buffered saline (PBS). A dilution of 1:20 dimethylsulfoxide solution in PBS was prepared such that the final concentration was 10 µg/ml. Serial 1:3 dilutions using PBS (.5ml previous sample of 1ml PBS) were prepared. Falcon 2054 tubes were used for the assay.

A 10ul sample of each dilution of test compound was added in triplicate to wells of GC3 plates. The plates were incubated for 72 hours at about 37°C. A 10µl sample of stock 3-[4,5-dimethyl-2-yl]-2,5-diphenyltetrazolium bromide salt ("MTT" 5 mg/ml in PBS) was added to each well. The plates were incubated for about an hour at 37°. The plates were centrifuged, media was decanted from the wells and 100µl acid-isopropanol (0.04 N HCl in isopropanol) was added to each well. The plate was read within one hour using a test wavelength of 570nm (SpectraMax reader).

Evaluation of compounds of Formula I suggest that the compounds can be useful in the treatment methods described herein. Further, the compounds will be useful for disrupting the microtubule system.

The preparation of the compounds of this invention can be completed using several protocols involving an activated ester followed by chromatography and acid induced deblocking where necessary.

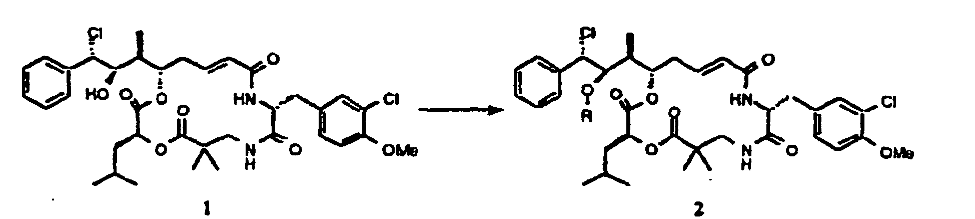

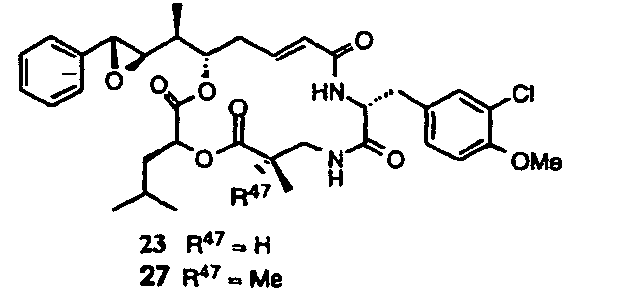

For example, the treatment of 1 (wherein the bolded numbers refer to the compound numbers indicated in the Example section) with acetic anhydride in the presence of triethylamine and 4-dimethylamino pyridine provides **3** in 89% yield after flash chromatography. Similarly, **4** is prepared from **1** *via* the agency of succinic anhydride followed by reverse phase HPLC purification. Exposure of pyridine solution of **1** to commercially available nicotinoyl chloride hydrochloride in the presence of triethylamine and 4-dimethylamino pyridine followed by chromatogrpahy and hydrogen chloride treatment gives rise to 7 in high yield. Pyridinium salt 8 is prepared in 47% yield according to the method of Nicolaou, Angew. Chem. Int. Ed. Engl., **1994,** 33 whereby 1 is treated with commercially available 2-fluoro-1-methylpyridinium *p*-toluenesulfonate followed by reverse phase HPLC purification with concomitant anion exchange (acetate for p-toluenesulfonate) and lyophilization. Esters **5, 9, 11, 13, 15, 17, 19,** and **21** are all prepared in moderate to high yields from 1 and commercially available (except in the case of **5** and **9)** N-*t*-boc protected amino acids with activation via the agency 1,3-dicyclohexylcarbodiimide in the presence of 4-dimethylamino pyridine. Hydrochloride salts **10, 12, 14, 16, 18, 20** and **22** are prepared in high yield from **9, 11, 13, 15, 17, 19,** and **21** respectively upon treatment with a 4.0 M solution of hydrogen chloride in dioxane and removal of solvent *in vacuo*. Di-sodium salt **6** is derived from 5 following hydrochloric acid induced t-butyl ester cleavage and sodium hydroxide treatment. The requisite acid **24** for the preparation of 5 is synthesized in 63% yield by way of a 5 step sequence featuring the method of Johns, Synthesis, 1988, 142, for installing the phosphate functionality. 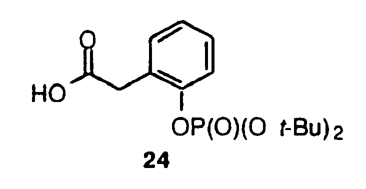 Several of the novel conjugates have been assayed for in vitro cytotoxicity in the GC3 tumor cell model. From the results depicted in Table 2 it is clear that good to excellent activity relative to that of cryptophycin 55 (1) is inherent to this series of compounds.

Preparation of any ester of type 2 (R¹ or R² derived from a carboxylic acid) includes a variety of technologies employing acid chlorides, anhydrides, and common activating reagents (e.g., carbodiimides).² Any solvent other than participating alcohols can be used. Any mild bases and/or catalysts (amines, carbonates) can be used to aid in esterification.

The conversion of carbamates **9, 11, 13, 15, 17**, **19,** and **21** to the corresponding salts could be effected with any strong acid, namely, mineral acids comprised of hydrogen halides, hydrogen sulfates, hydrogen phosphates, hydrogen nitrates, hydrogen perchlorates, or strong organic acids such as trifluoroacetic, p-toluenesulfonic, and methanesulfonic. The same acids could be used to produce salts of type **7** from the corresponding free base. A variety of counterions (cations) could comprise salts of type **6** including any of the alkali and alkaline earth metals. A variety of counterions (anions) could comprise salts of type **8**, namely, any conjugate base of an acid (organic or mineral).

**Table 2. In vitro cytotoxicity data for cryptophycin derivatives using the assay described supra.**

| Compound | GC3 IC₅₀ (nM) |
|---|---|
| 1 | 0.065 |
| 3 | 83 |
| 4 | 31 |
| 6 | 3.7 |
| 7 | 116 |
| 8 | 2.2 |
| 10 | --- |
| 12 | 0.10 |
| 14 | 21 |
| 16 | 230 |
| 18 | 2.6 |
| 20 | --- |
| 22 | 7.2 |
| 26 | --- |

Additional compounds of this invention and the GC3 assay results are indicated:

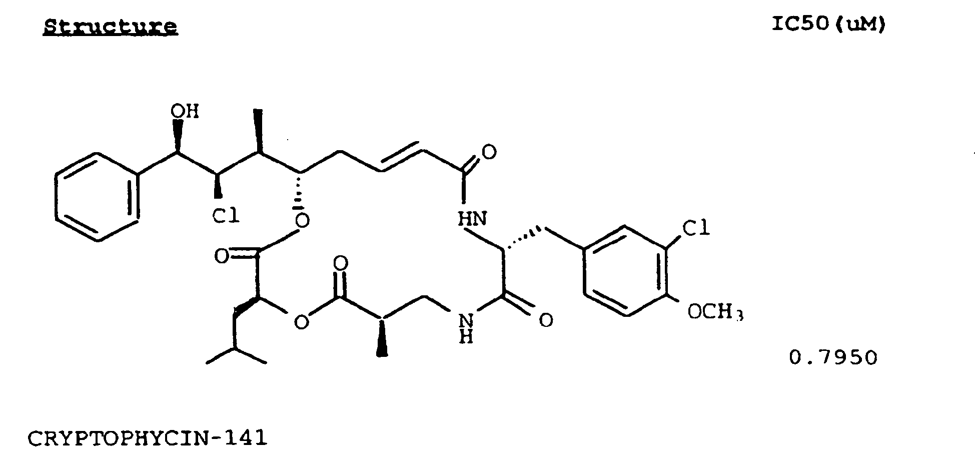

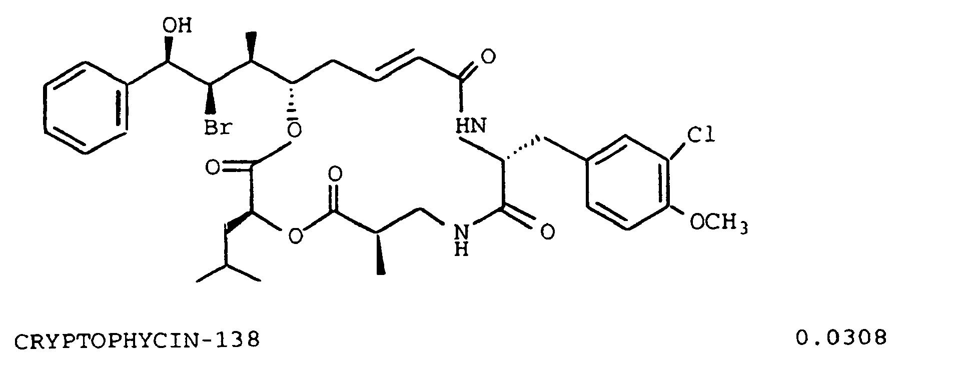

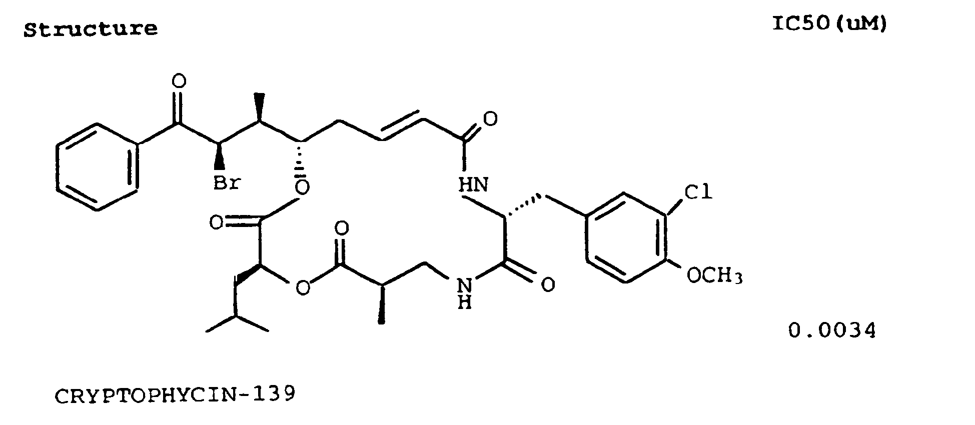

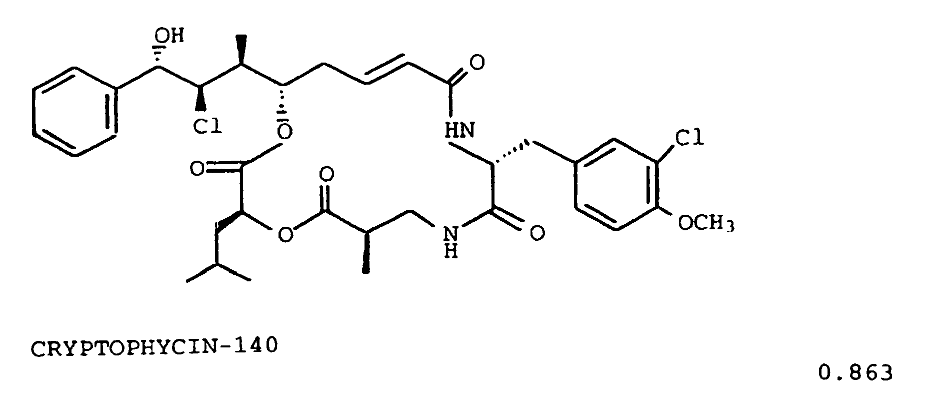

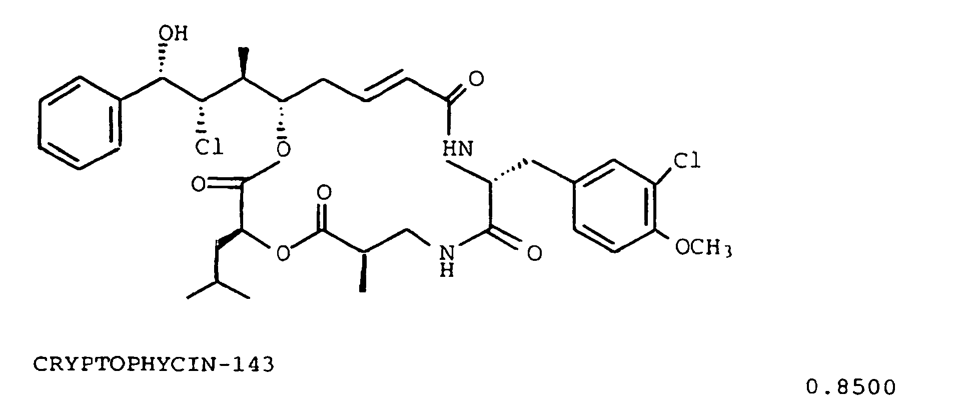

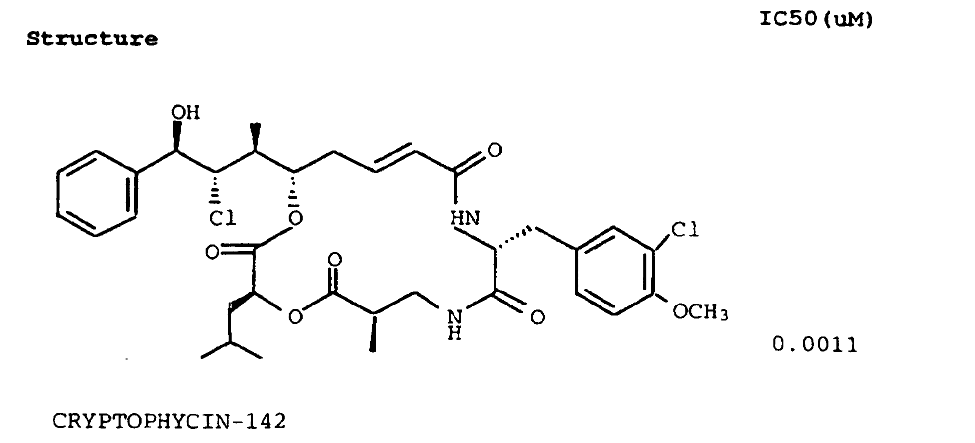

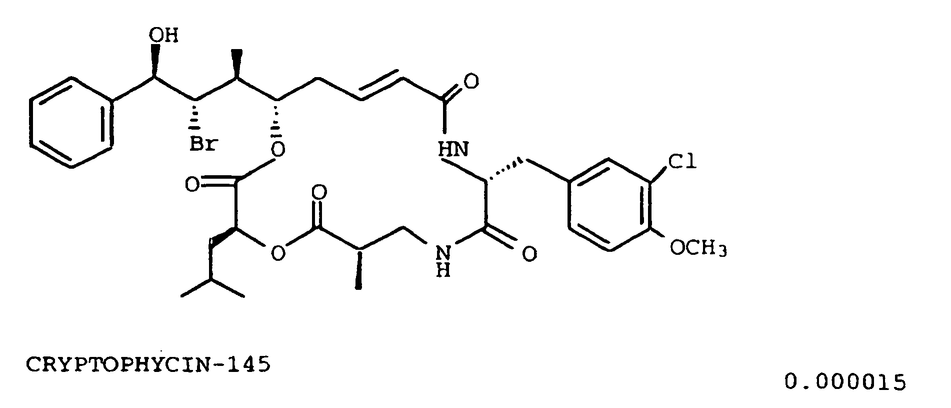

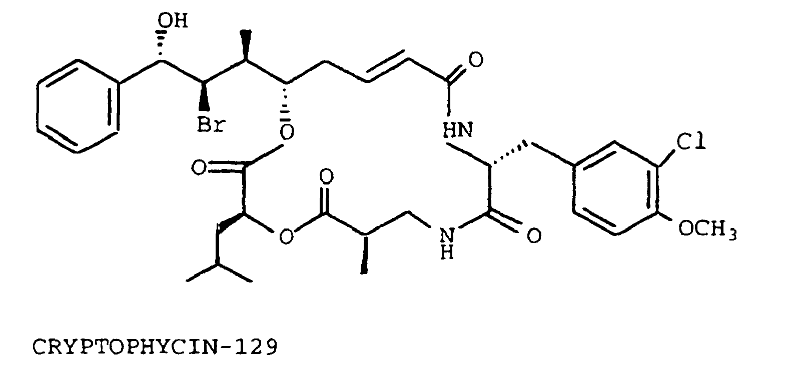

Additional compounds of this invention have been tested in the GC3 assay and had IC₅₀ values ranging from less than one to about 700 nM; however, the most typical values were less than 100 nM.
Results from comparative preliminary solubility studies among **27**, **1**, and 12 are shown in Table 3. These results indicate enhanced solubility of **12**.

**Table 3. Comparative solubilities of cryptophycin compounds 1, 12 and 27.**

| Excipient volume adjusted 0.05M acetate buffer | | %v/v | 27 (mg/ml) | 1 (mg/ml) | 12 (mg/ml) |
|---|---|---|---|---|---|
| | | | | | |
| 1 | Propylene Glycol | 15 | BQL | BQL | >2.0 |
| | Ethanol | 10 | | | |
| | | | | | |
| 2 | Propylene Glycol | 15 | BQL | 0.0026 | >2.0 |
| | Polyethylele Glycol 3000 | 20 | | | |
| | | | | | |
| 3 | Polysorbate 80 | 1(w/v) | BQL | 0.3113 | 1-2 |
| | | | | | |
| 4 | Emulphor EL719 | 1 (w/v) | 0.0272 | 0.3611 | 1.5 |
| | | | | | |
| 5 | 0.05M Acetate buffer pH 4.0 | 100 | <0.001 | <0.001 | 0.03 |

| | | | | | |
|---|---|---|---|---|---|
| (BQL = Below quantitation limit, approximately 0.001 mg/ml) | | | | | |

A comparison of stability characteristics between 1 and 12 in aqueous Ph ranging from 4-8 was determined at room temperature for 6 hours. Results from these studies clearly demonstrated the superior aqueous solubility and stability profile of **12**. For example, at pH 8, Cryptophycin 55 had a solubility value of 10 mg/mL at room temperature compared to a solubility of 30 mg/mL for **12** under substantially the same conditions. At a pH of 4, Cryptophycin 55 had an aqueous solubility value of about 50 mg/mL; however, the aqueous solubility of Cryptophycin 55 declined as the pH bacame basic, while the aqueous solubility of 12 remained substantially steady over the pH range studied.

Based on results from solubility and stability studies, appropriate parenteral vehicles were chosen to determine absolute solubility/stability characteristics of **12**. Table 4 illustrates the solubility profile of **12** in these vehicles. Preliminary results indicate acceptable stability of **12** in formulation 6 for up to 3 weeks.

**Table 4. Solubility of cryptophycin glycinate 12 in various parenteral vehicles.**

| | Excipient | %v/v | **12** (mg/ml) |
|---|---|---|---|
| 1 | Ethanol | 10 | 0.11 |
| | Propylene Glycol | 10 | |
| | 0.05 M Citrate buffer pH 4.0 | qs | |
| 2 | Ethanol | 10 | 1.99 |
| | Propylene Glycol | 10 | |
| | 0.05 M Acetate buffer pH 4.0 | qs | |
| 3 | Ethanol | 10 | 1.29 |
| | 0.05M Acetate buffer pH 4.0 | qs | |
| 4 | Propylene Glycol | 10 | 1.58 |
| | 0.05 M Acetate buffer pH 4.0 | qs | |
| 5 | Polysorbate 80 | 0.5 (w/v) | 2.21 |
| | 0.05 M Acetate buffer pH 4.0 | qs | |
| 6 | Ethanol | 10 | 10.27 |
| | Propylene Glycol | 25 | |
| | 0.05 M Acetate buffer pH 4.0 | qs | |
| 7 | Ethanol | 10 | 4.32 |
| | Propylene Glycol | 15 | |
| | 0.05 M Acetate buffer pH 4.0 | qs | |

Thus, it is feasible to achieve high concentrations of a compound of this invention in a vehicle containing no surfactant or an emulsifier which in turn should enable facile toxicological and clinical evaluations of these compounds. The glycinate ester (**12**) also affords better stability in an aqueous environment in physiological pH range over longer periods of time indicating enhanced shelf-life under normal conditions. Whereas it is necessary to prepare concentrates of 1 and 27 for storage and then diluted prior to administration, it is feasible to prepare ready to use solutions of compounds of this invention.

Compounds of Formula I can be prepared using a compound of the formula **II** 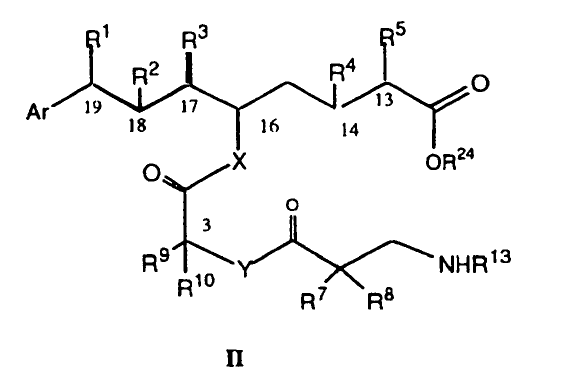 wherein
Ar, R¹, R², R³, R⁴, R⁵, R⁷, R⁸, R⁹, R¹⁰ have the meanings set for *supra* in Formula **I**.
R¹³ is selected from the group consisting of t-butylcarbamate (BOC);
R²⁴ is selected from the group consisting of 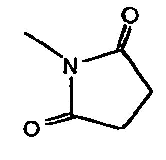 (N-hydroxysuccinimide, herein "NHS"), N-hydroxysulfosuccinimide and salts thereof, 2-nitrophenyl, 4-nitrophenyl, and 2,4-dichlorophenyl;
X is O, NH or alkylamino;
Y is O, NH, or alkylamino.

Compounds of Formula **III** 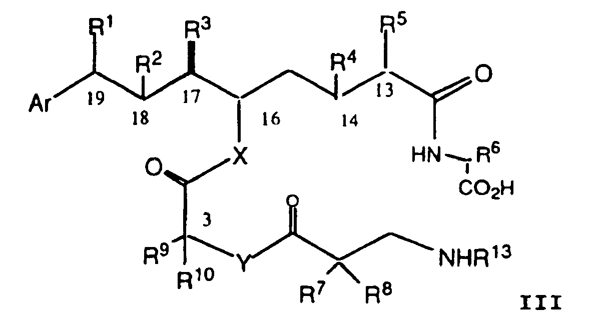 wherein the R groups and various substituents are as defined hereinbefore and throughout the specification; can be prepared by contacting a compound of the formula IV 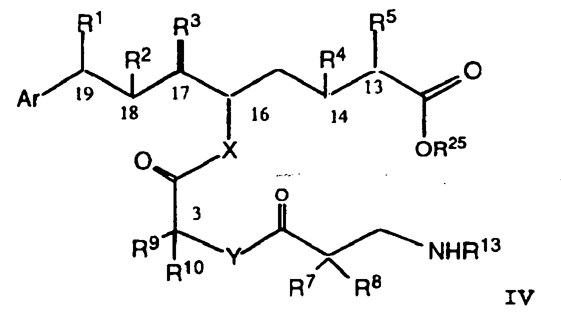 R²⁵ is
with an acid of the formula 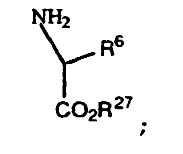 R²⁷ is selected from the group consisting of H, C₁-C₁₂ alkyl, and aryl;
and a silylating agent. Bis N,O-trimethylsilyl acetamide (BSA) is an especially preferred silylating agent.

As used herein, the phrase "active ester substituent" refers to a substituent which makes the indicated substituent a good leaving group. Appropriate substituents can be selected with guidance from standard reference guides, for example, "Protective Groups in Organic Chemistry", Plenum Press, (London and New York, 1973); Greene, T.W. "Protecting Groups in Organic Synthesis", Wiley (New York, 1981). See especially pages 180 through 184 of Greene. An especially preferred active ester substituent group is N-hydroxy-succinimide. (NHS) Other preferred groups include, but are in no way limited to: 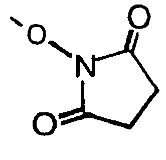 (ON-hydroxysuccinimide), O-N-hydroxysulfosuccinimide and salts thereof, O-2-nitrophenyl, O-4-nitrophenyl, and O-2,4-dichlorophenyl, wherein the "O" refers to the oxygen group necessary to form the ester functionality.

As used herein the term "amide" refers to an amide functionality that can be cleaved using alkaline conditions. For example, the term refers to but is in no way limited to, -NMe₂. For additional guidance, see for example Greene, T.W. "Protecting Groups in Organic Synthesis", Wiley (New York, 1981).

As used herein the phrase "active ester substituent" refers to a substituent which makes the OR²⁴ substituent a good leaving group. Appropriate substituents can be selected with guidance from standard reference guides, for example, "Protective Groups in Organic Chemistry", Plenum Press, (London and New York, 1973); Greene, T.W. "Protecting Groups in Organic Synthesis", Wiley (New York, 1981). An especially preferred R²⁵ group is N-hydroxy-succinimide. (NHS)

The processes described herein are most preferably completed in the presence of a solvent. The artisan can select an appropriate solvent for the above described process. Inert organic solvents are particularly preferred; however, under certain conditions an aqueous solvent can be appropriate. For example, if R²⁷ is hydrogen and and R¹³ is BOC an aqueous base as solvent will be effective.

When the desired R⁶ substituent in the compound of Formula I contains an amine, then the amine substituent of the R⁶ group must be protected using an amino protecting group. The artisan can readily select an appropriate amino protecting group using guidance from standard works, including, for example, "Protective Groups in Organic Chemistry", Plenum Press, (London and New York, 1973); Greene, T.W. "Protecting Groups in Organic Synthesis", Wiley (New York, 1981).

R²⁷ should be a group that allows for the removal of the -CO₂R²⁷ substituent using acidic, neutral, or mild basic conditions. Preferred R²⁷ groups include, but are in no way limited to, hydrogen, C₁-C₆ alkyl, tricholoromethyl, trichloroethyl, and methylthiomethyl. It is especially preferred that R²⁷ is hydrogen.

To provide further guidance for the artisan, the following schemes are provided:
Compounds of Formula I can be prepared using a compound of the formula II 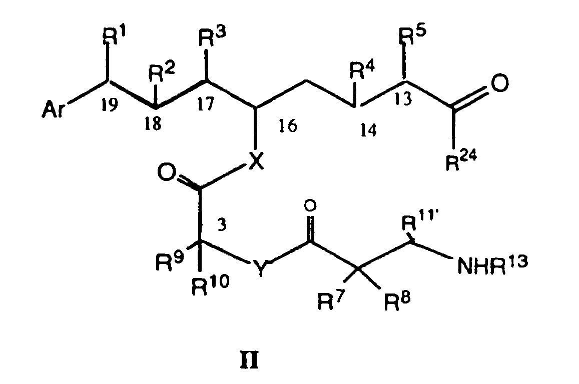 wherein
   Ar, R¹, R², R³, R⁴, R⁵, R⁷, R⁸, R⁹, R¹⁰ have the meanings set for *supra* in Formula I. The term R^{11'} is as defined for R¹¹, *supra.*
   R¹³ is a selected amino protecting group;
   R²⁴ is selected from the group consisting of active ester substituent, amide substituent, O-2-nitrophenyl, O-4-nitrophenyl, and O-2,4-dichlorophenyl;
   X is O, NH or alkylamino;
   Y is C, O, NH, S, SO, SO₂ or alkylamino.

Compounds of Formula **III** 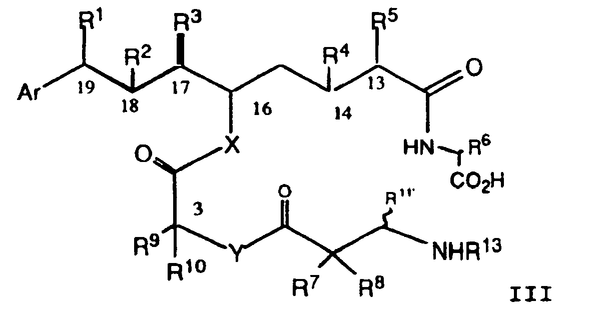 wherein the R groups and various substituents are as defined hereinbefore and throughout the specification; can be prepared by contacting a compound of the formula **IV** 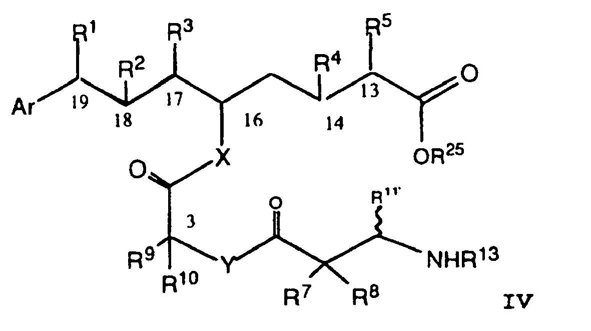 R²⁵ is
with an acid of the formula 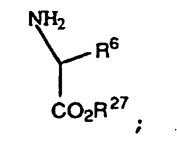 R²⁷ is selected from the group consisting of H, C₁-C₁₂ alkyl, and aryl;
and a silylating agent. Bis N,O-trimethylsilyl acetamide (BSA) is an especially preferred silylating agent.

As used with regard to R²⁵ the phrase "active ester substituent" refers to a substituent which makes the OR²⁴ substituent a good leaving group. Appropriate substituents can be selected with guidance from standard reference guides, for example, "Protective Groups in Organic Chemistry", Plenum Press, (London and New York, 1973); Greene, T.W. "Protecting Groups in Organic Synthesis", Wiley (New York, 1981). An especially preferred R²⁵ group is N-hydroxy-succinimide. (NHS)

The processes described herein are most preferably completed in the presence of a solvent. The artisan can select an appropriate solvent for the above described process. Inert organic solvents are particularly preferred; however, under certain conditions an aqueous solvent can be appropriate. For example, if R²⁷ is hydrogen and R¹³ is BOC an aqueous base as solvent will be effective.

When the desired R⁶ substituent in the compound of Formula **I** contains an amine, then the amine substituent of the R⁶ group must be protected using an amino protecting group. The artisan can readily select an appropriate amino protecting group using guidance from standard works, including, for example, "Protective Groups in Organic Chemistry", Plenum Press, (London and New York, 1973); Greene, T.W. "Protecting Groups in Organic Synthesis", Wiley (New York, 1981).

R²⁷ should be a group that allows for the removal of the -CO₂R²⁷ substituent using acidic, neutral, or mild basic conditions. Preferred R²⁷ groups include, but are in no way limited to, hydrogen, C₁-C₆ alkyl, tricholoromethyl, trichloroethyl, and methylthiomethyl. It is especially preferred that R²⁷ is hydrogen.

To provide further guidance for the artisan, the following schemes are provided:

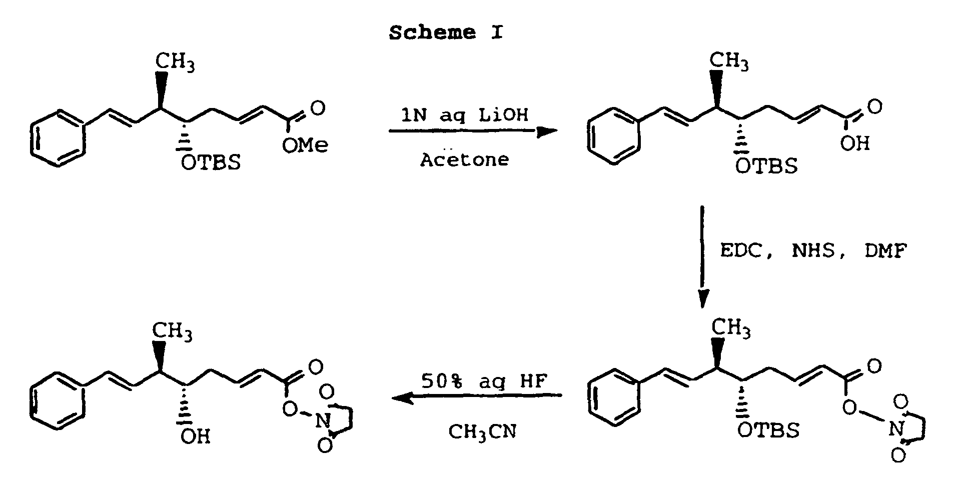

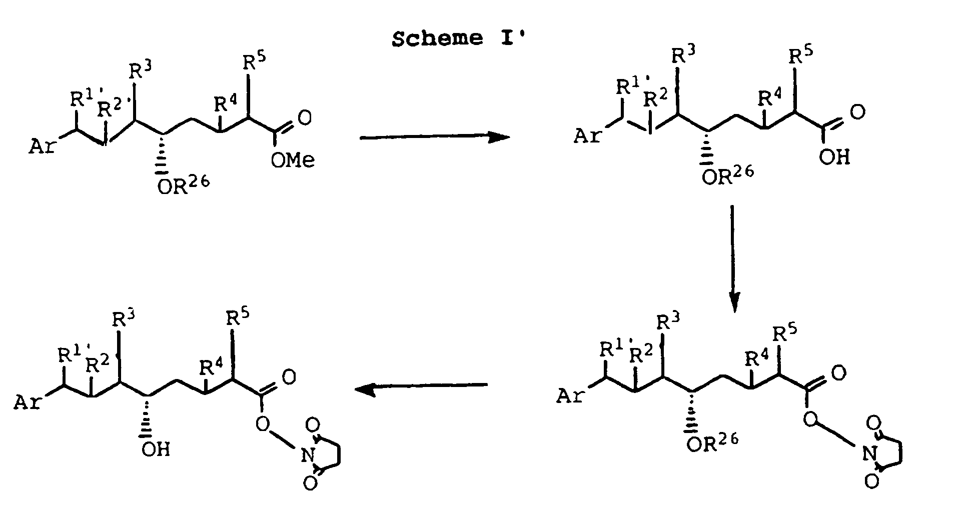

As used in Scheme I' and throughout the specification, R^{1'} is halogen, SH, amino, monoalkylamino, dialkylamino, trialkylammonium, alkylthio, dialkylsulfonium, sulfate, phosphate or a protected OH or protected SH group; R² is OH or SH; R²⁶ is an alcohol protecting group introduced during a portion of the synthetic process to protect an alcohol group which might otherwise react in the course of chemical manipulations, and is then removed at a later stage of the synthesis. Numerous reactions for the formation and removal of such a protecting groups are described in a number of standard works, including, for example, "Protective Groups in Organic Chemistry", Plenum Press, (London and New York, 1973); Greene, T.W. "Protecting Groups in Organic Synthesis", Wiley (New York, 1981). The skilled artisan can select an appropriate alcohol protecting group particularly with guidance provided from such works. One particularly useful alcohol protecting group is tert-butyldimethylsilyl (TBS). The products of such schemes can be derivatized using standard methods to provide other cryptophycin compounds.

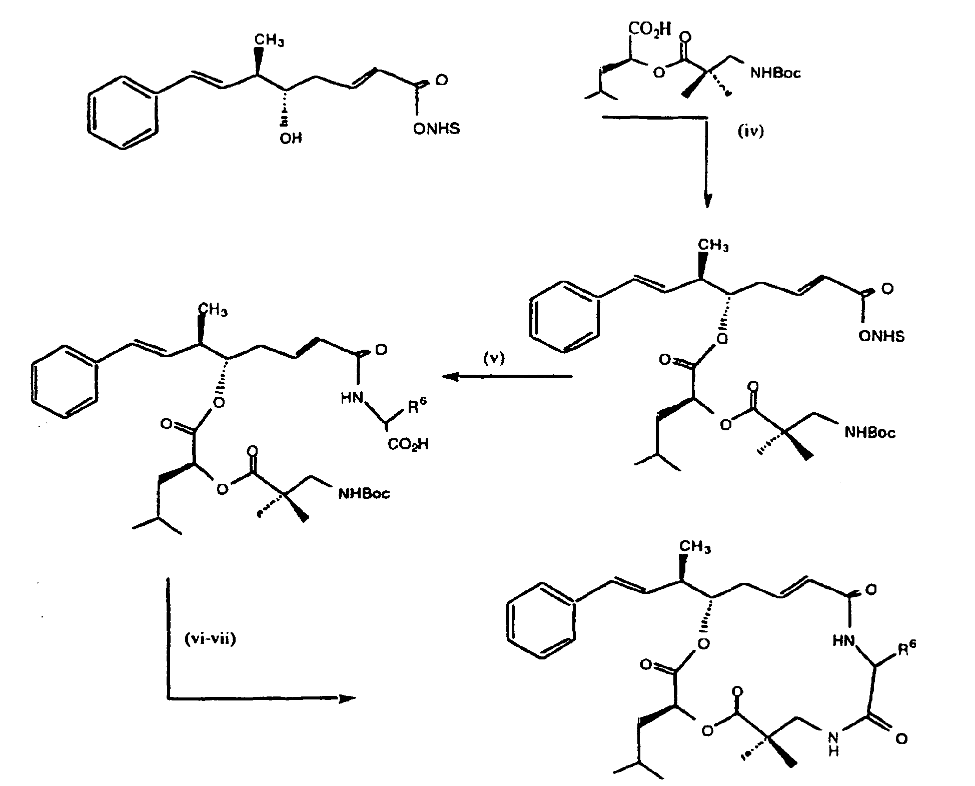

In general terms processes of this invention are as illustrated below:

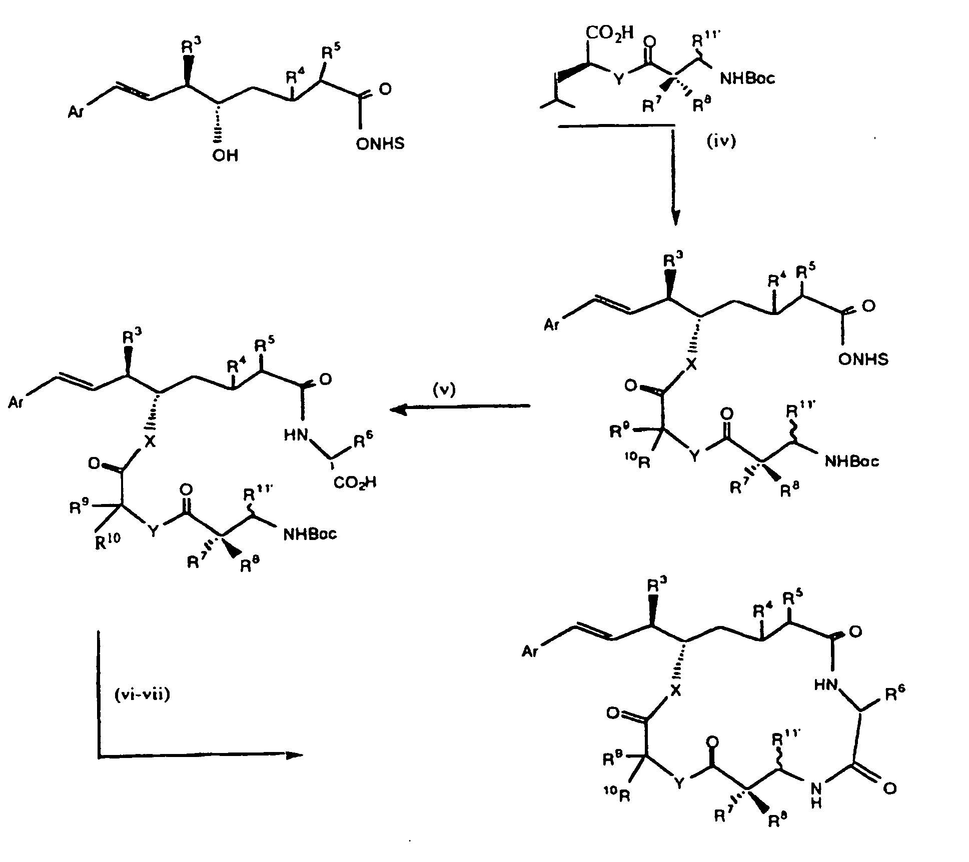

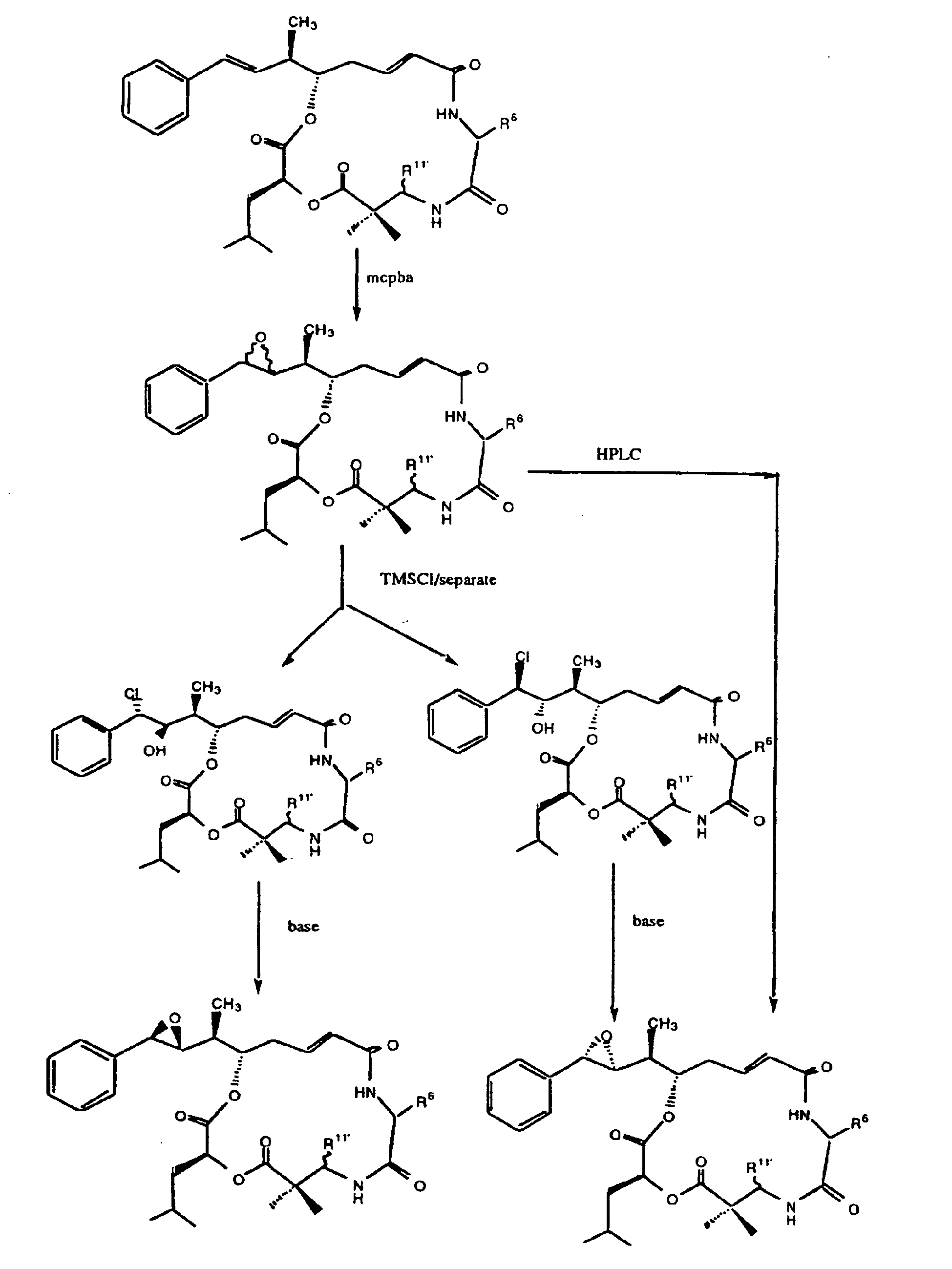

R⁶ and R^{11'} is as defined herein throughout the specification.

The product of the schemes provided herein can be further derivatized using standard methods to provide further cryptophycin compounds.

The artisan can utilize appropriate starting materials and reagents to prepare desired compounds using the guidance of the previous schemes and following examples.

The ester starting material can be prepared, for example, as follows: 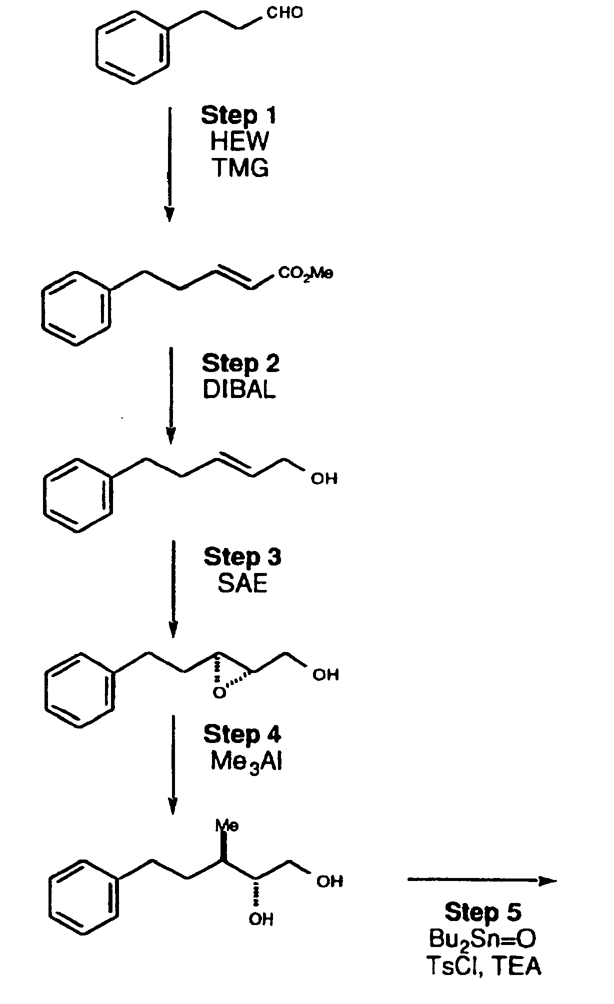 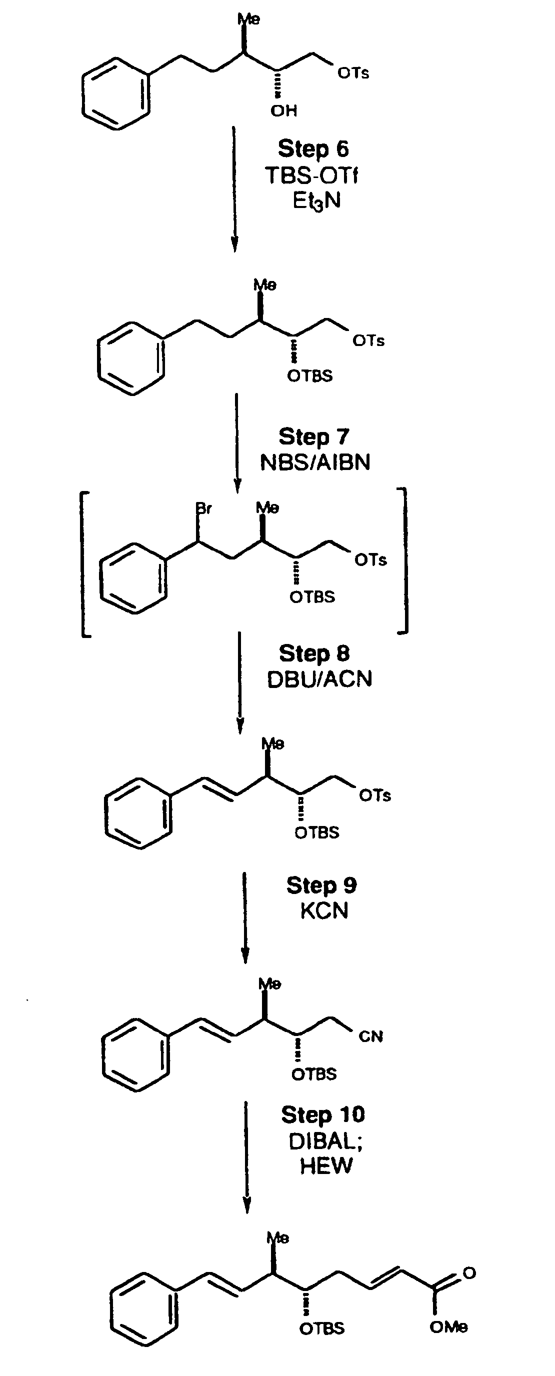 R⁶ has the meaning defined *supra*.

To provide further guidance, the following schemes are provided. Certain abbreviations are used in the Schemes, Preparations and Examples which are generally known in the art. For convenience, these abbreviations include:
- **DMAP**: 4-dimethylaminopyridine
- **BOC**: tert-butoxycarbonyl
- **mcpba**: m-chloroperbenzoic acid
- **TMSCl**: chlorotrimethylsilane
- **HEW**: Horner-Emmons-Wadsworth reaction (standard reaction for olefination of an aldehyde using a phosphonate and a base)
- **TMG**: 1,1,3,3-tetramethylguanidine (standard base used for the HEW reaction)
- **DIBAL**: diisobutylaluminum hydride (standard reagent for the reduction of an unsaturated ester to an allylic alcohol)
- **SAE**: Sharpless Asymmetric Epoxidation (established reaction for the enantioselective epoxidation of allylic alcohols)
- **TBS**: tert-butyldimethylsilyl
- **TBS-Otf**: TBS trifluoromethanesulfonate (standard reagent for the t-butyldimethylsilylation of alcohols)
- **AIBN**: 2,2'-azobis(isobutyronitrile) (standard radical initiator)
- **ACN**: acetonitrile
- **DBU**: 1,8-diazabicyclo[5.4.0]undec-7-ene (standard amine base)
- **EDCI**: 1-ethyl-3-(3-dimethylaminopropyl)carbodiimide

The scheme for preparing the ester is further explained by the Preparation Section herein which provides one specific application of the scheme for the convenience of the skilled artisan. 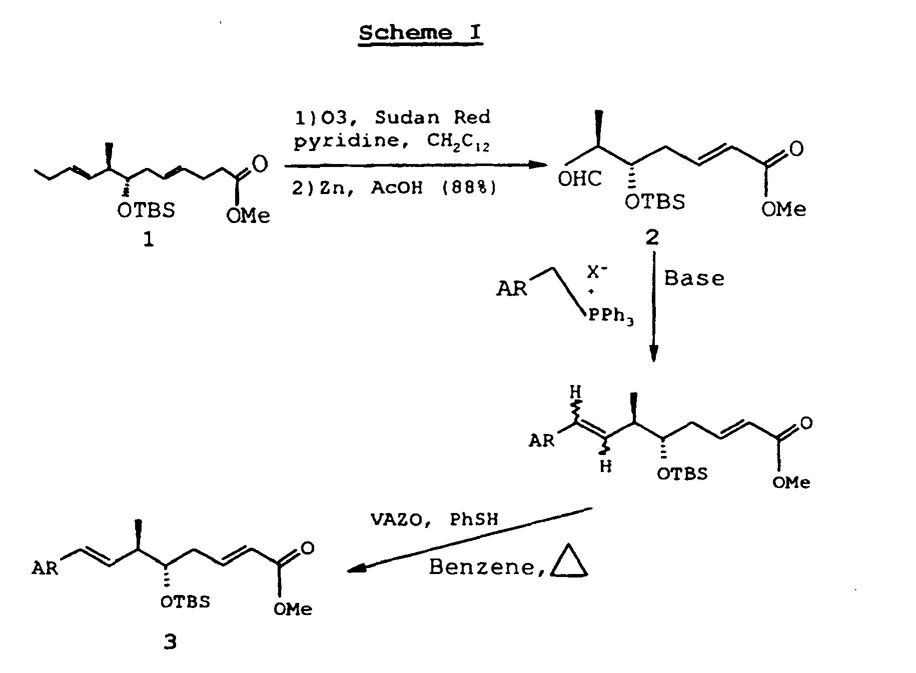 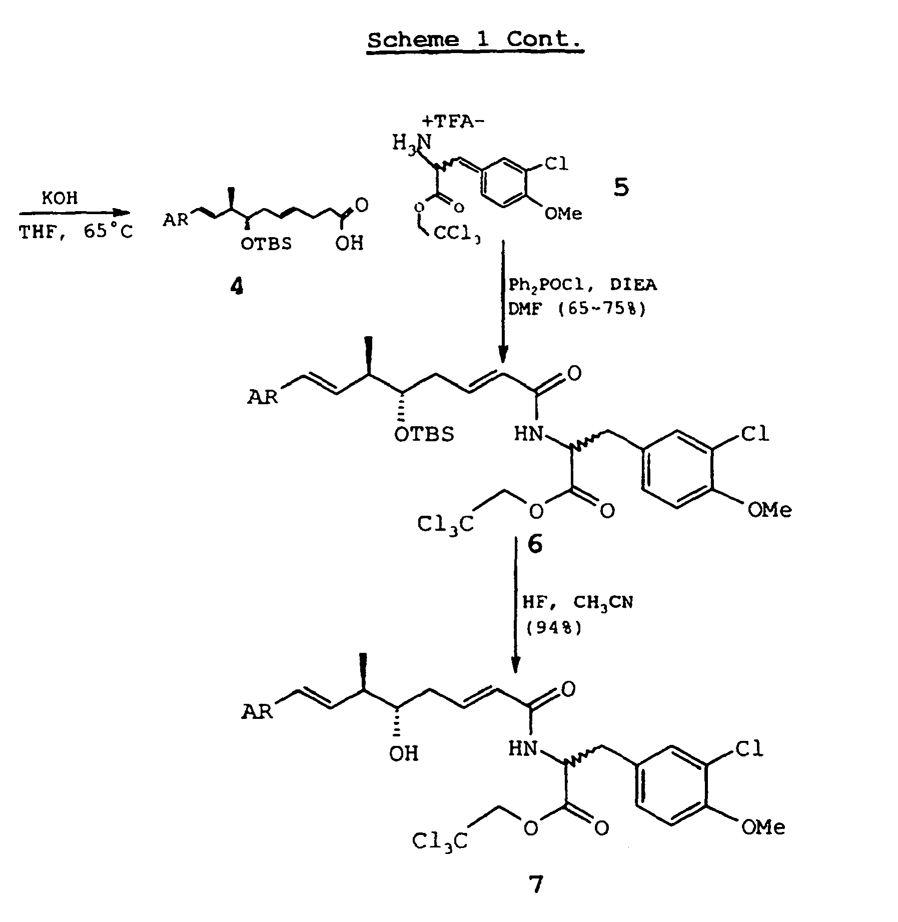 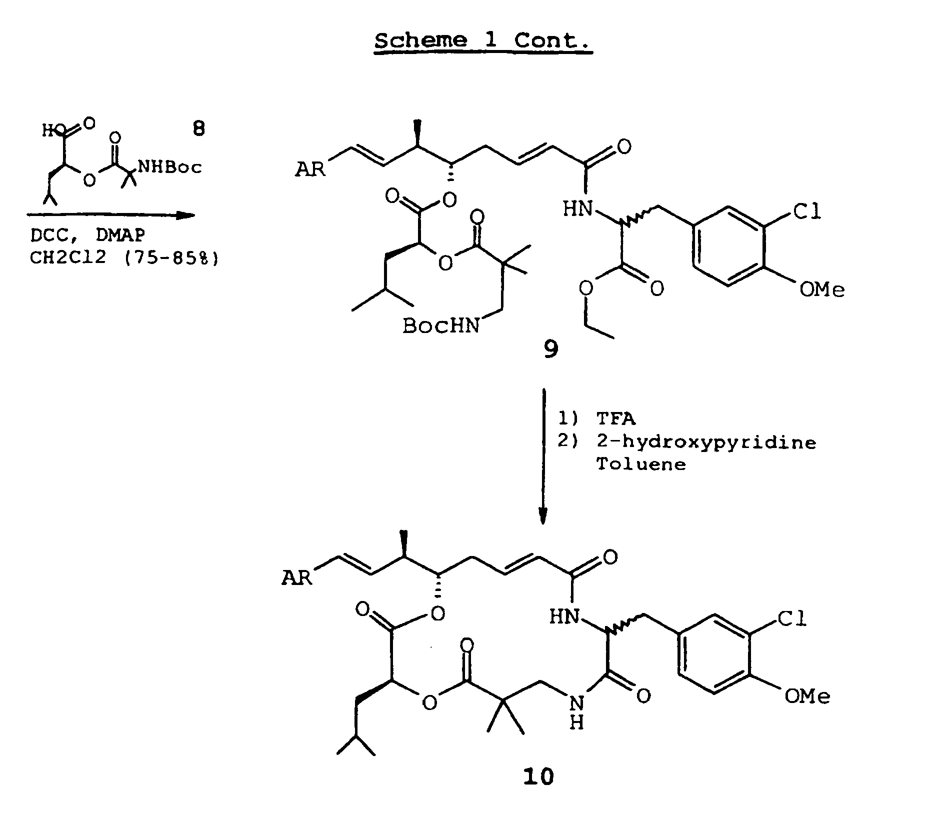 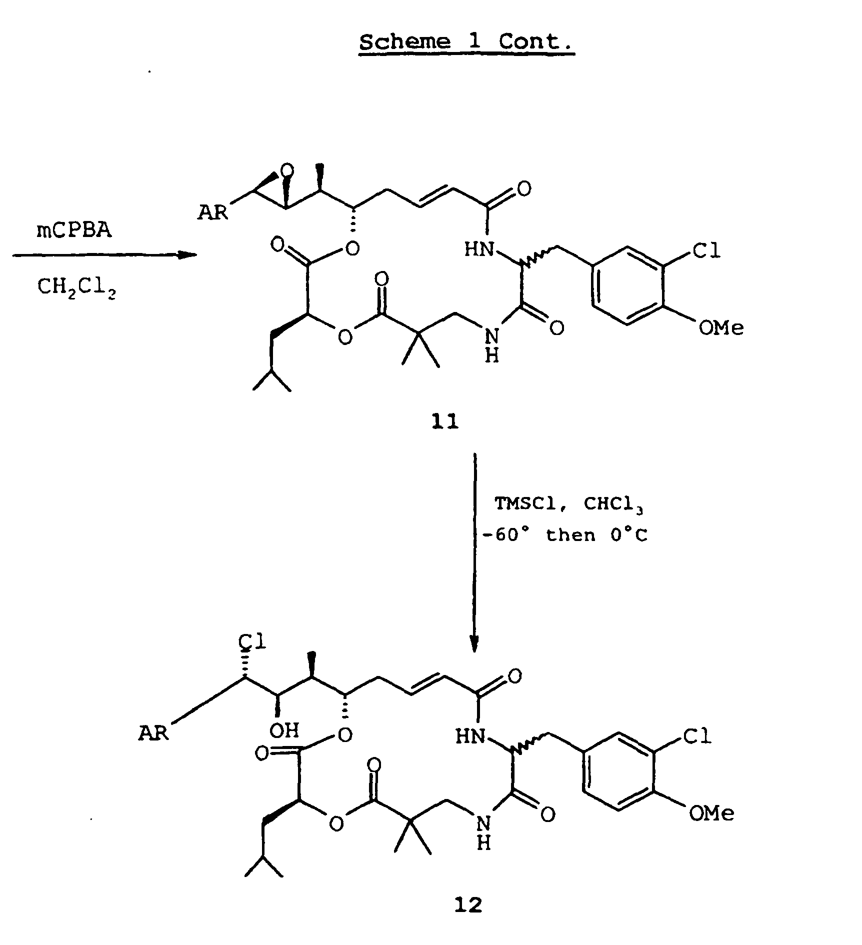 the Ar substituents claimed herein. The scheme illustration is not intended to limited the synthesis scheme only to the phenyl ring illustrated. Rather, the artisan can broadly apply this process to provide desired starting materials for the compounds claimed herein.

The necessary reaction time is related to the starting materials and operating temperature. The optimum reaction time for a given process is, as always, a compromise which is determined by considering the competing goals of throughput, which is favored by short reaction times, and maximum yield, which is favored by long reaction times.

Certain compounds of this invention can be prepared using a biosynthetic route. For example, a solution of cryptophycin 3 (about 0.24mmol) in DME-H₂O (2:1, 15.0 mL) at about 0C is treated with bromosuccinimide (about 65mg) and allowed to warm to room temperature. After about 24 hours the solvent is removed and the residue dissolved in a solvent such as CH₃CN and subject to reverse phase HPLC (65:35 CH₃CN/H₂O, 6 mL/min is preferred) to give a mixture of cryptophycins. The mixture is rechromatographed by normal phase HPLC (1:1 hexane/ethyl acetate, 3 mL/min preferred) to give the desired cryptophycins.

Likewise, cryptophycin 3 (about 40mg, 0.063 mmol) is put into 2:1 DME/H₂O (about 6mL) and N-chlorosuccinimide (0.075 mmol) is added and is heated at about 60-70 C for about 24 hours. A further quantity of NCS (8mg) is added and continue heating for about another 24 hours. The solvent is removed and the residue subject to HPLC (35%H₂O/CH₃CN 6mL/min) to give the desired cryptophycin compounds. The resulting mixture is further purified by HPLC using methanol/water (about 4:1) to obtain the desired purified cryptophycin compounds.

To further illustrate the invention the following examples are provided. The scope of the invention is in no way to be construed as limited to or by the following examples.

### Preparation 1

**Step 1. Methyl 5-Phenylpent-2(E)-enoate.** A solution of trimethyl phosphonoacetate (376 g, 417 mL, 2.07 mol) in THF (750 mL) was stirred at 0 °C in a 3L 3-neck round bottom flask equipped with a mechanical stirrer and N₂ inlet. To the chilled solution, neat tetramethyl guanidine (239 g, 260 mL, 2.07 mol) was added dropwise via an addition funnel. The chilled clear pale yellow solution was stirred for 25 minutes at 0 °C. A solution of hydrocinnamaldehyde (90%, 253 g, 248 mL, 1.9 mol) in THF (125 mL) was added dropwise to the reaction solution slowly. Upon completion of addition, the reaction was stirred for 10 h rising to room temperature. GC indicated a 95:5 ratio of product to starting material. 500ml of water was added to the reaction vessel and the reaction stirred overnight separating into two layers. The organic layer was isolated and the aqueous layer was extracted with t-BuOMe. The organic layers were combined and dried over MgSO₄ then concentrated *in vacuo* to yield an orange oil. The crude product was distilled at 129 °C/0.3mm Hg yielding 360.5g, 91.7% yield, of a clear slightly yellow oil.
EIMS *m*/*z* 190(13; M+), 159(410, 158(39), 131(90), 130(62), 117(22), 104(12), 95(57), 91(100), 77(21), 65(59); HREIMS *m*/*z* 190.0998 (C₁₂H₁₄O₂ D -0.4 mnu); UV lmax (e) 210 (8400), 260 (230) nm; IR nmax 3027, 2949, 1723, 1658, 1454, 1319, 1203, 978, 700 cm⁻¹; ¹H NMR d (CDCl₃) 7.15-7.3 (Ph-H5;bm), 7.00 (3-H;dt, 15.6/6.6), 5.84 (2-H;dt, 15.6/1.2), 3.70 (OMe;s), 2.76 (5-H2;t, 7.2), 2.51 (4-H2; bdt, 6.6/7.2); ¹³C NMR d (CDCl₃) 166.9 (1), 148.3(3), 140.6(Ph-1'), 128.4/128.2 (Ph2'/3'/5'6'), 126.1 (Ph 4'), 121.4 (2). 51.3 (OMe), 34.2/33.8 (4/5).

**Step 2. 5-phenyl-pent-2-en-1-ol.** To a 12L 4-neck round bottom flask equipped with a thermocouple, mechanical stirrer and N₂ inlet, a solution of enoate ester (310.5 g, 1.5 mol) in THF (1.5 L) was charged and chilled to -71 °C via a *i*-PrOH/CO₂ bath. To the reaction vessel, was added dropwise DIBAL (2.5 L, 1.5 M in toluene, 3.75 mol) at a rate to maintain the reaction temperature < -50 °C. Upon complete addition, the reaction was stirred overnight with the reaction temperature < -50 °C. TLC (3:1 Hexanes:EtOAc, SiO₂) indicated absence of starting material after 16 h. The reaction temperature was allowed to raise to -15°C. The reaction was quenched slowly with1N HCl (150 mL). At this point the reaction setup into a gelatinous solid. A spatula was employed to breakup the the semi-solid and 1N HCl (200 mL) was added making the mixture more fluid. Concentrated HCl (625 mL) was charged to form a two phase system. The layers were separated and the product extracted with t-BuOMe. The organic layer was dried over MgSO₄ and concentrated *in vacuo* to yield a clear pale yellow oil, 247.8g. The crude product was distilled at 145 °C/0.25mm Hg yielding 209.7g, 86.2%.
EIMS *m*/*z* 162 (1:M+) 144 (16), 129 (7), 117 (9) 108 (6), 92 (17), 91 (100), 75 (5), 65 (12), HREIMS *m*/*z* 162, 1049 (C₁₁H₁₄O, D -0.4 mmu); UV lmax (e) 206 (9900), 260 (360); IR nmax 3356, 2924, 1603, 1496, 1454, 970, 746, 700 cm⁻¹; ¹H NMR d 7.15-7.3 (Ph-H5;m), 5.70 (3-H;dt, 15.6/6.0), 5.61 (2-H;dt, 15.6/4.8), 4.02 (1-H2;d 4.8), 2.68 (5-H2; t, 7.2), 2.40 (OH;bs), 2.36(4-H2; dt, 6.0/7.2); ¹³C NMR d141.6 (Ph 1'), 131.8(3), 129.5 (2), 128.3/128.2 (Ph 2'/3'/5'/6'), 125.7 (Ph 4'), 63.3 (1), 35.4/33.8 (4/5).

**Step 3. (2S,3S)-2,3-Epoxy-5-phenyl-1-pentanol.** To a 1L 3 neck round bottom flask equipped with a mechanical stirrer, thermocouple and nitrogen inlet was added CH₂Cl₂ (350 mL), dried 4 Å molecular sieves (30 g) and L-(+)-diethyl tartrate (7.62 g, 0.037 mol). The resulting mixture was cooled to-20 °C and treated with Ti(O-*i*-Pr)₄ (9.2 mL, 0.031 mol), followed by the addition of t-butylhydroperoxide (4.0 M in CH₂Cl₂, 182 mL, 0.78 mol) at a rate to maintain the temperature ² -20 °C. Upon complete addition, the reaction mixture was stirred for another 30 min, and then treated with a solution of the allylic alcohol (50 g, 0.31 mol) in CH₂Cl₂ (30 mL) at a rate to maintain the temperature ² -20 °C. The reaction was stirred at the same temperature for 5 h, then filtered into a solution of ferrous sulfate heptahydrate (132 g) and tartaric acid (40 g) in water (400 mL) at 0 °C. The mixture was stirred for 20 min, then transferred to a separatory funnel and extracted with t-BuOMe (2x200 mL). The combined organic phase was stirred with 30% NaOH solution containing NaCl, for 1 h at 0 °C. The layers were again separated, and the aqueous phase extracted with t-BuOMe. The combined organic phase was washed with brine, dried over MgSO₄ and concentrated to yield 52.8 g as an amber oil.

**Step 4. (2*R*, 3*R*)-2-hydroxy-3-methyl-5-phenylpentan-1-ol** . To a 5L 3 neck round bottom flask equipped with a mechanical stirrer, thermocouple and nitrogen inlet was added hexanes (1L) and cooled to 0 °C. A 2.0M solution of Me₃Al in hexanes (800 mL, 1.6 mol) was added, followed by a solution of the epoxide (120 g, 0.677 mol) in hexanes (250 mL)/CH₂Cl₂ (50 mL) maintaining the temperature below 20 °C. Upon complete addition, the cloudy reaction mixture was stirred at 5 °C for 35 min, whereupon a solution of 10% HCl (300 mL) was added dropwise, followed by the addition of concd HCl (350 mL). The layers were separated, and the organic phase was washed with brine and dried over MgSO₄. After removal of the volatiles *in vacuo,* 122.1 gram of an oil was obtained.

**Step 5. (2*R*, 3*R*)-2-hydroxy-3-methyl-5-phenylpent-1-yl Tosylate.** To a 2L 3 neck round bottom flask equipped with a mechanical stirrer and nitrogen inlet was added the diol (58 g, 0.30 mol), dibutyltin oxide (1.5 g, 0.006 mol, 2 mol%), toluenesulfonyl chloride (57.5 g, 0.30 mol), CH₂Cl₂ (580 mL) and triethylamine (42.0 mL, 0.30 mol). The resulting mixture was stirred at room temperature for 2 h (although the reaction was complete within 1 h), filtered, washed with water and dried over MgSO₄. Concentration of the volatiles *in vacuo* afforded 104.1 gram of a slightly amber oil.

**Step 6. (2R, 3R)-2-[(tert-Butyldimethylsilyl)oxy]-3-methyl-5-phenylpent-l-yl Tosylate.** A solution of the tosylate (100 g, 0.29 mol) and triethylamine (81.0 mL, 0.58 mol) in CH₂Cl₂ (1200 mL) was treated with neat TBS-OTf (99 mL, 0.43 mol) dropwise with continued stirring for another 20 min. The reaction was washed twice with brine, dried over MgSO₄ and concentrated to dryness. The oil was dissolved in a minimal amount of hexanes and filtered over a silica pad, eluting with hexanes:EtOAc (9:1) to yield a slightly amber oil, 134 g.

**Step 7. (2R, 3R,5RS)-2-[(tert-Butyldimethylsilyl)oxy]-3-methyl-5-bromo-5-phenylpent-1-yl Tosylate.** To a 5L 3 neck round bottom flask equipped with a mechanical stirrer, reflux condenser and nitrogen inlet was added CCl₄ (1680 mL), TBS Ts (140 g, 0.30 mol), NBS (65g, 0.365 mol) and AIBN (16.5 g, 0.10 mol). The mixture was degassed by evacuation under full vacuum with stirring, and backfilling with nitrogen (3x). The reaction mixture was then heated to reflux, whereupon the color became dark brown. After 15 min at vigorous reflux, the reaction mixture became light yellow, and chromatographic analysis indicated the reaction was complete. After cooling to room temperature, the reaction was filtered and the filtrate concentrated to dryness. The residue was redissolved in hexanes and filtered again, and concentrated to dryness to afford 170.3 gram as an amber oil.

**Step 8. (2*R*, 3*R*)-2-[(tert-Butyldimethylsilyl)oxy]-3-methyl-5-phenylpent-4(*E*)-en-1-yl Tosylate.** To a 2L 3 neck round bottom flask equipped with a mechanical stirrer, reflux condenser and nitrogen inlet was added a solution of the bromide (100 g, 0.186 mol) in acetonitrile (700 mL). DBU (83.6 mL, 0.557 mol) was added and the resulting dark brown solution was stirred at reflux for 15 min. After cooling to room temperature, the solvent was removed *in vacuo,* and the residue digested in CH₂Cl₂ (200 mL) and filtered through a silica pad. The volatiles were again evaporated, and the residue dissolved in EtOAc and washed with water, brine and dried over MgSO₄ and concentrated to dryness. Preparative mplc (Prep 500) chromatography afforded the desired unsaturated compound (50.3 g, 60% yield over 4 steps).

**Step 9. (3S, 4R)-3-[(tert-Butyldimethylsilyl)oxy]-4-methyl-6-phenylhex-5(E)-en-1-nitrile.** The tosylate (50 g, 0.11 mol) was dissolved in DMSO (1 L) and treated with KCN (14.2 g, 0.22 mol) and water (25 mL), and the resulting mixture was stirred at 60°C under nitrogen for 18 h. After cooling to room temperature, the reaction mixture was partitioned between EtOAc (1 L) and water (1 L). The aqueous phase was extracted with EtOAc (500 mL), and the combined organic phase was washed with brine and dried over Na₂SO₄. Flash chromatography over silica with CH₂Cl₂ afforded the desired nitrile in 92% yield.

**Step 10. Methyl (5S, 6R)-5-[(tert-Butyldimethylsilyl)oxy]-6-methyl-8-phenylocta-2(E),7(E)-dienoate.** The nitrile (14.67 g, 46.5 mmol) was dissolved in toluene (200 mL) and cooled to -78 °C under nitrogen. A 1.5M solution of DIBAL in toluene (37.2 mL, 55.8 mmol) was added dropwise with vigorous stirring. Upon complete addition, the cooling bath was removed and the reaction was stirred at room temperature for 1 h. The reaction mixture was carefully poured into 1N HCl and the mixture stirred at room temperature for 30 min. The layers were separated, and the organic phase was washed with a saturated aqueous solution of sodium potassium tartrate (2x), brine and dried over Na₂SO₄. The volatiles were removed *in vacuo,* and the crude pale yellow oil was used directly in the subsequent condensation.
The crude aldehyde from above was dissolved in THF (90 mL) and treated with trimethyl phosphonoacetate (9.03 mL, 55.8 mmol) and tetramethylguanidine (7.0 mL, 55.8 mmol) at room temperature under nitrogen. The reaction mixture was stirred for 16 h, then partitioned between EtOAc (200 mL) and water (100 mL). The aqueous phase was back extracted with EtOAc (100 mL), and the combined organic phase was washed with water, brine and dried over Na₂SO₄. The volatiles were removed *in vacuo,* and the crude yellow oil (17.0 g) was chromatographed over silica gel with CH₂Cl₂ : cyclohexane (1 : 1 to 2 : 1) to afford 13.67 grams of the desired ester, 78.5%.

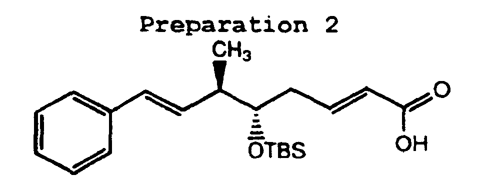

Methyl ester (2.673 mmol) was dissolved in acetone and then 1N aqueous LiOH (26mL) added at room temperature. The cloudy mixture was further diluted with acetone (20mL) and the resulting yellow mixture stirred at room temperature for 23.5h. The reaction was diluted with diethylether (400mL) and the organics washed with 1N HCl (120mL), brine (200mL) and H₂O (160mL). The organics were dried and concentrated *in vacuo* to leave a yellow oil which was purified by column chromatography (gradient: 5% AcOH + 20%-40% EtOAc/Hexanes ) to give carboxylic acid as a yellow oil (960mg, 100%).
¹H NMR (CDCl₃) d 7.38-7.19 (m, PhH₅), 7.09 (ddd,J=15.2,7.6 and7.9 Hz,3-H), 6.38 (d,J=16 Hz,8-H), 6.16 (dd,J=16 and8 Hz, 7-H), 5.85 (d,J=15.8Hz,2-H),3.81-3.75 (m,5-H), 2.49-2.37 (m,6-H,4-CH₂), 1.12 (d,J=6.7Hz,6-Me), 0.91 (s,SiCMe₃), 0.065 (s,SiMe), 0.068 (s, SiMe) ppm;
IR u (CHCl₃) 2957, 2930, 2858, 1697, 1258, 1098, 838 cm⁻¹.
MS (FD) 360.2 (M⁺, 100) ;
[a]_{D}+87.6° (c 10.5, CHCl₃);
Anal. calcd. for C₂₁H₃₂O₃ requires: C,69.95; H,8.95%. Found: C,69.19; H,8.39%.

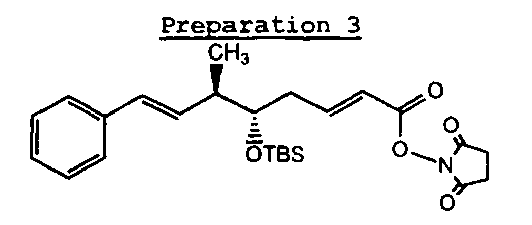

To a stirred solution of carboxylic acid (2mmol) in dry dimethylformamide (5.50mL) was added 1-ethyl-3-(3-dimethyaminopropyl)carbodiimide (2.4mmol) and N-hydroxysuccinimide (2.6mmol) at room temperature. The mixture was stirred for 28h and then diluted with EtOAc (100mL) and washed with 1N aqueous HCl (2x50mL), H₂O (75mL), dried and concentrated *in vacuo* to leave an oil. Crude product was purified by column chromatography (gradient: 5-30% EtOAc/Hexanes) to give active ester as a pale yellow oil (724mg, 80%).
¹H NMR (CDCl₃) d 7.36-7.20 (m, PhH₅, 3-H), 6.38 (d, J=16Hz, 8-H), 6.14 (dd,J=16.1 and 8.0 Hz,7-H). 6.03 (d,J=16Hz,2-H), 3.79 (q,J=4.3Hz,5-H), 2.94 (brs,CH₂CH₂), 2.58-2.42 (m,6-H,4-CH₂), 1.10 (d,J=6.8Hz,6-Me), 0.90 (s,SiCMe₃), 0.05 (s, SiMe₂) ppm;
IR u (CHCl₃)
2957,2931,2858,1772,1741,1648,1364,1254,1092,1069,838 cm⁻¹;
MS (FD) 457 (M⁺, 100) ;
[a]_{D} +71.3° (c 10.1, CHCl₃);
Anal. calcd. for C₂₅H₃₅NO₅ requires: C,65.61;H,7.71;N,3.06%. Found: C,65.51;H,7.56; N, 3.02%.

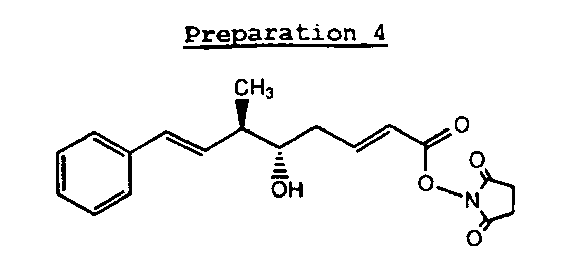

To a stirred solution of silyl ether (2.50g,5.47mmol) in CH₃CN (130mL) was added 48% aqueous HF (15mL) at 0C. The solution was stirred at 0 C for 0.75h and then at room temperature for 4h. The reaction was diluted with diethylether (300mL) and washed with H₂O until the wash was ~pH7. Organics were dried (MgSO₄) and concentrated in vacuo to give a yellow residue which was recrystallized from Et2O to give alcohol as white crystals (1.46g,7B%).
¹H NMR (CDCl₃) d 7.41-7.20 (m,PhH₅,3-H), 6.48 (d,J=16Hz,8-H), 6.15-6.07 (m,7-H,2-H), 3.71-3.65 (m,5-H), 2.83 (brs,CH₂CH₂), 2.60-2.33 (m,6-H,4-CH₂),1.95 (brs, 5-OH), 1.14 (d, J=6. BHz, 6-Me) ppm;
IR u (KBr)
3457, 1809, 1773, 1735, 1724, 1209, 1099, 1067, 1049, 975, 744, 694 cm¹;
UV (EtOH) lₘₐₓ 250 (e =20535) nm;
MS (FD) 343.2 (M⁺, 100);
[a]_{D} -57.8° (c 10.56, CHCl₃);
Anal. calcd. for C₁₉H₂₁NO₅S requires: C,66.46;H,6.16;N,4.08%. Found: C,66.49; H,6.16; N, 4.07%.

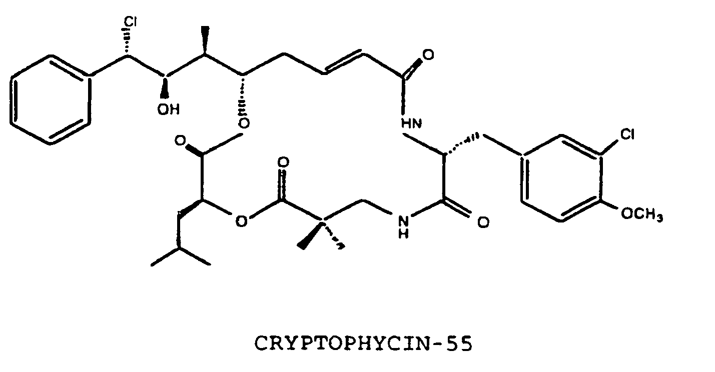

### Example 1

**Preparation of Cryptophycin 55 acetate (3) .** To a solution of 1 (93 mg, 0.13 mmol) in 659 ml of methylene chloride at 0 °C was added triethylamine (55 ml, 0.40 mmol), 4-dimethylamino pyridine (1.6 mg, 0.013 mmol), and acetic anhydride (19 ml, 0.20 mmol). After stirring at 0 °C for 1h the reaction was quenched with 19 ml of methanol, concentrated to 0.5 volume, and applied directly to a flash chromatography column (19 g of flash silica gel). Elution with ethyl acetate-hexanes (3:1) provided 88 mg (89%) of the title compound as a white foam: 500 MHz ¹H NMR (CDCl₃) d 7.38-7.31 (m, 5H), 7.24 (d, 1H, J = 2.1 Hz), 7.22-7.18 (m, 1H), 7.10 (dd, 1H, J = 8.5, 2.1 Hz), 6.88 (d, 1H, J = 8.5 Hz), 6.75 (ddd, 1H, J = 15, 13, 4.6 Hz), 5.78 (dd, 1H, J = 15, 1.0 Hz), 5.55 (d, 1H, J = 7.9 Hz), 5.46 (dd, 1H, J = 9.8, 1.2 Hz), 4.95 (dd. 1H, J = 11, 2.9 Hz), 4.89 (ddd, 1H, J = 9.9, 9.9, 1.7 Hz), 4.81 (d, 1H, J = 9.8 Hz), 4.79-4.74 (m, 1H), 3.91 (s, 3H), 3.39 (dd, 1H, J = 13,8.1 Hz), 3.22 (dd, 1H, J = 13, 4.1 Hz), 3.16 (dd, 1H, J = 14, 5.1 Hz), 3.07 (dd, 1H, J = 14, 7.6 Hz), 2.65-2.55 (m, 2H), 2.47-2.39 (m, 1H), 1.95 (ddd, 1H, J = 14, 13, 4.6 Hz), 1.86-1.77 (m, 1H), 1.73-1.66 (m, 1H), 1.68 (s, 3H), 1.27 (s, 3H), 1.19 (s, 3H), 1.09 (d, 3H, J = 7.1 Hz), 1.03 (d, 3H, J = 6.7 Hz), 0.97 (d, 3H, J = 6.6 Hz).

### Example 2

**Preparation of Cryptophycin 55 succinate (4)** To a solution of **1** (27 mg, 0.038 mmol) and succinic anhydride (5.7 mg, 0.057 mmol) in 383 ml of methylene chloride at room temperature was added triethylamine (16 ml, 0.115 mmol) and 4-dimethylamino pyridine (4.7 mg, 0.038 mmol). After stirring for 19 h, another 5.7 mg (0.057 mmol) of succinic anhydride and 4.7 mg (0.038 mmol) of 4-dimethylamino pyridine were added followed by stirring an additional 29 h. The reaction was treated with 0.5 ml of 1 N aqueous hydrochloric acid and washed with methylene chloride (3 X 0.5 ml). The combined organic extracts were dried (Na₂SO₄), filtered, and concentrated in *vacuo* to white foam. Reverse phase HPLC⁷ purification provided 10 mg (32%) of the title compound as a white foam: 500 MHz ¹H NMR (CDCl₃) d 7.36-7.31 (m, 6H), 7.22 (br s, 1H), 7.08 (d, 1H, J = 8.4 Hz), 7.02 (br s, 1H), 6.87 (d, 1H, J = 8.4 Hz), 6.61 (m, 1H), 5.94-5.87 (m, 2H), 5.51 (d, 1H, J = 9.8 Hz), 4.95 (dd, 1H, J = 10, 2.8 Hz), 4 .87-4.76 (m, 3H), 3.90 (s, 3H), 3.39 (dd, 1H, J = 16, 5.6 Hz), 3.28 (dd, 1H, J = 16, 8.2 Hz), 3.17 (dd, 1H, J = 16, 5.6 Hz), 3.05 (dd, 1H, J = 16, 8.2 Hz), 2.68-2.62 (m, 1H), 2.60-2.46 (m, 2H), 2.45-2.28 (m, 3H), 2.01-1.93 (m, 2H), 1.88-1.70 (m, 2H), 1.26 (s, 3H), 1.18 (s, 3H), 1.12 (d, 3H, J = 7.0 Hz), 1.04 (d, 3H, J = 6.6 Hz), 1.00 (d, 3H, J = 6.5 Hz).

### Example 3

### Preparation of Cryptophycin 55 (2'-di-t-butylphosphatyl)phenylacetate (5)

To a solution of **1** (0.102 mmol), **24** (46 mg, 0.134 mmol), and 4-dimethylamino pyridine (12 mg, 0.102 mmol) in 250 ml of anhydrous methylene chloride at room temperature was added a solution of 1,3-dicyclohexylcarbodiimide (27 mg, 0.134 mmol) in 50 ml of methylene chloride. After stirring at room temperature for 6 h the reaction was diluted with 1 ml of ethyl acetate-hexanes (3:1) and filtered through a plug of celite, washing with ethyl acetate-hexanes (3:1). The filtrate and washings were concentrated in *vacuo* to a purple foam. Chromatography (15 g of flash silica gel), eluting with ethyl acetate-hexanes (4:1) provided 86 mg (82%) of the title compound as a white foam: 500 MHz ¹H NMR (CDCl₃) d 7.35 (d, 1H, J = 8.3 Hz), 7.30-7.19 (m, 8H), 7.11 (dd, 1H, J = 8.4, 2.0 Hz), 7.02 (t, 1H, J = 7.5 Hz), 6. 87 (d, 1H, J = 8.4 Hz), 6.84 (d, 1H, J = 7.5 Hz), 6.73 (ddd, 1H, J = 15, 13, 4.7 Hz), 5.92 (d, 1H, J = 7.9 Hz), 5.79 (dd, 1H, J = 15, 1.0 Hz), 5.43 (dd, 1H, J = 9.4, 1.8 Hz), 4.98 (dd, 1H, J = 12, 3.1 Hz), 4.81 (ddd, 1H, J = 9.9, 9.9, 1.8 Hz), 4.75 (d, 1H, J = 9.4 Hz), 4.73-4.67 (m, 1H), 3.90 (s, 3H), 3.49 (d, 1H, J = 16 Hz), 3.44-3.38 (m, 1H), 3.38 (d, 1H, J = 16 Hz), 3.27-3.17 (m, 2H), 3.10 (dd, 1H, J = 14, 8.2 Hz), 2.55-2.46 (m, 2H), 2.37-2.27 (m, 1H), 1.95 (ddd, 1H, J = 14, 12, 4.5 Hz), 1.83-1.70 (m, 2H), 1.49 (s, 18H), 1.27 (s, 3H), 1.20 (s, 3H), 1.03 (d, 3H, J = 6.5 Hz), 0.97 (d, 3H, J = 6.4 Hz), 0.92 (d, 3H, J = 7.0 Hz) .

### Example 4

### Preparation of Cryptophycin 55 (2'-phosphatyl)phenylacetate di-sodium salt (6)

To a solution of 5 (84 mg, 0.081 mmol) in 400 ml of methylene chloride at room temperature was added a 4.0 M solution of hydrogen chloride in 1,4-dioxane (81 ml, 0.33 mmol). The faint yellow solution was allowed to stir at room temperature for 2 h. After concentration in *vacuo* to an off-white foam, the crude dihydrogen phosphate was dissolved in 614 ml of tetrahydrofuran and treated with a 5.00 N aqueous solution of sodium hydroxide (33 ml, 0.163 mmol). After stirring for 10 min the mixture was concentrated *in vacuo* to a tan foam. The crude salt was taken up in 1 ml of hot acetonitrile and 0.1 ml of hot water. The insolubles were filtered off and the filtrate was concentrated *in vacuo* to provide 69 mg (87 %) of the title compound as a white solid: 500 MHz ¹H NMR (MeOH-d₄) d 7.58 (d, 1H, J = 8.2 Hz), 7.38-7.32 (m, 2H), 7.32-7.28 (m, 3H), 7.28 (d, 1H, J = 2.1 Hz), 7.17 (dd, 1H, J = 8.5, 2.1 Hz), 7.09 (ddd, 1H, J = 7.8, 7.8, 1.7 Hz), 6.98 (d, 1H, J = 8.5 Hz), 6.79-6.70 (m, 2H), 6.67 (d, 1H, J = 7.4 Hz), 5.91 (dd, 1H, J = 15, 1.7 Hz), 5.45 (dd, 1H, J = 9.4, 1.6 Hz), 5.06 (dd, 1H, J = 10, 2.7 Hz), 5.01 (d, 1H, J = 9. 4 Hz), 4.89-4.80 (m, 1H), 4.47 (dd, 1H, J = 11, 3.8 Hz), 3.84 (s, 3H), 3.67 (d, 1H, J = 16 Hz), 3.45 (d, 1H, J = 14 Hz), 3.42 (d, 1H, J = 16 Hz), 3.18 (dd, 1H, J = 14, 3.8 Hz), 3.12 (d, 1H, J = 14 Hz), 2.77 (dd, 1H, J = 14, 11 Hz), 2.67-2.60 (m, 1H), 2.56-2.48 (m, 1H), 2.31-2.22 (m, 1H), 1.96-1.88 (m, 1H), 1.85-1.77 (m, 2H), 1.22 (s, 3H), 1.20 (s, 3H), 1.03 (d, 3H, J = 6.2 Hz), 0.98 (d, 3H, J = 6.1 Hz), 0.93 (d, 3H, J = 7.1 Hz).

### Example 5

### Preparation of Cryptophycin 55 nicotinoate hydrochloride salt (7)

To a solution of 1 (50 mg, 0.071 mmol) in 354 ml of pyridine at room temperature was added nicotinoyl chloride hydrochloride (15 mg, 0.085 mmol) followed by triethylamine (23 ml, 0.170 mmol). After stirring for 1.5 h, 4-dimethylamino pyridine (8.6 mg, 0.071 mmol) was added. After stirring 5 h, additional triethylamine (23 ml, 0.170 mmol), 4-dimethylamino pyridine (8.6 mg, 0.071 mmol), and nicotinoyl chloride hydrochloride (15 mg, 0.085 mmol) was added along with a 50 ml pyridine rinse. After stirring 18 h the reaction was treated with 0.5 ml of saturated aqueous sodium bicarbonate and washed with methylene chloride (4 X 1 ml). The combined organic extracts were dried (Na₂SO₄), filtered, and concentrated *in vacuo* to a light brown oil. Chromatography (14 g of flash silica gel), eluting with ethyl acetate-hexanes (10:1) provided 49 mg (85%) of the free base as a white foam. The nicotinoate was dissolved in 1 ml of methylene chloride and treated with a 1.0 M solution of hydrogen chloride in diethyl ether (90 ml, 0.090 mmol). The clear, colorless soution was allowed to stand at room temperature for 5 min. Removal of the solvent *in vacuo* produced 51 mg of the title compound as a white foam: 500 MHz ¹H NMR (CDCl₃) d 8.94 (s, 1H), 8.78 (br s, 1H), 8.29 (d, 1H, J = 7.0 Hz), 7.57 (br s, 1H), 7.38 (d, 2H, J = 7.1 Hz), 7.30-7.16 (m, 5H), 7.10 (dd, 1H, J = 8.4, 1.7 Hz), 6.88 (d, 1H, J = 8.4 Hz), 6.71 (m, 1H), 5.80 (d, 1H, J = 15 Hz), 5.74 (d, 1H, J = 9.6 Hz), 5.56 (br s, 1H), 5.00 (d, 1H, J = 9.6 Hz), 4.95 (t, 1H, J = 8.9 Hz), 4.84 (d, 1H, J = 9.8 Hz), 4.77-4.72 (m, 1H), 3.91 (s, 3H), 3.39 (dd, 1H, J = 13, 8.2 Hz), 3.23-3.14 (m, 2H), 3.06 (dd, 1H, J = 14, 7.6 Hz), 2.81-2.74 (m, 1H), 2.62-2.45 (m, 2H), 1.93 (ddd, 1H, J = 14, 12, 4.8 Hz), 1.78-1.70 (m, 1H), 1.66-1.59 (m, 1H), 1.25 (s, 3H), 1.20 (d, 3H, J = 7.0 Hz), 1.19 (s, 3H), 0.98 (d, 3H, J = 6.7 Hz), 0.84 (d, 3H, J = 6.5 Hz).

### Example 6

### Preparation of Cryptophycin 55 N-methylpyridinium acetate salt (8)

To a solution of **1** (53 mg, 0.075 mmol) in 751 ml of methylene chloride at 0 °C was added triethylamine (13 ml, 0.090 mmol) followed by 2-fluoro-l-methylpyridinium p-toluenesulfonate (23 mg, 0.083 mmol). The heterogeneous reaction mixture was warmed to room temperature and stirred for 3.5 h at which time another 11 mg (0.039 mmol) of 2-fluoro-1-methylpyridinium *p*-toluenesulfonate was added. After stirring for 14.5 h another 11 mg (0.039 mmol) of 2-fluoro-1-methylpyridinium p-toluenesulfonate was added followed by another 11 mg (0.039) of 2-fluoro-1-methylpyridinium *p*-toluenesulfonate and 13 ml (0.090) of triethylamine after 2.5 h. After stirring an additional 1h the reaction was concentrated in *vacuo* to an orange foam. Purification by reverse phase HPLC⁸ with concomitant anion exchange (acetate for p-toluenesulfonate) followed by lyophilization yielded 30 mg (47%) of the title compound as a white solid: 500 MHz ¹H NMR (DMSO-d₆) d 8.65-8.58 (m, 2H), 8.36 (t, 1H, J = 7.8 Hz), 7.68 (d, 1H, J = 8.9 Hz), 7.60 (d, 1H, J = 6.6 Hz), 7.48 (t, 1H, J = 6.6 Hz), 7.35-7.21 (m, 6H), 7.19 (dd, 1H, J = 8.5, 1.9 Hz), 7.05 (d, 1H, J = 8.5 Hz), 6.49 (ddd, 1H, J = 16, 13, 4.0 Hz), 5.91 (d, 1H, J = 16 Hz), 5.72 (d, 1H, J = 8.0 Hz), 5.66 (dd, 1H, J = 8.0, 1.9 Hz), 5.32-5.27 (m, 1H), 4.73 (dd, 1H, J = 9.7, 4.3 Hz), 4.24 (ddd, 1H, J = 11, 9.8, 3.7 Hz), 3.93 (s, 3H), 3.81 (s, 3H), 3.32 (dd, 1H, J = 13, 9.3 Hz), 3.05-2.97 (m, 2H), 2.77-2.57 (m, 3H), 2.54-2.47 (m, 1H), 1.76 (s, 3H), 1.68-1.62 (m, 1H), 1.55-1.46 (m, 1H), 1.37-1.30 (m, 1H), 1.15 (d, 3H, J = 7.0 Hz), 1.13 (s, 3H), 1.00 (s, 3H), 0.88 (d, 3H, J = 6.7 Hz), 0.73 (d, 3H, J = 6.5 Hz).

### Example 7

### Preparation of Cryptophycin 55 N-t-Boc-3-(3-chloro-4-methoxyphenyl)-(D)-alaninate (9)

To a solution of 1 (23 mg, 0.033 mmol), N-t-Boc-3-(3-chloro-4-methoxyphenyl)-(D)-alanine^{4c} (16 mg, 0.049 mmol), and 4-dimethylamino pyridine (few crystals) in 143 ml of anhydrous methylene chloride at room temperature was added a solution of 1,3-dicyclohexylcarbodiimide (10 mg, 0.049 mmol) in 20 ml of methylene chloride. After stirring for 2 h, the cloudy white reaction mixture was diluted with ethyl acetate-hexanes (2:1, 1 ml), stirred for 10 min, and filtered through a plug of celite, washing with ethyl acetate:hexanes (2:1). The filtrate and washings were concentrated *in vacuo* to a colorless oil. Chromatography (14 g of flash silica gel, 2:1 ethyl acetate-hexanes) afforded 29 mg (88%) of the title compound as a white foam: 500 MHz ¹H NMR (CDCl₃) d 7.42-7.27 (m, 5H), 7.24 (d, 1H, J = 2.0 Hz), 7.25-7.18 (m, 1H), 7.09 (dd, 1H, J = 8.4, 1.9 Hz), 6.91-6.86 (m, 2H), 6.84-6.70 (m, 3H), 5.75 (d, 1H, J = 15 Hz), 5.53 (d, 1H, J = 9.6 Hz), 5.47 (d, 1H, J = 7.6 Hz), 5.00 (dd, 1H, J = 10, 2.9 Hz), 4.90-4.80 (m, 2H), 4.78-4.71 (m, 1H), 4.63 (d, 1H, J = 8.3 Hz), 4.19-4.12 (m, 1H), 3.91 (s, 3H), 3.88 (s, 3H), 3.40 (dd, 1H, J = 13, 8.1 Hz), 3.25-3.12 (m, 2H), 3.07 (dd, 1H, J = 14, 7.6 Hz), 2.67-2.57 (m, 2H), 2.39-2.27 (m, 2H), 2.15 (dd, 1H, J = 14, 8.0 Hz), 2.01 (ddd, 1H, J = 14, 12, 4.2 Hz), 1.87-1.76 (m, 2H), 1.39 (s, 9H), 1.27 (s, 3H), 1.20 (s, 3H), 1.09-1.04 (m, 6H), 1.01 (d, 3H, J = 6.3 Hz).

### Example 8

### Preparation of Cryptophycin 55 3-(3-chloro-4-methoxyphenyl)-(D)-alaninate hydrochloride salt (10)

To a solution of 9 (27 mg, 0.027 mmol) in 265 ml of methylene chloride at room temperature was added a 4.0 M solution of hydrogen chloride in 1,4-dioxane (33 ml, 0.133 mmol). After stirring for 3 h, the clear, colorless reaction mixture was concentrated *in vacuo* to provide 26 mg (96%, corrected for 5 wt% dioxane) of the title compound as a white foam: 500 MHz ¹H NMR (MeOH-d₄) d 7.79 (d, 1H, J = 7.3 Hz), 7.49-7.45 (m, 2H), 7.43-7.48 (m, 3H), 7.31 (d, 1H, J = 2.0 Hz), 7.20 (dd, 1H, J = 8.4, 2.1 Hz), 7.13 (d, 1H, J = 2.2 Hz), 7.07-6.95 (m, 3H), 6.71 (ddd, 1H, 15, 13, 3.8 Hz), 5.98 (dd, 1H, J = 15, 1.8 Hz), 5.69 (d, 1H, J = 10 Hz), 5.22 (d, 1H, J = 10 Hz), 5.18 (dd, 1H, J = 10, 2.5 Hz), 4.89-9.80 (m, 1H), 4.53 (dd, 1H, J = 11, 3.7 Hz), 4.16 (dd, 1H, J = 10, 4.4 Hz), 3.88 (s, 3H), 3.87 (s, 3H), 3.51 (dd, 1H, J = 13, 9.9 Hz), 3.20 (dd, 1H, J = 14, 3.7 Hz), 3.14 (dd, 1H, J = 13, 2.3 Hz), 2.82-2.75 (m, 3H), 2.45 (dd, 1H, J = 15, 4.5 Hz), 2.42-2.34 (m, 1H), 2.08-2.00 (m, 1H), 1.97-1.86 (m, 3H), 1.27 (s, 3H), 1.21 (s, 3H), 1.16 (d, 3H, J = 7.1 Hz), 1.10 (d, 3H, J = 6.1 Hz), 1.06 (d, 3H, J = 6.0 Hz).

### Example 9

### Preparation of Cryptophycin 55 N-t-Boc-glycinate (11)

To a solution of 1 ( 118 mg, 0.167 mmol), N-*t*-Boc-glycine (44 mg, 0.251 mmol), and 4-dimethylamino pyridine (2.0 mg, 0.0167 mmol) in 490 ml of anhydrous methylene chloride at room temperature was added a solution of 1,3-dicyclohexylcarbodiimide (52 mg, 0.251 mmol) in 67 ml of methylene chloride. After stirring for 50 min, the cloudy white reaction mixture was diluted with ethyl acetate-hexanes (3:1, 1 ml), stirred for 10 min, and filtered through a plug of celite, washing with ethyl acetate-hexanes (3:1). The filtrate and washings were concentrated *in vacuo* to a colorless oil. Chromatography (19 g of flash silica gel, 3:1/ethyl acetate-hexanes) afforded 138 mg (96%) of the title compound as a white foam: 500 MHz ¹H NMR (CDCl₃) d 7 .39 (s, 5H), 7.24 (d, 1H, J = 2.0 Hz), 7. 23-7 . 19 (m, 1H), 7.10 (dd, 1H, J = 8.4, 2.0 Hz), 6.88 (d, 1H, J = 8.4 Hz), 6.79-6.70 (m, 1H), 5.77 (d, 1H, J = 13 Hz), 5.50 (d, 1H, J = 8.0 Hz), 5.47 (d, 1H, J = 9.8 Hz), 4.97 (dd, 1H, J = 11, 2.7 Hz), 4.89 (t, 1H, J = 10 Hz), 4.83 (d, 1H, J = 9.8 Hz), 4.79-4.72 (m, 1H), 4.68 (br s, 1H), 3.91 (s, 3H), 3.66 (dd, 1H, J = 18, 5.3 Hz), 3.42-3.35 (m, 2H), 3.21 (dd, 1H, J = 13, 4.0 Hz), 3.17 (dd, 1H, J = 15, 5.1 Hz), 3.08 (dd, 1H, J = 15, 7.6 Hz), 2.66-2.57 (m, 2H), 2.47-2.38 (m, 1H), 1.95 (ddd, 1H, J = 14, 12, 4.7 Hz), 1.85-1.77 (m, 1H), 1.75-1.67 (m, 1H), 1.43 (s, 9H), 1.27 (s, 3H), 1.20 (s, 3H), 1.08 (d, 3H, J = 7.0 Hz), 1.03 (d, 3H, J = 6.7 Hz), 0.98 (d, 3H, J = 6.5 Hz).

### Example 9

### Preparation of Cryptophycin 55 glycinate hydrochloride salt (12)

To a solution of **11** (122 mg, 0.141 mmol) in 471 ml of methylene chloride at room temperature was added a 4.0 M solution of hydrogen chloride in 1,4-dioxane (178 ml, 0.707 mmol). After stirring for 1h 20 min, the clear, colorless reaction mixture was concentrated in *vacuo* to provide 120 mg (99%, corrected for 7 wt% dioxane) of the title compound as a white foam: 500 MHz ¹H NMR (MeOH-d₄) d 7.81 (dd, 1H, J = 8.5, 2.2 Hz), 7.46-7.41 (m, 2H), 7.40-7.36 (m, 3H), 7.31 (d, 1H, J = 2.1 Hz), 7.20 (dd, 1H, J = 8.4, 2.1 Hz), 7.01 (d, 1H, J = 8.4 Hz), 6.70 (ddd, 1H, J = 15, 13, 3.7 Hz), 5.97 (dd, 1H, J = 15, 1.7 Hz), 5.55 (d, 1H, J = 9.9 Hz), 5.18 (d, 1H, J = 9.9 Hz), 5.14 (dd, 1H, J = 10, 2.8 Hz), 4.84 (t, 1H, J = 10 Hz), 4.52 (dd, 1H, J = 11, 3.7 Hz), 3.87 (s, 3H), 3.78 (d, 1H, J = 18 Hz), 3.50 (dd, 1H, J = 13, 9.8 Hz), 3.23 (d, 1H, J = 18 Hz), 3.20 (dd, 1H, J = 14, 3. 6 Hz), 3. 13 (dd, 1H, J = 13, 2.4 Hz), 2.80-2.69 (m, 3H), 2.41-2.32 (m, 1H), 1.99-1.92 (m, 1H), 1.91-1.81 (m, 2H), 1.25 (s, 3H), 1.20 (s, 3H), 1.12 (d, 3H, J = 7.0 Hz) . 1.06 (d, 3H, J = 6.2 Hz), 1.04 (d, 3H, 6.2 Hz).

### Example 10

### Preparation of Cryptophycin 55 N-t-Boc-b-alaninate (13)

To a solution of 1 (102 mg, 0.145 mmol), N-*t*-Boc-b-alanine (41 mg, 0.217 mmol), and 4-dimethylamino pyridine (18 mg, 0.145 mmol) in 400 ml of anhydrous methylene chloride at room temperature was added a solution of 1,3-dicyclohexylcarbodiimide (45 mg, 0.217 mmol) in 82 ml of methylene chloride. After stirring for 3.5 h, the cloudy white reaction mixture was diluted with ethyl acetate-hexanes (3:1, 1 ml), stirred for 10 min, and filtered through a plug of celite, washing with ethylacetate-hexanes (3:1). The filtrate and washings were concentrated *in vacuo* to a colorless oil. Chromatography (21 g of flash silica gel, 2:1 then 4:1/ethyl acetate-hexanes) afforded 121 mg (95%) of the title compound as a white foam: 500 MHz ¹H NMR (CDCl₃) d 7.44-7.39 (m, 2H), 7.37-7.31 (m, 3H), 7.32 (d, 1H, J = 2.1 Hz), 7.20 (dd, 1H, J = 8.4, 2.1 Hz), 7.01 (d, 1H, J = 8.4 Hz), 6.72 (ddd, 1H, J = 15, 13, 3.7 Hz), 5.96 (dd, 1H, J = 15, 1.6 Hz), 5.51 (d, 1H, J = 9.8 Hz), 5.11-5.06 (m, 1H), 5.08 (d, 1H, J = 9.8 Hz), 4.90-4.83 (m, 1H), 4.50 (dd, 1H, J = 11, 3.6 Hz), 3.86 (s, 3H), 3.52-3.46 (m, 1H), 3.20 (dd, 1H, J = 14, 3.6 Hz), 3.13 (br d, 1H, J = 14 Hz), 3.05-2.92 (m, 2H), 2.79-2.63 (m, 3H), 2.45-2.37 (m, 1H), 2.24 (dt, 1H, J = 16, 7.0 Hz), 2.08-1.99 (m, 1H), 1.96-1.79 (m, 3H), 1.43 (s, 9H), 1.25 (s, 3H), 1.21 (s, 3H), 1.12 (d, 3H, J = 7.0 Hz), 1.06 (d, 3H, J = 6.2 Hz), 1.02 (d, 3H, J = 6.1 Hz).

### Example 11

### Preparation of Cryptophycin 55 b-alaninate hydrochloride salt (14)

To a solution of **13** (119 mg, 0.136 mmol) in 452 ml of methylene chloride at room temperature was added a 4.0 M solution of hydrogen chloride in 1,4-dioxane (170 ml, 0.679 mmol). After stirring for 2 h 15 min, the cloudy, white reaction mixture was concentrated in *vacuo* to provide 110 mg (96%, corrected for 4 wt% dioxane) of the title compound as a white foam: 500 MHz ¹H NMR (MeOH-d₄) d 7.80 (dd, 1H, J = 9.7, 2.3 Hz), 7.45-7.40 (m, 2H), 7.39-7.32 (m, 3H), 7.20 (dd, 1H, J = 8.4, 2.1 Hz), 7.01 (d, 1H, J = 8.4 Hz), 6.68 (ddd, 1H, J = 15, 13, 3.8 Hz), 5.98 (dd, 1H, J = 15, 1.7 Hz), 5.48 (dd, 1H, J = 9.4 1.0 Hz), 5. 15-5. 11 (m, 1H), 5. 13 (d, 1H, J = 9.4 Hz), 4.82 (t, 1H, J = 10 Hz), 4.51 (dd, 1H, J = 11, 3.7 Hz), 3.90 (s, 3H), 3.50 (dd, 1H, J = 14, 9.8 Hz), 3.20 (dd, 1H, J = 14, 3.7 Hz), 3.14 (dd, 1H, J = 14, 2.4 Hz), 2.85 (t, 2H, J = 7.0 Hz), 2.80-2.65 (m, 5H), 2.54 (dt, 1H, J = 17, 7.4 Hz), 2.42-2.33 (m, 1H), 2.22 (dt, 1H, *J* = 17, 6.7 Hz), 1.90-1.81 (m, 3H), 1.25 (s, 3H), 1.20 (s, 3H), 1.13 (d, 3H, J = 7.1 Hz), 1.08 (d, 3H, J = 6.3 Hz), 1.04 (d, 3H, J = 6.2 Hz.

### Example 12

### Preparation of Cryptophycin 55 N-t-Boc-g-aminobutyrate (15)

To a solution of 1 (48 mg, 0.068 mmol), N-t-Boc-4-aminobutyric acid (18 mg, 0.088 mmol), and 4-dimethylamino pyridine (8 mg, 0.068 mmol) in 150 ml of anhydrous methylene chloride at room temperature was added a solution of 1,3-dicyclohexylcarbodiimide (18 mg, 0.088 mmol) in 50 ml of methylene chloride. After stirring for 45 min, the cloudy white reaction mixture was diluted with ethyl acetate-hexanes (3:1, 0.5 ml), stirred for 5 min, and filtered through a plug of celite, washing with ethyl acetate-hexanes (3:1). The filtrate and washings were concentrated in *vacuo* to a colorless oil. Chromatography (15 g of flash silica gel, 3:1/ethyl acetate-hexanes) afforded 55 mg (90%) of the title compound as a white foam: 500 MHz ¹H NMR (CDCl₃) d 7.38-7.32 (m, 5H), 7.24 (d, 1H, J = 1.9 Hz), 7.22-7.19 (m, 1H), 7.10 (dd, 1H, J = 8.4, 1.9 Hz), 6.88 (d, 1H, J = 8.4 Hz), 6.75 (ddd, 1H, J = 15, 13, 3.9 Hz), 5.78 (d, 1H, J = 15 Hz), 5.60-5.55 (m, 1H), 5.49 (dd, 1H, J = 9.8, 1.4 Hz), 4.96 (dd, 1H, J = 11, 3.0 Hz), 4. 89 (t, 1H, J = 9.2 Hz), 4.81 (d, 1H, J = 9.8 Hz), 4.78-4.70 (m, 1H), 4.44 (br s, 1H), 3.91 (s, 3H), 3.40 (dd, 1H, J = 14, 8.1 Hz), 3.22 (dd, 1H, J = 14, 4.1 Hz), 3.22-3.15 (m, 1H), 3.08 (dd, 1H, J = 14, 7.8 Hz), 2.89-2.82 (m, 2H), 2.67-2.56 (m, 2H), 2.47-2.38 (m, 1H), 2.11-2.04 (m, 1H), 2.00-1.77 (m, 3H), 1.75-1.67 (m, 1H), 1.45 (s, 9H), 1.50-1.40 (m, 2H), 1.27 (s, 3H), 1.20 (s, 3H), 1.09 (d, 3H, J = 7.0 Hz), 1.04 (d, 3H, J = 6.6 Hz), 0.98 (d, 3H, J = 6.6 Hz).

### Example 13

### Preparation of Cryptophycin 55 g-aminobutyrate hydrochloride salt (16)

To a solution of 15 (53 mg, 0.059 mmol) in 297 ml of methylene chloride at room temperature was added a 1.0 M solution of hydrogen chloride in diethyl ether (297 ml, 0.297 mmol). The starting material precipitated as a white paste which was redissolved with an additional 150 ml of methylene chloride. After stirring for 4 h, another 59 ml (0.059 mmol) of hydrogen chloride solution was added. Stirring was continued for another 14 h and the reaction mixture was concentrated *in vacuo* to provide 49 mg (100%) of the title compound as a white foam: 500 MHz ¹H NMR (DMSO-d₆) d 8.49 (d, 1H, J = 8.0 Hz), 7.72 (br s, 3H), 7.44-7.33 (m, 5H), 7.32 (d, 1H, J = 1.9 Hz), 7.29 (dd, 1H, J = 9.4, 2.6 Hz), 7.20 (dd, 1H, J = 8.5, 1.9 Hz), 7.06 (d, 1H, J = 8.5 Hz), 6.48 (ddd, 1H, J = 15, 13, 3.9 Hz), 5.87 (d, 1H, J = 15 Hz), 5.37.(d, 1H, J = 9.7 Hz), 5.33 (d, 1H, J = 9.7 Hz), 5.04-5.01 (m, 1H), 4.73 (t, 1H, J = 11 Hz), 4.25 (ddd, 1H, J = 12, 9.8, 3.5 Hz), 3.82 (s, 3H), 3.40-3.30 (m, 1H), 3.07-3.01 (m, 2H), 2.72 (dd, 1H, J = 14, 12 Hz), 2.65-2.47 (m, 4H), 2.38-2.28 (m, 1H), 2.21 (dt, 1H, J = 17, 7.5 Hz), 1.97 (dt, 1H, J = 17, 7.5 Hz), 1.80-1.70 (m, 3H), 1.54-1.46 (m, 2H), 1.17 (s, 3H), 1.03 (s, 3H), 1.01 (d, 3H, J = 7.0 Hz), 0.99 (d, 3H, J = 5.8 Hz), 0.95 (d, 3H, J = 5.8 Hz).

### Example 14

### Preparation of Cryptophycin 55 N-t-Boc-(L)-alaninate (17)

To a solution of 1 (103 mg, 0.146 mmol), N-t-Boc-(L)-alanine (41 mg, 0.219 mmol), and 4-dimethylamino pyridine (18 mg, 0.146 mmol) in 400 ml of anhydrous methylene chloride at room temperature was added a solution of 1,3-dicyclohexylcarbodiimide (45 mg, 0.219 mmol) in 87 ml of methylene chloride. After stirring for 5 h 50 min, the cloudy white reaction mixture was treated with another 5.5 mg (0.029 mmol) of N-t-Boc-(L)-alanine, 6.0 mg (0.029 mmol) of 1,3-dicyclohexylcarbodiimide, and a few crystals of 4-dimethylamino pyridine. After stirring an additional 1 h, the reaction was diluted with ethyl acetate-hexanes (3:1, 1 ml), stirred for 10 min, and filtered through a plug of celite, washing with ethyl acetate:hexanes (3:1). The filtrate and washings were concentrated in *vacuo* to a colorless oil. Chromatography (22 g of flash silica gel, 1.5:1 then 2:1 then 4:1/ethyl acetate-hexanes) afforded 96 mg (75%) of the title compound as a white foam: 500 MHz ¹H NMR (CDCl₃) d 7.35-7.30 (m, 5H), 7 .26-7 .21 (m, 2H), 7.10 (dd, 1H, J = 8.4, 1.9 Hz), 6.88 (d, 1H, J = 8.4 Hz), 6.76 (ddd, 1H, J = 15, 13, 4.2 Hz), 5.77 (d, 1H, J = 15 Hz), 5.52 (d, 1H, 7.6 Hz), 5.44 (d, 1H, J = 9.7 Hz), 4.98 (dd, 1H, J = 11, 2.5 Hz), 4.85-4.81 (m, 2H), 4.75 (q, 1H, J = 6.8 Hz), 4.56 (d, 1H, J = 7.8 Hz), 4.01-3.96 (m, 1H), 3.91 (s, 3H), 3.91 (dd, 1H, J = 13, 8.3 Hz), 3.20 (dd, 1H, J = 13, 4.0 Hz), 3.16 (dd, 1H, J = 15, 5.9 Hz), 3.08 (dd, 1H, J = 15, 7.6 Hz), 2.65-2.57 (m, 2H), 2.40-2.31 (m, 1H), 2.02-1.96 (m, 1H), 1.87-1.73 (m, 2H), 1.43 (s, 9H), 1.27 (s, 3H), 1.20 (s, 3H), 1.11-1.02 (m, 9H), 0.99 (d, 3H, J = 6. 3 Hz) .

### Example 15

### Preparation of Cryptophycin 55 (L)-alaninate hydrochloride salt (18)

To a solution of 17 (95 mg, 0.108 mmol) in 361 ml of methylene chloride at room temperature was added a 4.0 M solution of hydrogen chloride in 1,4-dioxane (135 ml, 0.542 mmol). After stirring for 2.5 h, the cloudy, white reaction mixture was concentrated *in vacuo* to provide 90 mg (96%, corrected for 6 wt% dioxane) of the title compound as a white foam: 500 MHz ¹H NMR (MeOH-d₄) d 8.54 (d, 1H, 7.6 Hz), 7.81 (br d, 1H, J = 9.7 Hz), 7.46-7.44 (m, 2H), 7.39-7.37 (m, 3H), 7.32 (d, 1H, J = 2.0 Hz), 7.20 (dd, 1H, J = 8.4, 2.0 Hz), 7.01 (d, 1H, J = 2.0 Hz), 6.69 (ddd, 1H, J = 15, 11, 3.7 Hz), 5.99 (d, 1H, 15 Hz), 5. 55 (d, 1H, J = 9.8 Hz), 5.20 (d, 1H, J = 9.8 Hz), 5.15 (dd, 1H, J = 11, 2.7 Hz), 4.78 (t, 1H, J = 11 Hz), 4.53-4.50 (m, 1H), 3.87 (s, 3H), 3.65 (q, 1H, J = 7.3 Hz), 3.50 (dd, 1H, J = 13, 9.8 Hz), 3.20 (dd, 1H, J = 14, 3.5 Hz), 3.14 (br d, 1H, J = 13 Hz), 2.81-2.71 (m, 3H), 2.41-2.34 (m, 1H), 1.98-1.93 (m, 1H), 1.88-1.82 (m, 2H), 1.41 (d, 3H, J = 7.3 Hz), 1.25 (s, 3H), 1.20 (s, 3H), 1.13 (d, 3H, J = 7.0 Hz), 1.06 (d, 3H, J = 6.2 Hz), 1.04 (d, 3H, 6.0 Hz).

### Example 16

### Preparation of Cryptophycin 55 N-t-Boc-(D)-alaninate (19)

To a solution of 1 (25 mg, 0.035 mmol), N-t-Boc-(D)-alanine (10 mg, 0.053 mmol), and 4-dimethylamino pyridine (0.4 mg, 0.0035 mmol) in 130 ml of anhydrous methylene chloride at room temperature was added a solution of 1,3-dicyclohexylcarbodiimide (11 mg, 0.053 mmol) in 47 ml of methylene chloride. After stirring for 5.5 h, the cloudy white reaction mixture was diluted with ethyl acetate-hexanes (3:1, 0.5 ml), stirred for 10 min, and filtered through a plug of celite, washing with ethyl acetate:hexanes (3:1). The filtrate and washings were concentrated *in vacuo* to a colorless oil. Chromatography (15 g of flash silica gel, 2:1/ethyl acetate-hexanes) afforded 26 mg (83%) of the title compound as a white foam: 500 MHz ¹H NMR (CDCl₃) d 7.49-7.29 (m, 5H), 7.24 (d, 1H, J = 2.0 Hz), 7.22-7.18 (m, 1H), 7.09 (dd, 1H, 8.4, 2.0 Hz), 6.87 (d, 1H, J = 8.4 Hz), 6.76 (ddd, 1H, J = 15, 13, 4.4, Hz), 5.77 (d, 1H, 15 Hz), 5.56 (d, 1H, J = 9.9 Hz), 5.48 (d, 1H, J = 7.7 Hz), 5.01 (dd, 1H, J = 10, 2.6 Hz), 4.91 (t, 1H, J = 9.4 Hz), 4.84 (d, 1H, J = 9.9 Hz), 4.81-4.73 (m, 2H), 3.99-3.93 (m, 1H), 3.91 (s, 3H), 3.39 (dd, 1H, J = 13, 8.0 Hz), 3.22 (dd, 1H, J = 13, 3.6 Hz), 3.17 (dd, 1H, J = 14, 5.0 Hz), 3.08 (dd, 1H, J = 14 Hz), 2.68-2.58 (m, 2H), 2.42-2.35 (m, 1H), 2.04-1.94 (m, 1H), 1.87-1.50 (m, 2H), 1.42 (s, 9H), 1.27 (s, 3H), 1.20 (s, 3H), 1.09 (d, 3H, J = 7.1 Hz), 1.04 (d, 3H, J = 6.4 Hz), 0.99 (d, 3H, J = 6.3 Hz), 0.65 (d, 3H, J = 6.8 Hz).

### Example 17

### Preparation of Cryptophycin 55 (D)-alaninate hydrochloride salt (20)

To a solution of **18** (24 mg, 0.027 mmol) in 274 ml of methylene chloride at room temperature was added a 4.0 M solution of hydrogen chloride in 1,4-dioxane (34 ml, 0.137 mmol). After stirring for 3.5 h, the clear, colorless reaction mixture was concentrated in *vacuo* to provide 24 mg (100%, corrected for 8 wt% dioxane) of the title compound as a white foam: 500 MHz ¹H NMR (MeOH-d₄) d 7.79 (d, 1H, J = 9.5 Hz), 7.47-7.40 (m, 2H), 7.40-7.36 (m, 3H), 7.31 (d, 1H, J = 2.0 Hz), 7.20 (dd, 1H, J = 8.4, 2.0 Hz), 7.01 (d, 1H, J = 8.4 Hz), 6.71 (ddd, 1H, J = 15, 13, 3.7 Hz), 5.98 (dd, 1H, J = 15, 1.6 Hz), 5.65 (d, 1H, J = 10 Hz), 5.20 (d, 1H, J = 10 Hz), 5.17 (dd, 1H, J = 11, 2.5 Hz), 4.88-4.78 (m, 1H), 4.53 (dd, 1H, J = 11, 3.7 Hz), 3.95 (q, 1H, J = 7.2 Hz), 3.87 (s, 3H), 3.51 (dd, 1H, J = 13, 9.8 Hz), 3.20 (dd, 1H, J = 14, 3.6 Hz), 3.14 (dd, 1H, J = 13, 2.3 Hz), 2.81-2.74 (m, 3H), 2.41-2.34 (m, 1H), 2.07-1.99 (m, 1H), 1.96-1.84 (m 2H), 1.26 (s, 3H), 1.21 (s, 3H), 1.15 (d, 3H, J = 7.1 Hz), 1.09 (d, 3H, J = 6.0 Hz), 1.05 (d, 3H, J = 6.0 Hz), 0.80 (d, 3H, J = 7.4 Hz).

### Example 18

### Preparation of Cryptophycin 55 Na-Ne-di-t-Boc-(L)-lysinate (21)

To a solution of 1 (105 mg, 0.149 mmol), Na-Ne-di-t-Boc-(L)-lysine (67 mg, 0.193 mmol), and 4-dimethylamino pyridine (18 mg, 0.149 mmol) in 400 ml of anhydrous methylene chloride at room temperature was added a solution of 1,3-dicyclohexylcarbodiimide (40 mg, 0.193 mmol) in 96 ml of methylene chloride. After stirring for 4 h, the cloudy white reaction mixture was treated with another 10 mg (0.030 mmol) of Na-Ne-di-*t*-Boc-(*L*)-lysine and 6.1 mg (0.030 mmol) of 1,3-dicyclohexylcarbodiimide as a soln in 100 ml of methylene chloride. After stirring an additional 1 h, the cloudy white reaction mixture was diluted with ethyl acetate-hexanes (3:1, 1 ml), stirred for 10 min, and filtered through a plug of celite, washing with ethyl acetate:hexanes (3:1). The filtrate and washings were concentrated in *vacuo* to a white foam which was resubmitted to the above conditions using 34 mg (0.097 mmol) of Na-Ne-di-*t*-Boc-(L)-lysine, 20 mg (0.097 mmol) of 1,3-dicyclohexylcarbodiimide, and 9.1 mg (0.075 mmol) of 4-dimethylamino pyridine. After stirring for 1.5 h, the reaction was processed as above to provide a crude white foam. Chromatography (21 g of flash silica gel, 1:1 then 4:1/ethyl acetate-hexanes) afforded 112 mg (73%) of the title compound as a white foam: 500 MHz ¹H NMR (MeOH -d₄) d 7.42-7.37 (m, 2H), 7.36-7.29 (m, 3H), 7.27 (br s, 1H), 7.16 (br d, 1H, J = 8.5 Hz), 6.97 (d, 1H, J = 8.5 Hz), 6.72 (ddd, 1H, J = 15, 13, 3.5 Hz), 5.92 (d, 1H, J = 15 Hz), 5.50 (d, 1H, J = 11 Hz), 5.11-5.04 (m, 2H), 4.84 (t, 1H, J = 10 Hz), 4.48 (dd, 1H, J = 11, 3.6 Hz), 3.84 (s, 3H), 3.75 (br s, 1H), 3.50-3.43 (m, 1H), 3.17 (dd, 1H, J = 14, 3.6 Hz), 3.11 (d, 1H, J = 14 Hz), 2.97-2.91 (m, 2H), 2.76-2.58 (m, 3H), 2.36-2.27 (m, 1H), 1.98-1.80 (m, 3H), 1.48-1.38 (m, 2H), 1.43 (s, 9H), 1.40 (s, 9H), 1.35-1.25 (m, 2H), 1.23 (s, 3H), 1.20 (s, 3H), 1.15-1.09 (m, 2H), 1.07 (d, 3H, J = 6.8 Hz), 1.06 (d, 3H, J = 6.0 Hz), 1.01 (d, 3H, J = 6.1 Hz).

### Example 19

### Preparation of Cryptophycin 55 (L)-lysinate di-hydrochloride salt (22)

To a solution of 21 (107 mg, 0.103 mmol) in 345 ml of methylene chloride at room temperature was added a 4.0 M solution of hydrogen chloride in 1,4-dioxane (155 ml, 0.621 mmol). After stirring for 4 h, the cloudy white reaction mixture was filtered. The collected white solid was washed with methylene chloride (2 x 1 ml) and dried *in vacuo* at room temperature to provide 87 mg (93%) of the title compound: 500 MHz ¹H NMR (MeOH-d₄) d 8.61 (d, 1H, J = 7.7 Hz), 7.81 (d, 1H, J = 7.7 Hz), 7.47-7.44 (m, 2H), 7.40-7.38 (m, 3H), 7.31 (d, 1H, J = 2.2 Hz), 7.20 (dd, 1H, J = 8.4, 2.2 Hz), 7.00 (d, 1H, J = 8.4 Hz), 6.63 (ddd, 1H, J = 15, 13, 4.0 Hz), 6.00 (dd, 1H, J = 15, 1.6 Hz), 5.55 (d, 1H, J = 9.8 Hz), 5.20 (d, 1H, J = 9.8 Hz), 5.15 (dd, 1H, J = 10, 2.9 Hz), 4.68 (t, 1H, J = 11 Hz), 4.55-4.49 (m, 1H), 3.87 (s, 3H), 3.79 (t, 1H, J = 5.6 Hz), 3.52 (dd, 1H, J = 14, 9.9 Hz), 3.20 (dd, 1H, J = 14, 3.6 Hz), 3.13 (dd, 1H, J = 13, 2.4 Hz), 3.06-2.98 (m, 1H), 2.94-2.87 (m, 1H), 2.85-2.74 (m, 3H), 2.45-2.38 (m, 1H), 1.98-1.76 (m, 5H), 1.71-1.64 (m, 2H), 1.39-1.30 (m, 2H), 1.25 (s, 3H), 1.18 (d, 3H, J = 8.2 Hz), 1.17 (s, 3H), 1.08 (d, 3H, J = 6.2 Hz), 1.05 (d, 3H, J = 6.1 Hz).

### Example 20

### Preparation of 2'-(di-t-Butylphosphatyl)phenylacetic acid (24)

To a solution of 2'-hydroxyphenethyl alcohol (1.05 g, 7.60 mmol) in 15.2 ml of N, N-dimethylformamide at 0 °C was added imidazole (621 mg, 9.11 mmol) and tert-butyldimethylsilyl chloride (1.26 g, 8.34 mmol). After stirring at 0 °C for 40 min and at room temperature for 45 min, another 155 mg (2.28 mmol) of imidazole and 229 mg (1.52 mmol) of *tert*-butyldimethylsilyl chloride were added. The reaction was allowed to stir for an additional 15 min at which time 150 ml of tert-butyl methy ether was added. The mixture was washed with cold 1N aqueous hydrochloric acid (1 X 15 ml) followed by water (1 X 15 ml). The organic layer was dried (Na₂SO₄), filtered, and concentrated in *vacuo* to a yellow oil. Chromatography (70 g of flash silica gel), eluting with hexanes-ethyl acetate (5:1) provided 1.81 g (94%) of the 1° silyl ether as a faintly off-white oil. To a solution of the silyl ether (506 mg, 2.00 mmol) and di-tert-butyl diethylphosphoramidite (600 ml of 93%, 2.00 mmol) in 2 ml of tetrahydrofuran at room temperature was added 1-H-tetrazole (421 mg, 6.01 mmol). After stirring for 45 min the reaction mixture was cooled to -10 °C and rapidly treated with a solution of m-chloroperbenzoic acid (450 mg of 99%, 2.61 mmol) in 3.6 ml of methylene chloride. The cloudy white reaction was allowed to warm to room temperature and stir for 15 min. The reaction was quenched with 4 ml of 10% aqueous sodium bisulfite, stirred vigorously for 10 min, diluted with 15 ml of tert-butyl methy ether, and washed with 10% aqueous sodium bisulfite (2 X 10 ml) followed by 0.5 N aqueous sodium hydroxide (2 X 10 ml). The organic layer was dried (Na₂SO₄), filtered, and concentrated *in vacuo* to a colorless oil (941 mg) which was used directly in the next step. The crude phosphate was dissolved in 10 ml of tetrahydrofuran, cooled to 0 °C, and treated with a 1 M solution of tetra-n-butylammonium fluoride in tetrahydrofuran (2.4 ml, 2.4 mmol). After stirring at 0 °C for 20 min and at room temperature for 1.5 h, the reaction was diluted 60 ml of tert-butyl methy ether and washed with water (1 X 10 ml) followed by brine (1 X 10 ml). The organic layer was dried (Na₂SO₄), filtered, and concentrated *in vacuo* to a yellow oil. Chromatography (50 g of flash silica gel), eluting with ethyl acetate-hexanes (3:1) provided 525 mg (79%) of the 1° alcohol as an off-white oil. To a solution of the alcohol (123 mg, 0.372 mmol) in acetonitrile-carbon tetrachloride (1:1, 1.49 ml) at room temperature was added water (1.1 ml) followed by sodium periodate (239 mg, 1.12 mmol) and ruthenium(III)chloride hydrate (1.8 mg, 0.0082 mmol). The brown mixture was allowed to stir rapidly at room temperature for 55 min. Upon concentration *in vacuo* and chromatography (8 g of flash silica gel, eluting with 10% methanol-ethyl acetate) 109 mg (85%) of the title compound was obtained as a purple oil: 500 MHz ¹H NMR (CDCl₃) d 7.49 (d, 1H, J = 7.53 Hz), 7.30-7.15 (m, 3H), 3.77 (s, 2H), 1.51 (s, 18H) .

### Example 21

The compound of Examples 21-26 were prepared using the methods substantially as hereinbefore described.

### Spectral Properties of Cryptophycin-129

EIMS m/z (rel intensity) 654/656 (2.1/0.8, M-HBr), 412/414 (9.7/3.6), 280/282 (10.3/3.5), 227 (15.2), 195/197 (50.1/16.3), 105 (100), 91 (100); high resolution EIMS m/z 654.2687(C₃₅H₄₃ClN₂O₈, D 2.1 mmu). ¹H NMR (CDCl₃) *amino or hydroxy acid unit* d (carbon position, multiplicity; *J* in Hz) *7-bromo-5,8-dihydroxy-6-methyl-8-phenyl-2-octenoic acid (A)* 5.76 (2,d; 15.3), 6.68 (3,ddd; 15.3,9.9 and 5.6, 2.29 (4,ddd; 14.2, 10.6 and 10.4), 2.67 (4,dd; 14.2 and 5.4), 4.95 (5,ddd;10.3, 10.3 and 1.8), 2.47 (6,m), 1.15 (6-Me, d; 6.5), 4.29 (7,dd; 9. 1 and 2.1), 4.84 (8,d; 9.0, 7.34-7.39 (10/11/12/13/14,m); *3-chloro-4-methoxyphenylalanine (B)* 4.80 (2,m), 5.73 (2-NH, d; 8.8), 3.01 (3, dd; 14.6 and 7.6), 3.16 (3, dd; 14.3 and 5.6), 7.23 (5, d; 2.0), 3.87 (7-OMe, s), 6.84 (8, d; 8.4), 7.09 (9, dd; 8.4 and 2.0); *3-amino-2-methylpropionic acid (C)* 2.73 (2, m), 1.23 (2-Me, d; 7.2), 3.25 (3, ddd; 13.5, 6.5 and 6.5), 3.53 (3, ddd; 13.4, 5.4 and 3.9), 6.93 (3-NH, brt; 6.1); *leucic acid (D)* 4.91 (2, dd; 10.5 and 2.8, 1.49 (3,m), 1.80 (3, m), 1.73 (4, m). 0.92 (4-Me, d; 6.5), 0.89 (5, d; 6.5). ¹³C NMR (CDCl₃) unit d (carbon position) A 165.4(1), 125.2 (2), 141.3 (3), 36.6 (4), 75.7 (5), 37.9 (6), 11.8 (6-Me), 60.3 (7), 76.7 (8), 141.6 (9), 126.8 (10/14), 128.7 (11/13), 128.8 (12); **B** 171.0 (1), 53.6 (2), 35.0 (3), 130.0 (4), 131.0 (5), 122.4 (6), 153.9 (7), 56.1 (7-OMe), 112.3 (8), 128.4 (9); C 175.4 (1), 38.4 (2), 14.0 (2-Me), 41.3 (3), D 170.2 (1), 71.3 (2), 39.6 (3), 24.8 (4), 23.2 (4-Me), 21.4 (5) .

### Example 22

### Spectral Properties of Cryptophycin-138

EIMS m/z (rel intensity) 734/736 (0.3/0.1), 654/656 (0.6/0.2), 412/414 (12.6/4.7), 313/315 (20.4/12.5) 280 (7.6), 227 (3.5), 195/197 (44.7/15.6), 155/157 (35.4/13.6), 105 (33.6), 91 (34.2), 80/82 (100/100); high resolution EIMS m/z 734.1985 (C₃₅H₄₄ClBrN₂O₈, D-1.6 mmu), 654.2722(C₃₅H₄₃ClN₂O₈, D-1.4 mmu). ¹H NMR (CDCl₃) *amino or hydroxy acid unit* d (carbon position, multiplicity; *J* in Hz) *7-bromo-5,8-dihydroxy-6-methyl-8-phenyl-2-octenoic acid (A)* 5.78 (2, d; 15.5), 6.69 (3, ddd; 15.1, 9.8 and 5.3), 2.33 (4,m), 2.71 (4,m), 4.97 (5, ddd; 10.9, 10.4 and 1.6), 2.45 (6, m), 1.17 (6-Me, d; 6.3), 4.40 (7, dd; 10.1 and 2.2), 5.06 (8, d; 10.1), 7.36-7.42 (10/11/12/13/14, m); *3-chloro-4-methoxyphenylalanine (B)* 4.82 (2, m), 5.69 (2-NH, d; 8.7), 3.02 (3, dd; 14.5 and 7.5), 3.17 (3, dd; 14.5 and 5.9), 7.23 (5, d; 2.1), 3.88 (7-OMe, s), 6.85 (8, d; 8.4), 7.09 (9, dd; 8.4 and 1.9) ; *3-amino-2-methylpropionic acid (C)* 2.73 (2,m), 1.23 (2-Me, d; 7.3), 3.25 (3, ddd; 13.9, 6.5 and 6.5), 3.54 (3, m), 6.92 (3-NH, brt; 5.7); *leucic acid (D)* 4.91 (2, dd; 10.6 and 2.7), 1.43 (3, m), 1.82 (3, m), 1.75 (4, m), 0.93 (4-Me, d; 6.6), 0.90 (5, d; 6.6). ¹³C NMR (CDCl₃) unit d (carbon position) A 165.4 (1), 125.2 (2), 141.2 (3), 36.6 (4), 76.5 (5), 38.2 (6), 11.7 (6-Me), 55.3 (7), 87.4 (8), 137.4 (9), 128.0 (10/14), 128.7 (11/13), 129.3 (12); **B** 171.0 (1), 53.6 (2), 35.0 (3), 129.9 (4), 131.0 (5), 122.4 (6), 153.9 (7), 56.1 (7-OMe), 112.2 (8), 128.4 (9) ; C 175.5 (1), 38.4 (2), 14.0 (2-Me), 41.2 (3), D 170.2 (1), 71.2 (2), 39.6 (3), 24.8 (4), 23.2 (4-Me), 21.4 (5).

### Example 23

### Spectral Properties of Cryptophycin-139

EIMS m/z (rel intensity) 732/734 (0.3/0.3), 652/654 (0.9/0.5), 533 (13.6), 445(5.7), 195/197 (9.2/11.4), 105 (96.2), 80/82 (100/100); high resolution EIMS m/z 732,1783(C₃₅H₄₂ClBrN₂O₈, Æ 3.0 mmu), 652.2573 (C₃₅H₄₁ClN₂O₈, Æ-2.1 mmu). ¹H NMR (CDCl₃) *amino or hydroxy acid unit d* (carbon position, multiplicity; J in Hz) *7-bromo-8-oxo-5-hydroxy-6-methyl-8-phenyl-2-octenoic acid* (**A**) 5.75 (2, d; 15.4), 6.68 (3, ddd; 15.2, 10.1 and 5.0), 2.31 (4, ddd; 14.2, 10.7 and 10.7), 2.63 (4, dd; 14.4 and 5.1), 5.09 (5, ddd; 9.7, 9.7 and 1.5), 2.46 (6, m), 1.18 (6-Me, d; 6.6), 5.41 (7, d; 3.5), 7.94 (10/14, brd; 8.6), 7.50 (11/13, t; 7.8), 7.63 (12, brt; 7.5); *3-chloro-4-methoxyphenylalanine* (**B**) 4.80 (2,m), 5.64 (2-NH, d; 8.3), 3.04 (3, dd; 14.6 and 7.2), 3.14 (3, dd; 19.4 and 5.5), 7.22 (5,d; 2.0), 3.87 (7-OMe, s), 6.84 (8, d; 8.3), 7.08 (9, dd; 8.3 and 2.3); 3-*amino-2-methylpropionic acid* (C) 2.76 (2,m), 1.26 (2-Me, d; 7.1),3.31 (3, ddd; 13.7, 6.8 and 6.6), 3.53 (3 m), 6.95 (3-NH, brt; 5.9); leucic acid (D) 4.93 (2, dd; 10.6 and 3.0), 1.51 (3, m), 1.95 (3, m), 1.80 (4,m), 0.97 (4-Me, d; 6.8), 0.91 (5, d, 6.6), 13C NMR (CD13) unit d (carbon position) **A** 165.2 (1), 125.3 (2), 141.0 (3), 36.2 (4), 76.0 (5), 39.3 (6), 13.8 (6-Me), 51.8 (7), 192.3 (8), 134.2 (9), 128.7 (10/14), 129.0 (11/13), 134.0 (12); **B** 170.9(1), 53.7 (2), 35.0 (3), 129.8 (4), 131.0 (5), 122.4 (6), * (7, not observed)), 56.2 (7-OMe), 112.3 (8), 128.4 (9); C 175.6 (1), 38.3 (2), 14.2 (2-Me), 41.1 (3), D 170.2 (1), 71.1 (2), 39.8 (3), 24.7, 23.2 (4-Me), 21.3 (5) .

### Example 24

### Spectral Properties of Cryptophycin-145

EIMS m/z (rel intensity) 734/736/738 (0.5/0.6/0.2), 654,656 (1.5/1.1), 412/414 (2.2/1.0), 313/315 (19.5/12.9), 227 (4.1), 195/197 (11.5/3.7), 105 (14.6), 91 (25.2), 80/82 (100/100); high resolution EIMS m/z 736.1980 (C₃₅H₄₄ClBrN₂O₈, Æ -3.1 mmu), 654.2714 (C₃₅H₄₃ClN₂O₈, Æ -0.6 mmu). ¹H NMR (CDCl₃) *amino or hydro amino or hydroxy acid unit* d (carbon position, multiplicity; J in Hz) *7-bromo-5,8-dihydroxy-6-methyl-8-phenyl-2-octenoic acid* (A) 5.81 (2, d; 15.7), 6.72 (3, m), 2.31 (4, m), 2.77 (4, m), 5.62 (5, m), 2.42 (6, m), 1.22 (6-Me, d; 7.0), 4.23 (7, dd; 8.0 and 3.2), 4.94 (8, d; 7.9), 7.32-7.28 (10/11/12/13/14, m); *3-chloro-4-methoxyphenylalanine* (B) 4.82 (2, m), 5.72 (2-NH, brs), 3.05 (3, m), 3.15 (3, m), 7.23 (5, brs), 3.88 (7-OMe, s), 6.84 (8, brd; 7), 7.09 (9, m); *3-amino-2-methylpropionic acid* (C) 2.75 (2, m), 1.25 (2-Me, d; 7.0), 3.30 (3, m), 3.53 (3, m), 6.97 (3-N, brs); *leucic acid* (D) 4.90 (2, dd; 9.7 and 3.6), 1.58 (3, m), 1.82 (3, m), 1.70 (4, m). 0.95 (4-Me, d; 6.6), 0.89 (5, d; 6.6). ¹³C NMR (CDCl₃) unit d (carbon position) A 165.14 (1), 125.2 (2), 141.3 (3), 35.8 (4), 75.8 (5), 41.2 (6), 13.4 (6-Me), 60.7 (7), 76.5 (8), 141.6 (9), 126.8 (10/14), 128.5 (11/13), 128.6 (12); **B** 170.5 (1), 53.7 (2), 35.0 (3), 129.9 (4), 131.0 (5), 122.4 (6), 154.0 (7), 56.2 (7.OMe), 112.3 (8), 128.5 (9); C 175.4 (1), 38.4 (2), 14.1 (2-Me), 41.2 (3), D 170.5 (1), 71.3 (2), 39.7 (3), 24.6 (4), 22.9 (4-e), 21.5 (5).

### Example 25

### Spectral Properties of Cryptophycin-140

EIMS m/z (rel intensity) 690/692 (2.3/1.8), 654/656 (3.8/2.2), 412/414 (5.4/2.1), 280/282 (5.4/2.2), 227 (10.2), 195/197 (27.8/10/4), 155/157 (53.7/16/4), 105 (81.0), 91 (100); high resolution EIMS m/z 690.2427 (C35H44Cl2N2O8, Æ 4.8 mmu), 654.2760 (C35H43ClN2O8, Æ -5.2 mmu). 1H NMR (CDCl3) amino or hydroxy acid unit d (carbon position, multiplicity; J in Hz) 7-chloro-5,8-dihydroxy-6-methyl-8-phenyl-2-octenoic acid (A) 5.77 (2, d; 15.4), 6.68 (3, ddd; 15.3, 9.8 and 5.6), 2.29 (4, m), 2.67 (4, m), 4.98 (5, ddd; 10.3, 10.3 and 1.7), 2.64 (6, m), 1.15 (6-Me, d; 6.6), 4.19 (7, dd; 9.2 and 2.1), 4.72 (8, d; 9.2), 7.33-7.39 (10/11/12/13/14, m); *3-chloro-4-methoxyphenylalanine* (**B**) 4.81 (2, m), 5.69 (2-NH, d; 8/8), 3.01 (3, dd; 14.5 and 7.5), 3.16 (3, dd; 14.5 and 5.5), 7.23 (5, d; 2.1), 3.87 (7-OMe, s), 6.84 (8, d; 8.3), 7.09 (9, dd; 8.4 and 2.0); *3-*amino-2-methylpropionic acid (C) 2.73 (2, m), 1.22 (2-Me, d; 7.3), 3.29 (3, ddd; 13.5, 6.8 and 6.8), 3.53 (3, m), 6.91 (3-NH, brt; 6.0); *leucic acid* (D) 4.89 (2, dd; 10.4 and 2.9), 1.39 (3, m), 1.69-1.80 (3/4, m). 0.90 (4-Me, d; 6.4), 0.87 (5, d; 6.6). 13 C NMR (CDCl₃) unit d (carbon position) A 165.4 (1), 125.2, 141.4 (3), 36.7 (4), 75.5 (5), 38.1 (6), 10.4 (6-Me), 65.0 (7), 75.8 (8), 141.4 (9), 126.8 (10/14), 128.7 (11/13), 128.8 (12); **B** 171.0 (1), 53.6 (2), 35.0 (3), 129.9 (4), 131.0 (5), 122.4 (6), 153.9 (7), 56.1 (7-OMe), 112.2 (8), 128.4 (9); C 175.5 (1), 38.4 (2), 14.1 (2-Me), 41.3 (3), D 170.3 (1), 71.3 (2), 39.5 (c), 24.7 (4), 23.2 (4-Me), 21.4 (5).

### Example 26

### Spectral Properties of Cryptophycin-141

EIMS m/z (rel intensity) 654/656 (1.8/0.8, M-HCl), 412/414 (7.6/3.1), 280/282 (3.2/2.0), 227 (8.4), 195/197 (35.6/11.9), 155/157 (100/37.2), 105 (68.1), 91 (88.4); high resolution EIMS m/z 690.2468 (C₃₅H₄₄Cl₂N₂O₈, Æ 0.6 mmu), 654.2706 (C₃₅H₄₃ClN₂O₈, Æ 0.2 mmu). ¹H NMR (CDCl₃) *amino or hydroxy acid unit* d (carbon position, multiplicity; J in Hz) *7-chloro-5,8-dihydroxy-6-methyl-8-phenyl-2-octenoic acid* (**A**) 5.69 (2, d; 15.4), 6.62 (3, ddd; 15.1, 9.9 and 5.2), 2.07 (4, m), 2.49 (4, dd, 14.4 and 5.4), 4.96 (5, ddd; 10.5, 10.5 and 1.8), 1.88 (6, m), 1.07 (6-Me, d; 6.8, 4.24 (7, dd; 8.9 and 1.9), 4.73 (8, d; 8.8), 2.80 (8-OH, broad Peak), 7.32 (10/14, dd; 7.6 and 1.9), 7.36-7.42 (11/12/13, m); *3-chloro-4-methoxyphenylalanine* (**B**) 4.78 (2, m), 5.65 (2-NH, d; 8.6), 3.00 (3, dd; 14.4 and 7.2), 3.13 (3, dd; 14.4 and 5.6), 7.20 (5, d; 2.3), 3.86 (7-OMe, s), 6.82 (8, d; 8.3), 7.06 (9, dd; 8.3 and 2.2); *3-amino-2-methylpropionic acid* (C) 2.73 (2, ), 1.23 (2-Me, d; 7.2) 3.27 (3, ddd; 13.5, 6.8 and 6.8), 3.51 (3, ddd; 13.5, 5.0 and 3.8), 6.91 (3-NH, brt; 6.0); leucic acid (D) 4.83 (2, dd; 10.4 and 2.9), 1.48 (3, m), 1.70 (3, m), 1.76 (4, m). 0.99 (4-Me, d; 6.5), 0.95 (5, d; 6.5). ¹³C NMR (CDCl₃) unit d (carbon position) **A** 165.3 (1), 125.2 (s), 141.0 (3), 36.2 (4), 75.5 (5), 39.3 (6), 10.8 (6-Me), 70.0 (7), 76.2 (8), 138.9 (9), 126.7 (10/14), 129.0 (11/13), 129.0 (12); **B** 170.9 (1), 53.6 (2), 35.0 (3), 129.8 (4), 131.0 (5), 122.4 (6), 153.9 (7), 56.1 (7-)Me), 112.2 (8), 128.4 (9); C 175.5 (1), 38.2 (2), 14.1 (2-Me), 41.1 (3), D 170.2 (1), 71.2 (2), 39.6 (3), 24.8 (4), 23.2 (4-Me), 21.4 (5).

### Example 27

### Scheme 1 Experimentals

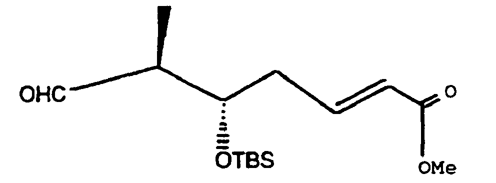

To a solution of alkene 1 (1.15 g, 3.52 mmol) in 50 mL of CH₂Cl₂ at -78 °C, was added pyridine (0.3 mL, 3.9 mmol) and 0.98 mL of a 0.1% solution of Sudan Red 7B in CH₂Cl₂. Ozone was slowly bubbled in until the red color changed to a yellow. The reaction progress was monitored by TLC. Upon completion, zinc dust (1.63 g, 24.9 mmol) and 3.4 mL of glacial acetic acid were added. The cold bath was removed and the mixture was allowed to warm up slowly to room temperature and was stirred an additional 2 h. The reaction mixture was filtered through Celite and washed with CuSO₄ (3 x 30 mL), followed by water (3 x 20 mL) and finally saturated aqueous NaHCO₃ (2 x 20 mL). The organic layer was dried over MgSO₄, filtered and concentrated in vacuo to give 0.937 g (88%) of the desired aldehyde which was used in the next step without further purification: ¹H NMR (300 MHz, CDCl₃) d 9.79-9.73 (d, 1 H, *J* = 1.97 Hz), 7.0-6.82 (m, 1 H), 5.91-5.86 (d, 1 H, *J* = 15.9 Hz), 9.1-9.0 (m, 1 H), 3.73 (s, 3 H), 2.55-2.4 (m, 3 H), 1.09-1.07 (d, 3 H, *J* = 6.8 Hz), 0.87 (s, 9 H), 0.08 (s, 3 H), 0.06 (s, 3 H).

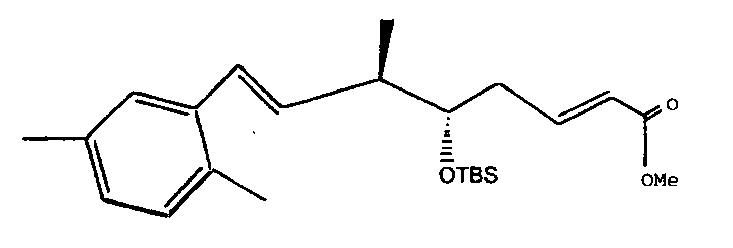

Methyllithium (9.6 mL, 14.4 mmol), as a 1.5 M solution in diethyl ether complexed with lithium bromide, was added dropwise to a -78 °C solution of 2,5-dimethylbenzyl triphenylphosphonium chloride in 120 mL of THF. The solution was allowed to warm up slowly to 0 °C and was cooled back down to -78 °C. Aldehyde **2** in 40 mL of THF, was added dropwise to the ylide at -78 °C. The mixture was stirred at -78°C for 15 min and then was warmed up slowly to room temperature. Following 1 h at rt, sat. NH₄Cl (50 mL) was added and the solution was extracted with ether. The ether layer was washed with water (2 x 50 mL) then brine, dried over MgSO₄, filtered and concentrated in vacuo. The crude styrene as a mixture of E:Z isomers was purified on silica gel using 2% EtOAc/hexanes to yield 2.32 g (58%) of a clear oil.
The E:Z mixture was refluxed for 8 h in 120 mL of benzene in the presence of thiophenol (0.3 mL) and 1,1'-azobis(cyclohexanecarbonitrile) (VAZO) (0.16 g). The solution was then cooled to ambient temperature, concentrated under vacuum and purified by column chromatography (silica gel, 2-5% EtOAc/hexanes) to yield 2.2 g (95%) of the pure E isomer as a clear oil: [a]²⁰D +34.6° (c1.0, MeOH); ¹H NMR (300 MHz, CDCl₃) d 7.3-7.0 (m, 4 H), 6.62-6.57 (d, 1 H, *J* = 15.8 Hz), 6.11-6.03 (dd, 1 H, *J =* 15.8, 8.1 Hz), 5.93-5.88 (d, 1 H, *J* = 15.5 Hz), 3.82-3.78 (m, 1 H), 3.78 (s, 3 H), 2.6-2.34 (m, 3 H), 2.37 (s, 3 H), 2.34 (s, 3 H), 1.18-1.16 (d, 3 H, *J* = 6.8 Hz), 0.96 (s, 9 H), 0.13 (s, 3 H), 0.11 (s, 3 H); ¹³C NMR (75 MHz, CDCl₃) d 167.2, 146.9, 136.9, 135.7, 133.3, 132.4, 130.5, 128.8, 128.1, 126.5, 123.2, 75.5, 51.8, 43.4, 37.9, 26.2, 21.4, 19.7, 18.5, 16.7, - 4.0, - 4.2; IR (CHCl₃) 2955, 2930, 2858, 1718, 1658, 1603,1496,1472, 1438, 1362, 1325, 1280, 1258, 1097, 1040 cm⁻¹; Anal. Calcd for C₂₄H₃₈O₃Si: C, 71.59; H, 9.51. Found: C, 71.38; H, 9.30.

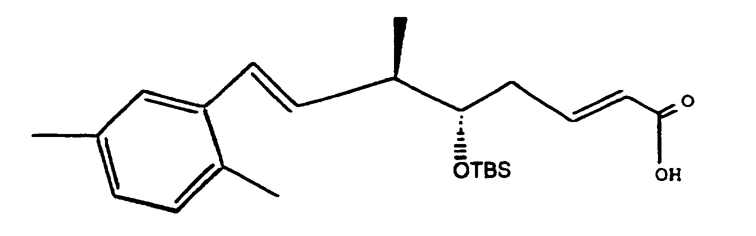

To a solution of ester 3 (2.2 g, 5.46 mmol) in 10 mL of THF was added 11 mL of a 2 M KOH solution. While being stirred vigorously, the resulting mixture was heated at 65 °C for 24 h. Upon cooling to ambient temperature, 11 mL of a 2 M solution of HCl was added and the resulting mixture was stirred vigorously for an additional 30 min. Ethyl acetate (50 mL) was added to the mixture and the layers separated. The aqueous layer was further extracted with ethyl acetate (2 x 30 mL). The combined organic layers were washed with 50% brine (3 x 30 mL), dried over Na₂SO₄, filtered and concentrated in vacuo to yield 2.12 g (100%) of the desired acid as a thick yellow oil: ¹H NMR (300 MHz, CDCl₃) d 7.26-6.95 (m, 4 H), 6.62-6.57 (d, 1 H, *J* = 15.8 Hz), 6.1-6.02 (dd, 1 H, *J* = 15.8, 8.0 Hz), 5.92-5.87 (d, 1 H, *J* = 15.7 Hz), 3.9-3.8 (m, 1 H), 2.6-2.2 (m, 3 H), 2.36 (s, 3 H), 2.34 (s, 3 H), 1.18-1.15 (d, 3 H, J = 6.8 Hz), 0.94 (s, 9 H), 0.12 (s, 3 H), 0.11 (s, 3 H).

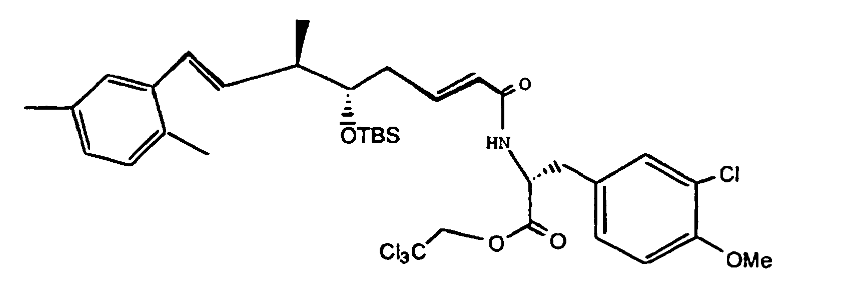

To a 0 °C solution of acid **4** (2.12 g, 5.46 mmol) and diisopropylethylamine (2.9 mL, 16.4 mmol) in 7 mL of DMF was added diphenylphosphinic chloride (1.1 mL, 6.01 mmol) dropwise. Following 5 min of stirring at 0 °C and 30 min at room temperature, the TFA salt of 3-(3-chloro-4-methoxyphenyl)-D-alanine-2,2,2-trichloroethyl ester 5 in 7 mL of DMF was added dropwise. The resulting mixture was stirred at room temperature for 2 h, poured into 100 mL of water and washed with diethyl ether (3 x 50 mL). The combined organic layers were washed with brine, dried over MgSO₄, filtered through Celite and concentrated in vacuo. Purification by column chromatography (silica gel, 10-30% EtOAc/hexanes) gave 2.86 g (72%) of amide 6 as a white foam: [a]²⁰_{D} + 44.8° (cl.0, MeOH); ¹H NMR (300 MHz, CDCl₃) d 7.25 (s, 1 H), 7.22 (s, 1 H), 7.1-6.83 (m, 5 H), 6.6-6.55 (d, 1 H, *J* = 15.7 Hz), 6.11-6.03 (dd, 1 H, *J* = 15.6, 8.1 Hz), 5.9-5.8 (m, 2 H), 5.18-5.05 (m, 1 H), 9.83-4.75 (q, 2 H, *J* = 12.0 Hz), 3.9 (s, 3 H), 3.83-3.68 (m, 1 H), 3.3-3.1 (m, 2 H), 2.6-2.36 (m, 3 H), 2.36 (s, 3 H), 2.32 (s, 3 H), 1.16-1.19 (d, 3 H, *J* = 6.7 Hz), 0.95 (s, 9 H), 0.11 (s, 3 H), 0.05 (s, 3H); ¹³C NMR (75 MHz, CDCl₃) d 170.0, 165.1, 154.2, 143.1, 136.4, 135.3, 133.0, 132.0, 131.0, 130.9, 130.0, 128.4, 128.3, 127.7, 126.1, 124.6, 112.3, 112.1, 94.2, 75.0, 74.7, 56.0, 52.9, 42.8, 37.5, 36.5, 25.8, 20.9, 19.3, 18.0, 16.4, - 4.3, - 4.7; IR (CHCl₃) 3428, 2957, 2930, 2857, 1759, 1676, 1645, 1606, 1503, 1464, 1442, 1380, 1349, 1281, 1259, 1173, 1067 cm⁻¹.

### Example 27

### Scheme 1 Experimentals

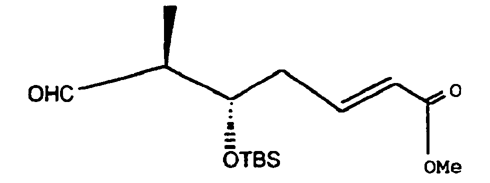

To a solution of alkene 1 (1.15 g, 3.52 mmol) in 50 mL of CH₂Cl₂ at -78 °C, was added pyridine (0.3 mL, 3.9 mmol) and 0.98 mL of a 0.1% solution of Sudan Red 7B in CH₂Cl₂. Ozone was slowly bubbled in until the red color changed to a yellow. The reaction progress was monitored by TLC. Upon completion, zinc dust (1.63 g, 24.9 mmol) and 3.4 mL of glacial acetic acid were added. The cold bath was removed and the mixture was allowed to warm up slowly to room temperature and was stirred an additional 2 h. The reaction mixture was filtered through Celite and washed with CuSO₄ (3 x 30 mL), followed by water (3 x 20 mL) and finally saturated aqueous NaHCO₃ (2 x 20 mL). The organic layer was dried over MgSO₄, filtered and concentrated in vacuo to give 0.937 g (88%) of the desired aldehyde which was used in the next step without further purification: ¹H NMR (300 MHz, CDCl₃) d 9.74-9.73 (d, 1 H, *J* = 1.97 Hz), 7.0-6.82 (m, 1 H), 5.91-5.86 (d, 1 H, *J* = 15.4 Hz), 4.1-4.0 (m, 1 H), 3.73 (s, 3 H), 2.55-2.4 (m, 3 H), 1.09-1.07 (d, 3 H, *J* = 6.8 Hz), 0.87 (s, 9 H), 0.08 (s, 3 H), 0.06 (s, 3 H).

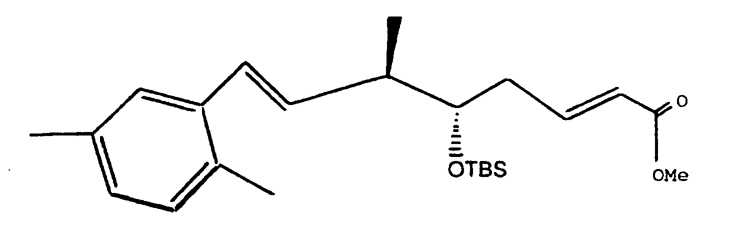

Methyllithium (9.6 mL, 14.4 mmol), as a 1.5 M solution in diethyl ether complexed with lithium bromide, was added dropwise to a -78 °C solution of 2,5-dimethylbenzyl triphenylphosphonium chloride in 120 mL of THF. The solution was allowed to warm up slowly to 0 °C and was cooled back down to -78 °C. Aldehyde 2 in 40 mL of THF, was added dropwise to the ylide at -78 °C. The mixture was stirred at -78°C for 15 min and then was warmed up slowly to room temperature. Following 1 h at rt, sat. NH₄Cl (50 mL) was added and the solution was extracted with ether. The ether layer was washed with water (2 x 50 mL) then brine, dried over MgSO₄, filtered and concentrated in vacuo. The crude styrene as a mixture of E:Z isomers was purified on silica gel using 2% EtOAc/hexanes to yield 2.32 g (58%) of a clear oil.
The E:Z mixture was refluxed for 8 h in 120 mL of benzene in the presence of thiophenol (0.3 mL) and 1,1'-azobis(cyclohexanecarbonitrile) (VAZO) (0.16 g). The solution was then cooled to ambient temperature, concentrated under vacuum and purified by column chromatography (silica gel, 2-5% EtOAc/hexanes) to yield 2.2 g (95%) of the pure E isomer as a clear oil: [a]²⁰D +34.6° (c1.0, MeOH); ¹H NMR (300 MHz, CDCl₃) d 7.3-7.0 (m, 4 H), 6.62-6.57 (d, 1 H, *J =* 15.8 Hz), 6.11-6.03 (dd, 1 H, *J =* 15.8, 8.1 Hz), 5.93-5.88 (d, 1 H, *J =* 15.5 Hz), 3.82-3.78 (m, 1 H), 3.78 (s, 3 H), 2.6-2.34 (m, 3 H), 2.37 (s, 3 H), 2.34 (s, 3 H), 1.18-1.16 (d, 3 H, *J =* 6.8 Hz), 0.96 (s, 9 H), 0.13 (s, 3 H), 0.11 (s, 3 H); ¹³C NMR (75 MHz, CDCl₃) d 167.2, 146.9, 136.9, 135.7, 133.3, 132.4, 130.5, 128.8, 128.1, 126.5, 123.2, 75.5, 51.8, 43.4, 37.9, 26.2, 21.4, 19.7, 18.5, 16.7, - 4.0, - 4.2; IR (CHCl₃) 2955, 2930, 2858, 1718, 1658, 1603,1496,1472, 1438, 1362, 1325, 1280, 1258, 1097, 1040 cm⁻¹; Anal. Calcd for C₂₄H₃₈O₃Si: C, 71.59; H, 9.51. Found: C, 71.38; H, 9.30.

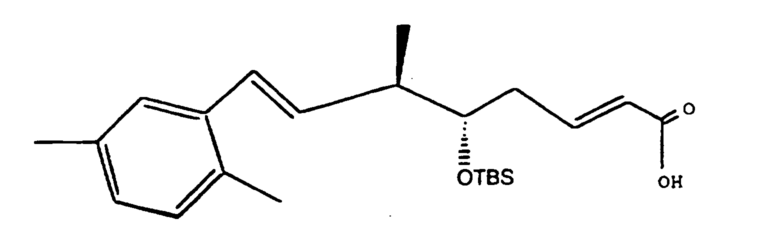

To a solution of ester 3 (2.2 g, 5.46 mmol) in 10 mL of THF was added 11 mL of a 2 M KOH solution. While being stirred vigorously, the resulting mixture was heated at 65 °C for 24 h. Upon cooling to ambient temperature, 11 mL of a 2 M solution of HCl was added and the resulting mixture was stirred vigorously for an additional 30 min. Ethyl acetate (50 mL) was added to the mixture and the layers separated. The aqueous layer was further extracted with ethyl acetate (2 x 30 mL). The combined organic layers were washed with 50% brine (3 x 30 mL), dried over Na₂SO₄, filtered and concentrated in vacuo to yield 2.12 g (100%) of the desired acid as a thick yellow oil: ¹H NMR (300 MHz, CDCl₃) d 7.26-6.95 (m, 4 H), 6.62-6.57 (d, 1 H, *J* = 15.8 Hz), 6.1-6.02 (dd, 1 H, *J =* 15.8, 8.0 Hz), 5.92-5. 87 (d, 1 H, *J =* 15.7 Hz), 3.9-3.8 (m, 1 H), 2.6-2.2 (m, 3 H), 2.36 (s, 3 H), 2.34 (s, 3 H), 1.18-1.15 (d, 3 H, *J* = 6.8 Hz), 0.94 (s, 9 H), 0.12 (s, 3 H), 0.11 (s, 3 H).

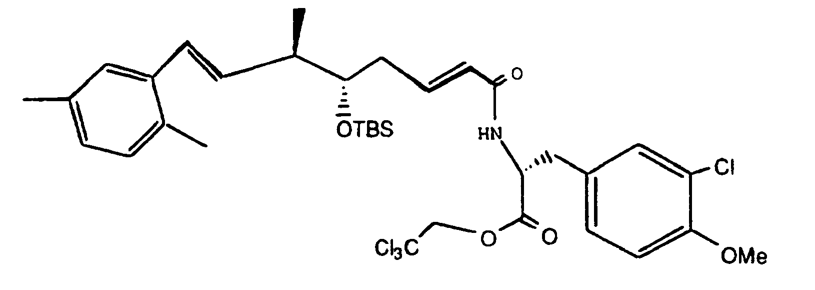

To a 0 °C solution of acid **4** (2.12 g, 5.46 mmol) and diisopropylethylamine (2.9 mL, 16.4 mmol) in 7 mL of DMF was added diphenylphosphinic chloride (1.1 mL, 6.01 mmol) dropwise. Following 5 min of stirring at 0 °C and 30 min at room temperature, the TFA salt of 3-(3-chloro-4-methoxyphenyl)-D-alanine-2,2,2-trichloroethyl ester 5 in 7 mL of DMF was added dropwise. The resulting mixture was stirred at room temperature for 2 h, poured into 100 mL of water and washed with diethyl ether (3 x 50 mL). The combined organic layers were washed with brine, dried over MgSO₄, filtered through Celite and concentrated in vacuo. Purification by column chromatography (silica gel, 10-30% EtOAc/hexanes) gave 2.86 g (72%) of amide 6 as a white foam: [a]²⁰_{D} + 44.8° (cl.0, MeOH); ¹H NMR (300 MHz, CDCl₃) d 7.25 (s, 1 H), 7.22 (s, 1 H), 7.1-6.83 (m, 5 H), 6.6-6.55 (d, 1 H, *J =* 15.7 Hz), 6.11-6.03 (dd, 1 H, *J =* 15.6, 8.1 Hz), 5.9-5.8 (m, 2 H), 5.18-5.05 (m, 1 H), 4.83-4.75 (q, 2 H, *J =* 12.0 Hz), 3.9 (s, 3 H), 3.83-3.68 (m, 1 H), 3.3-3.1 (m, 2 H), 2.6-2.36 (m, 3 H), 2.36 (s, 3 H), 2.32 (s, 3 H), 1.16-1.14 (d, 3 H, *J* = 6.7 Hz), 0.95 (s, 9 H), 0.11 (s, 3 H), 0.05 (s, 3H); ¹³C NMR (75 MHz, CDCl₃) d 170.0, 165.1, 154.2, 143.1, 136.4, 135.3, 133.0, 132.0, 131.0, 130.9, 130.0, 128.4, 128.3, 127.7, 126.1, 124.6, 112.3, 112.1, 94.2, 75.0, 74.7, 56.0, 52.9, 42.8, 37.5, 36.5, 25.8, 20.9, 19.3, 18.0, 16.4, - 4.3, - 4.7; IR (CHCl₃) 3428, 2957, 2930, 2857, 1759, 1676, 1645, 1606, 1503, 1464, 1442, 1380, 1399, 1281, 1259, 1173, 1067 cm⁻¹.

### Example 29

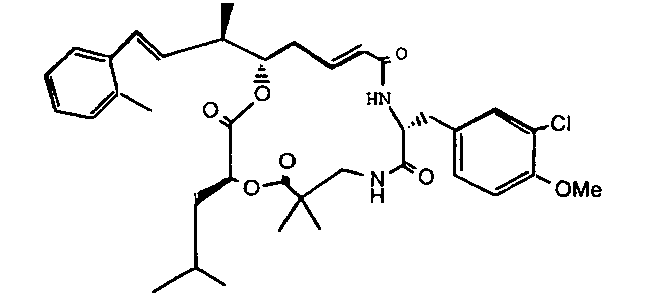

The ester (2.1 g) was prepared from 2.5 g of aldehyde 2 and 5.0 g of 3-methylbenzyl triphenylphosphonium chloride in 65% yield using the procedure described above: [a]²⁰_{D} +45.55° (c 1.0, MeOH): ¹H NMR (300 MHz, CDCl₃) d 7.22-6.88 (m, 5 H), 6.37-6.31 (d, 1 H, *J* = 16.0 Hz), 6.18-6.10 (dd, 1 H, *J* = 16.0, 8.0 Hz), 5.86-5.81 (d, 1 H, *J* = 15.5 Hz), 3.8-3.7 (m, 4 H), 2.5-2.3 (m, 6 H), 1.11-1.08 (d, 3 H, *J* = 6.9 Hz), 0.91 (s, 9H), 0.061 (s, 3 H), 0.052 (s, 3 H); ¹³C NMR (75 MHz, CDCl₃) d 166.7, 146.4, 137.5, 135.9, 131.6, 130.4, 128.3, 127.7, 126.7, 123.1, 122.8, 75.0, 51.3, 42.7, 37.4, 33.8, 25.8, 21.9, 21.3, 18.0, 16.0, -4.5, -4.6; IR (CHCl₃) 2953, 2931, 2859, 1718, 1658, 1604, 1472, 1454, 1938, 1258 cm⁻¹.

The acid (1.93 g) was prepared from 2.0 g of ester in 100% yield using the procedure described above: ¹H NMR (300 MHz, CDCl₃) d 7.22-7.0 (m, 5 H), 6.38-6.33 (d, 1H, *J =* 16.0 Hz), 6.18-6.10 (dd, 1 H, *J =* 16.0, 8.0 Hz), 5.87-5.82 (d, 1 H, *J* = 15.7 Hz), 3.8-3.7 (m, 1 H), 2.5-2.35 (m, 3 H), 2.34 (s, 3 H), 1.12-1.09 (d, 3 H, *J =* 6.8 Hz), 0.9 (s, 9 H), 0.068 (s, 3 H), 0.061 (s, 3 H).
The amide (2.9 g) was prepared from 1.93 g of acid in 72% yield using the procedure described above: ¹H NMR (300 MHz, CDCl₃) d 7.22-7.1 (m, 4 H), 7.1-7.0 (m, 2 H), 6.9-6.8 (m, 2 H), 6.36-6.30 (d, 1 H, J = 16.0 Hz), 6.19-6.11 (dd, 1 H, *J* = 16.0, 8.0 Hz), 5.82-5.77 (m, 2 H), 5.1-5.0 (m, 1 H), 4.78-4.71 (q, 2 H, *J* = 12.0 Hz), 3.86 (s, 3 H), 3.8-3.7 (m, 1 H), 3.25-3.15 (m, 2 H), 2.5-2.3 (m, 6 H), 1.1-1.08 (d, 3 H, *J* = 6.8 Hz), 0.88 (s, 9 H), 0.056 (s, 3 H), 0.044 (s, 3 H).

The alcohol (2.0 g) was prepared from 2.4 g of the starting amide in 100% yield using the procedure described above: ¹H NMR (300 MHz, CDCl₃) d 7.25-6.8 (m, 8 H), 6.46-6.41 (d, 1 H, *J* = 15.9 Hz), 6. 16-6.07 (dd, 1 H, *J* = 16.0, 8.8 Hz), 5.95-5.8 (m, 2 H), 5.1-5.0 (m, 1 H), 4.81-4.7 (q, 2 H, *J =* 12.0 Hz), 3.87 (s, 3 H), 3.7-3.6 (m, 1 H), 3.22-3.05 (m, 2 H), 2.5-2.34 (m, 3 H), 2.34 (s, 3 H), 1 .8-1 .7 (bs, 1 H), 1.15-1.13 (d, 3 H, *J =* 6.8 Hz).
The substrate (2.3 g) was prepared from 2.0 g of the starting alcohol in 76% yield using the procedure described above: [a]²⁰_{D} + 31.6 ° (c 1.08, MeOH); ¹H NMR (300 MHz, CDCl₃) d 7.2-7.0 (m, 6 H), 6.89-6.81 (d, 1 H, *J =* 8.4 Hz), 6.80-6.73 (m, 1 H), 6.53-6.50 (bd, 1 H, *J =* 7.3 Hz), 6.4-6.35 (d, 1 H, J = 15.8 Hz), 6.03-5.95 (dd, 1 H, *J =* 15.8, 8.5 Hz), 5.9-5.85 (d, 1 H, *J =* 15.7 Hz), 5.42-5.35 (bt, 1 H, *J =* 6.38 Hz), 5.1-4.82 (m, 3 H), 4.8-4.67 (q, 2 H, *J =* 12.0 Hz), 3.85 (s, 3 H), 3.28-3.26 (d, 2 H, *J =* 6.46 Hz), 3.23-3.16 (dd, 1 H, *J =* 14.3, 5.8 Hz), 3.1-3.03 (dd, 1 H, *J* = 14.2, 6.7 Hz), 2.7-2.4 (m, 3 H), 2.33 (s, 3 H), 1.8-1.5 (m, 3 H), 1.43 (s, 9 H), 1.2 (s, 3 H), 1.15 (s, 3 H), 1.12-1.1 (d, 3 H, *J =* 6.7 Hz), 0.88-0.86 (d, 3 H, *J =* 6.2 Hz), 0.84-0.82 (d, 3 H, J = 6.4 Hz); ¹³C NMR (75 MHz, CDCl₃) d 170.6, 170.0, 165.2, 154.0, 139.2, 138.0, 136.7, 135.8, 131.8, 131.1, 129.7, 128.8, 128.4, 128.2, 126.8, 125.3, 123.3, 122.23, 112.1, 94.2, 78.9, 74.5, 71.3, 56.0, 53.1, 48.6, 43.9, 41.0, 39.4, 36.5, 33.3, 28.3, 24.7, 22.9, 22.7, 22.3, 21.3, 21.2; 16.5; IR (CHCl₃) 3426, 3383, 2967, 2935, 2874, 2841, 1727, 1710, 1680, 1646, 1605, 1504, 1368, 1280, 1259, 1169, 1151 cm⁻¹.

The styrene (0.86 g) was prepared from 2.2 g of the starting carbamate in 54% yield using the procedure described above: [a]²⁰_{D} + 33.1 ° (c 1.03, MeOH); ¹H NMR (300 MHz, CDCl₃) d 7.24-7.0 (m, 7 H), 6.85-6.82 (d, 1 H, *J =* 8.4 Hz), 6.82-6.70 (m, 1 H), 6.39-6.34 (d, 1 H, *J =* 15.8 Hz), 6.03-5.95 (dd, 1 H, *J =* 15.8, 8.7 Hz), 5.78-5.73 (d, 1 H, *J =* 15.2 Hz), 5.67-5.69 (d, 1 H, *J =* 7.8 Hz), 5.1-5.0 (m, 1 H), 4.87-4.83 (dd, 1 H, *J =* 10.2, 3.5 Hz), 4.8-4.7 (m, 1 H), 3.9 (s, 3 H), 3.95-3.38 (dd, 1 H, *J =* 13.4, 8.6 Hz), 3.2-3.0 (m, 3 H), 2.6-2.3 (m, 3 H), 2.32 (s, 3 H), 1.75-1.25 (m, 3 H), 1.22 (s, 3 H), 1.15 (s, 3 H), 1.13-1.11 (d, 3 H, *J = 6.8* Hz), 0.75-0.72 (t, 6 H, *J =* 5.7 Hz); ¹³C NMR (75 MHz, CDCl₃) d 177.9, 170.5, 170.3, 165.1, 154.0, 142.1, 138.0, 136.6, 135.6, 131.8, 130.8, 129.9, 129.6, 128.4, 128.22, 128.17, 126.7, 124.5, 123.3, 122.5, 112.3, 71.4, 56.1, 54.3, 46.4, 42.7, 42.2, 39.4, 36.5, 35.3, 24.5, 22.8, 22.6, 22.5, 21.2, 21.1, 17.2; IR (CHCl₃) 3424, 3021, 3017, 2965, 1747, 1711, 1680, 1652, 1528, 1503, 1485, 1259, 1151, 1067 cm⁻¹ ; Anal. Calcd for C₃₇H₄₇ClN₂O₇: C, 66.60; H, 7.10; N, 4.20. Found: C, 66.79; H, 7.03; N, 4.25.

### Example 30

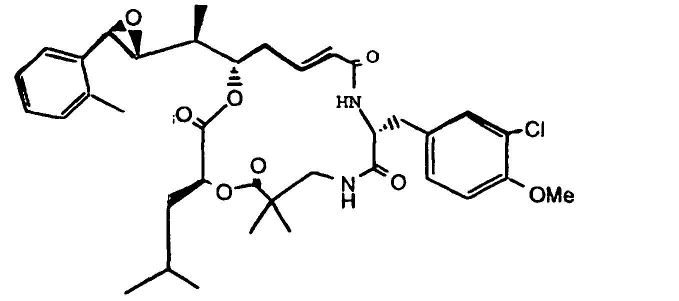

The *b* epoxide, title compound (0.20 g) was prepared from 0.667 g of the starting styrene in 29% yield using the procedure described above: ¹H NMR (300 MHz, CDCl₃) d 7.3-7.0 (m, 7 H), 6.90-6.87 (d, 1 H, *J =* 8.4 Hz), 6.87-6.75 (m, 1 H), 5.79-5.74 (d, 1 H, *J =* 14.8 Hz), 5.54-5.51 (d, 1 H, *J* = 7.8 Hz), 5.28-5.22 (m, 1 H), 4.89-9.85 (dd, 1 H, *J* = 10.4, 3.5 Hz), 4.82-4.75 (m, 1 H), 3.92 (s, 3 H), 3.69-3.68 (d, 1 H, *J* = 1.63), 3.51-3.44 (dd, 1 H, *J* = 13.4, 8.6 Hz), 3.2-3.1 (m, 2 H), 2.98-2.95 (dd, 1 H, *J* = 7.6, 1.6 Hz), 2.65-2.32 (m, 3 H), 2.32 (s, 3 H), 1.85-1.6 (m, 3 H), 1.4-1.25 (m, 1 H), 1.27 (s, 3 H), 1.21 (s, 3 H), 1.21-1.18 (d, 3 H, *J* = 7.5 Hz), 0.90-0.86 (t, 6 H, *J* = 6.13 Hz).

### Example 31

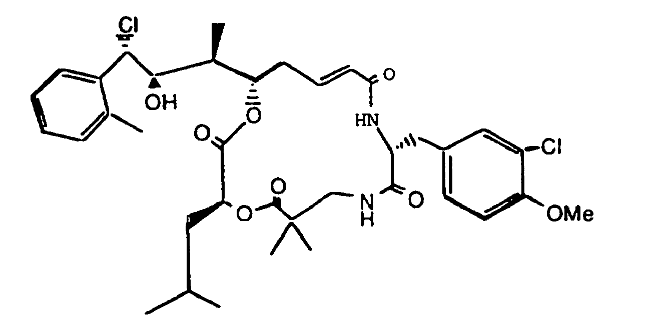

To a solution of the *b* epoxide (0.1 g, 0.147 mmol) in 5.0 mL of CHCl₃ at -60 °C, was added chlorotrimethyl silane (0.093 mL, 0.74 mmol). The solution was stirred at -60 °C for 30 min and at room temperature for 1.5 h before being concentrated under vacuum. The resulting residue, containing a 50:50 mixture of the syn and anti chlorohydrins, was purified via reverse phase HPLC to yield 0.028 g (27%) of the desired trans isomer: ¹H NMR (300 MHz, CDCl₃) d 7.28-7.2 (m, 5 H), 7.13-7.1 (dd, 1 H, *J* = 8.32, 1.95 Hz), 6.91-6.88 (d, 1H, *J* = 8.5 Hz), 6.88-6.78 (m, 1 H), 5.86-5.81 (d, 1 H, *J =* 15.0 Hz), 5.73-5.71 (d, 1 H, *J =* 7.8 Hz), 5.24-5.17 (t, 1 H, *J =* 9.4 Hz), 5.0-4.96 (dd, 1 H, *J =* 9.58, 2.93 Hz), 4.81-4.74 (m, 1 H), 4.67-4.64 (d, 1 H, *J* = 9.73 Hz), 4.06-9.03 (dd, 1 H, *J =* 9.6, 1.1 Hz), 3.92 (s, 3 H), 3.47-3.39 (dd, 1 H, *J =* 13.2, 8.3 Hz), 3.24-3.0 (m, 3 H), 2.8-2.4 (m, 2 H), 2.4 (s, 3 H), 1.9-1.4 (m, 4 H), 1.28 (s, 3H), 1.22 (s, 3 H), 1.09-1.07 (d, 3 H, *J* = 6.95 Hz), 0.98-0.96 (d, 6 H, *J* = 6.4 Hz).

### Example 32

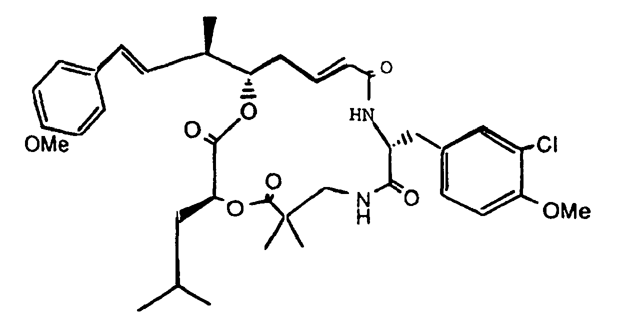

The ester (2.75 g) was prepared from 3.39 g of aldehyde 2 and 7.7 g of 4-methoxybenzyl triphenylphosphonium chloride in 54% yield using the procedure described above.: [a]²⁰_{D} + 71.85 ° (*c* 1.03, MeOH): ¹H NMR (300 MHz, CDCl₃) d 7.25-7.21 (m, 2 H, J = 9.0 Hz), 7.0-6.85 (m, 1 H), 6.81-6.78 (d, 2 H, *J* = 8.5 Hz), 6.30-6.23 (d, 1 H, *J* = 16.3 Hz), 6.0-5.9 (dd, 1 H, J = 16.3, 8.2 Hz), 5.81-5.75 (d, 1 H, *J* = 14.26 Hz), 3.75 (s, 3 H), 3.67 (s, 4 H), 2.42-2.22 (m, 3 H), 1.05-1.02 (d, 3 H, *J* = 6.8 Hz), 0.86 (s, 9 H), 0.005 (s, 3 H), -0.001 (s, 3 H); ¹³C NMR (75 MHz, CDCl₃) d 166.7, 158.8, 146.4, 130.4, 129.7, 129.7, 127.0, 122.7, 113.8, 75.0, 55.2, 51.3, 42.7, 37.4, 25.8, 18.0, 16.1, -4.5, -4.6; IR (CHCl₃) 3010, 2955, 2930, 2898, 2857, 1718, 1658, 1607, 1511 cm⁻¹.

The acid (1.58 g) was prepared from 1.7 g of ester in 96% yield using the procedure described above: ¹H NMR (300 MHz, CDCl₃) d 7.3-7.27 (d, 2 H, *J* = 8.7 Hz), 7.13-7.03 (m, 1H), 6.86-6.83 (d, 2 H, *J* = 8.6 Hz), 6.35-6.29 (d, 1 H, *J* = 16.0 Hz), 6.04-5.96 (dd, 1 H, *J* = 15.9, 8.0 Hz), 5.87-5.81 (d, 1 H, *J* = 15.8 Hz), 3.8 (s, 3 H), 3.79-3.7 (m, 1 H), 2.5-2.33 (m, 3 H), 1.1-1.08 (d, 3 H, *J* = 6.8 Hz), 0.9 (s, 9 H), 0.058 (s, 3 H), 0.055 (s, 3 H).
The amide (2.09 g) was prepared from 1.58 g of acid in 70% yield using the procedure described above: [a]²⁰_{D} + 2.0 ° (c 1.0, CHCl₃); ¹H NMR (300 MHz, CDCl₃) d 7.28-7.26 (d, 2 H, *J* = 7.33 Hz), 7.18-7.17 (d, 1 H, *J* =1.8 Hz), 7.04-7.0 (dd, 1 H, *J =* 8.5, 1.8 Hz), 6.90-6.79 (m, 3 H), 6.32-6.27 (d, 1 H, *J =* 16.0 Hz), 6.05-5.97 (dd, 1 H, *J* = 16.0, 8.1 Hz), 5.83-5.76 (m, 3 H), 5.09-5.05 (m, 1 H), 4.82-4.7 (q, 2 H, *J =* 11.9 Hz), 3.86 (s, 3 H), 3.8 (s, 3 H), 3.77-3.7 (m, 1 H), 3.24-3.17 (dd, 1 H, *J =* 14.2, 5.7 Hz), 3.13-3.07 (dd, 1 H, *J=* 14.2, 5.94 Hz), 2.5-2.3 (m, 3 H), 1.09-1.06 (d, 3 H, *J =* 6.8 Hz), 0.89 (s, 9 H), 0.048 (s, 3 H), 0.038 (s, 3 H); ¹³C NMR (75 MHz, CDCl₃) d 169.9, 165.1, 158.7, 154.2, 143.0, 135.5, 131.0, 130.4, 129.6, 128.4, 127.0, 124.6, 122.5, 113.8, 112.1, 94.2, 75.0, 74.7, 56.0, 55.2, 52.9, 42.5, 37.4, 36.4, 25.8, 18.0, 16.4, -9.4, -9.7; IR (CHCl₃) 2957, 2931, 2857, 1757, 1676, 1694, 1607, 1511, 1503 cm⁻¹.

The alcohol (1.35 g) was prepared from 1.63 g of the starting amide in 98% yield using the procedure described above: [a]²⁰_{D} + 55.7 ° (c 1.0, MeOH); ¹H NMR (300 MHz, CDCl₃) d 7.31-7.29 (d, 2 H, *J* = 7.9 Hz), 7.18 (s, 1 H), 7.05-7.02 (d, 1 H, *J =* 8.3 Hz), 7.0-6.87 (m, 1 H), 6.86-6.83 (d, 3 H, *J =* 8.2 Hz), 6.44-6.38 (d, 1 H, *J =* 15.8 Hz), 6.0-5.82 (m, 3 H), 5.10-5.0 (m, 1 H), 4.81-4.7 (q, 2 H, *J =* 11.8 Hz), 3.87 (s, 3 H), 3.81 (s, 3 H), 3.67-3.6 (m, 1 H), 3.24-3.19 (dd, 1 H, *J =* 14.1, 6.1 Hz), 3.12-3.07 (dd, 1 H, *J* =14.4, 5.9 Hz), 2.5-2.25 (m, 3 H), 1.8-1.6 (bs, 1 H), 1.14-1.12 (d, 3 H, *J =6.6* Hz); ¹³C NMR (75 MHz, CDCl₃) d 170.1, 165.2, 159.0, 154.2, 142.5, 131.2, 131.0, 129.8, 128.7, 128.4, 127.2, 125.0, 122.4, 113.9, 112.2, 94.2, 74.7, 73.8, 56.05, 55.2, 53.0, 43.2, 37.1, 36.4, 16.8; IR (CHCl₃) 3428, 2964, 2936, 2912, 2874, 2840, 1758, 1677, 1645, 1607, 1512, 1503, 1258, 1175 cm⁻¹.

The substrate (1.69 g) was prepared from 1.26 g of the starting alcohol in 89% yield using the procedure described above: [a]²⁰_{D} + 35.2 ° (c 1.02, MeOH); ¹H NMR (300 MHz, CDCl₃) d 7.26-7.23 (d, 21 H, *J =* 8.0 Hz), 7.18-7.17 (d, 1 H, *J =* 1.7), 7.07-7.03 (dd, 1 H, *J =* 8.4, 1.6), 6.85-6.7 (m, 4 H), 6. 53-6. 5 (d, 1 H, *J =* 7.9 Hz), 6.36-6.31 (d, 1 H, *J* = 15.8 Hz), 5.9-5.81 (m, 2 H), 5.92-5.35 (t, 1 H), 5.1-4,95 (m, 2 H), 9.94-4.90 (dd, 1 H, *J* = 9.6, 3.4 Hz), 4.81-4.67 (q, 2 H, *J* = 11.9 Hz), 3.85 (s, 3 H), 3.79 (s, 3 H), 3.28-3.26 (d, 2 H, *J* = 6.5 Hz), 3.23-3.16 (dd, 1 H, *J* = 19.3, 5.8 Hz), 3.09-3.02 (dd, 1 H, *J* = 14.1, 6.7 Hz), 2.61-2.4 (m, 3 H), 1.8-1.5 (m, 3 H), 1.43 (s, 9 H), 1.20 (s, 3 H), 1.15 (s, 3 H), 1.11-1.09 (d, 3 H, *J =* 6.7 Hz), 0.87-0.85 (d, 3 H, *J =* 6.4 Hz), 0.84-0.82 (d, 3 H, *J* = 6.5 Hz); ¹³C NMR (75 MHz, CDCl₃) d 174.9, 170.6, 169.9, 165.3, 159.0, 154.0, 139.3, 135.4, 131.1, 131.0, 129.6, 128.8, 128.4, 127.8, 127.2, 125.2, 122.2, 113.9, 112.2, 94.3, 74.5, 71.3, 56.0, 55.2, 53.1, 48.6, 43.9, 41.0, 39.4, 36.5, 28.3, 24.7, 22.9, 22.7, 22.3, 21.4, 16.6; IR (CHCl₃) 3426, 3383, 2965, 2936, 2874, 2840, 1728, 1711, 1680, 1646, 1607, 1512, 1465, 1367, 1254, 1175, 1067 cm⁻¹.

The styrene product (0.676 g) was prepared from 1.43 g of the starting carbamate in 65% yield using the procedure described above: ¹H NMR (300 MHz, CDCl₃) 7.26-7.23 (d, 3 H, *J* = 8.4 Hz), 7.20-7.19 (d, 1 H, *J* = 1.8 Hz), 7.07-7.03 (dd, 1 H, *J* = 8.4, 1.9 Hz), 6.84-6.81 (d, 3 H, *J* = 8.5 Hz), 6.8-6.7 (m, 1 H), 6.36-6.31 (d, 1 H, *J* = 15.8 Hz), 5.89-5.81 (dd, 1 H, J = 15.8, 8.8 Hz), 5.78-5.73 (d, 1 H, J = 13.7 Hz), 5.68-5.66 (d, 1 H, J = 7.9 Hz), 5.05-4.99 (ddd, 1 H, *J* = 10.6, 6.6, 1.6 Hz), 4.87-4.82 (dd, 1 H, J = 9.7, 3.1 Hz), 4.78-4.7 (m, 1 H), 3.86 (s, 3 H), 3.79 (s, 3 H), 3.45-3.37 (dd, 1H, J = 13.4, 8.6 Hz) 3.15-3.0 (m, 3 H), 2.6-2.25 (m, 3 H), 1.7-1.3 (m, 3 H), 1.22 (s, 3 H), 1.15 (s, 3 H), 1.12-1.1 (d, 3 H, J = 6.8 Hz), 0.76-0.75 (d, 3 H, *J =* 2.9 Hz), 0.74-0.73 (d, 3 H, J = 2.8 Hz).

### Example 33

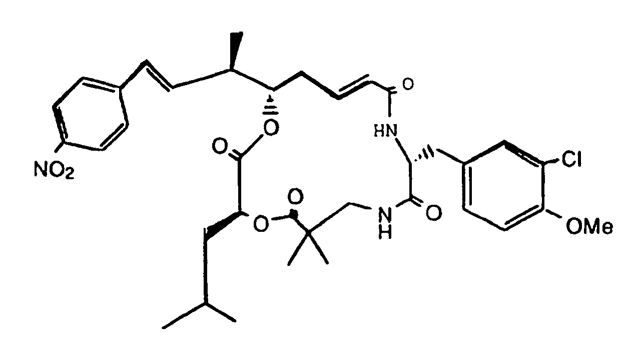

To a 0 °C solution of potassium tert-butoxide (1.17 g, 10.5 mmol) in 120 mL of THF was added p-nitro-benzyl triphenylphosphonium bromide (5.0 g, 10.5 mmol), in small portions over a period of 30 min. The mixture was stirred at 0 °C for 1 h. Aldehyde 2 in 20 mL of THF, was added dropwise to the preformed ylide. The mixture was stirred at 0°C for 15 min, then warmed up slowly to room temperature and stirred overnight. Saturated aqueous NH₄Cl (50 mL) was added and the solution extracted with ethyl acetate. The organic layer was washed with brine, dried over MgSO₄, filtered and concentrated in vacuo. The crude styrene as a mixture of E:Z isomers was purified on silica gel using 2% EtOAc/hexanes to yield 0.5 g of the Z isomer and 1.1 g of the E isomer (42%) as a yellow oil: [a]²⁰_{D} + 63.81 ° (c 1.05, MeOH); ¹H NMR (300 MHz, CDCl₃) d 8.18-8.15 (d, 2 H, *J* =8.6 Hz), 7.47-7.44 (d, 2 H, *J* = 8.7 Hz), 7.0-6.85 (m, 1 H), 6.5-6.3 (m, 2 H), 5.87-5.82 (d, 1 H, *J =* 15.6 Hz), 3 . 8-3.73 (m, 1 H), 3.73 (s, 3 H), 2.55-2.35 (m, 3 H), 1.13-1.11 (d, 3 H, J = 6.9 Hz), 0.90 (s, 9 H), 0.059 (s, 3 H), 0.047 (s, 3 H); ¹³C NMR (75 MHz, CDCl₃) 166.5, 145.5, 143.9, 137.3, 128.7, 126.4, 123.9, 123.1, 74.6, 51.3, 42.8, 37.8, 33.8, 25.7, 24.3, 21.9, 18.0, 16.3, -4.4, -4.7; IR (CHCl₃) 2953, 2931, 2859, 1719, 1658, 1596, 1518, 1472, 1438, 1345, 1259, 1110 cm⁻¹.

The amide (3.06 g) was prepared from 2.55 g of acid in 65% yield using the procedure described above: [α]²⁰_{D} + 70.67 ° (c 1.05, MeOH); ¹H NMR (300 MHz, CDCl₃) d 8.15-8.12 (d, 2 H, *J* = 8.7 Hz), 7.95-7.92 (d, 2 H, *J =* 8.7 Hz), 7.17-7.16 (d, 1 H, *J* = 2.0 Hz), 7.04-7.01 (dd, 1 H, *J* = 8.45, 1.9 Hz), 6.88-6.82 (m, 2 H), 6.43-6.3 (m, 2 H), 5.93-5.91 (d, 1 H, *J* = 7.5 Hz), 5.84-5.79 (d, 1 H, *J* = 15.3 Hz), 5.1-5.0 (m, 1 H), 4.82-4.69 (q, 2 H, *J* = 11.9 Hz), 3.85 (s, 3 H), 3.8-3.7 (m, 1 H), 3.24-3.18 (dd. 1 H, *J* = 14.3, 5.7 Hz), 3.13-3.06 (dd, 1 H, *J* = 14.2, 6.05 Hz), 2.52-2.3 (m, 3 H), 1.11-1.09 (d, 3 H, *J* = 6.8 Hz), 0.88 (s, 9 H), 0.041 (s, 3 H), 0.032 (s, 3 H); ¹³C NMR (75 MHz, CDCl₃) d 170.0, 165.0, 154.2, 146.5, 144.0, 142.2, 137.4, 136.0, 131.0, 128.7, 128.4, 126.4, 124.9, 123.9, 122.5, 112.1, 94.1, 74.72, 74.67. 56.0, 52.9, 42.7, 37.8, 36.4, 25.7, 18.0, 16.5, -4.7, -4.3; IR (CHCl₃) 3429, 2957, 2931, 2858, 1757, 1677, 1645, 1597, 1517, 1503, 1345, 1259, 1180, 1110, 1067, 1026 cm⁻¹.

The substrate (3.3 g) was prepared from 2.54 g of the starting alcohol in 87% yield using the procedure described above: [a]²⁰_{D} + 38.2° (c 1.07, MeOH); ¹H NMR (300 MHz, CDCl₃) d 8.23-8.2 (d, 2 H, *J =* 8.7 Hz), 7.52-7.49 (d, 2 H, *J* = 8.7 Hz), 7.21 (s, 1 H), 7.12-7.08 (dd, 1 H, *J* = 8.2, 1.8 Hz), 6.89-6.86 (d, 1 H, *J* = 8.4 Hz), 6.86-6.75 (m, 1 H), 6.6-6.58 (d, 1 H, *J* = 8.0 Hz), 6.55-6.5 (d, 1 H, *J* = 15.9 Hz), 6.31-6.23 (dd, 1 H, J = 15.8, 8.5 Hz), 5.97-5.92 (d, 1 H, *J* = 15.5 Hz), 5.4-5.3 (bt, 1H), 5.2-5.0 (m, 2 H), 4.98-4.94 (dd, 1 H, *J* = 9.5, 3.6 Hz), 4.86-4.72 (q, 2 *H, J* = 12 Hz), 3.9 (s, 3 H), 3.33-3.3 (d, 1 H, *J* = 6.6 Hz), 3.27-3.22 (dd, 1 H, *J* = 14.1, 5.7 Hz), 3.14-3.07 (dd, 2 H, *J* = 14.0, 6.7 Hz), 2.8-2.5 (m, 3 H), 2.9-1.47 (m, 3 H), 1.47 (s, 9 H), 1.3-1.2 (m, 9 H), 0.91-0.89 (d, 3 H, J= 6.4 Hz), 0.87-0.85 (d, 3 H, *J* = 6.4 Hz); ¹³C NMR (75 MHz, CDCl₃) d 176.8, 170.6, 169.9, 165.1, 156.2, 154.0, 146.8, 143.2, 138.8, 135.8, 135.4, 131.1, 129.7, 128.7, 128.4, 126.7, 126.6, 125.5, 123.9, 122.2, 112.1, 94.2, 79.0, 76.2, 74.5, 71.2, 56.0, 53.1, 48.5, 43.9, 41.2, 39.4, 36.5, 33.3, 28.3, 24.7, 22.9, 22.7, 22.3, 21.4, 16.3; IR (CHCl₃) 3426, 3385, 2967, 2936, 2874, 2841, 1712, 1681, 1646, 1597, 1519, 1500, 1345, 1280, 1259, 1170, 1150, 1067, 1024 cm⁻¹.
The styrene product (0.55 g) was prepared from 1.77 g of the starting carbamate in 42% yield using the procedure described above: ¹H NMR (300 MHz, CDCl₃) 8.23-8.21 (d, 2 H, *J =* 8.7 Hz), 7.52-7.49 (d, 2 H, *J =* 8.7 Hz), 7.24-7.2 (m, 2 H), 7.11-7.08 (dd, 1 H, J = 8.5, 1.92 Hz), 6.89-6.87 (d, 1 H, J = 8.4 Hz), 6.86-6.72 (m, 1 H), 6.56-6.51 (d, 1 H, *J* = 15.9 Hz), 6.33-6.25 (dd, 1 H, *J =* 15.9, 8.7 Hz), 5.94-5.91 (d, 1 H, *J =* 7.8 Hz), 5.83-5.78 (d, 1 H, J = 15.2 Hz), 5.17-5.12 (m, 1 H), 4.92-4.88 (dd, 1 H, *J =* 9.7, 3.6 Hz), 4.8-4.75 (m, 1 H), 3.92 (s, 3 H), 3.5-3.43 (dd, 1 H, *J =* 13.5, 8.73 Hz), 3.2-3.1 (m, 3 H), 2.7-2.35 (m, 3 H), 1.8-1.6 (m, 2 H), 1.4-1.19 (m, 1H), 1.2 (s, 3 H), 1.16-1.14 (d, 6 H), 0.82-0.81 (d, 3 H, *J =* 3.73 Hz), 0.80-79 (d, 3 H, *J =* 3.87 Hz).

### Example 34

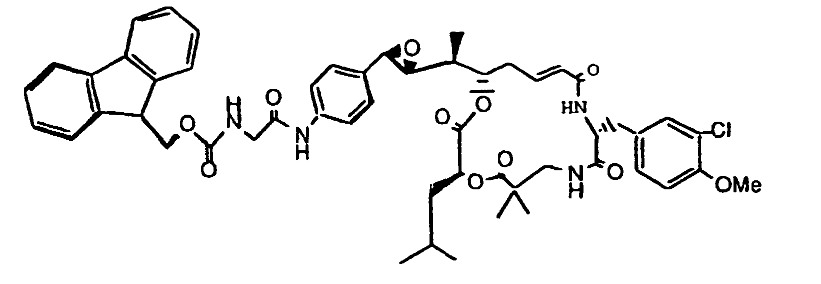

¹H NMR (300 MHz, CDCl₃) 8.5 (s, 1 H), 7.76-7.74 (d, 2 H, *J* = 7.4 Hz), 7.58-7.56 (d, 2 H, *J* = 7.4 Hz), 7.5-7.1 (m, 8 H), 7.03-7.0 (d, 1 H, *J* = 8.2 Hz), 6.81-6.78 (d, 1 H, *J* = 8.5 Hz), 6.78-6.65 (m, 1 H), 6.37-6.34 (d, 1 H, *J* = 7.3 Hz), 5.89-5.81 (t, 1 H, *J* = 5.2 Hz), 5.69-5.69 (d, 1 H, *J* = 15.0 Hz), 5.18-5.15 (bd, 1 H, *J* = 9.0 Hz), 4.83-9.6 (m, 2 H), 9.95-9.93 (d, 2 H, *J* = 6.7 Hz), 4.29-4.19 (t, 1 H, *J =* 6.6 Hz), 3.99 (bs, 2 H), 3. 81 (s, 3 H), 3.61 (s, 1 H), 3.96-3.39 (dd, 1 H, *J* = 13.9, 8.8 Hz), 3.2-2.8 (m, 5 H), 2.6-2.3 (m, 3 H), 1.8-1.3 (m, 3 H), 1.2 (s, 3 H), 1.14 (s, 3 H), 1.12-1.1 (d, 3 H, *J =* 6.8 Hz), 0.86-0.81 (t, 6 H, *J =* 7.3 Hz) .

### Example 35

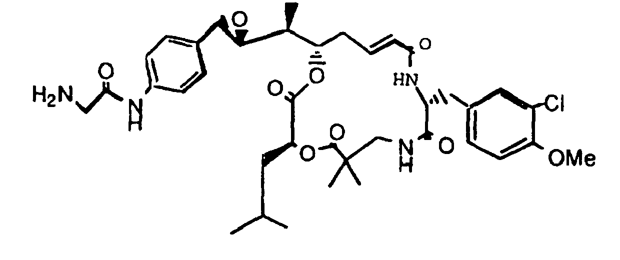

¹H NMR (300 MHz, CDCl₃) d 9.5 (bs, 2 H), 7.6-7.57 (d, 2 H, *J* = 8.1 Hz), 7.24-7.18 (m, 5 H), 7.05-7.03 (d, 1 H, *J* = 8.3 Hz), 6.84-6.82 (d, 1 H, *J* = 8.4 Hz), 6.82-6.65 (m, 1 H), 6.02-6.0 (d, 1 H, *J* = 7.6 Hz), 5.72-5.67 (d, 1 H, *J* = 15.3 Hz), 5.19-5.16 (bd, 1 H, *J* = 10.5 Hz), 4.9-4.6 (m, 2 H), 3.85 (s, 3 H), 3.64 (s, 1 H), 3.55-3.4 (m, 3 H), 3.2-2.95 (m, 3 H), 2.90-2.88 (d, 1 H, 7.11 Hz), 2.6-2.3 (m, 3 H), 2.0-1.3 (m, 3 H), 1.2 (s, 3 H), 1.15 (s, 3 H), 1.13-1 .11 (d, 3 H, *J* = 6.9 Hz), 0.90-0.84 (t, 6 H, *J* = 7.2 Hz) .

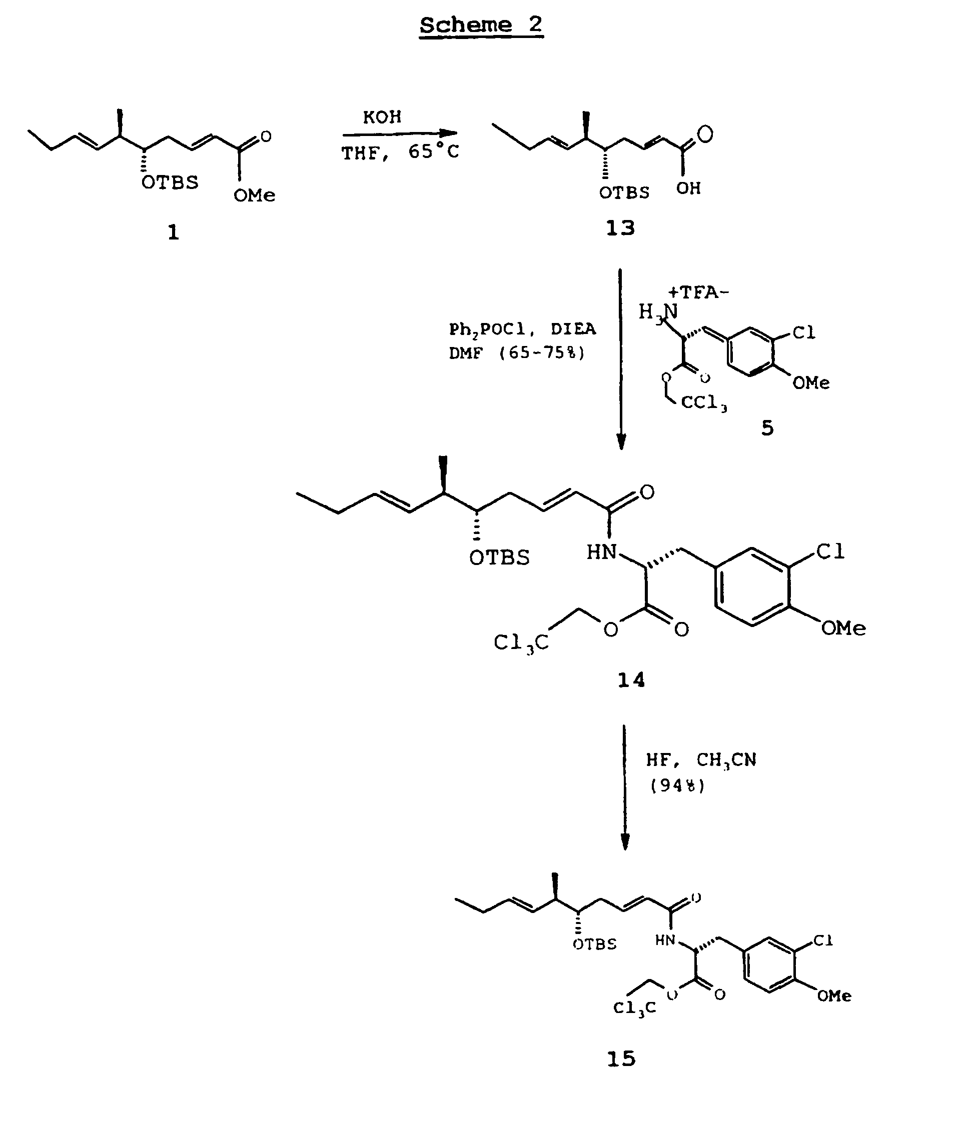

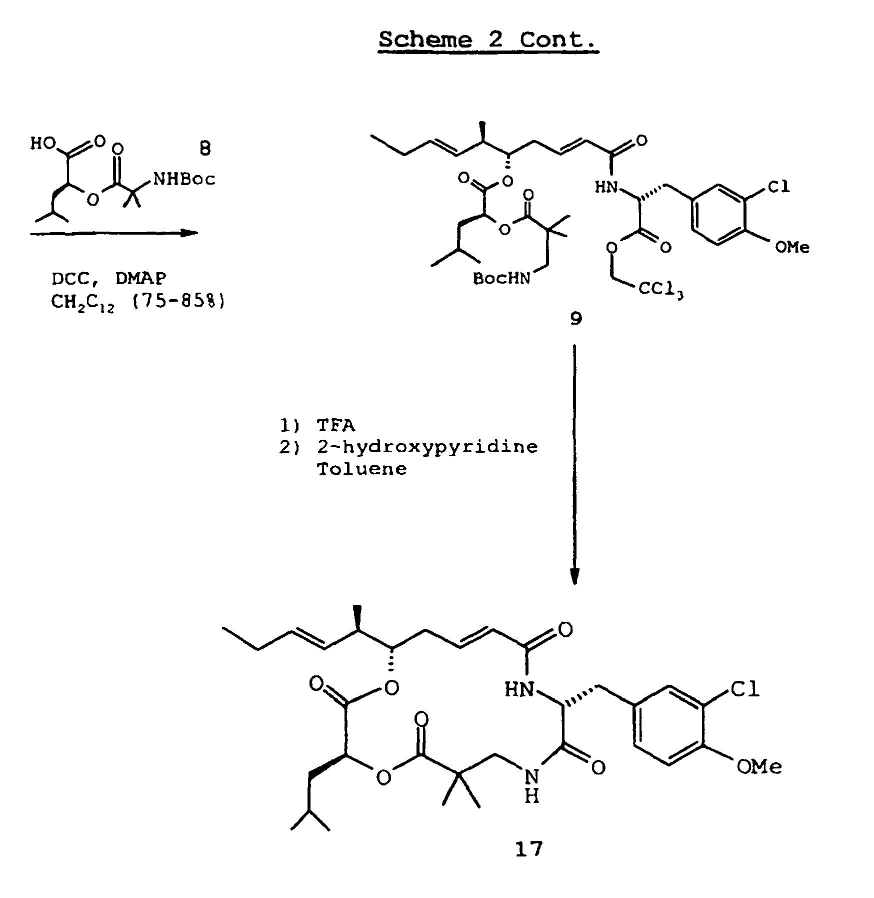

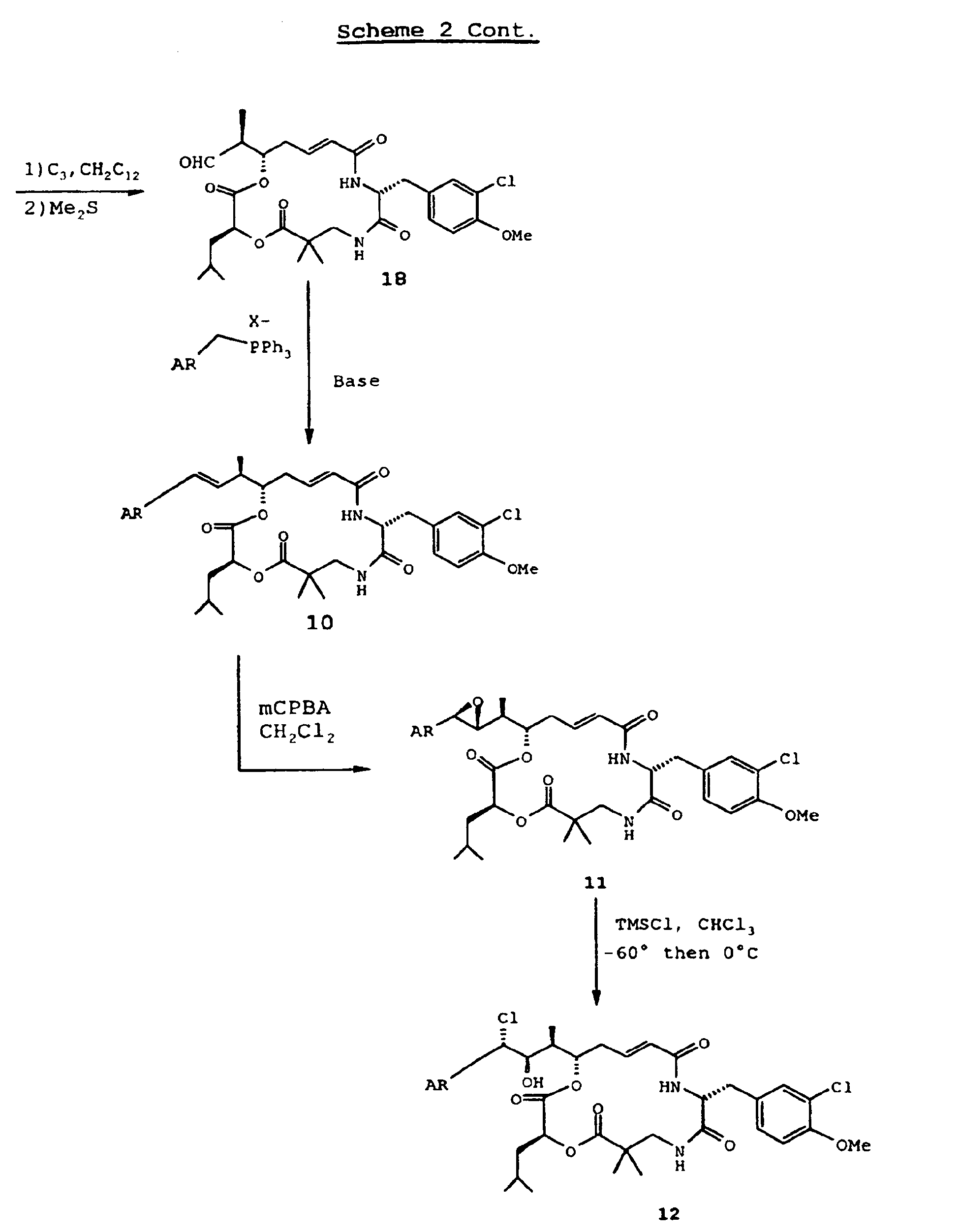

### Example 36

### Via Scheme 2

To alkene 17 (0.3 g, 0.50 mmol) at -78 °C in a 9.0 mL CH₂Cl₂/ 1.0 mL MeOH was added 2-picoline (0.07 mL, 0.074 mmol). This solution was subjected to 1.2 equivalence of ozone and the resulting ozonide was quenched with dimethyl sulfide (1.1 mL, 2.2 mmol). The mixture was slowly warmed up to room temperature and stirred overnight. The solution was washed with water (2 x 20 mL), dried over Na₂SO₄, filtered and concentrated in vacuo to yield 0.251 g (87%) of aldehyde **18:** ¹H NMR (300 MHz, CDCl₃) d 9.64-9.63 (d, 1 H, *J* = 1.05 Hz), 7.25-7.2 (m, 2 H), 7.08-7.05 (d, 1 H, *J =* 8.2 Hz), 6.86-6.83 (d, 1 H, *J =* 8.4 Hz), 6.83-6.7 (m, 1 H), 5.81-5.76 (d, 1 H, *J =* 15.1 Hz), 5.75-5.65 (bs, 1 H), 5.38-5.3 (m, 1 H), 4.85-4.81 (dd, 1 H, *J =* 10.2, 3.2 Hz), 4.80-4.7 (m, 1 H), 3.87 (s, 3 H), 3.46-3.39 (dd, 1 H, *J =* 13.5, 8.7 Hz), 3.2-3.0 (m, 3 H), 2.7-2.4 (m, 3 H), 1.8-1.6 (m, 2 H), 1.4-1.3 (m, 1 H), 1.23 (s, 3 H), 1.18-1.16 (d, 6 H), 0.94-0.92 (d, 3 H, *J* = 6.5 Hz), 0.88-0.86 (d, 3 H, *J* = 6.4 Hz).

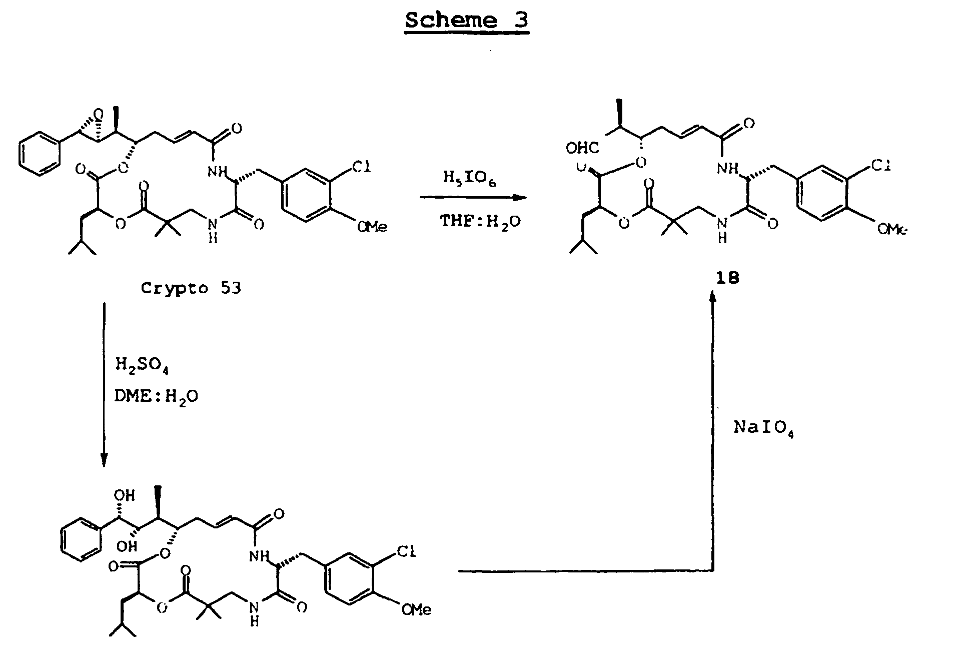

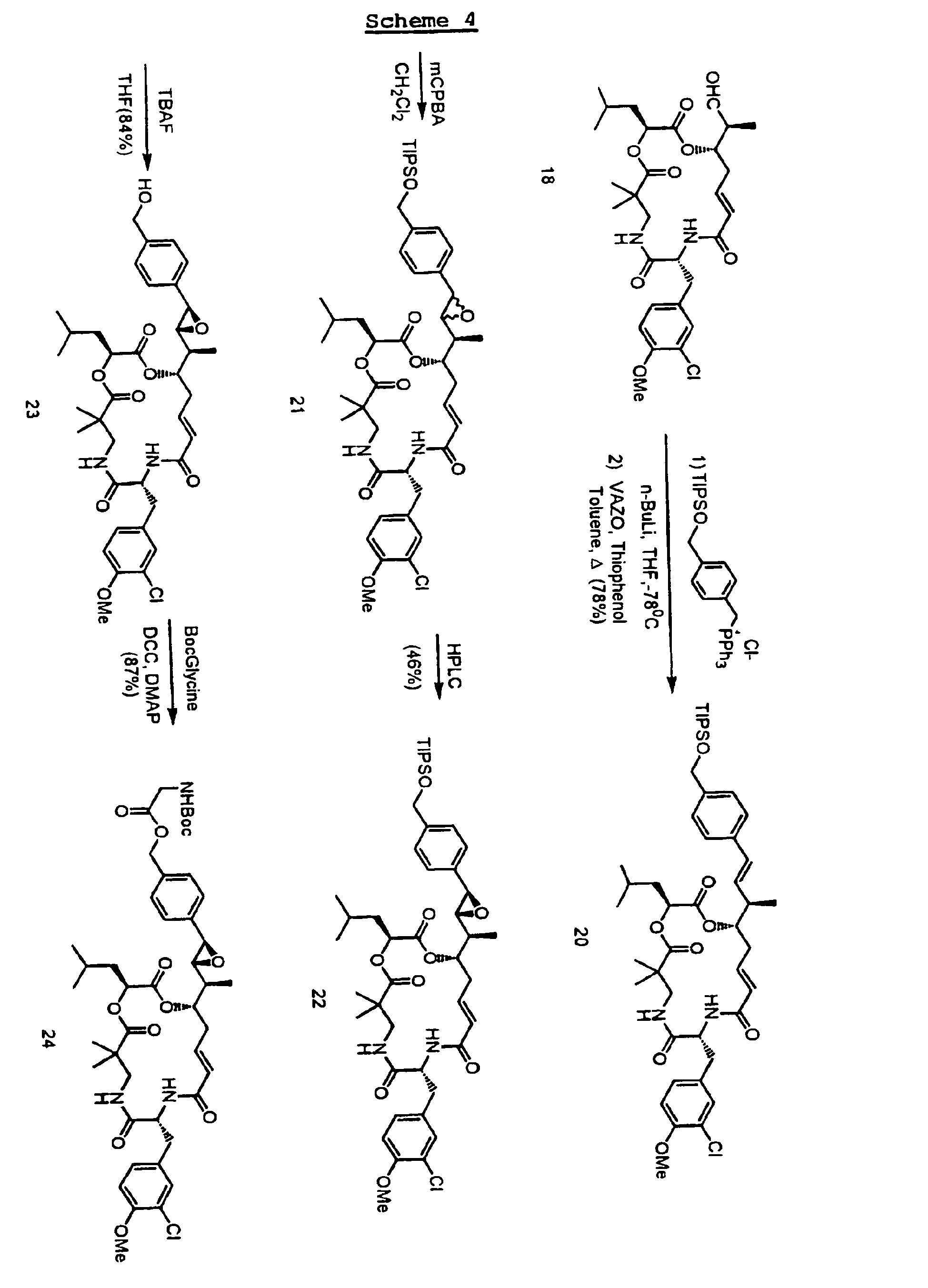

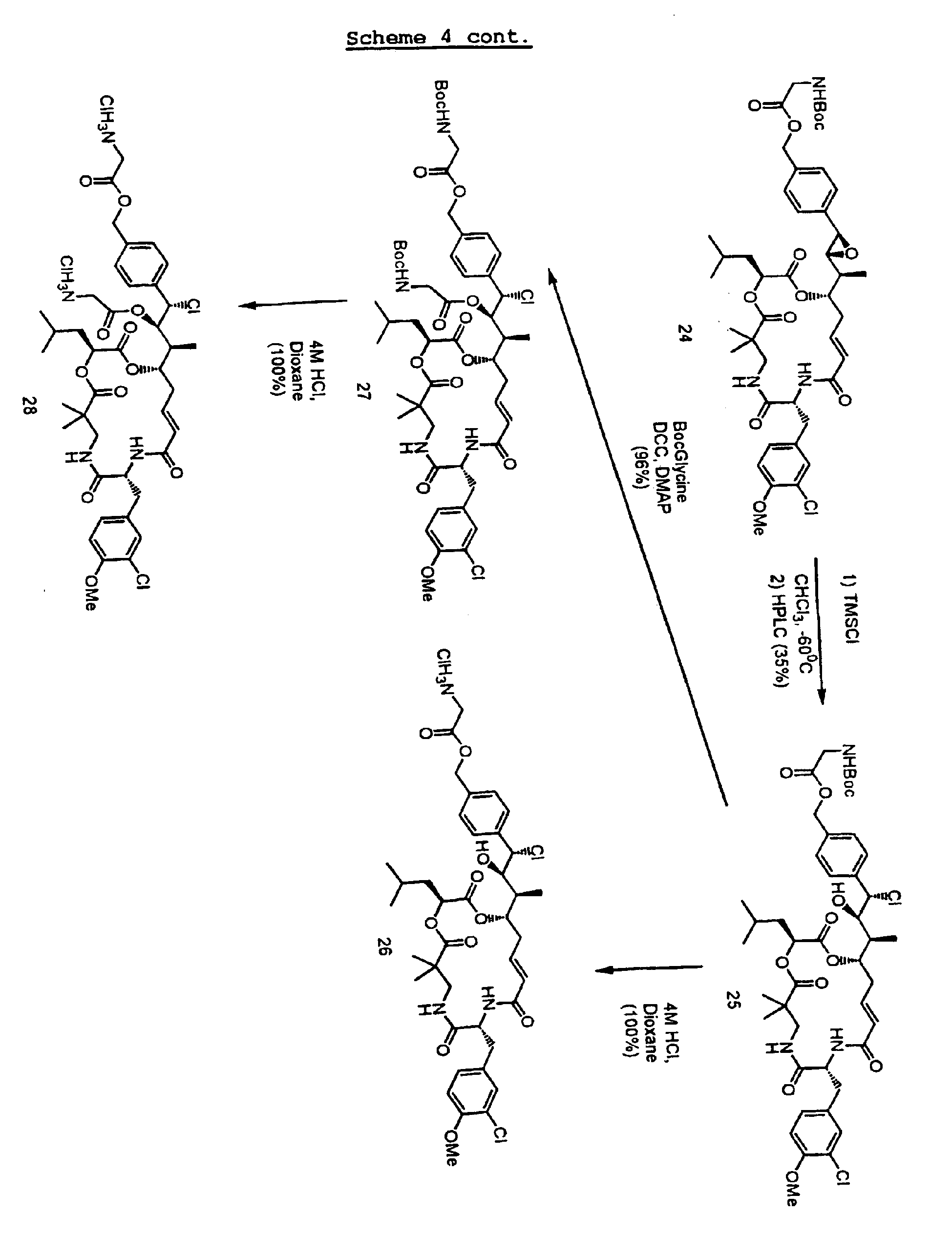

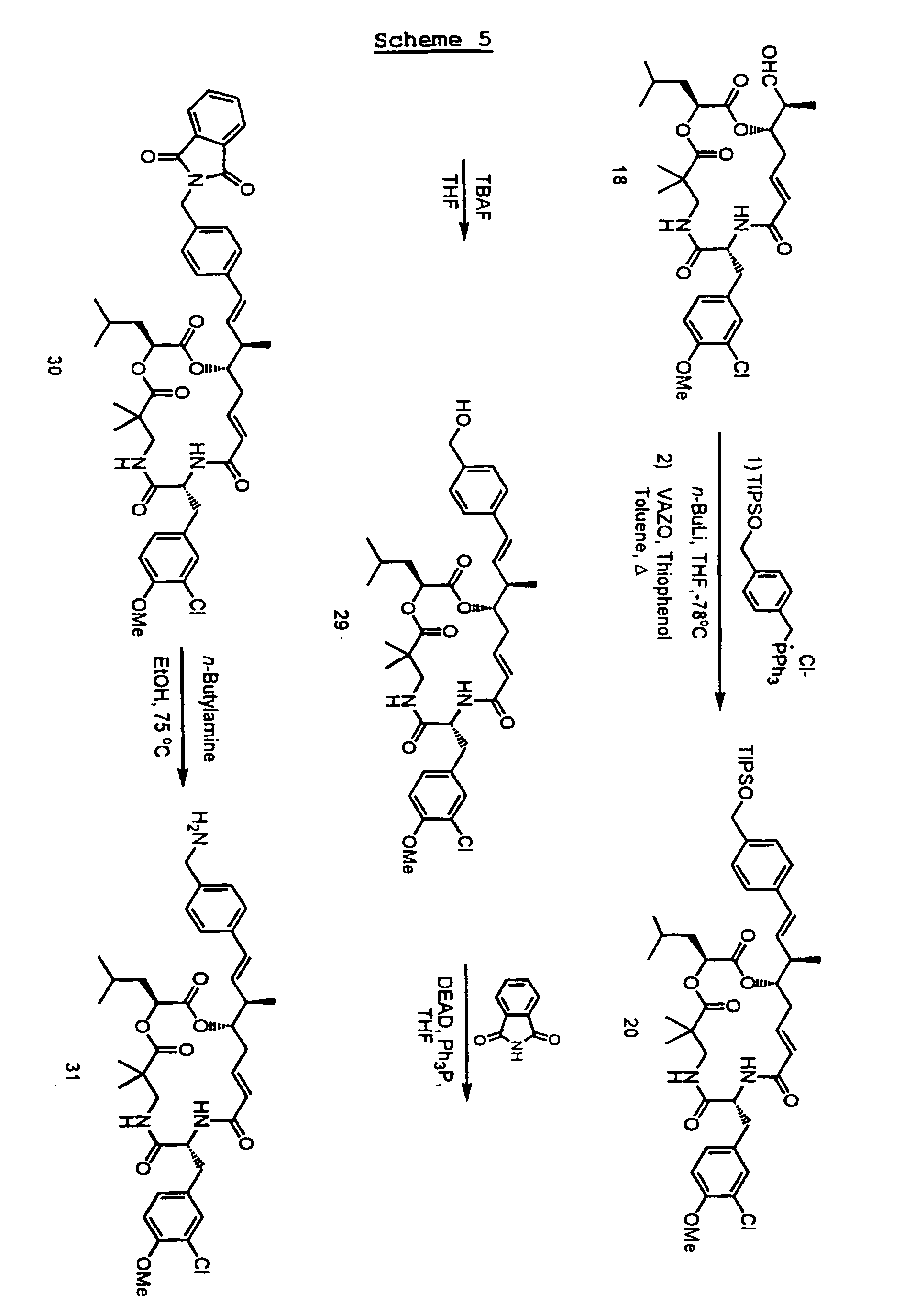

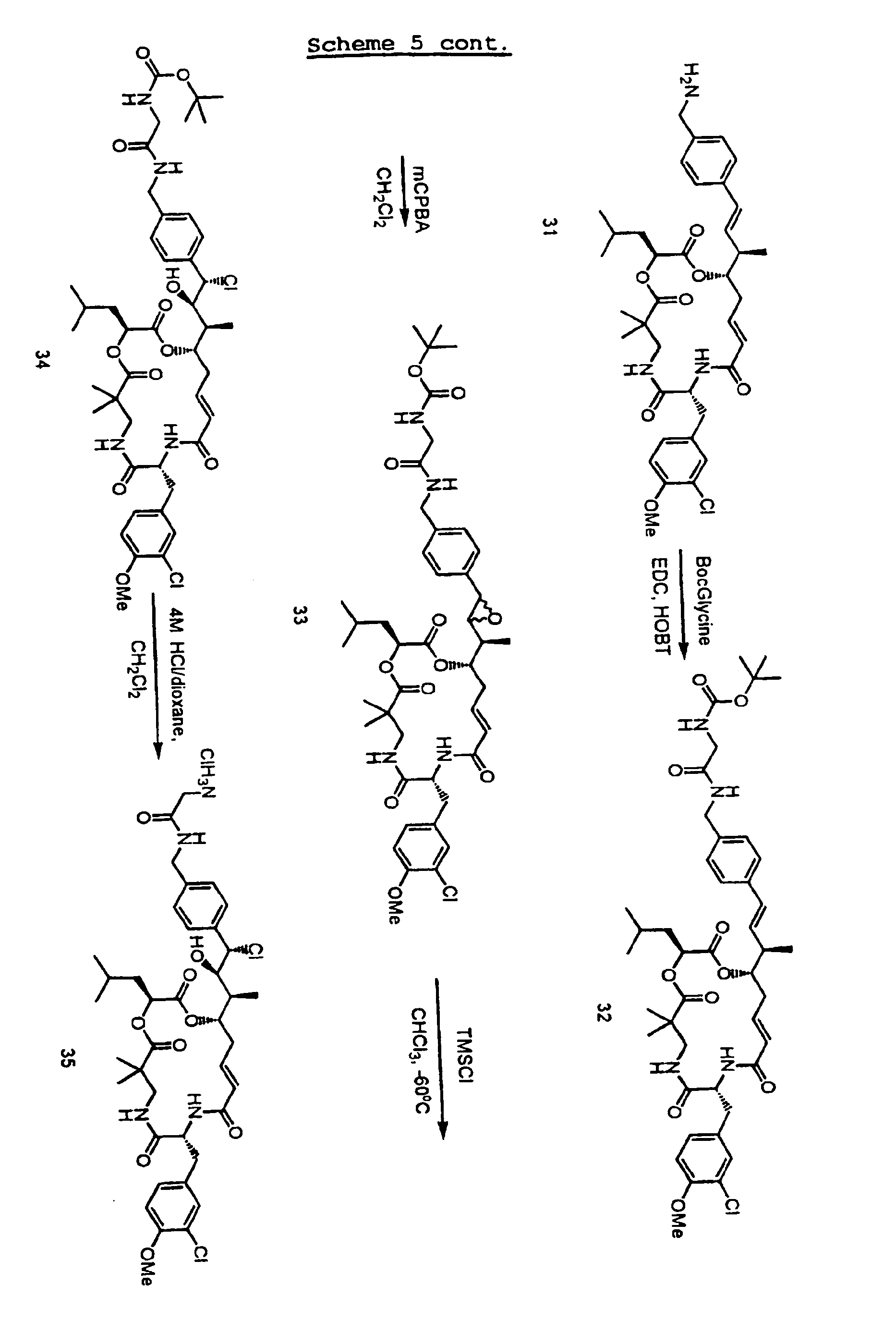

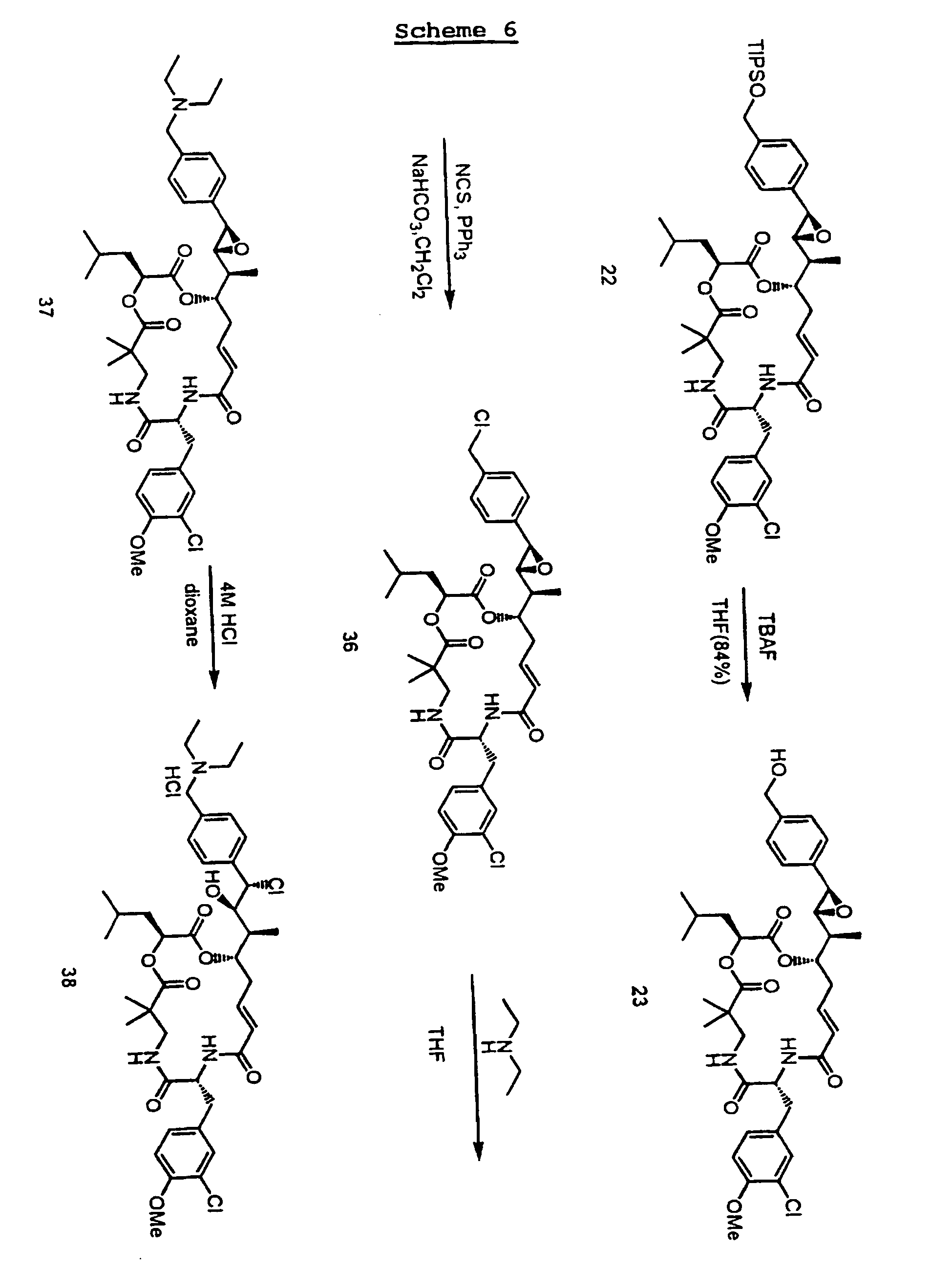

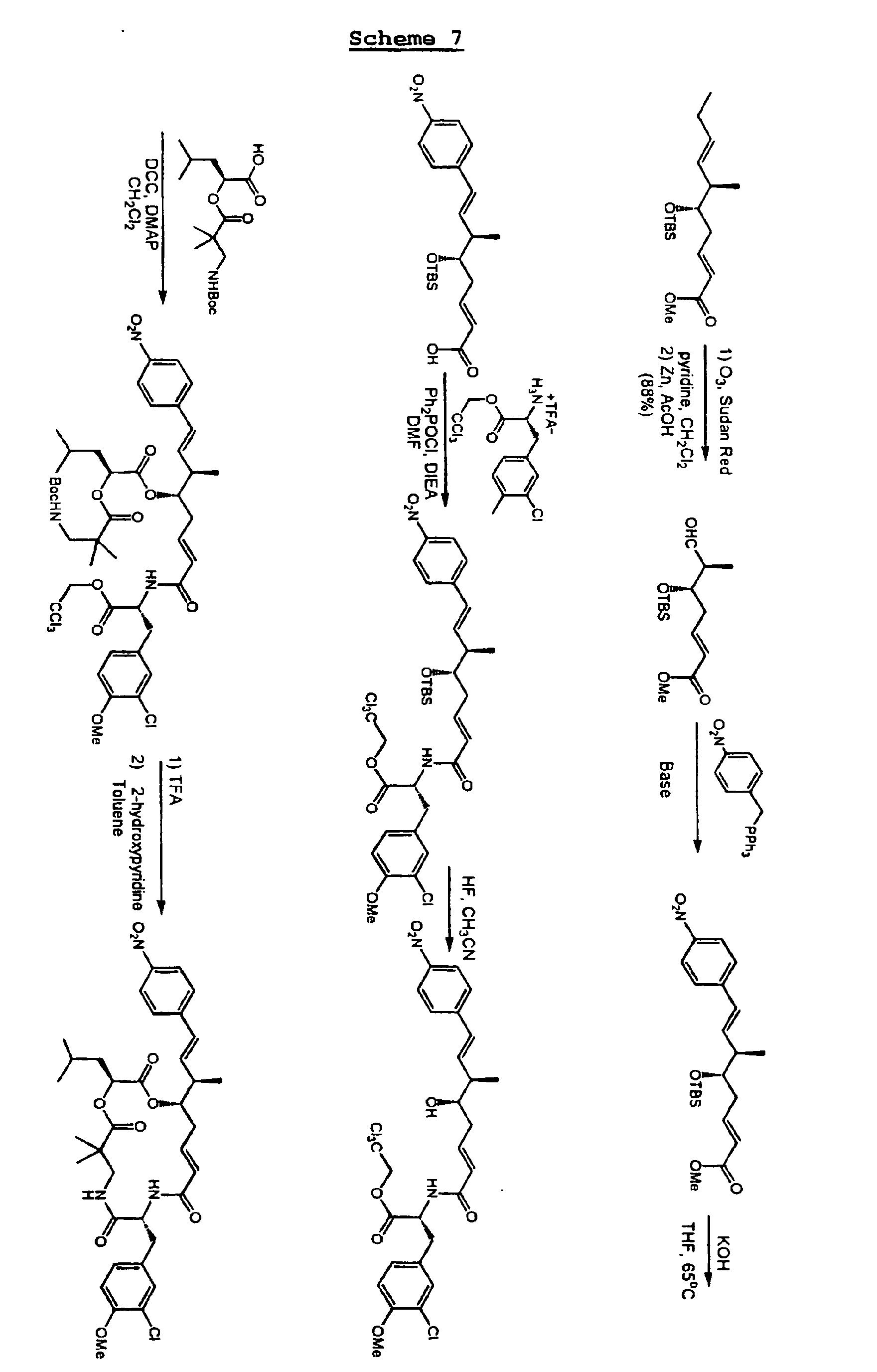

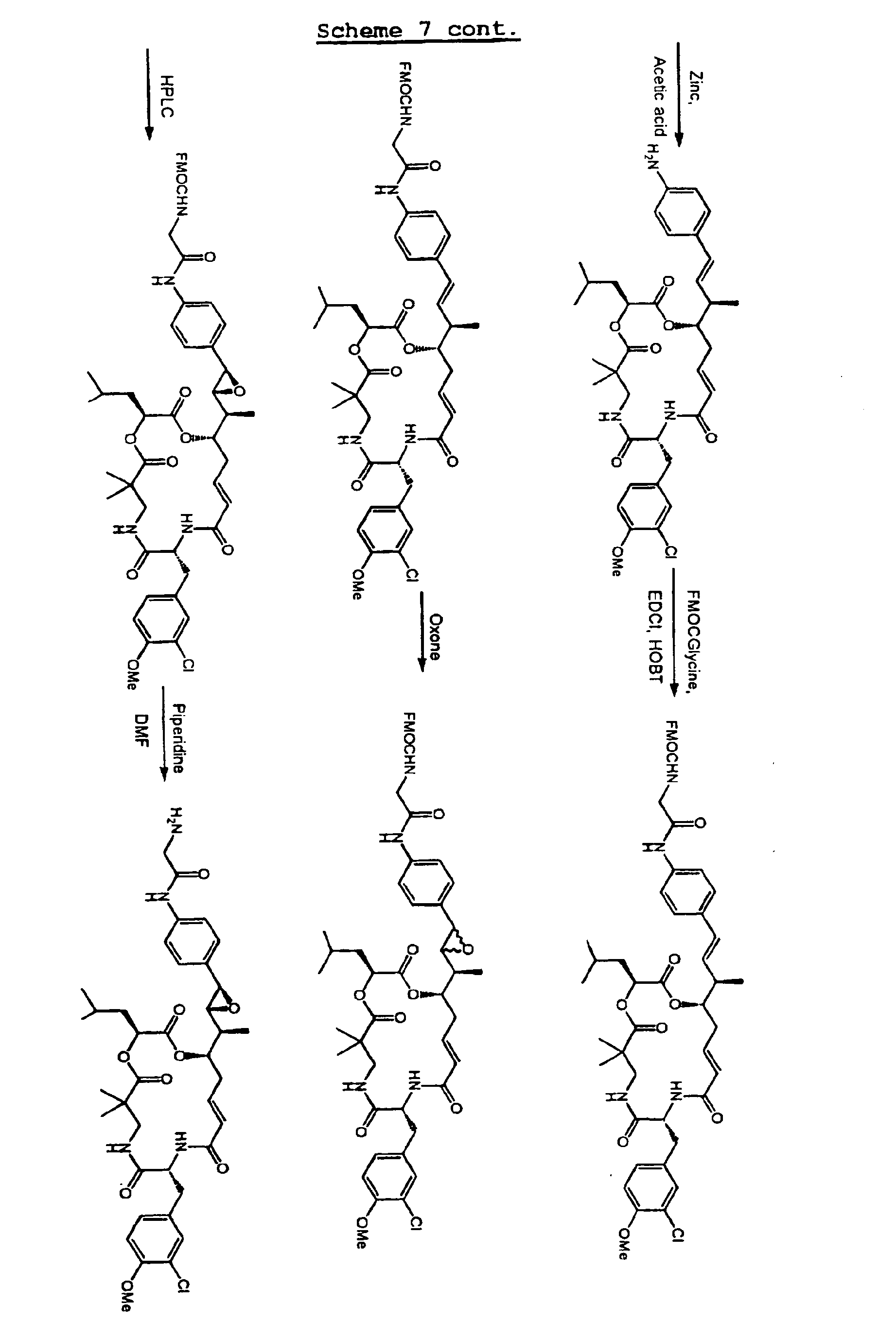

### Example 37

### Via Scheme 3

To a solution of Cryptophycin **53** (0.15 g, 0.23 mmol) in 3 mL of DME and 2.0 mL of H₂O, was added 5 drops of concentrated H₂SO₄. The mixture was stirred overnight and an additional 5 drops of H₂SO₄ were added and stirring continued for another 24 h. Saturated NaHCO₃ was added slowly until all reactivity subsided and the mixture was extracted with CH₂Cl₂. The organic layer was washed with brine, dried over Na₂SO₄, filtered and concentrated. The resulting residue was purified by column chromatography (silica gel, 2% MeOH/CH₂Cl₂) to yield 0.13 g of the diols. To the mixture of diols in 4.0 mL of THF and 2.0 mL of H₂O was added NaIO₄ (0.144 g, 0.675 mmol). The resulting mixture was stirred overnight at room temperature. The mixture was concentrated in vacuo and 5 mL of H₂O were added and extracted with CH₂Cl₂. The combined organic layers were dried over Na₂SO₄, filtered and concentrated in vacuo to give 0.10 g (77%) of aldehyde **18**.

To the TIPS protected 4-hydroxymethylbenzyl triphenylphosphonium chloride (0.23 g, 0.4 mmol) in 3.0 mL of THF at -78 °C was added dropwise 0.25 mL of a 1.6 M solution of n-butyllithium. The mixture was warmed slowly to 0 °C and stirred for an additional 10 min. To aldehyde 18 in 4.0 mL of THF and at -78 °C was added dropwise 2.5 mL of the 0.13 M orange ylide solution. The resulting mixture was stirred at -78 °C for 2 h and at room temperature for 30 min. Saturated NH₄Cl (20 mL) was added along with ethyl acetate (10 mL), the layers separated and the organic one was washed with water (3 x 10 mL) and brine. Finally upon drying it over MgSO₄, the organic phase was filtered, concentrated in vacuo and the resulting residue was purified using column chromatography (silica gel, 70% EtOAc/hexanes) to give 0.09 g (62%) of the desired styrene.

### Example 38

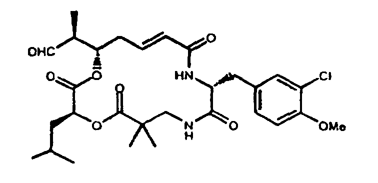

To a solution of Cryptophycin **53** (2.0 g, 2.99 mmol) in 30 mL of DME, was added a 2 M aqueous perchloric acid solution (15 mL, 30 mmol) and the resulting mixture was stirred for 6 hours. Upon careful neutralization with saturated NaHCO₃ (50 mL) the mixture was extracted with CH₂Cl₂ (4 x 100 mL) and the combined organic layers were dried over Na₂SO₄, filtered and concentrated in vacuo. Purification by column chromatography (silica gel, 5% MeOH/CH₂Cl₂) gave diols **19** (1.5 g) in 72% yield as a 3:1 anti/syn mixture. To a solution of the diols (1.0 g, 1.46 mmol), in 20 mL of THF and 15 mL of water, was added NaIO₄ (1.9 g, 8.9 mmol) and the mixture was stirred under nitrogen overnight. Upon removing the bulk of the THF under reduced pressure, the residue was diluted with water (100 mL) and extracted with CH₂Cl₂ (4 x 50 mL). The combined organic extracts were washed with brine (1 x 25 mL), dried over Na₂SO₄, filtered and concentrated under vacuum. Residual benzaldehyde was removed by dissolving the solid in 100 mL of toluene and subsequently removing the toluene at 40 °C on a rotary evaporator. Two additional evaporations from toluene gave aldehyde **18** as a yellow foam (0.828 g) in 98% yield. The resulting aldehyde was used without further purification and was stored at -23 °C for stability reasons: [α]²⁰_{D}+23.0 ° (*c* 0.565, CHCl₃) ; ¹H NMR (300 MHz, CDCl₃) δ 9.64-9.63 (d, 1 H, *J* = 1.4 Hz), 7.28-7.26 (m, 1 H), 7.21-7.20 (d, 1 H, *J =* 1.9 Hz), 7.08-7.05 (dd, 1 H, *J* = 7.1, 1.7 Hz), 6.87-6.84 (d, 1 H, *J =* 8.5 Hz), 6.82-6.72 (m, 1 H), 5.80-5.75 (d, 1 *H, J =* 15.0 Hz), 5.54-5.51 (d, 1 H, *J* = 7.7 Hz), 5.40-5.33 (m, 1 H), 4.85-4.81 (dd, 1 H, *J* = 9.7, 3.2 Hz), 4.78-4.71 (m, 1 H), 3.88 (s, 3 H), 3.46-3.39 (dd, 1 H, *J* = 13.5, 8.6 Hz), 3.15-3.03 (m, 3 H), 2.68-2.35 (m, 3 H), 1.82-1.63 (m, 2 H), 1.45-1.37 (m, 1 H), 1.24 (s, 3 H), 1.19-1.16 (d, 3 H, *J* = 7.1 Hz), 1.18 (s, 3 H), 0.94-0.92 (d, 3 H, *J =* 6.5 Hz), 0.89-0.87 (d, 3 H, *J* = 6.5 Hz); ¹³C NMR (63 MHz, CDCl₃) δ 200.7, 177.8, 170.6, 170.1, 165.1, 153.9, 141.1, 130.7, 129.8, 128.1, 124.9, 122.3, 112.3, 73.4, 71.1, 56.0, 54.6, 49.9, 46.4, 42.7, 39.2, 36.1, 35.2, 24.7, 22.8, 22.7, 21.3, 10.7; IR (CHCl₃) 3422, 2964, 2936, 1755, 1730, 1718, 1678, 1529, 1504, 1487,1474, 1464, 1442, 1320, 1303, 1281, 1259, 1244, 1185, 1151, 1127, 1067 cm⁻¹; Anal. (C₂₉H₃₉ClN₂O₆) : C, H, N.

### Example 39

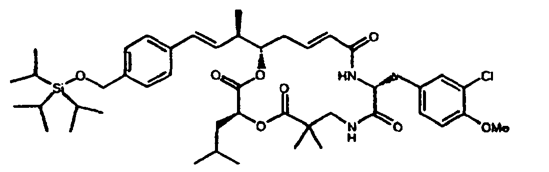

To 4-(triisopropylsiloxymethyl)benzyl triphenylphosphonium bromide (7.6 g, 12.2 mmol) in 100 mL of THF at -50°C was added dropwise 8.0 mL of a 1.5 M solution of n-butyllithium (8.1 mL, 12.2 mmol). The mixture was warmed slowly to room temperature and stirred for an additional 30 min. To aldehyde **18** (2.95 g, 5.1 mmol), in 100 mL of THF and at -78 °C, was added dropwise the red ylide solution via a double tipped needle. The resulting mixture was stirred at -78 °C for 3 h and at room temperature for 45 min. Saturated NH₄Cl (100 mL) was added along with ethyl acetate (100 mL), the layers separated and the aqueous one extracted with ethyl acetate (2 x 50 mL). The combined organic layers were washed with water (3 x 40 mL) and brine, dried over MgSO₄, filtered and concentrated in vacuo. The resulting yellow residue was purified using column chromatography (silica gel, 10-20-50% EtOAc/hexanes) to give 3.6 g (84%) of the desired styrene as a white solid and as a mixture of E:Z isomers.
The mixture of isomers (7.3 g, 8.7 mmol) was dissolved in 240 mL of benzene and heated to reflux in the presence of 1,1'-azobis(cyclohexanecarbonitrile) (VAZO) (0.32 g, 0.87 mmol) and thiophenol (3.7 mL, 4.0 mmol). Following 5 h of reflux, the solution was concentrated and the residue purified by column chromatography (silica gel, 5-50% EtOAc/hexanes) to give 6.7 g (92%) of the E isomer **20** as a white solid: [α]²⁰_{D} +31.9 ° (c1.0, CHCl₃) ; ¹H NMR (300 MHz, CDCl₃) δ 7.3-7.22 (m, 5 H), 7.20-7.19 (d, 1 H, J = 1.95 Hz), 7.07-7.04 (dd, 1 H, J = 8.4, 2.0 Hz), 6.85-6.82 (d, 1 H, *J* = 8.5 Hz), 6.8-6.7 (m, 1 H), 6.4-6.38 (d, 1 H, *J* = 15.8 Hz), 6.02-5.94 (dd, 1 H, J = 15.8, 8.8 Hz), 5.77-5.72 (d, 1 H, *J* = 14.9 Hz), 5.56-5.54 (d, 1 H, *J* = 7.9 Hz), 5.1-4.7 (m, 5 H), 3.9 (s, 3 H), 3.45-3.37 (dd, 1 H, *J* = 13.5, 8.5 Hz), 3.2-3.0 (m, 3 H), 2.6-2.3 (m, 3 H), 1.7-1.5 (m, 2 H), 1.4-1.0 (m, 31 H), 0.75-0.71 (t, 6 H, *J* = 6.1 Hz); ¹³C NMR (63 MHz, CDCl₃) δ 177.8, 170.5, 170.4, 165.2, 153.9, 142.1, 141.1, 135.2, 131.5, 130.8, 129.7, 129.6, 128.1, 125.9, 124.5, 122.4, 112.2, 77.0, 71.4, 64.7, 56.0, 54.4, 46.4, 42.7, 42.2, 39.4, 36.5, 35.3. 24.5, 22.8, 22.6, 22.5, 21.2, 17.9, 17.2, 11.9; IR (CHCl₃) 3423, 2962, 2945, 2867, 1746, 1712, 1681, 1652, 1528, 1503, 1485, 1473, 1464, 1303, 1259 cm⁻¹; Anal. (C₄₆H₆₇ClN₂O₈Si): C, H, N.

### Example 40

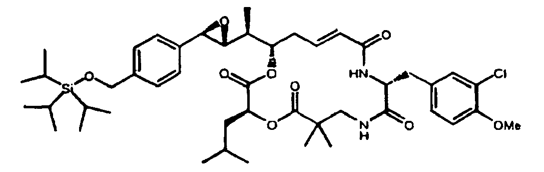

3-Chloroperoxybenzoic acid (0.27 g, 1.59 mmol) was added to a 0 °C solution of styrene **20** (1.25 g , 1.49 mmol) in 20 mL of CH₂Cl₂. The solution was stirred for 1 h at 0 °C and overnight at room temperature. It was concentrated in vacuo and the resulting epoxides separated by reverse phase HPLC to yield 0.67 g of the β epoxide **22** (57%) as a white solid: [α]²⁰_{D} +20.9 ° (c 0.765, CHCl₃); ¹H NMR (300 MHz, CDCl₃) δ 7.35-7.33 (d, 2 H, *J =* 7.8 Hz), 7.26-7.2 (m, 4 H), 7.05-7.02 (bd, 1 H, *J* = 8.2 Hz), 6.84-6.81 (d, 1 H, *J* = 8.4 Hz), 6.81-6.65 (m, 1 H), 5.8-5.65 (m, 2 H), 5.25-5.15 (m, 1 H), 4.9-4.7 (m, 4 H), 3.9 (s, 3 H), 3.7 (s, 1 H), 3.46-3.42 (dd, 1 H, *J =* 13.4, 8.8 Hz), 3.15-3.0 (m, 3 H), 2.93-2.9 (d, 1 H, *J* = 7.3 Hz), 2.6-2.4 (m, 2 H), 1.8-1.6 (m, 3 H), 1.4-1.0 (m, 31 H), 0.83-0.79 (t, 6 H, *J* = 5.3 Hz); ¹³C NMR (63 MHz, CDCl₃) δ 177.7, 170.5, 170.4, 165.1, 153.9, 142.1, 141.6, 136.7, 135.1, 130.7, 129.8, 128.1, 125.9, 125.5, 124.6, 122.3, 112.2, 75.9, 71.0, 64.6, 63.0, 58.9, 56.0, 54.6, 46.3, 42.7, 40.5, 39.2, 36.8, 35.2. 24.2, 22.8, 22.7, 22.6, 18.0, 13.4, 11.9; IR (CHCl₃) 3424, 2962, 2945, 2867, 1751, 1712, 1682, 1528, 1503, 1485, 1473, 1464, cm⁻¹; Anal. (C₄₆H₆₇ClN₂O₉Si): C, H, N.

### Example 41

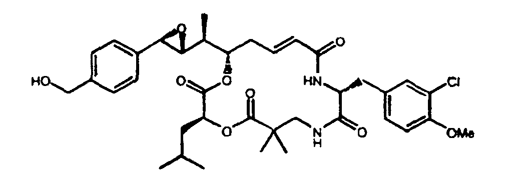

Tetrabutylammonium fluoride (0.14 mL, 0.14 mmol), as a 1.0 M solution in THF, was added dropwise to a 0 °C solution of the β epoxide **22** ( 0.1 g, 0.117 mmol) in 3.5 mL of THF. The solution was allowed to warm up to room temperature and stirring was continued for another 20 min, followed by the addition of water (10 mL) and ethyl acetate (20 mL). The layers were separated and the aqueous one was extracted with CH₂Cl₂ (3 x 20 mL). The combined organic layers were dried over anhydrous Na₂SO₄, filtered and concentrated in vacuo to yield the free alcohol. Purification by column chromatography (silica gel, 70-100% EtOAc-hexanes) yielded 0.068 g (84%) of the pure alcohol **23** as a white solid: [α]²⁰_{D}+26.2° (*c*0.435, CHCl₃); ¹H NMR (300 MHz, CDCl₃) δ 7.39-7.36 (d, 2 H, *J* = 7.8 Hz), 7.26-7.23 (d, 3 H, *J* = 9.1 Hz), 7.18 (s, 1 H), 7.05-7.02 (d, 1 H, *J* = 8.5 Hz), 6.85-6.82 (d, 1 H, *J* = 8.2 Hz), 6.82-6.7 (m, 1 H), 5.72-5.67 (d, 1 H, *J* = 15.1 Hz), 5.55-5.52 (d, 1 H, J = 7.8 Hz), 5.22-5.17 (m, 1 H), 4.85-4.7 (m, 4 H), 3.9 (s, 3 H), 3.7 (s, 1 H), 3.45-3.38 (dd, 1 H, J = 13.4, 9.3 Hz), 3.2-3.0 (m, 3 H), 2.92-2.89 (d, 1 H, J = 7.6 Hz), 2.65-2.4 (m, 2 H), 1.8-1.6 (m, 4 H), 1.4-1.2 (m, 1 H), 1.22 (s, 3 H), 1.16 (s, 3 H), 1.16-1.13 (d, 3 H, J = 7.2 Hz), 0.86-0.82 (t, 6 H, J = 6.5 Hz); ¹³C NMR (63 MHz, CDCl₃) δ 177.8, 171.0, 170.4, 165.5, 153.8, 141.5, 141.4, 135.7, 133.5, 130.6, 130.0, 128.0, 127.1, 125.6, 124.6, 122.2, 112.3, 77.2, 76.5, 76.0, 71.0, 64.2, 63.1, 58.8, 56.0, 54.7, 46.3, 42.7, 40.5, 39.3, 36.9, 35.1, 24.5, 22.7, 22.5, 22.1, 13.4; IR (CHCl₃) 3422, 2992, 2963, 2936, 2874, 1751, 1713, 1682, 1651, 1504, 1486, 1303, 1259, 1186, 1165, 1151, 1067 cm⁻¹; FAB HRMS [M + H] cacld for (C₃₇H₄₈ClN₂O₉) 699.3048, found 699.3054.

### Example 42

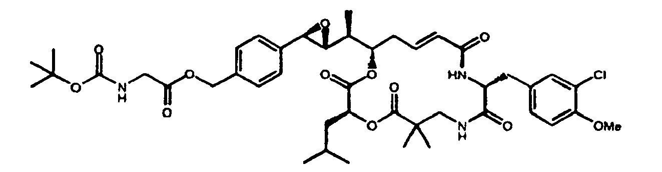

To a 0 °C solution of alcohol **23** (0.08 g, 0.114 mmol), N-(tert-butoxycarbonyl)glycine (0.034 g, 0.194 mmol) and 4-dimethylaminopyridine (DMAP) (0.004 g, 0.034 mmol) in 2.0 mL CH₂Cl₂ was added 1,3-dicyclohexylcarbodiimide (DCC) (0.040 g, 0.194 mmol). The mixture was stirred at 0 °C for 10 min and at room temperature for 45 min, filtered and concentrated in vacuo. The resulting residue was purified using column chromatography (silica gel, 70-80% EtOAc/hexanes) to give 0.07 g (72%) of the ester as a white solid: [α]²⁰_{D} +18.5°(*c* 0.65, CHCl₃) ¹H NMR (300 MHz, CDCl₃) δ 7.4-7.2 (m, 6 H), 7.11-7.08 (dd, 1 H, *J =* 8.4, 1.8 Hz), 6.9-6.87 (d, 1 H, *J* = 8.4 Hz), 6.86-6.7 (m, 1 H), 5.78-5.73 (d, 1 H, *J =* 15.2 Hz), 5.64-5.62 (d, 1 H, *J* = 7 .4 Hz), 5.3-5.22 (m, 1 H), 5.22 (s, 2 H), 5.1-5.0 (bs, 1 H), 4.9-4.7 (m, 2 H), 4.0-3.99 (d, 2 H, *J =* 5.4 Hz), 3.9 (s, 3 H), 3.73-3.72 (d, 1 H, *J* = 1.0 Hz), 3.5-3.43 (dd, 1 H, *J* = 13.4, 8.6 Hz), 3.2-3.0 (m, 3 H), 2.95-2.92 (d, 1 H, *J* = 6.4 Hz), 2.65-2.4 (m, 2 H), 1.8-1.6 (m, 3 H), 1.5 (s, 9 H), 1.45-1.3 (m, 1 H), 1.26 (s, 3 H), 1.2 (s, 3 H), 1.2-1.17 (d, 3 H, *J =* 8.7 Hz), 0.9-0.86 (t, 6 H, *J* = 6.3 Hz); ¹³C NMR (63 MHz, CDCl₃) δ 177.7, 170.6, 170.3, 170.2, 165.1, 155.6, 153.8, 141.4, 137.1, 135.6, 130.6, 129.9, 128.6, 128.0, 125.7, 124.7, 122.2, 112.2, 79.9, 75.8, 70.9, 66.4, 63.1, 58.5, 56.0, 54.7, 48.9, 46.3, 42.7, 42.4, 40.5, 39.3, 36.8, 35.2, 28.2, 24.5, 22.8, 22.7, 22.6, 21.2, 13.5; Anal. (C₄₄H₅₈ClN₃O₁₂): C, H, N.

### Example 43

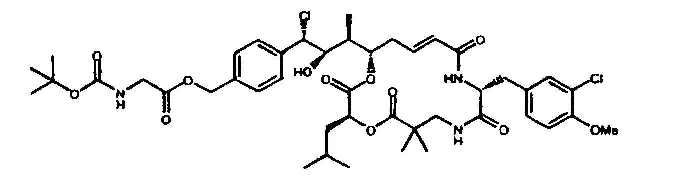

Trimethylsilyl chloride (0.09 mL, 0.75 mmoI) was added to a -60 °C solution of b epoxide **24** (0.16 g, 0.187 mmol) in 5.0 mL of CHCl₃. Following 2 h of stirring between -60 °C to -40 °C an additional 0.09 mL of TMSC1 was added and stirring continued for 3 h. The solution was allowed to warm up to room temperature, concentrated and purified by reverse phase preparative HPLC (55:45) CH₃CN:H₂O to separate the two resulting chlorohydrins. This purification gave 0.058 g (35%) of the desired chlorohydrin **25**: [α]²⁰_{D} + 50.5 ° (*c* 1.075, CHCl₃) ; ¹H NMR (300 MHz, CDCl₃) δ 7.42-7.2 (m, 6 H), 7.13-7.09 (dd, 1 H, *J =* 8.4, 1.8 Hz), 6.9-6.87 (d, 1 H, *J* = 8.4 Hz), 6.85-6.7 (m, 1 H), 5.9-5.8 (m, 2 H), 5.2 (s, 3 H), 5.15-5.05 (m, 1 H), 5.0-4.9 (m, 1 H), 4.8-4.72 (m, 1 H), 4.71-4.68 (d, 1 H, *J* = 9.7 Hz), 4.07-4.03 (d, 1 H, *J* = 9.3 Hz), 3.99-3.97 (d, 2 H, *J =* 5.5 Hz), 3.9 (s, 3 H), 3.44-3.37 (dd, 1 H, J = 13.6, 8.3 Hz), 3.23-3.14 (m, 2 H), 3.08-3.0 (dd, 1 H, *J* = 14.5, 8.0 Hz), 2.75-2.4 (m, 3 H), 2.0-1.7 (m, 3 H), 1.5 (s, 10 H), 1.26 (s, 3 H), 1.21 (s, 3 H), 1.08-1.06 (d, 3 H, *J* = 7.0 Hz), 0.977-0.963 (d, 3 H, J = 4.0 Hz), 0.956-0.942 (d, 3 H, J = 4.1 Hz); ¹³C NMR (63 MHz, CDCl₃) 177.5, 170.5, 170.2, 170.1, 165.3, 153.9, 142.2, 139.0, 138.3, 136.1, 130.8, 129.9, 128.7, 128.2, 128.1, 124.5, 122.3, 112.2, 80.0, 76.1, 73.9, 71.1, 66.2, 61.7, 56.1, 54.6, 46.4, 42.7, 42.3, 39.6, 38.4, 36.3, 35.1, 28.2, 24.8, 23.0, 22.9, 22.7, 21.5, 8.6; IR (CHCl₃) 3428, 3009, 2966, 2935, 1750, 1714, 1683, 1504, 1486, 1369, 1259, 1193, 1162, 1127, 1067; FAB HRMS [M + H] cacld for (C₄₄H₆₀ClN₃O₁₂) 892.3554, found 892.3565.

### Example 44

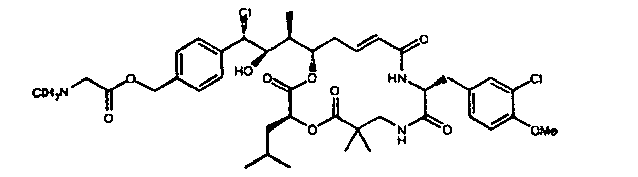

A 4 M solution of hydrogen chloride in 1,4-dioxane (0.08 mL, 0.33 mmol) was added to a solution of the glycinate **25** (0.058 g, 0.065 mmol) in 0.2 mL of CH₂Cl₂. The resulting mixture was stirred at room temperature for 3 h, concentrated in vacuo and maintained under vacuum for 3 days to remove the 1,4-dioxane thus giving the desired hydrochloride salt **26** in quantitative yield: [α]²⁰_{D} + 26.2 ° (c 0.58, MeOH ) ; ¹H NMR (500 MHz, CD₃OD) δ 7.48-7.42 (q, 4 H, *J* = 11.2 Hz), 7.31-7.3 (d, 1 H, *J* = 2.0 Hz), 7.21-7.19 (dd, 1 H, *J* = 8.5, 2.0 Hz), 7.01-7.0 (d, 1 *H, J* = 8.4 Hz), 6.8-6.7 (m, 1 H), 6.0-5.95 (dd, 1 H, *J* = 15.2, 1.5 Hz), 5.3 (d, 2 H, *J =* 1.3 Hz), 5.16-5.1 (m, 1 H), 5.09-5.07 (dd, 1 H, *J* = 10.0, 3.6 Hz), 4.84-4.82 (d, 1 H, *J* = 9.8 Hz), 4.54-4.51 (dd, 1 H, *J =* 11.3, 3.7 Hz), 4.05-4.03 (dd, 1 H, *J =* 9.5, 1.8 Hz), 3.9 (s, 2 H), 3.86 (s, 3 H), 3.5-3.47 (d, 1 H, *J* = 13.5 Hz), 3.22-3.18 (dd, 1 H, *J =* 14.5, 3.6 Hz), 3.14-3.11 (d, 1 H, J = 13.5 Hz), 2.8-2.77 (d, 1 H, *J* = 14.4 Hz), 2.78-2.75 (m, 2 H), 2.55-2.35 (m, 2 H), 1.9-1.55 (m, 4 H), 1.4-1.3 (m, 1 H), 1.24 (s, 3 H), 1.2 (s, 3 H), 1.04-1.03 (d, 3 H, *J* = 7.0 Hz), 1.02-1.0 (t, 6 H, *J* = 7.2 Hz); ¹³C NMR (75 MHz, CDCl₃) δ 178.9, 173.8, 171.9, 168.3, 155.3, 144.2, 141.8, 136.7, 132.3, 131.5, 129.75, 129.7, 129.4, 125.2, 123.3, 113.5, 77.2, 74.7, 72.6, 68.5, 63.5, 57.6, 56.7, 47.6, 44.1, 41.1, 40.4, 37.9, 36.5, 26.3, 23.6, 23.5, 22.2, 9.0; IR (KBr) 3412, 2961, 2935, 1752, 1722, 1669, 1504, 1473, 2279, 1259, 1207, 1151. 1126, 1065 cm⁻¹.

### Example 45

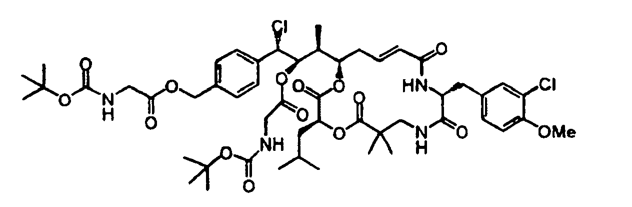

To chlorohydrin **25** (0.14 g, 0.16 mmol), *N-*(*tert-*butoxycarbonyl)glycine (0.041 g, 0.24 mmol) and 4-dimethylaminopyridine (DMAP) (0.002 g, 0.016 mmol) in 0.7 mL of CH₂Cl₂ was added 1,3-dicyclohexylcarbodiimide (DCC) (0.049 g, 0.24 mmol). The resulting mixture was stirred at room temperature for 1h, filtered using ethyl acetate and concentrated in vacuo. The resulting residue was purified using column chromatography (silica gel, 50-60-70% EtOAc/hexanes) to give 0.158 g (97%) of the diglycinate **27** as a white solid: [α]²⁰_{D} + 44.0 ° (*c* 1.25, CHCl₃); ¹H NMR (300 MHz, CDCl₃) δ 7.4-7.2 (m, 7 H), 7.13-7.10 (bd, 1 H, *J =* 9.8 Hz), 6.91-6.88 (d, 1 H, *J* = 8.4 Hz), 5.8-5.77 (d, 1 H, *J* = 15.3 Hz), 5.6-5.58 (m, 1 H), 5.49-5.46 (d, 1 H, *J =* 9.6 Hz), 5.19 (s, 2 H), 5.1-4.7 (m, 6 H), 3.98-3.97 (d, 2 H, *J =* 5.0 Hz), 3.93 (s, 3 H), 3.69-3.62 (dd, 1 H, *J* = 18.2, 4.0 Hz), 3.5-3.0 (m, 5 H), 2.7-2.35 (m, 3 H), 2.0-1.7 (m, 3 H), 1.49 (s, 9 H), 1.44 (s, 9 H), 1.28 (s, 3 H), 1.22 (s, 3 H), 1.1-1.08 (d, 3 H, *J =* 7.0 Hz), 1.06-1.04 (d, 3 H, *J =* 6.4 Hz), 1.0-0.98 (d, 3 H, *J* = 6.2 Hz); ¹³C NMR (63 MHz, CDCl₃) δ 177.6, 170.4, 169.9, 168.5, 165.0, 155.2, 153.9, 141.6, 138.6, 137.3, 136.3, 130.8, 129.8, 128.2, 128.1, 124.6, 122.3, 112.2, 79.9, 74.7, 71.1, 66.2, 60.0, 56.0, 54.5, 46.5, 42.8, 42.4, 41.8, 39.5, 38.0, 36.5, 35.2, 28.2, 28.15, 24.8, 23.1, 22.8, 22.6, 21.4, 9.9; IR (CHCl₃) 3431, 2982, 2966, 2935, 2872, 1756, 1713, 1685, 1504, 1369, 1258, 1193, 1161; FAB HRMS [M - BOC + H] cacld for (C₄₆H₆₃Cl₂N₄O₁₃) 949.3769, found 949.3777.

### Example 46

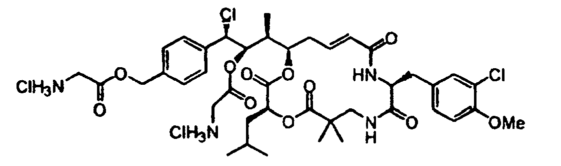

A 4 M solution of hydrogen chloride in 1,4-dioxane (0.1 mL, 0.42 mmol) was added to a solution of the diglycinate **27** (0.044 g, 0.042 mmol) in 0.2 mL of CH₂Cl₂. The resulting mixture was stirred at room temperature for 3 h, concentrated in vacuo and maintained under vacuum for 3 days to remove residual 1,4-dioxane thus giving the desired hydrochloride salts **28** in quantitative yield: [α]²⁰_{D} + 33.1° (c 0.865, MeOH); ¹H NMR (500 MHz, CD₃OD) δ 7.77-7.74 (d, 1 H), 7.46-7.41 (q, 4 H, *J =* 20.3, 8.4 Hz), 7.29-7.28 (d, 1 H, *J* = 2.1 Hz), 7.18-7.16 (dd, 1 H, *J* = 8.6, 2.1 Hz), 6.99-6.97 (d, 1 H, *J* = 8.5 Hz), 6.7-6.6 (m, 1 H), 5.95-5.92 (d, 1 H, *J* = 15.3 Hz), 5.54-5.52 (dd, 1 H, *J =* 9.5, 1.4 Hz), 5.5 (s, 1 H), 5.28 (s, 2 H), 5.21-5.19 (d, 1H, *J* = 9.4 Hz), 5.12-5.09 (dd, 1 H, *J* = 10.5, 3.1 Hz), 4.87-4.85 (d, 1 H, *J* = 12.4 Hz), 4.5-4.47 (dd, 1H, *J =* 11.3, 3.7 Hz), 3.9 (s, 2 H), 3.84 (s, 3 H), 3.82-3.79 (d, 1 H, *J* = 17.8 Hz), 3.48-3.45 (d, 1 H, *J =* 13.7 Hz), 3.35-3.3 (m, 1 H), 3.19-3.15 (dd, 1 H, *J* = 14.5, 3.8 Hz), 3.11-3.09 (d, 1 H, *J* = 13.8 Hz), 2.77-2.6 (m, 3 H), 2.37-2.3 (m, 1 H), 1.95-1.75 (m, 3 H), 1.22 (s, 3 H), 1.17 (s, 3 H), 1.08-1.07 (d, 3 H, *J* = 7.0 Hz), 1.03-1.02 (d, 3 H, *J* = 6.4 Hz), 1.0-0.99 (d, 3 H, *J* = 6.2 Hz); ¹³C NMR (63 MHz, CDCl₃) δ 178.8, 173.8, 171.9, 168.5, 168.2, 167.8, 155.4, 143.5, 139.3, 137.6, 132.2, 131.4, 129.7, 129.5, 129.4, 125.3, 123.3, 113.5, 77.9, 76.2, 72.4, 68.2, 60.8, 57.7, 56.7, 47.5, 44.2, 41.2, 40.5, 39.8, 37.9, 36.5, 26.3, 23.7, 23.4, 22.0, 9.9; IR (KBr) 3417, 3234, 2959, 2873, 2622, 1757, 1724, 1673, 1504, 1473, 1303, 1259, 1221, 1150, 1065 cm⁻¹.

### Example 47

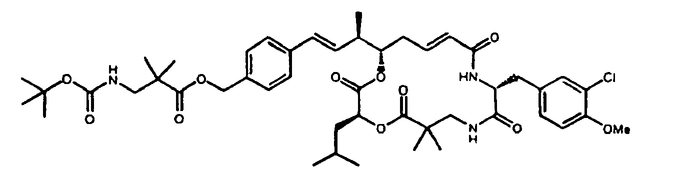

A mixture of the free acid of fragment C' (0.22 g, 1.02 mmol), DMAP (0.032 g, 0.26 mmol) and DCC (0.21 g, 1.02 mmol) was stirred for 30 min at 0 °C in 6.5 mL of CH₂Cl₂. The free alcohol **29** (0.35 g, 0.51 mmol) in 6.0 mL of CH₂Cl₂ was added dropwise. The mixture was stirred at 0 °C for 10 min and at room temperature for 24 h and finally was heated at reflux for 3 h and cooled back to room temperature. The reaction mixture was concentrated in vacuo and filtered through Celite using EtOAc. The resulting residue was purified using column chromatography (silica gel, 60-70% EtOAc/hexanes) to give 0.38 g (85%) of the ester above as a white solid: [α]²⁰_{D}+ 23.2 ° (c 1.0, CHCl₃ ) ; ¹H NMR (300 MHz, CDCl₃) δ 7.4-7.2 (m, 6 H), 7.12-7.08 (dd, 1 H, J = 8.4, 2.0 Hz), 6.9-6.87 (d, 1 H, *J =* 8.5 Hz), 6.87-6.75 (m, 1 H), 6.47-6.42 (d, 1 H, *J =* 15.8 Hz), 6.11-6.03 (dd, 1 H, *J =* 15.8, 8.8 Hz), 5.82-5.77 (d, 1 H, *J =* 14.9 Hz), 5.61-5.58 (d, 1 H, *J =* 7.8 Hz), 5.12 (s, 2 H), 5.12-4.75 (m, 4 H), 3.9 (s, 3 H), 3.49-3.42 (dd, 1 H, J = 13.4, 8.7 Hz), 3.29-3.27 (d, 1 H, *J =* 6.5 Hz), 3.2-3.1 (m, 3 H), 2.65-2.3 (m, 3 H), 1.8-1.6 (m, 3 H), 1.47 (s, 9 H), 1.45-1.3 (m, 1 H), 1.3-1.15 (m, 15 H), 0.79-0.75 (t, 6 H, *J* =6.4 Hz); ¹³C NMR (75 MHz, CDC1₃) δ 177.8, 170.4, 165.1, 153.9, 141.9, 136.7, 135.1, 131.1, 130.8, 129.7, 128.3, 128.2, 126.2, 124.6, 122.4, 112.2, 76.9, 71.3, 66.0, 56.0, 54.4, 48.2, 46.4, 43.7, 42.6, 42.2, 39.4, 36.4, 35.2, 28.3, 24.5, 22.8, 22.6, 21.2, 17.2; IR (CHCl₃) 3426, 2968, 2935, 2874, 2841, 1746, 1713, 1684, 1652, 1504, 1486, 1474, 1368, 1318, 1304, 1259, 1244, 1165, 1151, 1067 cm⁻¹; FAB HRMS [M - BOC + H] calcd for (C₄₂H₅₇ClN₃O₉) 782.3783, found 782.3788.

### Example 48

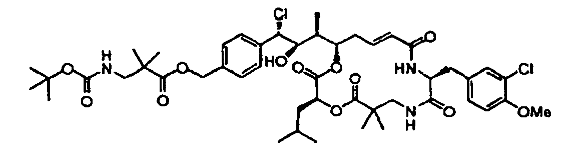

3-Chloroperoxybenzoic acid (0.092 g, 0.54 mmol) was added to a 0 °C solution of the starting styrene (0.45 g , 0.51 mmol) in 9.0 mL of CH₂Cl₂. The solution was stirred for 1 h at 0 °C and overnight at room temperature. It was concentrated in vacuo and the resulting epoxides dissolved in 10 mL of CHCl₃ and cooled to -60 °C.
Freshly distilled TMSCl (0.25 mL, 1.95 mmol) was added to the -60 °C solution and the mixture was stirred for 1h. More TMSCl (0.5 mL, 3.9 mmol) was added and stirring continued between -60 °C to -40 °C for an additional 2 h. The solution was allowed to warm up to room temperature and additional TMSCl (0.25 mL, 1.95 mmol) was added. Following 30 min of stirring at room temperature the solution was concentrated and purified by preparative HPLC to separate the resulting chlorohydrins. This purification gave 0.1 g (22%) of the desired chlorohydrin: [α]²⁰_{D} + 47.9 °(*c* 0.75, CHCl₃); ¹H NMR (300 MHz, CDCl₃) δ 7.5-7.2 (m, 6 H), 7.13-7.10 (dd, 1 H, *J* = 8.4, 1.9 Hz), 6.91-6.88 (d, 1 H, *J* = 8.5 Hz), 6.87-6.75 (m, 1 H), 5.85-5.8 (d, 1 H, *J* = 14.9 Hz), 5.75-5.6 (m, 1 H), 5.22-5.1 (m, 3 H), 5.0-4.9 (m, 2 H), 4.8-4.72 (m, 1 H), 4.71-4.68 (d, 1 H, *J* = 9.6 Hz), 4.07-4.03 (d, 1 H, *J* = 9.5 Hz), 3.9 (s, 3 H), 3.45-3.38 (dd, 1 H, *J* = 13.4, 8.5 Hz), 3.31-3.26 (d, 1 H, *J* = 6.4 Hz), 3.25-3.0 (m, 4 H), 2.8-2.65 (bd, 1 H), 2.6-2.35 (m, 2H), 1.9-1.7 (m, 3 H), 1.47 (s, 10 H), 1.27 (s, 3 H), 1.25 (s, 6 H), 1.22 (s, 3 H), 1.09-1.07 (d, 3 H, *J* = 7.0 Hz), 0.98-0.96 (m, 6 H); ¹³C NMR (75 MHz, CDCl₃) δ 177.5, 170.5, 165.2, 153.9, 142.5, 137.0, 130.8, 129.8, 128.2, 124.5, 122.4, 112.2, 79.1, 76.1, 73.9, 71.1, 65.6, 61.7, 56.1, 54.5, 48.2, 46.4, 43.7, 42.7, 39.6, 38.4, 36.3, 35.2, 28.3, 24.8, 23.0, 22.9, 22.8, 22.7, 21.5, 8.6; IR (CHCl₃) 3426, 2967, 2934, 2873, 2841, 1715, 1684, 1605, 1504, 1485, 1474, 1442, 1368, 1305, 1258, 1151 cm⁻¹; FAB HRMS [M - BOC + H] calcd for (C₄₂H₅₈Cl₂N₃O₁₀) 834.3499, found 834.3487.

### Example 49

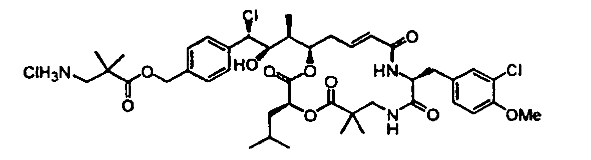

A 4 M solution of hydrogen chloride in 1,4-dioxane (0.11 mL, 0.43 mmol) was added to a solution of the chlorohydrin (0.08 g, 0.086 mmol) in 0.35 mL of CH₂Cl₂. The resulting mixture was stirred at room temperature for 3 h, concentrated in vacuo and maintained under vacuum for 3 days to remove the 1,4-dioxane thus giving the desired hydrochloride salt **(shown above)** (0.075 g) in quantitative yield: [α]²⁰_{D} + 28.0 ° (c 0.5, CH₃OH ); ¹H NMR (300 MHz, CD₃OH) δ 8.52-8.49 (d, 1 H, *J* = 7.5 Hz), 7.84-7.81 (d, 2 H, *J* = 9.6 Hz), 7.49-7.39 (q, 4 H, *J=* 8.3 Hz), 7.32-7.31 (d, 1 H, *J* = 1.9 Hz), 7.22-7.19 (dd, 1 H, *J* = 8.4, 2.1 Hz), 7.02-7.0 (d, 1 *H, J* = 8.4 Hz), 6.8-6.7 (m, 1 H), 6.0-5.9 (dd, 1 H, *J =* 15.4, 1.0 Hz), 5.22 (s, 2 H), 5.2-5.0 (m, 2 H), 4.85-4.81 (d, 1 H, *J =* 9.6 Hz), 4.6-4.5 (m, 1 H), 4.06-4.03 (dd, 1 H, *J* = 9.6, 1.5 Hz), 3.9 (s, 3 H), 3.54-3.46 (dd, 1 H, *J* = 13.5, 9.7 Hz), 3.25-3.11 (m, 2 H), 3.11 (s, 2 H), 2.8-2.7 (m, 2 H), 2.6-2.3 (m, 2 H), 1.9-1.5 (m, 3 H), 1.3 (s, 7 H), 1.25 (s, 3 H), 1.2 (s, 3 H), 1.06-1.03 (d, 3 *H, J* = 6.9 Hz), 1.02-1.01 (d, 3 H, *J =* 3.2 Hz), 1.0-0.99 (d, 3 *H, J* = 3.4 Hz); ¹³C NMR (63 MHz, CD₃OH) δ 178.9, 176.5, 173.8, 171.8, 168.3, 155.3, 144.2, 141.6, 137.4, 132.3, 131.5, 129.8, 129.4, 129.3, 125.2, 123.3, 113.5, 77.2, 74.7, 72.6, 67.8, 63.5, 57.6, 56.7, 47.7, 47.6, 44.1, 42.3, 41.1, 40.4, 37.9, 36.5, 26.2, 23.7, 23.4, 22.2, 9.0; IR (CHCl₃) 3421, 2964, 2935, 2873, 2841, 1717, 1676, 1528, 1504, 1477, 1464, 1405, 1282, 1259, 1185, 1152, 1067 cm⁻¹; FAB HRMS [M - Cl] calcd for (C₄₂H₅₈Cl₃N₃O₁₀) 834.3499, found 834.3504.

### Example 50

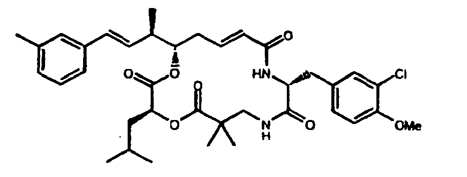

Styrene **(shown above)** (0.67 g) was prepared from aldehyde 18 (1.0 g, 1.73 mmol) and 3-methylbenzyl triphenylphosphonium chloride (0.886 g, 2.2 mmol) in 58% yield according to the procedure described above for styrene **20**: [α]²⁰_{D} + 33.1 ° (*c* 1.0, CH₃OH ) ; ¹H NMR (300 MHz, CDCl₃) δ 7.24-7.0 (m, 7 H), 6.85-6.82 (d, 1 H, *J* = 8.4 Hz), 6.82-6.7 (m, 1 H), 6.39-6.34 (d, 1 H, *J* = 15.8 Hz), 6.03-5.95 (dd, 1 H, *J* = 15.8, 8.7 Hz), 5.78-5.73 (d, 1 H, *J =* 15.2 Hz), 5.67-5.64 (d, 1 H, *J =* 7.8 Hz), 5.1-5.0 (m, 1 H), 4.87-4.83 (dd, 1 H, *J =* 10.2, 3.5 Hz), 4.8-4.7 (m, 1 H), 3.9 (s, 3 H), 3.45-3.38 (dd, 1 H, *J* = 13.4, 8.6 Hz), 3.2-3.0 (m, 3 H), 2.6-2.3 (m, 3 H), 2.32 (s, 3 H), 1.75-1.25 (m, 3 H), 1.22 (s, 3 H), 1.15 (s, 3 H), 1.13-1.11 (d, 3 H, *J* = 6.8 Hz), 0.75-0.72 (t, 6 H, *J* = 5.7 Hz); ¹³C NMR (63 MHz, CDCl₃) δ 177.9, 170.5, 170.3, 165.1, 154.0, 142.1, 138.0, 136.6, 135.6, 131.8, 130.8, 129.9, 129.6, 128.4, 128.22, 128.17, 126.7, 124.5, 123.3, 122.5, 112.3, 71.4, 56.1, 54.3, 46.4, 42.7, 42.2, 39.4, 36.5, 35.3, 24.5, 22.8, 22.6, 22.56, 21.2, 21.1, 17.2; IR (CHCl₃) 3424, 3021, 3017, 2965, 1747, 1711, 1680, 1652, 1528, 1503, 1485, 1259, 1151, 1067 cm⁻¹.

### Example 51

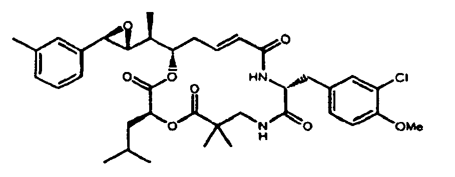

3-Chloroperoxybenzoic acid (0.19 g, 1.1 mmol) was added to the styrene **(shown above)** (0.667 g, 1.0 mmol) in 5.0 mL of CH₂Cl₂. The resulting solution was stirred overnight, concentrated in vacuo to give the β and α epoxides in a 1.8:1 ratio, in favor of the β. Separation of the two epoxides by reverse phase HPLC (70:30) CH₃CN:H₂O, gave 0.20 g of the major β epoxide as a white solid: ¹H NMR (300 MHz, CDCl₃) δ 7.3-7.0 (m, 7 H), 6.9-6.87 (d, 1 H, J = 8.4 Hz), 6.87-6.75 (m, 1 H), 5.79-5.74 (d, 1 H, *J* = 14.8 Hz), 5.54-5.51 (d, 1 H, *J* = 7.8 Hz), 5.28-5.22 (m, 1 H), 4.89-4.85 (dd, 1 H, J = 10.4, 3.5 Hz), 4.82-4.75 (m, 1 H), 3.9 (s, 3 H), 3.69-3.68 (d, 1 H, J = 1.6 Hz), 3.51-3.44 (dd, 1 H, *J* = 13.4, 8.6 Hz), 3.2-3.1 (m, 2 H), 2.98-2.95 (dd, 1 H, *J* = 7.6, 1.6 Hz), 2.65-2.32 (m, 3 H), 2.32 (s, 3 H), 1.85-1.6 (m, 3 H), 1.4-1.25 (s, 3 H), 1.27 (s, 3 H), 1.21 (s, 3 H), 1.21-1.18 (d, 3 H, *J* = 7.5 Hz), 0.90-0.86 (t, 6 H, *J* = 6.13 Hz) .

### Example 52

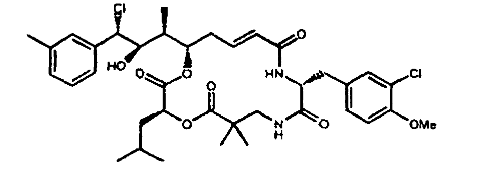

To a solution of the β epoxide **(shown above)** (0.1 g, 0.147 mmol) in 5.0 mL of CHCl₃, at -60 °C, was added chlorotrimethylsilane (0.093 mL, 0.74 mmol). The solution was stirred at -60 °C for 30 min and at room temperature for 1.5 h before being concentrated under vacuum. The resulting residue, containing a 50:50 mixture of the syn and anti chlorohydrins, was purified via reverse phase HPLC to yield 0.028 g (27%) of the desired trans isomer: ¹H NMR (300 MHz, CDCl₃) δ 7.28-7.2 (m, 5 H), 7.13-7.1 (dd, 1 H, *J* = 8.3, 1.9 Hz), 6.91-6.88 (d, 1 H, *J =* 8.5 Hz), 6.88-6.78 (m, 1 H), 5.86-5.81 (d, 1 H, *J =* 15.0 Hz), 5.73-5.71 (d, 1 H, *J* = 7.8 Hz), 5.24-5.17 (t, 1 H, *J* = 9.4 Hz), 5.0-4.96 (dd, 1 H, *J* = 9.6, 2.9 Hz), 4.81-4.74 (m, 1 H), 4.67-4.64 (d, 1 H, *J* = 9.7 Hz), 4.06-4.03 (dd, 1 H, *J =* 9.6, 1.1 Hz), 3.92 (s, 3 H), 3.47-3.39 (dd, 1 H, *J =* 13.2, 8.3 Hz), 3.42-3.0 (m, 3 H), 2.8-2.4 (m, 2 H), 2.4 (s, 3 H), 1.9-1.4 (m, 4 H), 1.28 (s, 3 H), 1.22 (s, 3 H), 1.09-1.07 (d, 3 H, *J =* 7.0 Hz), 0.98-0.96 (d, 6 H, *J* = 6.4 Hz) .

### Example 53

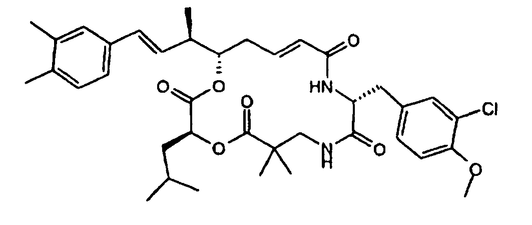

Styrene **(shown above)** (0.095 g) was prepared from aldehyde **18** (0.2 g, 0.345 mmol) and 3,4-dimethylbenzyl triphenylphosphonium chloride (0.26 g, 0.62 mmol) in 63% yield according to the procedure described above for styrene **20:** [α]²⁰_{D}+ 27.8 ° (*c* 0.576, CHCl₃ ) ; ¹H NMR (300 MHz, CDCl₃) δ 7.22-7.18 (d, 1 H, J = 1.6 Hz), 7.12-7.02 (m, 5 H), 6.87-6.81 (d, 1 H, *J* = 8.3 Hz), 6.80-6.71 (m, 1 H), 6.40-6.30 (d, 1 H, J = 15.8 Hz), 6.00-5.88 (dd, 1 H, *J* = 15.8, 8.8 Hz), 5.80-5.70 (d, 1 H, *J* = 15.1 Hz), 5.55-5.45 (d, 1 H, *J* = 7.7 Hz), 5.10-4.97 (m, 1 H), 4.90-4.80 (dd, 1 H, *J* = 9.4, 2.8 Hz), 4.80-4.70 (m, 1 H), 3.88 (s, 3 H), 3.50-3.35 (dd, 1 H, *J =* 13.3, 4.8 Hz), 3.20-3.00 (m, 3 H), 2.60-2.48 (m, 2 H), 2.45-2.30 (m, 1 H), 2.23 (s, 3 H), 2.24 (s, 3 H), 1.80-1.55 (m, 2 H), 1.40-1.30 (m, 1 H), 1.22 (s, 3 H), 1.16 (s, 3 H), 1.14-1.10 (d, 3 H, *J* = 6.8 Hz), 0.80-0.73 (m, 6 H); ¹³C NMR (63 MHz, CDCl₃) δ 177.9, 170.5, 170.3, 165.1, 154.0, 142.2, 136.5, 135.9, 134.3, 131.6, 130.8. 129.7, 129.5, 128.8, 128.1, 127.2, 124.4, 123.6, 122.4, 112.2, 71.4, 56.0, 54.2, 46.4, 42.6, 42.1, 39.4, 36.4, 35.2, 24.5, 22.7, 22.6, 22.5, 21.1, 19.6, 19.3, 17.2; IR (CHCl₃) 3424, 2965, 2935, 1746, 1711, 1681, 1652, 1527, 1503, 1485, 1259, 1187, 1164, 1151, 1067, 970, 727 cm⁻¹; Anal. (C₃₈H₄₉ClN₂O₇) C, H, N.

### Example 54

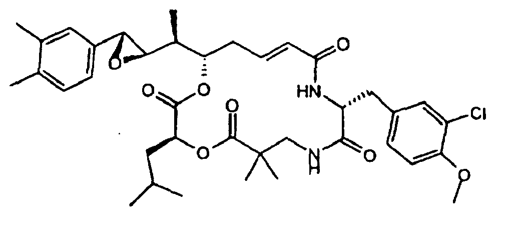

This β epoxide (0.06 g) was prepared from the styrene **(shown above)** (0.3 g, 0.44 mmol) using 3-chloroperoxybenzoic acid (0.081 g, 0.47 mmol) in 20% yield using the procedure described above: [α]²⁰_{D}+ 20.0° (c 1.43, CHCl₃); ¹H NMR (300 MHz, CDCl₃) δ 7.26-6.96 (m, 6 H), 6.98 (s, 1 H), 6.84-6.82 (d, 1 H, *J* = 8.5 Hz), 6.79-6.65 (m, 1 H), 5.73-5.69 (d, 1 H, *J* = 11.9 Hz), 5.68-5.67 (d, 1 H, *J* = 4.6 Hz), 5.29-5.15 (m, 1 H), 4.83-4.76 (dd, 1 H, *J* = 9.7, 2.8 Hz), 4.75-4.58 (m, 1 H), 3.86 (s, 3 H), 3.61-3.60 (d, 1 H, *J =* 1.6 Hz), 3.46-3.38 (dd, 1 H, *J =* 13.4, 8.8 Hz), 3.14-2.97 (m, 3 H), 2.92-2.89 (dd, 1 H, *J =* 7.7, 1.6 Hz), 2.59-2.35 (m, 2 H), 2.25 (s, 6 H), 1.78-1.58 (m, 3 H), 1.28-1.09 (m, 1 H), 1.21 (s, 3 H), 1.15 (s, 3 H), 1.15-1.12 (d, 3 H, *J =* 7.8 Hz), 0.85-0.77 (m, 6 H); ¹³C NMR (63 MHz, CDCl₃) δ 177.8, 170.4, 170.3, 164.9, 154.0, 141.7, 137.0, 136.9, 134.0, 130.8, 129.9, 129.5 , 128.1, 126.7, 124.6, 123.2, 122.4, 122.3, 75.9, 71.04, 62.8, 59.1, 56.1, 54.3, 46.4, 42.7, 40.7, 39.2, 36.8, 35.2, 24.4, 22.8, 22.6, 21.0, 19.7, 19.4, 13.6; IR (KBr) 3419, 2962, 1752, 1721, 1681, 1654, 1534, 1504, 1473, 1442, 1302, 1282, 1259, 1192, 1126, 1066 cm⁻¹; Anal. (C₃₈H₄₉ClN₂O₈) C, H, N.

### Example 55

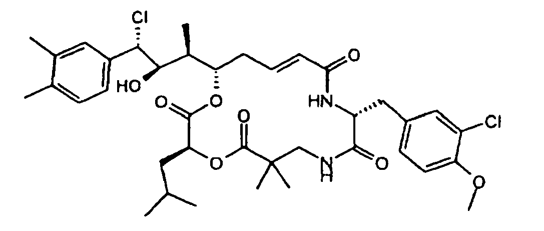

To a solution of the styrene **(shown above)** (0.492 g, 0.72 mmol) in 2.4 mL CH₂Cl₂ at 0 °C was added 3-chloroperoxybenzoic acid (0.137 g, 0.79 mmol) and toluene (1.2 mL) and stirring continued at 0 °C for 30 minutes. The ice-bath was removed and the reaction allowed to stir at room temperature for 24 hours. After diluting with 10 mL CH₂Cl₂, the solution was washed with 10% Na₂SO₃ (1 x 10 mL), H₂O (1 x 10 mL), 10% NaHCO₃ (1 x 10 mL) and dried over Na₂SO4. Concentration provided a mixture of the b/a crude epoxides in a 2:1 ratio.

The crude epoxides (0.445 g, 0.638 mmol) were dissolved in 10 mL dry CHCl₃, cooled to -60 °C and treated with trimethylsilyl chloride (0.2 mL, 1.5 mmol). Stirring was continued for 90 minutes and the solution was concentrated in vacuo. The crude chlorohydrins were purified by reverse-phase HPLC (CH₃CN/H₂O) to give the product **(shown above)** (0.115 g) in 21% yield as a white solid: [α]²⁰_{D}- 45.9 ° (c 0.59, CHCl₃ ); ¹H NMR (300 MHz, CDCl₃) δ 7.26-7.01 (m, 5 H), 6.85-6.82 (d, 1 H, *J =* 8.4 Hz), 6.80-6.71 (m, 1 H), 5.71-5.66 (d, 1 H, *J =* 15.1 Hz), 5.50-5.47 (d, 1 H, *J* = 7.6 Hz), 5.13-5.08 (t, 1 H, *J =* 8.8 Hz), 4.89-4.84 (m, 2 H), 4.81-4.71 (m, 1 H), 4.09-4.06 (d, 1 H, *J =* 9.4 Hz), 3.87 (s, 3 H), 3.44-3.37 (dd, 1 H, *J =* 13.4, 8.4 Hz), 3.16-3.06 (m, 3 H), 2.60-2.54 (m, 2 H), 2.26 (s, 6 H), 2.26-2.14 (m, 1 H), 1.89-1.81 (m, 1H), 1.70-1.62 (m, 2 H), 1.58-1.46 (m, 2 H)1.23 (s, 3 H), 1.17 (s, 3 H), 0.97-0.95 (d, 3 H, *J* = 6.6 Hz), 0.93-0.90 (m, 6 H); ¹³C NMR (63 MHz, CDCl₃) δ 177.7, 170.5, 170.1, 165.2, 153.9, 142.3, 137.7, 137.3, 135.1, 130.7, 130.2, 129.8, 128.5, 128.1, 124.6, 124.4, 122.3, 112.2, 75.9, 74.2, 71.2, 68.8, 56.0, 54.4, 46.4, 42.7, 39.6, 38.4, 36.2, 35.2, 24.8, 22.9, 22.8, 22.7, 21.7, 19.8, 19.5, 8.6; IR (KBr) 3421, 2960, 1756, 1721, 1675, 1504, 1258, 1195, 1151, 1126, 1066 cm⁻¹; Anal. (C₃₈H₅₀Cl₂N₂O₈) C, H, N.

### Example 56

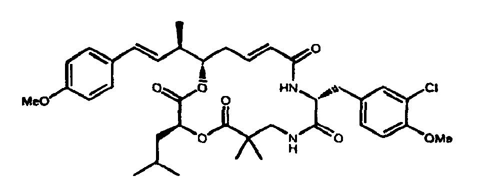

Styrene **(shown above)** (0.21 g) was prepared from aldehyde **18** (0.5 g, 0.87 mmol) and 4-methoxybenzyl triphenylphosphonium chloride (0.47 g, 1.12 mmol) in 36% yield according to the procedure described above for styrene **20**: [α]²⁰_{D}+ 31.6 ° (c 1.03, CHCl₃) ; ¹H NMR (300 MHz, CDCl₃) δ 7.26-7.23 (d, 3 H, *J* = 8.4 Hz), 7.20-7.19 (d, 1 H, *J* = 1.8 Hz), 7.07-7.03 (dd, 1 H, *J =* 8.4, 1.9 Hz), 6.84-6.81 (d, 3 H, *J =* 8.5 Hz), 6.80-6.7 (m, 1 H), 6.36-6.31 (d, 1 H, *J* = 15.8 Hz), 5.89-5.81 (dd, 1 H, *J =* 15.8, 8.8 Hz), 5.78-5.73 (d, 1 H, *J =* 13.7 Hz), 5.68-5.66 (d, 1 H, *J =* 7.9 Hz), 5.05-4.99 (ddd, 1 H, *J* = 10.6, 6.6, 1.6 Hz), 4.87-4.82 (dd, 1 H, *J =* 9.7, 3.1 Hz), 4.78-4.7 (m, 1 H), 3.86 (s, 3 H), 3.79 (s, 3 H), 3.45-3.37 (dd, 1 H, *J =* 13.4, 8.6 Hz), 3.15-3.0 (m, 3 H), 2.6-2.25 (m, 3 H), 1.7-1.3 (m, 3 H), 1.22 (s, 3 H), 1.15 (s, 3 H), 1.12-1.1 (d, 3 H, *J* = 6.8 Hz), 0.76-0.75 (d, 3 H, *J* = 2.9 Hz), 0.74-0.73 (d, 3 H, J = 2.8 Hz) ; ¹³C NMR (63 MHz, CDCl₃) δ 177.8, 170.5, 170.4, 165.1, 159.1, 153.9, 142.1, 135.8, 131.0, 130.8, 129.7, 129.5, 128.2, 127.9, 127.2, 124.5, 122.4, 113.9, 112.3, 77.1, 71.4, 56.0, 55.2, 54.4, 46.4, 42.7, 42.1, 39.4, 36.4, 35.3, 24.5, 22.8, 22.6, 21.2, 17.3; IR (CHCl₃) 3422, 3003, 2964, 2936, 2873, 2840, 1746, 1712, 1681, 1651, 1607, 1527, 1512, 1504, 1485, 1465, 1301, 1251 cm⁻¹; Anal. (C₃₇H₄₇ClN₂O₈) C, H, N.

### Example 57

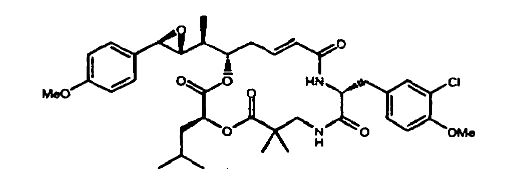

To styrene **(shown above)** (0.3 g, 0.44 mmol) was added 19 mL of acetone, 9 mL of H₂O, 9 mL of CH₂Cl₂ and solid NaHCO₃ (1.2 g, 14.5 mmol) and the mixture was cooled to 0 °C. A solution of Oxone (1.08 g, 1.8 mmol) in 9 mL of H₂O was prepared and added (2 mL) to the cold styrene mixture. Following 30 min of vigorous stirring at 0 °C an additional 2 mL of Oxone solution was added and again another 2.0 mL was added, following another 30 min, for a total of 6 mL of Oxone solution. The reaction progress was monitored by reverse phase HPLC and was found to be complete after 2.5 hrs of stirring. While still at 0 °C, the reaction was quenched with saturated aqueous NaHCO₃ (50 mL) and an additional 50 mL of CH₂Cl₂ was also added. The layers were separated and the organic layer was washed with aq. 10% Na₂SO₃ (50 mL), followed by saturated aq. NaHCO₃ (50 mL) then brine and finally was dried over Na₂SO₄, filtered and concentrated in vacuo. The mixture of b and a epoxides was separated by reverse phase HPLC (45:55) CH₃CN:H₂O to provide 0.12 g of the b epoxide as a white solid in 39% yield: [α]²⁰_{D} + 25.8 ° (*c* 0.66, CHCl₃); ¹H NMR (300 MHz, CDCl₃) δ 7.26-7.15 (m, 4 H), 7.05-7.03 (d, 1 H, *J =* 8.6 Hz), 6.9-6.87 (d, 2 H, *J* = 8.6 Hz), 6.85-6.82 (d, 1 H, *J* = 8.5 Hz), 6.82-6.7 (m, 1 H), 5.74-5.69 (d, 1 H, *J* = 15.1 Hz), 5.54-5.52 (d, 1 H, *J* = 7.8 Hz), 5.22-5.16 (m, 1 H), 4.84-4.7 (m, 2 H), 3.87 (s, 3 H), 3.81 (s, 3 H), 3.63 (s, 1 H), 3.46-3.38 (dd, 1 H, *J =* 13.5, 8.8 Hz), 3.2-3.0 (m, 3 H), 2.91-2.89 (d, 1 H, *J* = 7.4 Hz), 2.6-2.38 (m, 2 H), 1.8-1.6 (m, 3 H), 1.4-1.23 (m, 1 H), 1.22 (s, 3 H), 1.15 (s, 3 H), 1.15-1.12 (d, 3 H, *J* = 8.9 Hz), 0.84-0.80 (t, 6 H, *J* = 6.0 Hz); ¹³C NMR (63 MHz, CDCl₃) δ 177.7, 170.5, 170.4, 165.1, 159.8, 153.9, 141.6, 136.8, 130.7, 129.7, 128.6, 128.1, 126.9, 124.6, 122.3, 114.1, 112.2, 75.9, 71.0, 62.8, 58.9, 56.0, 55.2, 54.6, 46.3, 42.7, 40.6, 39.2, 36.8, 35.2, 24.4, 22.8, 22.7, 22.6, 21.1, 13.5; IR (CHCl₃) 3423, 3009, 2964, 2936, 2874, 2840, 1751, 1713, 1681, 1653, 1614, 1517, 1504, 1486, 1464, 1442, 1303, 1281, 1257, 1183, 1173, 1152 cm⁻¹; Anal. (C₃₇H₄₇ClN₂O₉) C, H, N.

### Example 58

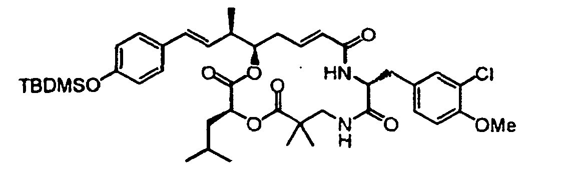

Styrene **(shown above)** (0.649 g) was prepared from aldehyde **18** (0.911 g, 1.57 mmol) and 4-(*tert-*butyldimethylsiloxy)benzyl triphenylphosphonium chloride (1.7 g, 3.27 mmol) in 53% yield according to the procedure described above for styrene **20**: [α]²⁰_{D}+ 30.83 ° (*c* 0.52, CHCl₃); ¹H NMR (300 MHz, CDCl₃) δ 7.25 (s, 1H), 7.26-7.20 (d, 2H, *J* = 8.5Hz), 7.12 - 7.06 (dd, 1H, *J* = 8.3, 1.6 Hz), 6.92-6.86 (d, 1H, *J* = 8.5 Hz), 6.84-6.78 (d, 2H, *J* = 8.5 Hz), 6.44-6.33 (d, 1H, *J* = 15.9 Hz), 5.95-5.85 (dd, 1H, *J* = 15.8, 8.8 Hz), 5.85-5.77 (d, 1H, *J* = 15.5 Hz), 5.68-5.55 (bd, 1H, *J* = 7.9 Hz), 5.15-5.00 (m, 1H), 4.95-4.80 (dd, 1H, *J* = 10.0, 3.0 Hz), 4.85-4.75 (m, 1H), 3.91 (s, 3H), 3.53-3.43 (dd, 1H, *J* = 13.4, 8.6 Hz), 3.23-3.08 (m, 3H), 2.65-2.50 (m, 2H), 2.50-2.35 (m, 3H), 1.75-1.60 (m, 2H), 1.45-1.36 (m, 1H), 1.27 (s, 3H), 1.20 (s, 3H), 1.16-1.13 (d, 3H, *J* = 6.8 Hz), 1.01 (s, 9H), 0.85-0.74 (m, 6H), 0.22 (s, 6H); ¹³C NMR (63 MHz, CDCl₃) δ 177.8, 170.5, 170.4, 165.1, 155.2, 153.9, 142.1, 131.1, 130.8, 130.1, 129.7, 128.2, 128.1, 127.1, 124.5, 122.4, 120.2, 112.2, 71.4, 56.0, 54.4, 46.4, 42.7, 42.2, 39.4, 36.5, 35.3, 25.6, 24.5, 22.8, 22.7, 21.2, 18.1, 17.3, -4.5; IR (CHCl₃) 3422, 3030, 3008, 2961, 2932, 2899, 2860, 1745, 1712, 1681, 1604, 1527, 1509, 1485, 1442, 1370, 1339, 1303, 1258, 1169, 1151, 1067, 1007, 970, 912, 841, 822, 792 cm⁻¹; Anal. (C₄₂H₅₉ClN₂O₈Si) C, H, N.

### Example 59

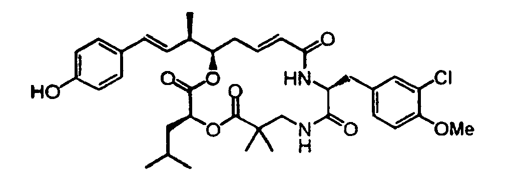

To a -70 °C solution of the silyl protected phenol (0.084 g, 0.107 mmol), in 4 mL of dry THF, was added a 1.0 M THF solution of tetrabutylammonium fluoride (TBAF) (0.11 mL, 0.11 mmol). The light yellow solution was stirred at -70 °C for 30 minutes, then quenched with 2 mL of saturated NH₄Cl and extracted with CH₂Cl₂ (3 x 25 mL). The combined organic extracts were dried over MgSO₄, filtered and concentrated in vacuo. Purification of the crude product by radial PLC (silica gel, 50-100% EtOAc/hexanes) gave the desired alcohol (0.067g) in 93% yield as a white solid: [α]²⁰_{D}+ 30.83 ° (*c* 0.52, CHCl₃); ¹H NMR (300 MHz, CDCl₃) δ 7.40-7.32 (m, 1H), 7.28-7.20 (m, 3H), 7.13-7.05 (dd, 1H, *J* = 8.3, 1.6 Hz), 6.95-6.75 (m, 5H), 6.57 (s, 1H), 6.42-6.33 (d, 1H, *J* = 15.9 Hz), 5.93-5.83 (dd, 1H, *J* = 15.8, 8.8 Hz), 5.83-5.78 (d, 1H, *J* = 15.5 Hz), 5.75-5.73(bd, 1H, *J* = 7.9 Hz), 5.15-5.00 (m, 1H), 4.93-4.85 (dd, 1H, *J* = 10.0, 3.0 Hz), 4.85-4.75 (m, 1H), 3.90 (s, 3H), 3.54-3.40 (dd, 1H, *J* = 13.4, 8.6 Hz), 3.25-3.02 (m, 3H), 2.65-2.35 (m, 3H), 1.80-1.60 (m, 2H), 1.45-1.36 (m, 1H), 1.27 (s, 3H), 1.20 (s, 3H), 1.17-1.12 (d, 3H, *J* = 6.8 Hz), 0.86-0.74 (d, 6H, J = 5.0 Hz); ¹³C NMR (63 MHz, CDCl₃) δ 177.9, 170.7, 170.6, 156.0, 154.0, 142.6, 137.1, 131.3, 130.8, 129.5, 128.9, 128.1, 127.4, 124.3, 122.5, 115.6, 112.3, 77.2, 71.5, 56.1, 54.5, 46.5, 42.7, 42.1, 39.4, 36.5, 35.3, 24.6, 22.8, 22.7, 21.2, 17.3; IR (CHCl₃) 3597, 3421,3319. 2964, 2935, 2874, 2841, 1746, 1711, 1680, 1652, 1610, 1513, 1504, 1485, 1464, 1259, 1170, 1152, 1067 cm⁻¹; Anal. (C₃₆H₄₆ClN₂O₈) C, H, N.

### Example 60

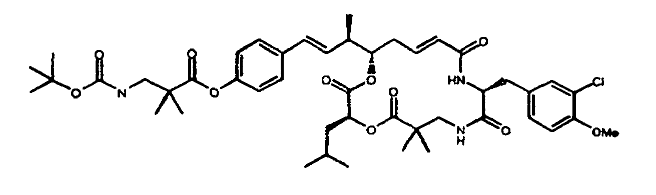

A solution of fragment C' acid (0.040 g, 0.184 mmole) and carbonyldiimidazole (CDI) (0.040 g, 0.25 mmole) in 2 mL of toluene was heated under nitrogen at 45 °C for 45 minutes. Following the addition of the alcohol **(shown above)** (0.10 g, 0.15 mmole) in 1 mL of toluene, the reaction was again heated at 45 °C for 4 hrs. Upon cooling to room temperature, the reaction mixture was diluted with EtOAc (100 mL), washed with 0.1 N HCl (1 x 10mL), water (1 x 10mL), saturated NaHCO₃ (1 x 10 mL) and brine (1 x 10mL). The organic layer was dried over MgSO₄, filtered and concentrated in vacuo to provide the crude ester as a yellow foam. Purification by radial PLC (silica gel, 50% EtOAc/hexanes) provided the pure ester (0.097) g in 75% yield as a yellow solid: [α]²⁰_{D}+ 17.2 ° (*c* 0.58, CHCl₃); ¹H NMR (300 MHz, CDCl₃) δ 7.40-7.34 (d, 2 H, *J =* 8.5 Hz), 7.29-7.22 (m, 3 H), 7.13-7.00 (m, 3 H), 6.92-6.86 (d, 1 H, *J* = 8.8 Hz), 6.86-6.76 (m, 1H), 6.50-6.38 (d, 1 H, *J =* 15.9 Hz), 6.10-5.97 (dd, 1 H, *J* = 15.8, 8.8 Hz), 5.85-5.75 (d, 1 H, *J* = 15.1 Hz), 5.55-5.45 (d, 1 H, J = 7.9 Hz), 5.15-5.06 (m, 1 H), 5.06-4.96 (m, 1 H), 4.95-4.85 (m, 1 H), 4.83-4.72 (dd, 1 H, *J* = 10.0, 3.0 Hz), 3.92 (s, 3 H), 3.53-3.35 (m, 3 H), 3.22-3.06 (m, 3 H), 2.65-2.50 (m, 2 H), 2.48-2.35 (m, 1 H), 1.80-1.65 (m, 2 H), 1.49 (s, 9 H), 1.40 (s, 6 H), 1.27 (s, 3 H), 1.21 (s, 3 H), 1.20-1.15 (d, 3 H, *J* = 6.9 Hz), 0.86-0.77 (d, 6 H, *J* = 6.3 Hz); ¹³C NMR (63 MHz, CDCl₃) δ 177.8, 170.5, 170.4 165.1, 156.0, 153.9, 150.0, 142.0, 134.5, 130.8, 130.6, 130.4, 129.6, 128.2, 126.9, 124.6, 122.4, 121.5, 112.2, 79.2, 71.3, 56.0, 54.4, 48.2, 46.4, 44.0, 42.7, 42.1, 39.5, 36.5, 35.2, 28.3, 24.9, 24.5, 22.9, 22.8, 22.7, 21.3, 17.2; IR (CHCl₃) 3425, 2970, 2934, 2874, 1746, 1711, 1684, 1604, 1505, 1442, 1394, 1368, 1305, 1258, 1166, 1123, 1067, 1015, 971 cm⁻¹; Anal. (C₄₁H₅₅ClN₃O₉) C, H, N.

### Example 61

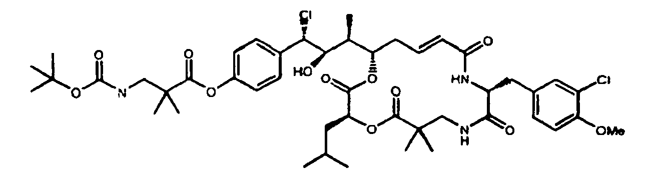

To the styrene **(shown above)** (0.276 g, 0.32 mmol) was added 12 mL of acetone, 6 mL of H₂O, 6 mL of CH₂Cl₂ and solid NaHCO₃ (0.84 g, 10 mmol) and the mixture was cooled to 0°C. A solution of Oxone (0.78 g, 1.27 mmol) in 6 mL of H₂O was prepared and added (2 mL) to the cold styrene mixture. Following 30 min of vigorous stirring at 0 °C an additional 2 mL of Oxone solution was added and again another 2.0 mL was added, following another 30 min, for a total of 6 mL of Oxone solution. The reaction progress was monitored by reverse phase HPLC and was found to be complete after 2.5 hrs of stirring. While still at 0 °C, the reaction was quenched with saturated aqueous NaHCO₃ (50 mL) and an additional 50 mL of CH₂Cl₂ was also added. The layers were separated and the organic layer was washed with aq. 10% Na₂SO₃ (50 mL), followed by saturated aq. NaHCO₃ (50 mL) then brine and finally was dried over Na₂SO₄, filtered and concentrated in vacuo to give 0.272 g of the crude epoxides as a yellow foam.

To a solution of the epoxides in 4 mL of CH₂Cl₂ at -60 °C was added trimethylsilyl chloride (0.2 mL, 1.54 mmol). After 3 hours at -60 °C, 5mL of 0.1 N HCl was added to hydrolyze any trimethylsilyl ether and the mixture warmed to room temperature. The layers were separated and the organic layer was dried over MgSO₄, filtered and concentrated in vacuo. The crude chlorohydrins were purified twice by radial PLC (silica gel, 50-60-70-100% EtOAc/hexanes) and finally by reverse-phase HPLC (CH₃CN/H₂O) to give the product **(shown above)** (0.090 g, 31%) as a white solid: [α]²⁰_{D} + 42.7 ° (*c* 3.0, CHCl₃); ¹H NMR (300 MHz, CDCl₃) δ 7.48-7.44 (d, 1 H, *J* = 8.4 Hz), 7.31-7.26 (m, 4 H), 7.16-7.10 (m, 3 H), 6.92-6.89 (d, 1 H, *J =* 8.4 Hz), 6.87-6.81 (m, 1 H), 5.84-5.79 (d, 1 H, *J* = 15.1 Hz), 5.58-5.55 (d, 1 H, *J* = 7.8 Hz), 5.24-5.19 (t, 1 H, *J* = 8.9 Hz), 5.05-4.95 (m, 2 H), 4.79-4.75 ( m, 1 H), 4.72-4.69 (d, 1 H, *J* = 9.5 Hz), 4.04-4.01 (dd, 1 H, *J =* 1.3, 9.3 Hz), 3.93 (s, 3 H), 3.47-3.40 (m, 3 H), 3.25-3.06 (m, 3 H), 2.75-2.70 (d, 1 H, *J* = 13.9 Hz), 2.55-2.41 (m, 2 H), 1.90-1.71 (m, 2 H), 1.63 (s, 1 H), 1.48 (s, 9 H), 1.39 (s, 6 H), 1.28 (s, 3 H), 1.22 (s, 3 H), 1.09-1.06 (d, 3 H, *J =* 6.9 Hz), 1.0-0.96 (m, 6 H); ¹³C NMR (63 MHz, CDCl₃) δ 177.6, 170.4, 170.2, 165.2, 153.9, 142.3, 136.1, 130.8, 129.7, 129.1, 128.2, 124.5, 122.4, 121.9, 112.2, 76.1, 74.0, 71.1, 61.5, 56.5, 54.4, 46.4, 44.1, 42.7, 39.6, 38.4, 36.3, 35.2, 28.3, 24.8, 22.9, 22.8, 22.7, 21.5, 8.6; IR (CHCl₃) 3417, 2974, 2934, 1755, 1720, 1677, 1505, 1473, 1368, 1320, 1258, 1205, 1167, 1153, 1123, 1066 cm⁻¹; FAB HRMS [M - BOC] calcd for (C₄₁H₅₆Cl₂N₃O₁₀) 820.3343, found 820.3354.

### Example 62

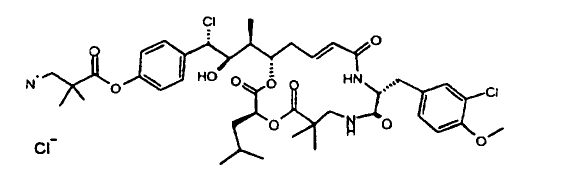

To the BOC protected amine **(shown above)** (0.070 g, 0.067 mmol) in CH₂Cl₂ (0.25 mL) was added a 4 M HCl solution (0.1 mL, 0.4 mmol) in 1,4-dioxane. Following 2 hrs of stirring at room temperature the solvents were removed under vacuum and the resulting residue was maintained under high vacuum for 2 days to give the product as a white solid (0.062 g) in 95% yield: [α]²⁰_{D}+ 27.68 ° (c 2.5, CHCl₃) ; ¹H NMR (300 MHz, CDCl₃) δ 7.79-7.76 (d, 1 H, J = 7.9 Hz), 7.48-7.38 (m, 2 H), 7.27-7.26 (d, 1 H, J = 1.2 Hz), 7.17-7.08 (m, 3 H), 6.98-6.95 (d, 1 H, *J* = 8.5 Hz), 6.71-6.60 (m, 1 H), 5.95-5.90 (d, 1 H, J = 15.2 Hz), 5.14-5.01 (m, 2 H), 4.50-4.46 (dd, 1 H, *J* = 3.0, 11.0 Hz), 3.99-3.96 (d, 1 H, *J* = 9.1 Hz), 3.82 (s, 3 H), 3.49-3.42 (m, 1 H), 3.19 (s, 2 H), 3.2-3.06 (m, 2 H), 2.77-2.68 (m, 2 H), 2.49-2.46 (t, 1 H, *J* = 6.8 Hz), 2.44-2.31 (q, 1 H, *J* = 11.4 Hz), 1.85-1.65 (m, 2 H), 1.60-1.50 (m, 1 H), 1.46 (s, 6 H), 1.20 (s, 3 H), 1.16 (s, 3 H), 1.08-0.94 (m, 9 H); ¹³C NMR (63 MHz, CDCl₃) δ 178.9, 175.6, 173.8, 171.9, 155.3, 151.8, 144.2, 139.5, 132.2, 131.5, 130.7, 129.4, 125.2, 123.3, 122.6, 113.5, 77.2, 74.8, 72.6, 63.2, 57.6, 56.7, 50.1, 48.3, 48.0, 47.4, 44.1, 42.7, 41.1, 40.4, 37.8, 36.5, 28.8, 26.2, 23.6, 23.4, 22.2, 9.0; IR (KBr) 3418, 2961, 2934, 1751, 1724, 1671, 1608, 1505, 1474, 1464, 1442, 1303, 1282, 1259, 1203, 1169, 1152, 1126, 1065, 1018; FAB HRMS [M - Cl] calcd for (C₄₁H₅₆Cl₂N₃O₁₀) 820.3343, found 820.3354.

### Example 63

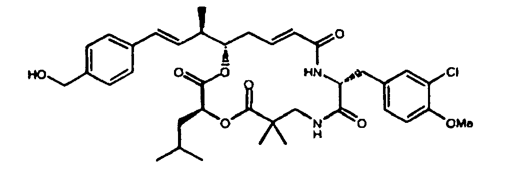

Tetrabutylammonium fluoride (TBAF) (4.0 mL, 4.1 mmol), as a 1.0 M solution in THF, was added dropwise to a -78 °C solution of protected alcohol **20** ( 3.1 g, 3.69 mmol) in 120 mL of THF. The solution was stirred at -78 °C for 10 min and the dry ice bath was removed allowing it to warm up to room temperature. Following 30 min at room temperature the reaction was quenched with water (80 mL) and ethyl acetate (100 mL). The layers were separated and the aqueous one was extracted with CH₂Cl₂ (3 x 50 mL). The combined organic layers were dried over anhydrous Na₂SO₄, filtered and concentrated in vacuo to yield the free alcohol.
Purification by column chromatography (silica gel, 50-70-100% EtOAc/hexanes) yielded 2.51 g (99%) of the pure alcohol **29** as a white solid: [α]²⁰_{D}⁺ 30.0 ° (c 1.0, CHCl₃); ¹H NMR (300 MHz, CDCl₃) δ 7.31 (s, 4 H), 7.26-7.21 (m, 1 H), 7.2-7.19 (d, 1 H, *J* = 1.8 Hz), 7.07-7.03 (dd, 1 H, *J* = 8.4, 1.7 Hz), 6.85-6.82 (d, 1 H, *J* = 8.4 Hz), 6.82-6.7 (m, 1 H), 6.43-6.37 (d, 1 H, *J =* 15.9 Hz), 6.05-5.97 (dd, 1 H, *J* = 15.9, 8.7 Hz), 5.77-5.72 (d, 1 H, *J =* 15.0 Hz), 5.58-5.55 (d, 1 H, *J =* 7.9 Hz), 5.08-5.02 (dd, 1 H, *J =* 9.4, 6.3 Hz), 4.87-4.83 (dd, 1 H, J = 10.2, 3.1 Hz), 4.8-4.67 (m, 1 H), 4.67 (s, 2 H), 3.87 (s, 3 H), 3.44-3.37 (dd, 1 H, *J =* 13.5, 8.5 Hz), 3.2-3.0 (m, 3H), 2.6-2.3 (m, 3 H), 1.8-1.6 (m, 3 H), 1.4-1.25 (m, 1 H), 1.22 (s, 3 H), 1.15 (s, 3 H), 1.14-1.12 (d, 3 H, J = 6.8 Hz), 0.76-0.73 (t, 6 H, J = 5.5 Hz) ; ¹³C NMR (63 MHz, CDCl₃) δ 177.7, 170.7, 170.5, 165.4, 153.8, 142.0, 140.5, 135.9, 131.3, 130.7, 130.0, 129.9, 128.1, 127.1, 126.1, 124.5, 122.2, 112.2, 77.0, 71.3, 64.6, 56.0, 54.6, 46.4, 42.7, 42.1, 39.4, 36.4, 35.2, 24.5, 22.8, 22.6, 21.2, 17.2; IR (CHCl₃) 3423, 3011, 2965, 2935, 2874, 2841, 1747, 1712, 1681, 1652, 1528, 1503, 1485, 1442, 1371, 1303, 1259, 1151 cm⁻¹; Anal. (C₃₇H₄₇ClN₂O₈) C, H, N.

### Example 64

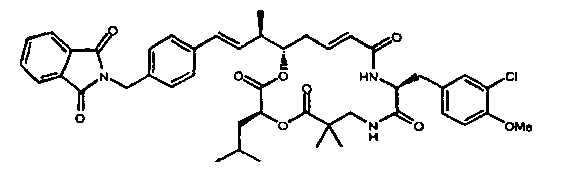

To a solution of alcohol **29** (0.13 g, 0.19 mmol) in 2.0 mL of THF, was added triphenyl phosphine (0.065 g, 0.25 mmol), phthalimide (0.037 g, 0.25 mmol) and finally diethyl azodicarboxylate (DEAD) (0.04 mL, 0.25 mmol). The resulting yellow solution was stirred at room temperature for 2 hrs and quenched with H₂O (10 mL) and CH₂Cl₂ (10 mL) . The aqueous layer was extracted with CH₂Cl₂ (2 x 10 mL) and the combined organic layers were dried over MgSO₄, filtered and concentrated in vacuo. The resulting residue was purified by radial PLC (silica gel, 50-60-70% EtOAc/hexanes) to give phthalimide **30** (0.14 g) as a white solid in 90% yield: [α]²⁰_{D}+ 19.2 ° (c 1.0, CHCl₃) ; ¹H NMR (300 MHz, CDCl₃) δ 7.9-7.8 (m, 2 H), 7.72-7.69 (m, 2 H), 7.39-7.36 (d, 2 H, J = 8.0 Hz), 7.28-7.26 (d, 2 H, J = 5.3 Hz), 7.25-7.2 (m, 1H), 7.19-7.18 (d, 1 H, *J* = 1.9 Hz), 7.06-7.03 (dd, 1 H, *J* = 8.5, 1.9 Hz), 6.85-6.82 (d, 1 H, J = 8.4 Hz), 6.81-6.7 (m, 1 H), 6.39-6.33 (d, 1 H, *J* = 15.9 Hz), 6.01-5.93 (dd, 1 H, *J* = 15.8, 8.8 Hz), 5.75-5.7 (d, 1 H, *J* = 15.4 Hz), 5.47-5.44 (d, 1 H, J = 7.9 Hz), 5.05-5.0 (dd, 1 H, *J* = 9.4, 6.3 Hz), 4.8 (s, 2 H), 4.83-4.7 (m, 2 H), 3.87 (s, 3 H), 3.4-3.36 (dd, 1 H, J = 13.4, 8.6 Hz), 3.18-3.02 (m, 3 H), 2.6-2.25 (m, 3 H), 1.65-1.5 (m, 2 H), 1.35-1.22 (m, 1 H), 1.21 (s, 3 H), 1.14 (s, 3 H), 1.12-1.09 (d, 3 H, *J* = 6.8 Hz), 0.71-0.69 (d, 3 H, *J* = 6.4 Hz), 0.65-0.63 (d, 3 H, *J* = 6.4 Hz); ¹³C NMR (63 MHz, CDCl₃) δ 177.8, 170.5, 170.4, 167.8, 165.2, 153.9, 142.0, 136.3, 135.6, 133.9, 131.9, 131.1, 130.7, 130.6, 129.8, 128.9, 128.6, 128.5, 128.1, 126.3, 124.6, 123.2, 122.3, 112.2, 76.9, 71.3, 56.0, 54.5, 46.4, 42.6, 42.1, 41.2, 39.4, 36.4, 35.2, 24.4, 22.8, 22.6, 22.5, 21.1, 17.2 ; IR (CHCl₃) 3421, 2967, 2935, 2873, 2840, 1747, 1716, 1682, 1527, 1503, 1485, 1433, 1395, 1259, 1151.

### Example 65

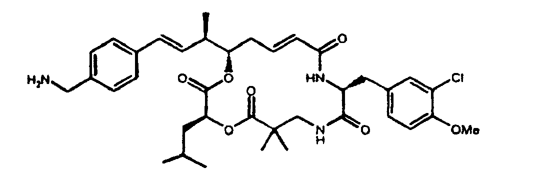

To phthalimide **30** (0.1 g, 0.123 mmol) in 1.8 mL of EtOH, was added n-butylamine (0.04 mL, 0.369 mmol). The solution was heated at 75 °C for 2 days, concentrated in vacuo and purified by radial PLC (silica gel, 10-25% MeOH/CH₂Cl₂) to provide the free amine **31** (0.048 g) in 57% yield.

### Example 66

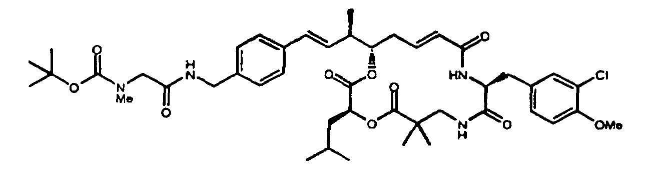

To *N*-(tert-butoxycarbonyl)sarcosine (0.07 g, 0.37 mmol) in 1.5 mL of DMF was added 1-hydroxybenzotriazole hydrate (HOBT) (0.05 g, 0.37 mmol) and 1-(3-dimethylaminopropyl)-3-ethylcarbodiimide hydrochloride (EDCI) (0.071 g, 0.37 mmol). Following 45 min of stirring at room temperature, amine **31** (0.17 g, 0.25 mmol) in 2.5 mL of DMF was added to the solution dropwise via a double-tipped needle. The resulting mixture was stirred for an additional 3 hrs, quenched with H₂O (10 mL) and extracted with CH₂Cl₂ (3 x 10 mL). The combined organic extracts were dried over MgSO4, filtered and concentrated in vacuo. Purification of the resulting crude product by radial PLC (silica gel, 70-80-100% EtOAc/hexanes) gave the desired amide **32** (0.15 g) in 71% yield as a white solid: [α]²⁰_{D}+ 22.4 ° (c 1.0, CHCl₃); ¹H NMR (300 MHz, CDCl₃) δ 7.27-7.1 (m, 6 H), 7.04-7.0 (dd, 1 H, *J* = 8.5, 1.9 Hz), 6.82-6.79 (d, 1 H, *J* = 8.5 Hz), 6.79-6.65 ( m, 1 H), 6.38-6.32 (d, 1 H, *J* = 15.9 Hz), 6.3-6.2 (bs, 1 H), 6.01-5.93 (dd, 1 H, *J =* 15.9, 8.7 Hz), 5.75-5.70 (d, 1 H, *J =* 15.0 Hz), 5.65-5.6 (m, 1 H), 5.0-4.99 (dd, 1 H, *J* = 9.3, 6.1 Hz), 4.84-4.79 (dd, 1 H, *J* = 9.6, 3.6 Hz), 4.74-4.67 (m, 1 H), 4.42-4.4 (d, 2 H, *J* = 5.7 Hz), 3.87 (s, 2 H), 3.84 (s, 3 H), 3.42-3.34 (dd, 1 H, *J* = 13.5, 8.6 Hz), 3.15-3.0 (m, 3 H), 2.9 (s, 3 H), 2.6-2.25 (m, 3 H), 1.8-1.5 (m, 2 H), 1.4 (s, 9 H), 1.39-1.25 (m, 1 H), 1.19 (s, 3 H), 1.12 (s, 3 H), 1.1-1.08 (d, 3 H, *J* = 6.8 Hz), 0.73-0.72 (d, 3 H, *J* = 4.4 Hz), 0.71-0.69 (d, 3 H, *J* = 4.3 Hz); ¹³C NMR (63 MHz, CDCl₃,) δ 177.7, 170.5, 170.4, 169.2, 165.2, 153.9, 141.9, 137.3, 136.0, 131.1, 130.7, 130.3, 129.8, 128.6, 128.1, 127.7, 126.3, 124.6, 122.3, 112.2, 80.6, 76.9, 71.3, 56.0, 54.5, 53.1, 46.4, 42.8, 42.7, 42.0, 39.4, 36.4, 35.8, 35.2, 28.2, 24.5, 22.8, 22.6, 21.2, 17.1; IR (CHCl₃) 3427, 2967, 2935, 2874, 2841, 1747, 1680, 1526, 1504, 1484, 1464, 1442, 1393, 1369, 1302, 1281, 1259, 1151, 1067

### Example 67

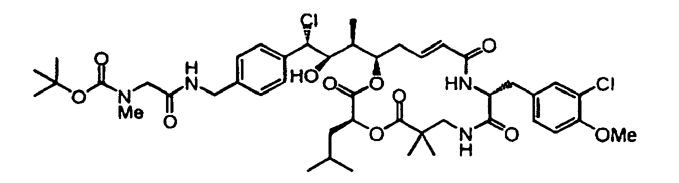

Amide **32** (0.34 g, 0.398 mmol) was epoxidized using mCPBA (0.072 g, 0.42 mmol) in 1.2 mL of CH₂Cl₂ according to previously described procedure to give the b and a epoxides in a 2:1 ratio. The resulting crude mixture of epoxides (0.3 g, 0.345 mmol) was dissolved in CHCl₃ and cooled to -60 °C. TMSC1 (0.22 mL, 1.73 mmol) was added and the solution was stirred between -50 °C and -20 °C for 2 hrs. More TMSCl (0.44 mL, 0.173 mmol) was added and the solution was allowed to warm up to room temperature. The solution was concentrated in vacuo and the resulting product was purified twice by column chromatography (70-80% EtOAc/hexanes) and twice by radial PLC (silica gel, 2-5% MeOH/CH₂Cl₂) to give the trans chlorohydrin **34** (0.1 g) in 48% yield as a white solid: [α]²⁰_{D}+ 46.9 ° (c 0.85, CHCl₃); ¹H NMR (300 MHz, CDCl₃) δ 7.36-7.34 (d, 2 H, *J* = 7.9 Hz), 7.29-7.26 (d, 2 H, *J* = 8.3 Hz), 7.21 (s, 2 H), 7.08-7.05 (d, 1 H, *J = 8.3* Hz), 6.86-6.83 (d, 1 H, *J =* 8.5 Hz), 6.8-6.7 (m, 1 H), 6.5-6.2 (bs, 1 H), 5.79-5.74 (d, 1 H, *J =* 15.2 Hz), 5.64-5.62 (d, 1 H, J = 7.7 Hz), 5.18-5.12 (t, 1 H, *J* = 9.1 Hz), 4.94-4.9 (dd, 1 H, *J* = 9.9, 3.5 Hz), 4.8-4.67 (m, 1 H), 9.66-4.63 (d, 1 H, *J =* 9.6 Hz), 4.47-4.45 (d, 2 *H, J* = 5.3 Hz), 4.02-3.98 (d, 1 H, *J* = 9.5 Hz), 3.89 (s, 2 H), 3.88 (s, 3 H), 3.41-3.34 (dd, 1 H, *J =* 13.6, 8.5 Hz), 3.2-3.0 (m, 3 H), 2.94 (s, 3 H), 2.69-2.68 (bdd, 1 H, J = 14.3, 2.1 Hz), 2.51-2.3 (m, 2 H), 1.8-1.6 (m, 3 H), 1.42 (s, 10 H), 1.22 (s, 3 H), 1.17 (s, 3 H), 1.03-1.01 (d, 3 H, J = 6.9 Hz), 0.94-0.9 (t, 6 H, *J =* 5.5 Hz); ¹³C NMR (63 MHz, CDCl₃) δ 177.6, 170.4, 170.2, 165.2, 153.9, 142.4, 139.1, 130.8, 129.7, 128.3, 128.2, 127.9, 124.4, 122.4, 112.2, 80.7, 76.1, 73.9, 71.2, 61.8, 56.1, 54.4, 53.1, 46.4, 42.7, 39.6, 38.4, 36.3, 35.9, 35.2, 28.2, 24.8, 23.0, 22.9, 22.7, 21.5, 8.5 ; IR (KBr) 3419, 3317, 2964, 2932, 1755, 1670, 1538, 1504, 1473, 1392, 1368, 1301, 1258, 1151, 1066; FAB HRMS [M + H] calcd for (C₄₅H₆₂Cl₂N₄O₁₁) 905.3870, found 905.3876.

### Example 68

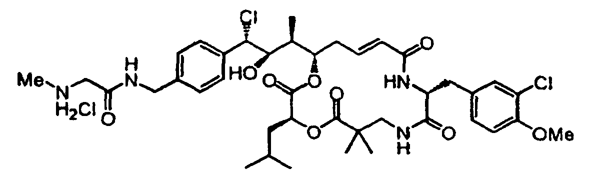

The hydrochloride salt **35** (0.041 g) of BOC protected amine **34** (0.045 g, 0.05 mmol) was prepared in quantitative yield according to the previously described procedure using 4 M HCl in 1,4-dioxane: ¹H NMR (300 MHz, MeOD) δ 8.47-8.45 (d, 1 H, *J* = 7.7 Hz), 7.79-7.76 (d, 1 H, *J =* 8.9 Hz), 7.39-7.36 (d, 2 H, *J* = 8.1 Hz), 7.3-7.27 (d, 3 H, *J* = 9.0 Hz), 7.17-7.14 (d, 1 H, *J* = 8.5 Hz), 6.98-6.95 (d, 1 H, *J* = 8.5 Hz), 6.75-6.6 (m, 1 H), 5.94-5.89 (d, 1 H, *J* = 15.1 Hz), 5.2-5.0 (m, 2 H), 4.78-4.75 (d, 1 *H, J* = 9.4 Hz), 4.5-4.42 (m, 1 H), 4.41 (s, 2 H), 4.01-3.98 (d, 1 H, *J* = 9.5 Hz), 3.82 (s, 3 H), 3.8 (s, 2 H), 3.5-3.4 (m,1 H), 3.19-3.13 (dd, 1 H, *J* = 14.4, 3.4 Hz), 3.11-3.06 (dd, 1 H, *J* = 13.2, 1.9 Hz), 2.8-2.6 (m, 2 H), 2.7 (s, 3 H), 2.5-2.2 (m, 2 H), 1.85-1.45 (m, 3 H), 1.3-1.2 (m, 1 H), 1.2 (s, 3 H), 1.15 (s, 3 H), 1.0-0.94 (q, 9 H, *J* = 11.3, 6.0 Hz); ¹³C NMR (63 MHz, CDCl₃) δ 178.9, 173.7, 171.9, 168.3, 166.3, 155.4, 144.2, 140.5, 139.7, 132.2, 131.5, 129.7, 129.4, 128.8, 125.2, 123.3, 113.5, 77.2, 74.7, 72.6, 63.7, 57.6, 56.6, 50.7, 47.4, 44.1, 43.9, 41.1, 40.4, 37.8, 36.5, 33.7, 26.2, 23.6, 23.4, 22.1, 9.0 ; IR (KBr) 3410, 3058, 2961, 2933, 1752, 1721, 1675, 1539, 1504, 1463, 1440, 1282, 1259, 1196, 1154, 1127, 1066

### Example 69

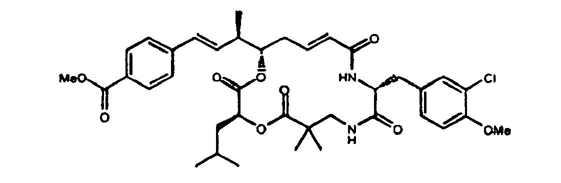

A suspension of sodium hydride (60% suspension) (0.041 g, 1.0 mmol) and 4-carbomethoxybenzyl triphenylphosphonium bromide (0.5 g, 1.0 mmol) in 10 mL of THF was heated at 65 °C for 1 h and cooled back to room temperature. This orange mixture was added dropwise to aldehyde **18** (0.46 g, 0.79 mmol) in THF (10 mL) at -78 °C. The resulting solution was warmed up to room temperature, stirred an additional 2 hrs and quenched with sat. NH₄Cl (30 mL) and EtOAc (30 mL). The layers were separated and the aqueous one was further extracted with EtOAc (2 x 20 mL). The combined organic extracts were washed with brine (30 mL), dried over MgSO₄, filtered and concentrated in vacuo. The crude styrene was purified by column chromatography (silica gel, 50-65% EtOAc/hexanes) to give clean styrene (0.129 g) in 23% yield: [α]²⁰_{D} + 29.7 ° (*c* 1.15, CHCl₃) ; ¹H NMR (300 MHz, CDCl₃) δ 8.03-8.0 (d, 2 H, *J =* 8.2 Hz), 7.44-7.41 (d, 2 H, *J* = 8.3 Hz), 7.3-7.25 (m, 1 H), 7.24-7.23 (d, 1 H, *J* = 1.6 Hz), 7.11-7.08 (dd, 1 H, *J* = 8.3, 1.9 Hz), 6.89-6.86 (d, 1 H, J = 8.5 Hz), 6.86-6.8 (m, 1 H), 6.52-6.46 (d, 1 H, *J* = 15.9 Hz), 6.23-6.15 (dd, 1 H, *J* = 15.8, 8.8 Hz), 5.83-5.8 (d, 1 H, *J =* 15.3 Hz), 5.64-5.61 (d, 1 H, *J =* 7.9 Hz), 5.14-5.09 (dd, 1 H, *J =* 9.4, 6.5 Hz), 4.91-4.87 (dd, 1 H, *J* = 10.2, 3.6 Hz), 4.85-4.75 (m, 1 H), 3.95 (s, 3 H), 3.91 (s, 3 H), 3.5-3.4 (dd, 1 H, *J* = 13.5, 8.7 Hz), 3.2-3.1 (m, 3 H), 2.9-2.3 (m, 3 H), 1.8-1.6 (m, 3 H), 1.4-1.3 (m, 1 H), 1.26 (s, 3 H), 1.2 (s, 3 H), 1.2-1.18 (d, 3 H, *J* = 6.9 Hz), 0.8-0.78 (d, 3 H, *J* = 5.6 Hz), 0.78-0.76 (d, 3 H, *J* = 6.0 Hz); ¹³C NMR (63 MHz, CDCl₃) δ 177.8, 170.4, 166.7, 165.1, 153.9, 141.8, 141.1, 133.1, 130.8, 129.9, 129.7, 128.9, 128.2, 125.9, 124.6, 122.4, 112.2, 76.8, 71.3, 56.0, 54.4, 52.0, 46.4, 42.7, 42.2, 39.5, 36.5, 35.2, 24.5, 22.8, 22.6, 21.2, 17.1; IR (CHCl₃) 3424, 2964, 2936, 2874, 2841, 1748, 1716, 1681, 1608, 1528, 1503, 1485, 1437, 1283, 1259 cm⁻¹; Anal. ( C₃₈H₄₇ClN₂O₉) C, H, N.

### Example 70

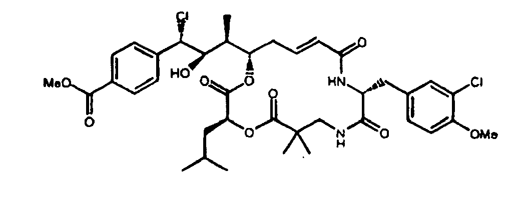

To styrene **(shown above)** (0.42 g, 0.59 mmol) was added 30 mL of acetone, 15 mL of H₂O, 15 mL of CH₂Cl₂ and solid NaHCO₃ (1.7 g, 20.2 mmol) and the mixture was cooled to 0°C. A solution of Oxone (1.4 g, 2.3 mmol) in 12 mL of H₂O was prepared and added (2 mL) to the cold styrene mixture. Following 30 min of vigorous stirring at 0 °C an additional 2 mL of Oxone solution was added and the solution was warmed up to room temperature. An additional 2 mL of Oxone solution was added every 2 hrs until the 12 mL was consumed. The reaction was stirred a total of 5 hrs and was quenched with saturated aqueous NaHCO₃ (50 mL) and 50 mL of CH₂Cl₂. The layers were separated and the organic layer was washed with aq. 10% Na₂SO₃ (50 mL), followed by saturated aq. NaHCO₃ (50 mL) then brine and finally was dried over Na₂SO₄, filtered and concentrated in vacuo. The crude mixture was purified by reverse phase HPLC (45:55) CH₃CN:H₂O to provide 0.14 g (33% yield) of the b and a epoxides and 0.14 g of a bisepoxidation mixture.
The mixture of b/a epoxides (0.14 g, 0.19 mmol) was dissolved in 3.0 mL of CHCl₃ and cooled to -60°C. Chlorotrimethyl silane (0.1 mL, 0.77 mmol) was added to the -60°C solution and the mixture was stirred for 1.5 h. More TMSCl (0.1mL, 0.77 mmol) was added and the solution was allowed to warm up to room temperature. Following 1 h of stirring at room temperature the solution was concentrated and purified by radial PLC (1-2% MeOH/CH₂Cl₂) to give 0.044 g (30% yield) of the desired chlorohydrin **(shown above)** as a white solid: [α]²⁰_{D} + 50.0 ° (c 0.75, CHCl₃) ; ¹H NMR (300 MHz, CDCl₃) δ 8.1-8.08 (d, 2 H, *J* = 8.1 Hz), 7.54-7.51 (d, 2 H, *J =* 8.2 Hz), 7.3-7.25 (m, 1 H), 7.25 (s, 1 H), 7.13-7.09 (dd, 1 H, *J =* 8.5, 1.5 Hz), 6.91-6.88 (d, 1 H, *J* = 8.3 Hz), 6.87-6.78 (m, 1 H), 5.86-5.8 (d, 1 H, *J* = 15.5 Hz), 5.7-5.6 (m, 1 H), 5.24-5.18 (t, 1 H, J = 9.2 Hz), 4.99-4.95 (dd, 1 H, *J* = 10.0, 3.6 Hz), 4.8-4.7 (m, 1 H), 4.76-4.73 (d, 1 H, *J* = 9.5 Hz), 4.09-4.06 (d, 1 H, *J* = 9.6 Hz), 3.97 (s, 3 H), 3.93 (s, 3 H), 3.45-3.38 (dd, 1 H, *J* = 13.6, 8.6 Hz), 3.25-3.0 (m, 3 H), 2.75-2.62 (m, 1 H), 2.6-2.4 (m, 2 H), 1.9-1.6 (m, 3 H), 1.5-1.4 (m, 1 H), 1.27 (s, 3 H), 1.22 (s, 3 H), 1.1-1.07 (d, 3 H, *J* = 6.95 Hz), 0.98-0.95 (t, 6 H, *J* = 5.3 Hz); ¹³C NMR (63 MHz, CDCl₃) δ 177.5, 170.6, 170.3, 166.3, 165.4, 153.9, 143.8, 142.2, 138.7, 130.7, 130.4, 129.9, 128.1, 128.08, 124.6, 122.3, 112.2, 76.1, 73.8, 71.1, 61.4, 56.0, 54.6, 52.2, 46.4, 42.7, 39.6, 38.4, 36.3, 35.1, 24.8, 23.0, 22.9, 22.7, 21.5, 8.7; IR (CHCl₃) 3425, 2962, 2935, 2873, 2842, 1750, 1720, 1680, 1528, 1504, 1484, 1438, 1284, 1259, 1194, 1152, 1114, 1067 cm⁻¹; Anal. ( C₃₈H₄₈Cl₂N₂O₁₀) C, H, N.

### Example 71

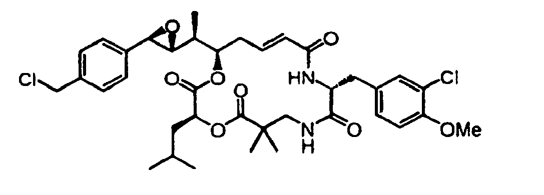

To a 0 °C solution of alcohol **23** (0.32 g, 0.46 mmol) in 8.5 mL of CH₂Cl₂ was added solid NaHCO₃ (0.19 g, 2.29 mmol), triphenyl phosphine (0.18 g, 0.69 mmol) and finally N-chlorosuccinimide (0.092 g, 0.69 mmol). The mixture was stirred at 0 °C for 20 min and quenched with sat. aq. NaHCO₃ (20 mL). The layers were separated and the aqueous was extracted with CH₂Cl₂ (3 x 10 mL). The combined organic layers were washed with brine, dried over Na₂SO₄, filtered and concentrated in vacuo. The crude yellow solid was purified by radial PLC (silica gel, 20-50% EtOAC/hexanes) to give 0.26 g of the benzyl chloride **36** in a 79% yield as a white solid: [α]²⁰_{D}+ 25.6 ° (c 0.9, CHCl₃); ¹H NMR (300 MHz, CDCl₃,) δ 7.4-7.38 (d, 2 H, *J* = 7.9 Hz), 7.26-7.23 (d, 3 H, *J* = 8.3 Hz), 7.19-7.18 (d, 1 H, *J =* 1.9 Hz), 7.06-7.03 (dd, 1 H, *J* = 8.3, 1.9 Hz), 6.85-6.82 (d, 1 H, *J =* 8.4 Hz), 6.8-6.7 (m, 1 H), 5.74-5.69 (d, 1 H, J = 15.4 Hz), 5.49-5.47 (d, 1 H, *J* = 7.8 Hz), 5.22-5.17 (m, 1 H), 4.85-4.8 (dd, 1 H, *J* = 9.7, 3.0 Hz), 4.78-4.7 (m, 1 H), 4.6 (s, 2 H), 3.9 (s, 3 H), 3.69-3.68 (d, 1 H, *J =* 1.3 Hz), 3.45-3.38 (dd, 1 *H, J* = 13.4, 8.6 Hz), 3.2-3.0 (m, 3 H), 2.92-2.89 (dd, 1 H, *J =* 7.6, 1.6 Hz), 2.6-2.4 (m, 2 H), 1.8-1.6 (m, 3 H), 1.4-1.3 (m, 1 H), 1.22 (s, 3 H), 1.16 (s, 3 H), 1.16-1.13 (d, 3 H, *J* = 8.6 Hz), 0.86-0.82 (t, 6 H, *J* = 6.8 Hz); ¹³C NMR (63 MHz, CDCl₃) δ 177.8, 175.1, 170.3, 164.9, 154.0, 141.6, 137.7, 137.1, 130.8, 129.5, 128.9, 128.1, 125.9, 124.6, 122.4, 112.3, 77.2, 75.8, 71.1, 63.1, 58.5, 56.1, 54.4, 46.4, 45.7, 42.7, 40.5, 39.3, 36.8, 35.2, 24.5, 22.8, 22.6, 21.2, 13.5; IR (CHCl₃) 3416, 3284, 2961, 2933, 2873, 2839, 1752, 1721, 1680, 1658, 1536, 1504, 1473, 1442, 1321, 1302, 1281, 1259, 1192, 1150, 1126, 1066 cm⁻¹; Anal. (C₃₇H₄₆Cl₂N₂O₈) C, H, N.

### Example 72

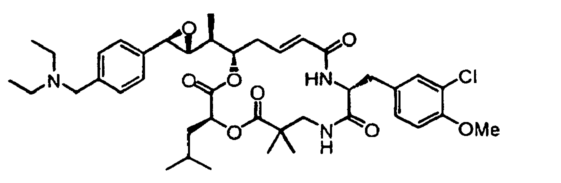

Diethyl amine (0.09 mL, 0.84 mmol) was added to benzyl chloride **36** (0.03 g, 0.042 mmol) in 0.3 mL of THF. The mixture was stirred overnight at room temperature and quenched with sat. aq. NaHCO₃ (5 mL). The aqueous layer was extracted with CH₂Cl₂ (3 x 5 mL). The combined organic layers were washed with brine, dried over Na₂SO₄, filtered and concentrated in vacuo. The crude yellow solid was purified by radial PLC (silica gel, 50-70-80% EtOAC/hexanes) to give 0.026 g of the amine 37 in a 82% yield as a white solid: [α]²⁰_{D}+ 25.9 ° (c 0.9, CHCl₃) ; ¹H NMR (300 MHz, CDCl₃) δ 7.35-7.32 (d, 2 H, *J* = 7.9 Hz), 7.25-7.12 (m, 4 H), 7.06-7.02 (dd, 1 H, *J* = 8.4, 1.6 Hz), 6.84-6.82 (d, 1 H, *J* = 8.5 Hz), 6.82-6.7 (m, 1 H), 5.74-5.69 (d, 1 H, *J* = 15.2 Hz), 5.57-5.55 (d, 1 H, *J* = 7.8 Hz), 5.22-5.17 (m, 1 H), 4.85-4.7 (m, 2 H), 3.86 (s, 3 H), 3.66 (s, 1 H), 3.57 (s, 2 H), 3.46-3.38 (dd, 1 H, *J* = 13.4, 8.7 Hz), 3.2-3.0 (m, 3 H), 2.93-2.91 (d, 1 H, *J* = 7.4 Hz), 2.6-2.4 (m, 6 H), 1.8-1.6 (m, 3 H), 1.4-1.3 (m, 1 H), 1.22 (s, 3 H), 1.15 (s, 3 H), 1.15-1.12 (d, 3 H, *J* = 9.2 Hz), 1.07-1.03 (t, 6 H, *J* = 7.1 Hz), 0.86-0.82 (t, 6 H, J = 6.3 Hz); ¹³C NMR (63 MHz, CDCl₃) δ 177.9, 170.4, 170.3, 164.9, 154.0, 141.7, 140.1, 135.2, 130.8, 129.6, 129.1, 128.1, 125.4, 124.6, 122.4, 112.3, 77.2, 75.8, 71.1, 62.9, 58.9, 57.1, 56.0, 54.4, 46.6, 46.4, 42.7, 40.6, 39.3, 36.8, 35.2, 24.5, 22.8, 22.6, 21.2, 13.5, 11.5; IR (CHCl₃) 3424, 2969, 2936, 2874, 1752, 1711, 1682, 1605, 1527, 1503, 1485, 1303, 1259, 1190, 1151, 1067 cm⁻¹; Anal. (C₄₁H₅₆ClN₃O₈) C, H, N.

### Example 73

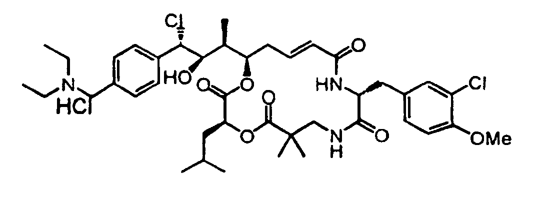

To a -66 °C solution of epoxide **37** (0.05 g, 0.066 mmol) in 0.8 mL of CHCl₃, was added dropwise a 4 M solution of HCl in 1,4-dioxane (0.04 mL, 0.166 mL). The mixture was stirred at -66 °C for 10 min upon which the dry ice bath was removed allowing the solution to slowly warm up to room temperature. The solvents were removed in vacuo and the resulting salt was placed under high vacuum for 3 days to remove residual dioxane hence yielding 0.054 g of the desired chlorohydin 38 exclusively in quantitative yield: [α]²⁰_{D}+ 29.3 ° (c 1.0, MeOH ); ¹H NMR (300 MHz, MeOD) δ 8.5-8.47 (d, 1 H, *J* = 7.5 Hz), 7.79-7.76 (d, 1 H, *J* = 8.8 Hz), 7.53 (s, 4 H), 7.26-7.25 (d, 1 H, *J* = 1.6 Hz), 7.17-7.14 (dd, 1 H, *J* = 8.6, 1.6 Hz), 6.97-6.94 (d, 1 H, *J* = 8.4 Hz), 6.75-6.6 (m, 1 H), 5.96-5.90 (d, 1 H, *J* = 15.3 Hz), 5.2-5.0 (m, 2 H), 4.85-4.82 (d, 1 H, *J =* 8.9 Hz), 4.5-4.4 (m, 1 H), 4.33 (s, 2 H), 4.02-3.98 (d, 1 H, *J =* 9.3 Hz), 3.8 (s, 3 H), 3.49-3.42 (dd, 1 H, *J* = 13.3, 9.9 Hz), 3.2-3.0 (m, 6 H), 2.8-2.6 (m, 2 H), 2.5-2.2 (m, 2 H), 1.8-1.5 (m, 3 H), 1.34-1.3 (m, 7 H), 1.2 (s, 3 H), 1.15 (s, 3 H), 1.01-0.99 (d, 3 H, *J =* 7.2 Hz), 0.98-0.94 (t, 6 H, *J* = 5.4 Hz); ¹³C NMR (63 MHz, CDCl₃) δ 178.9, 173.8, 171.8, 168.3, 155.4, 144.1, 143.4, 132.3, 132.2, 131.5, 131.1, 130.4, 129.4, 125.2, 123.3, 113.5, 77.2, 74.8, 72.6, 63.2, 57.6, 56.7, 56.66, 48.0, 47.5, 44.1, 41.1, 40.4, 37.8, 36.5, 26.2, 23.6, 23.4, 22.2, 9.07, 9.0; IR (KBr) 3414, 2960, 2934, 1751, 1721, 1671, 1521, 1504, 1463, 1443, 1259, 1197, 1155, 1127, 1065 cm⁻¹.

### Example 74

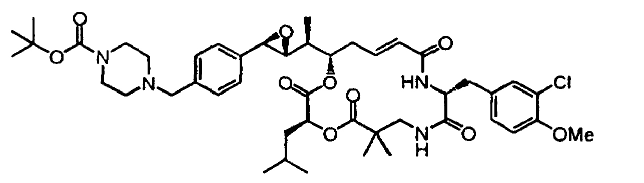

The epoxide **(shown above)** (0.147 g) was prepared in 81% yield, according to the procedure described above from benzyl chloride **36** (0.15 g, 0.21 mmol) and *N*-(tert-butoxycarbonyl)piperazine (0.195 g, 1.05 mmol): [α]²⁰_{D} + 25.4 ° (c 0.65, CHCl₃); ¹H NMR (300 MHz, CDCl₃) δ 7.37-7.23 (m, 5 H), 7.11-7.08 (d, 1 H, J = 8.6 Hz), 6.9-6.87 (d, 1H, *J* = 8.5 Hz), 6.86-6.72 (m, 1 H), 5.78-5.73 (d, 1 H, J =15.2 Hz), 5.53-5.5 (d, 1 H, J = 7.7 Hz), 5.28-5.23 (m, 1 H), 4.88-4.70 (m, 2 H), 3.9 (s, 3 H), 3.7 (s, 2 H), 3.54 (s, 2 H), 3.5-3.4 (m, 5 H), 3.2-3.05 (m, 3 H), 3.0-2.95 (d, 1 H, J = 7.4 Hz), 2.65-2.4 (m, 6 H), 1.85-1.6 (m, 3 H), 1.5 (s, 9 H), 1.45-1.4 (m, 1 H), 1.27 (s, 3 H), 1.2 (s, 3 H), 1.2-1.18 (d, 3 H, J = 8.3 Hz), 0.91-0.87 (t, 6 H, J = 6.1 Hz)); ¹³C NMR (63 MHz, CDCl₃) δ 177.9, 170.3, 170.26, 164.9, 154.7, 154.0, 141.7, 138.4, 135.6, 130.8, 129.5, 129.3, 128.1, 125.5, 124.6, 122.4, 112.3, 79.5, 77.2, 75.8, 71.1, 62.9, 62.6, 58.8, 56.1, 54.4, 52.8, 46.3, 42.7, 40.6, 39.3, 36.7, 35.2, 28.3, 24.5, 22.8, 22.6, 21.2, 13.4; IR (CHCl₃) 3425, 3008, 2965, 2937, 2874, 2817, 1752, 1709, 1683, 1527, 1484, 1463, 1459, 1427, 1367, 1259, 1167, 1150 cm⁻¹; FAB HRMS [M - BOC] calcd for (C₄₆H₆₄ClN₄O₁₀) 867.4311, found 867.4300.

### Example 75

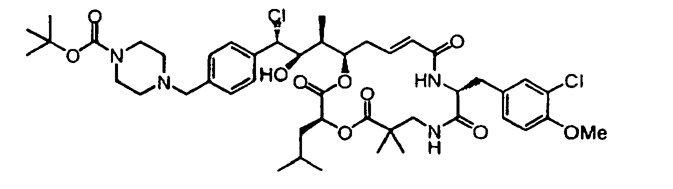

To a -66 °C solution of the epoxide **(shown above)** (0.135 g, 0.156 mmol) in 3.0 mL of CHCl₃, was added dropwise trimethylsilyl chloride (0.16 mL, 1.2 mL). The mixture was stirred at -66 °C for 2 hrs and additional TMSCl was added (0.16 mL, 1.2 mL). Following another 1 h at -66 °C the ice bath was removed allowing the solution to slowly warm up to room temperature. The solvents were removed in vacuo and the resulting solid was purified by radial PLC (silica gel, 2-5% MeOH/CH₂Cl₂) to give 0.13 g of the chlorohydrin **(shown above)** in 92% yield: [α]²⁰_{D} + 50.0 ° *(c* 1.0, CHCl₃ ); ¹H NMR (300 MHz, CDCl₃) δ 7.34 (s, 4 H), 7.21-7.2 (d, 2 H, *J* = 1.4 Hz), 7.08-7.05 (dd, 1 H, *J* = 8.6, 1.6 Hz), 6.86-6.83 (d, 1 H, *J* = 8.4 Hz), 6.82-6.7 (m, 1 H), 5.8-5.75 (d, 1 H, *J* = 15.1 Hz), 5.65-5.62 (d, 1 H, *J =* 7.8 Hz), 5.2-5.1 (m, 1 H), 5.0-4.7 (m, 2 H), 4.66-4.63 (d, 1 H, J = 9.7 Hz), 4.02-4.0 (d, 1 H, *J =* 9.6 Hz), 3.88 (s, 3 H), 3.49-3.48 (d, 2 H, *J* = 4.2 Hz), 3.45-3.3 (m, 5 H), 3.2-3.0 (m, 3 H), 2.7-2.3 (m, 7 H), 1.8-1.6 (m, 3 H), 1.45 (s, 10 H), 1.23 (s, 3 H), 1.17 (s, 3 H), 1.04-1.02 (d, 3 H, J = 6.9 Hz), 0.93-0.91 (d, 6 H, *J* = 6.3 Hz); ¹³C NMR (63 MHz, CDCl₃) δ 177.5, 170.5, 170.2, 165.2, 154.6, 153.9, 142.3, 139.1, 137.3, 130.8, 129.8, 129.5, 128.1, 127.9, 124.5, 122.3, 112.2, 79.5, 76.0, 73.9, 71.1, 62.4, 61.9, 56.1, 54.5, 52.8, 46.4, 42.7, 39.6, 38.4, 36.4, 35.2, 28.3, 24.7, 23.0, 22.9, 22.7, 21.5, 8.6; IR (CHCl₃) 3424, 3007, 2966, 2936, 2872, 2820, 1751, 1712, 1682, 1528, 1504, 1483, 1426, 1367, 1259, 1168, 1150, 1127, 1067, 1006 cm⁻¹; Anal. (C₄₆H₆₄Cl₂N₄O₁₀) C, H, N.

### Example 76

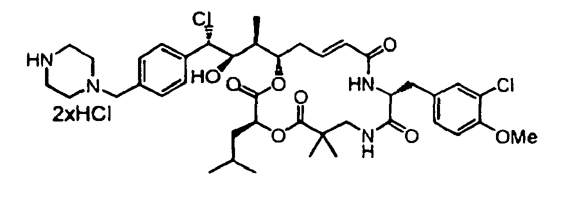

The dihydrochloride salts (0.116 g ) of BOC protected piperazine (0.122 g, 0.13 mmol) were prepared in quantitative yield according to the previously described procedure using 4 M HCl in 1,4-dioxane (0.32 ml, 1.3 mmol): [α]²⁰_{D} + 26.3 ° (c 0.7, MeOH ); ¹H NMR (300 MHz, MeOD) δ 8.47-8.45 (d, 1 H, *J* = 7.5 Hz), 7.78-7.75 (d, 1 *H, J* = 9.2 Hz), 7.6-7.52 (q, 4 H, *J* = 16.9, 7.9 Hz), 7.27-7.26 (d, 1 *H, J* = 1.15 Hz), 7.18-7.14 (dd, 1 H, *J* = 8.6, 1.8 Hz), 6.98-6.95 (d, 1 H, *J =* 8.4 Hz), 6.75-6.6 (m, 1 H), 5.95-5.9 (d, 1 H, *J* = 15.4 Hz), 5.2-5.0 (m, 2 H), 4.85-4.82 (d, 1 H, *J =* 9.9 Hz), 4.5-4.4 (m, 1 H), 4.4 (s, 2 H), 4.0-3.98 (d, 1 H, *J* = 9.3 Hz), 3.8 (s, 3 H), 3.6-3.4 (m, 9 H), 3.32-3.29 (d, 1 H, *J =* 11.3 Hz), 3.19-3.13 (dd, 1 H, J = 14.8, 3.5 Hz), 3.1-3.06 (d, 1 H, *J =* 13.7 Hz), 2.8-2.6 (m, 2 H), 2.5-2.3 (m, 2 H), 1.85-1.5 (m, 3 H), 1.3-1.2 (m, 1H), 1.2 (s, 3 H), 1.15 (s, 3 H), 1.02-0.95 (q, 9 H, *J =* 13.4, 6.3 Hz); ¹³C NMR (63 MHz, CDCl₃) δ 178.8, 173.7, 171.9, 168.3, 155.4, 144.2, 143.7, 132.8, 132.2, 131.5, 130.4, 129.9, 129.4, 125.2, 123.3, 113.5, 77.2, 74.8, 72.6, 63.1, 61.2, 57.6, 56.7, 49.2, 47.5, 44.1, 42.1, 41.1, 40.3, 37.8, 36.5, 26.3, 23.7, 23.4, 22.2, 9.0; IR (KBr) 3415, 2960, 2933, 2455, 1749, 1721, 1671, 1504, 1475, 1442, 1304, 1258, 1197, 1152, 1126, 1065, 1012 cm⁻¹; FAB HRMS [M - HCl₂] calcd for (C₄₁H₅₇Cl₂N₄O₈) 803.3553, found 803.3563.

### Example 77

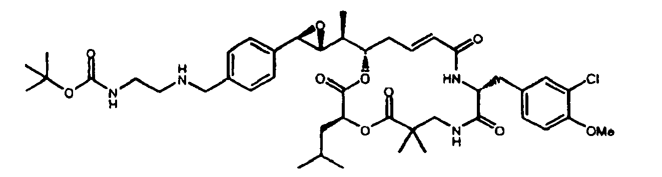

Epoxide **(shown above)** (0.15 g) was prepared in 78% yield, according to the procedure described above from benzyl chloride 36 (0.16 g, 0.22 mmol) and tert-butyl-N-(2-aminoethyl)carbamate (0.35 g, 2.22 mmol): [α]²⁰_{D} + 22.3 ° (c 1.0, CHCl₃) ; ¹H NMR (300 MHz, CDCl₃) δ 7.41-7.38 (d, 2 H, J = 7.8 Hz), 7.31 (s, 1 H), 7.26-7.24 (d, 3 H, J = 8.0 Hz), 7.11-7.08 (dd, 1 H, *J* = 8.4, 1.7 Hz), 6.88-6.86 (d, 1 H, *J* = 8.4 Hz), 6.86-6.72 (m, 1 H), 5.88-5.8 (bs, 1 H), 5.78-5.73 (d, 1 H, J = 15.2 Hz), 5.28-5.22 (m, 1 H), 5.2-5.08 (bs, 1 H), 4.95-4.7 (m, 2 H), 3.91 (s, 3 H), 3.87 (s, 2 H), 3.7 (s, 1 H), 3.45-3.38 (dd, 1 H, *J* = 13.4, 8.4 Hz), 3.35-3.0 (m, 5 H), 2.96-2.93 (dd, 1 H, *J =* 7.5, 1.2 Hz), 2.89-2.78 (m, 2 H), 2.65-2.4 (m, 2 H), 1.85-1.65 (m, 3 H), 1.49 (s, 9 H), 1.48-1.3 (m, 1 H), 1.27 (s, 3 H), 1.2 (s, 3 H), 1.19-1.17 (d, 3 H, 7.1 Hz), 0.91-0.87 (t, 6 H, *J* = 6.8 Hz); ¹³C NMR (63 MHz, CDCl₃) δ 177.7, 170.4, 165.0, 156.0, 153.9, 141.5, 139.9, 135.6, 130.7, 129.7, 128.5, 128.1, 125.6, 124.7, 122.3, 112.2, 77.2, 75.7, 71.0, 63.0, 58.8, 56.0, 54.5, 52.9, 48.5, 46.3, 42.7, 40.5, 39.3, 36.8, 35.2, 28.3, 24.5, 22.83, 22.8, 22.6, 21.2, 13.5; IR (CHCl₃) 3425, 3009, 2967, 2936, 2874, 2841, 1751, 1709, 1685, 1504, 1368, 1280, 1259, 1165, 1153, 1067 cm⁻¹; Anal. (C₄₄H₆₁ClN₄O₁₀) C, H, N.

### Example 78

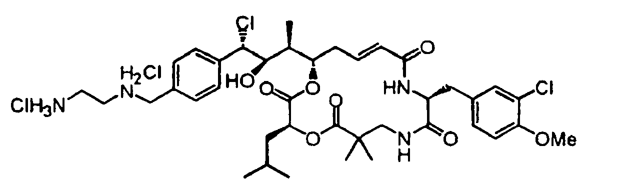

To a -78 °C solution of the epoxide **(shown above)** (0.065 g, 0.076 mmol) in 0.9 mL of CH₂Cl₂ was added dropwise 4 M HCl in 1,4-dioxane (0.09 ml, 0.38 mmol). The solution was stirred at -78 °C for 30 min and then was allowed to slowly warm up to room temperature. It was stirred an additional 2 hrs at room temperature and was concentrated in vacuo to yield the chlorohydrin **(shown above)** (0.063 g) in quantitative yield: [α]²⁰_{D} + 16.6 ° (c 1.0, MeOH ); ¹H NMR (300 MHz, CDCl₃) δ 8.54-8.52 (d, 1 H, *J* = 7.7 Hz), 7.84-7.81 (dd, 1 H, *J* = 8.8, 1.7 Hz), 7.63-7.53 (q, 4 H, *J* = 20.0, 8.2 Hz), 7.31-7.3 (d, 1 H, *J* = 2.0 Hz), 7.22-7.18 (dd, 1 H, *J* = 8.4, 2.0 Hz), 7.02-6.99 (d, 1 H, J = 8.5 Hz), 6.8-6.7 (m, 1 H), 6.0-5.92 (d, 1 H, *J* = 15.0 Hz), 5.2-5.0 (m, 2 H), 4.9-4.8 (m, 1 H), 4.6-4.4 (m, 1 H), 4.3 (s, 2 H), 4.07-4.03 (dd, 1 H, *J* = 9.5, 1.4 Hz), 3.86 (s, 3 H), 3.6-3.1 (m, 7 H), 2.82-2.7 (m, 2 H), 2.6-2.3 (m, 2 H), 1.9-1.6 (m, 3 H), 1.25 (s, 3 H), 1.2 (s, 3 H), 1.05-0.99 (m, 9 H); ¹³C NMR (63 MHz, CDCl₃) δ 178.8, 173.8, 171.9, 168.3, 155.3, 144.2, 143.1, 132.2, 131.5, 131.4, 130.3, 129.4, 125.2, 123.2, 113.5, 77.2, 74.7, 72.6, 63.2, 57.6, 56.7, 52.4, 47.5, 45.5, 44.1, 41.1, 40.3, 37.8, 36.9, 36.5, 26.3, 23.7, 23.5, 22.2, 9.0; IR (KBr) 3412, 2961, 2933, 1749, 1721, 1663, 1504, 1462, 1442, 1259, 1199, 1152, 1126, 1065 cm⁻¹; FAB HRMS [M - HCl₂] calcd for (C₃₉H₅₅Cl₂N₄O₈) 777.3397, found 777.3407.

### Example 79

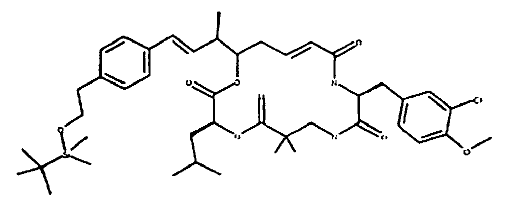

The styrene **(shown above)** (1.2 g) as a mixture of E:Z isomers was prepared from aldehyde **18** (1.0 g, 1.73 mmol) and 4-(ethyl-2-*tert*-butyldimethylsiloxy)benzyl triphenylphosphonium bromide (1.23 g, 2.08 mmol) in 86% yield according to the procedure described above for styrene **20**.
The mixture of isomers was dissolved in toluene (50 mL) and heated to reflux in the presense of 1,1'-azobis(cyclohexanecarbonitrile) (VAZO) (0.040 g, 0.16 mmol) and thiophenol (0.061 mL, 0.59 mmol) for 3 hours. After concentration the residue was purified by radial PLC (20-75% EtOAc/hexanes) to give the E isomer (0.813 g, 68%) as a white foam: [α]²⁰_{D}+ 35.6 ° (c 0.56, MeOH); ¹H NMR (300 MHz, CDCl₃) δ 7.26-7.12 (m, 6 H), 7.07-7.04 (d, 1 H, *J =* 8.5 Hz), 6.85-6.82 (d, 1 H, *J =* 8.4 Hz), 6.83-6.70 (m, 1 H), 6.40-6.35 (d, 1 H, *J =* 15.8 Hz), 6.0-5.92 (dd, 1 H, *J* = 15.4, 8.7 Hz), 5.77-5.72 (d, 1 H, *J* = 15.2 Hz), 5.46-5.43 (d, 1 H, *J =* 7.7 Hz), 5.07-5.02 (m, 1 H), 4.86-4.83 (m, 1 H), 4.82-4.74 (m, 1 H), 3.88 (s, 3 H), 3.78-3.74 (t, 2 H, *J* = 7.1 Hz), 3.44-3.37 (dd, 1 H, *J =* 12.5, 8.6 Hz), 3.15-3.08 (m, 3 H), 2.81-2.77 (t, 2 H, *J* = 7.1 Hz), 2.57-2.52 (m, 2 H), 2.43-2.35 (m, 1 H), 1.74-1.56 (m, 2 H), 1.38-1.23 (m, 1 H), 1.22 (s, 3 H), 1.16 (s, 3 H), 1.13-1.11 (d, 3 H, *J =* 6.8 Hz), 0.88 (s, 9 H), 0.84-0.72 (m, 6 H), 0.0 (s, 6 H); ¹³C NMR (75MHz, CDCl₃) δ 177.9, 170.5, 170.3, 165.1, 154.0, 142.2, 138.6, 134.6, 131.5, 130.9, 129.6, 129.4, 128.2, 126.0, 125.3, 124.5, 122.5, 112.3, 92.9, 77.0, 71.4, 64.4, 56.1, 54.3, 46.5, 42.7, 42.2, 39.4, 39.2, 36.5, 35.3, 25.9, 24.5, 22.8, 22.7, 22.6, 21.2, 17.2, -5.44; IR (CHCl₃) 3423, 2959, 2931, 2858, 1747, 1712, 1681, 1605, 1527, 1503, 1485, 1442, 1370, 1339, 1303, 1281, 1258, 1194, 1151, 1095, 1067, 1025, 1007, 838 cm⁻¹; Anal. (C₄₄H₆₃ClN₂O₈Si) C, H, N.

### Example 80

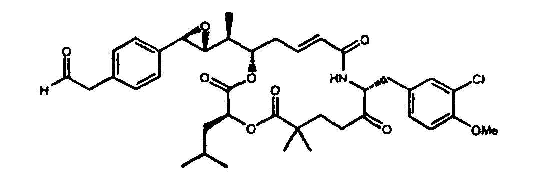

Pyridine (0.06 mL, 0.76 mmol) followed by Dess-Martin reagent (0.161g, 0.379 mmol) was added to a 0 °C of the free alcohol of the styrene **(shown above)** (0.135 g, 0.189 mmol) in 4.5 mL of CH₂Cl₂. The mixture was stirred for 30 min at 0 °C, 20 min at room temperature then was filtered using EtOAc through Celite and was finally concentrated in vacuo. Quick purification of the crude product by radial PLC (silica gel, 80-100% EtOAc/ CH₂Cl₂) provided the desired aldehyde (0.08 g), in 59% yield as a white solid:¹H NMR (300 MHz, CDCl₃) 9.77-9.76 (t, 1 H, *J* = 2.0 Hz), 7.3-7.2 (m, 6 H), 7.06-7.02 (dd, 1 H, *J* = 8.3, 2.0 Hz), 6.85-6.82 (d, 1 H, *J* = 8.4 Hz), 6.81-6.7 (m, 1 H), 5.74-5.68 (d, 1 H, *J =* 15.3 Hz), 5.51-5.48 (d, 1 H, *J* = 7.8 Hz), 5.22-5.17 (m, 1 H), 4.85-4.81 (dd, 1 H, *J =* 10.3, 3.6 Hz), 4.77-4.71 (m, 1 H), 3.87 (s, 3 H), 3.72-3.71 (d, 2 H, *J =* 2.0 Hz), 3.69-3.68 (d, 1 H, *J* = 1.5 Hz), 3.45-3.38 (dd, 1 H, *J =* 13.5, 8.7 Hz), 3.17-3.0 (m, 3 H), 2.92-2.89 (dd, 1 H, *J* = 7.6, 1.8 Hz), 2.6-2.4 (m, 2 H), 1.8-1.6 (m, 3 H), 1.4-1.3 (m, 1 H), 1.22 (s, 3 H), 1.16 (s, 3 H), 1.16-1.13 (d, 3 H, *J* = 8.3 Hz), 0.86-0.82 (t, 6 H, *J* = 6.3 Hz).

### Example 81

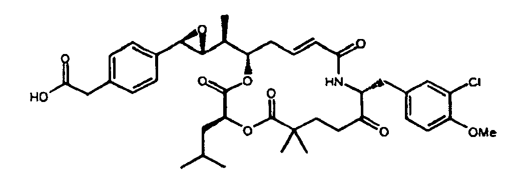

Tetrahydofuran (3.2 mL) and H₂O (3.2 mL) were added to the aldehyde **(shown above)** (0.08 g, 0.112 mmol) and the mixture was cooled to 0 °C. 2-Methyl-2-butene (3.2 mL), followed by NaClO₂ (0.081 g, 0.896 mmol) and NaH₂PO₄H₂O (0.139 g, 1.0 mmol) were also added consecutively. The mixture was allowed to warm to room temperature and was stirred vigorously for 5 hrs. The solution was diluted with 10 mL of CH₂Cl₂ and the layers separated. The aqueous layer was extracted with CH₂Cl₂ (3 x 10 mL) and the combined organic extracts were dried over Na₂SO₄, filtered and concentrated in vacuo. The crude product was purified twice by radial PLC (silica gel, 5-10-25% MeOH/CH₂Cl₂) to give 0.03 g of the carboxylic acid **(shown above)** in 37% yield as a white solid: [α]²⁰_{D} + 24.5° (*c* 0.33, MeOH); ¹H NMR (300 MHz, CD₃OD) δ 7.75-7.71 (dd, 1 H, *J =* 10.3, 1.9 Hz), 7.31-7.2 (m, 5 H), 7.16-7.13 (dd, 1 H, *J* = 8.4, 1.9 Hz), 6.97-6.95 (d, 1 H, *J =* 8.4 Hz), 6.8-6.6 (m, 1 H), 5.87-5.81 (d, 1 H, *J =* 15.3 Hz), 5.19-5.14 (dd, 1 H, *J* = 11.0, 5.0 Hz), 4.9-4.8 (m, 2 H), 4.48-4.43 (dd, 1 H, *J =* 11.5, 3.5 Hz), 3.8 (s, 3 H), 3.77 (s, 1 H), 3.53 (s, 2 H), 3.5-3.4 (m, 1 H), 3.17-3.11 (dd, 1 H, *J* = 14.3, 3.5 Hz), 3.05-3.0 (d, 1 H, *J =* 13.6 Hz), 2.95-2.92 (dd, 1 H, *J =* 7.7, 1.7 Hz), 2.8-2.6 (m, 2 H), 2.5-2.3 (m, 1 H), 1.8-1.6 (m, 3 H), 1.4-1.2 (m, 1 H), 1.17 (s, 3 H), 1.13 (s, 3 H), 1.13-1.1 (d, 3 H, J = 9.2 Hz), 0.83-0.81 (d, 6 H, *J* = 6.3 Hz); ¹³C NMR (126 MHz, CDCl₃) δ 178.8, 173.7, 172.0, 168.2, 155.4, 143.4, 138.1, 136.8, 132.2, 131.2, 130.8, 129.3, 126.8, 125.4, 123.3, 113.5, 77.7, 72.4, 64.4, 60.0, 57.5, 56.6, 47.4, 44.1, 41.7, 40.7, 38.6, 36.5, 25.9, 23.4, 23.3, 21.6, 14.0; IR (KBr) 3417, 2961, 2934, 2874, 1750, 1721, 1674, 1561, 1504, 1464, 1441, 1300, 1259, 1194, 1151, 1066 cm⁻¹; FAB HRMS [M + H] calcd for (C₃₈H₄₈ClN₂O₁₀) 727.2997, found 727.3005.

### Example 82

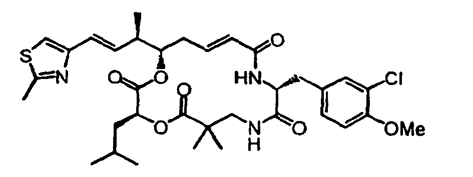

To a mixture of [(2-methyl-4-thiazolyl)methyl]triphenylphosphonium chloride (0.496 g, 1.2 mmol) in 10 mL of THF at -78 °C was added dropwise a 1.6 M solution of n-butyllithium (0.8 mL, 1.2 mmol). The mixture was warmed slowly to room temperature and stirred for an additional 45 min. To aldehyde **18** (0.5 g, 0.865 mmol), in 15 mL of THF and at -78 °C, was added dropwise the orange ylide solution via a double tipped needle. The resulting mixture was stirred at -78 °C for 2 h and at room temperature for 1.5 h. Saturated NH₄Cl (30 mL) was added along with ethyl acetate (30 mL), the layers separated and the aqueous one extracted with ethyl acetate (2 x 20 mL). The combined organic layers were washed with water (2 x 20 mL) and brine, dried over MgSO₄, filtered and concentrated in vacuo. The resulting yellow residue was purified using column chromatography (silica gel, 50-70-80% EtOAc/hexanes) to give 0.4 g of the desired styrene along with triphenylphosphine oxide. The triphenylphosphine oxide was easily removed by reverse phase HPLC using CH₃CN:H₂O (50:50) to give 0.2 g (34%) of pure thiazole **(shown above)** as a white solid: [α]²⁰_{D} + 16.7 ° (c 1.0, CHCl₃); ¹H NMR (300 MHz, CDCl₃) δ 7.3-7.2 (m, 1 H), 7.18-7.17 (d, 1 H, *J* = 1.7 Hz), 7.06-7.03 (dd, 1 H, *J* = 8.5, 1.8 Hz), 6.83 (s, 1 H), 6.83-6.8 (d, 1 H, *J =* 9.0 Hz), 6.8-6.67 (m, 1 H), 6.37-6.35 (m, 2 H), 5.85-5.82 (d, 1 H, *J =* 7.9 Hz), 5.76-5.71 (d, 1 H, *J* = 15.1 Hz), 5.05-5.0 (dd, 1 H, *J =* 9.0, 6.0 Hz), 4.86-4.82 (dd, 1 H, *J =* 10.2, 3.6 Hz), 4.77-4.68 (m, 1 H), 3.85 (s, 3 H), 3.44-3.37 (dd, 1 H, *J* = 13.4, 8.6 Hz), 3.2-3.0 (m, 3 H), 2.68 (s, 3 H), 2.6-2.3 (m, 3 H), 1.8-1.6 (m, 2 H), 1.43-1.3 (m, 1 H), 1.2 (s, 3 H), 1.14 (s, 3 H), 1.12-1.1 (d, 3 H, *J* = 6.9 Hz), 0.79-0.78 (d, 3 H, J = 3.1 Hz), 0.77-0.76 (d, 3 H, J = 3.1 Hz); ¹³C NMR (63 MHz, CDCl₃) δ 177.8, 170.5, 166.0, 165.2, 153.9, 153.0, 142.1, 136.7, 132.7, 130.8, 129.8, 128.1, 124.6, 124.4, 122.3, 114.1, 112.2, 76.9, 71.4, 56.0, 54.4, 46.4, 42.7, 41.9, 39.3, 36.5, 35.3, 24.5, 22.8, 22.7, 22.6, 21.2, 19.2, 17.1; IR (CHCl₃) 3423, 3027, 3008, 2965, 2935, 2874, 1747, 1712, 1681, 1652, 1604, 1528, 1504, 1485, 1259, 1181, 1152, 1067 cm⁻¹; Anal. (C₃₄H₄₄ClN₃O₇S) C, H, N.

### Example 83

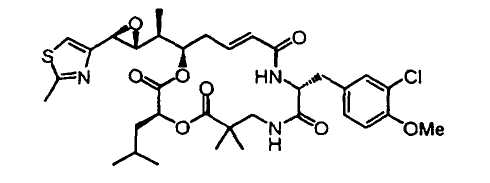

To the styrene **(shown above)** (0.25 g, 0.37 mmol) was added 15 mL of acetone, 6 mL of H₂O, 6 mL of CH₂Cl₂ and solid NaHCO₃ (1.0 g, 11.9 mmol) and the mixture was cooled to 0°C. A solution of Oxone (0.92 g, 1.5 mmol) in 8 mL of H₂O was prepared and added (2 mL) to the cold styrene mixture. Following 30 min of vigorous stirring at 0 °C an additional 2 mL of Oxone solution was added and again another 2.0 mL was added, following another 30 min, for a total of 6 mL of Oxone solution. The reaction progress was monitored by reverse phase HPLC and was found to be complete after 2.0 hrs of stirring. While still at 0 °C, the reaction was quenched with saturated aqueous NaHCO₃ (40 mL) and 40 mL of CH₂Cl₂. The layers were separated and the organic layer was washed with aq. 10% Na₂SO₃ (40 mL), followed by saturated aq. NaHCO₃ (40 mL) then brine and finally was dried over Na₂SO₄, filtered and concentrated in vacuo. The mixture of b and a epoxides (54:46) was separated by reverse phase HPLC (50:50) CH₃CN:H₂O to provide 0.09 g of the b epoxide **(shown above)** as a white solid in 35% yield: [α]²⁰_{D} + 26.0 ° (c 1.0, CHCl₃ ); ¹H NMR (300 MHz, CDCl₃) δ 7.19-7.18 (d, 2 H, *J* = 1.8 Hz), 7.1 (s, 1 H), 7.06-7.03 (dd, 1 H, *J* = 8.5, 1.9 Hz), 6.85-6.82 (d, 1 H, *J* = 8.4 Hz), 6.82-6.7 (m, 1 H), 5.76-5.71 (d, 1H, *J* = 15.2 Hz), 5.49-5.47 (d, 1 H, *J* = 7.8 Hz), 5.23-5.18 (m, 1 H), 4.88-4.84 (dd, 1 H, *J* = 10.3, 3.6 Hz), 4.8-4.7 (m, 1 H), 3.88 (s, 3 H), 3.79 (d, 1 H, *J* = 0.93 Hz), 3.45-3.38 (dd, 1 H, *J* = 13.4, 8.6 Hz), 3.35-3.32 (d, 1 H, *J* = 7.2 Hz), 3.2-3.0 (m, 3 H), 2.7 (s, 3 H), 2.6-2.4 (m, 2 H), 1.8-1.6 (m, 3 H), 1.4-1.3 (m, 1 H), 1.23 (s, 3 H), 1.16 (s, 3 H), 1.14-1.12 (d, 3 H, *J* = 6.8 Hz), 0.89-0.87 (d, 3 H, *J* = 6.5 Hz), 0.86-0.84 (d, 3 H, *J* = 6.4 Hz); ¹³C NMR (63 MHz, CDCl₃) δ 177.9, 170.33, 170.3, 166.9, 165.0, 154.0, 151.9, 141.8, 130.8, 129.5, 128.2, 124.5, 122.4, 116.4, 112.3, 75.8, 71.1, 61.3, 56.1, 55.2, 54.3, 46.4, 42.7, 40.3, 39.3, 36.6, 35.2, 24.5, 22.85, 22.8, 22.6, 21.2, 19.1, 13.3; IR (CHCl₃) 3425, 3007, 2964, 2936, 2874, 2841, 1751, 1711, 1682, 1604, 1528, 1503, 1485, 1464, 1303, 1259, 1185, 1152, 1067 cm⁻¹; Anal. (C₃₄H₄₄ClN₃O₈S) C, H, N.

### Example 84

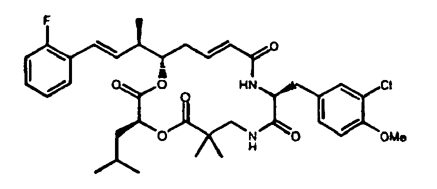

Styrene **(shown above)** (0.5 g) was prepared from aldehyde 18 (1.3 g, 2.2 mmol) and 2-fluorobenzyltriphenylphosphonium bromide (1.7 g, 3.8 mmol) in 33% yield according to the procedure described above for styrene 20: [α]²⁰_{D} + 17.0 ° (*c* 1.16, CHCl₃) ; ¹H NMR (300 MHz, CDCl₃) δ 7.40-7.34 (m, 1 H), 7.23-6.96 (m, 6 H), 6.83-6.80 (d, 1 H, *J =* 8.4 Hz), 6.78-6.70 (m, 1 H), 6.57-6.52 (d, 1 H, *J =* 16.0 Hz), 6.10-6.02 (dd, 1 H, *J =* 8.8, 16.0 Hz), 5.76-5.71 (d, 1 H, *J* = 15.3 Hz), 5.50-5.47 (d, 1 H, *J* = 7.8 Hz), 5.05-4.95 (m, 1 H), 4.85-4.80 (dd, 1 H, *J =* 9.6, 3.1 Hz), 4.75-4.69 (m, 1 H), 3.85 (s, 3 H), 3.42-3.34 (dd, 1 H, *J* = 13.5, 8.7 Hz), 3.13-3.05 (m, 3 H), 2.56-2.51 (m, 2 H), 2.37-2.33 (m, 1 H), 1.68-1.58 (m, 2 H), 1.34-1.23 (m, 1 H), 1.20 (s, 3 H), 1.13 (s, 3 H), 1.13-1.11 (d, 3 H, *J* = 7.3 Hz), 0.79-0.70 (m, 6 H); ¹³C NMR (63 MHz, CDCl₃) δ 177.9, 170.5, 170.4, 165.1, 154.0, 142.0, 137.4, 133.0, 132.9, 130.8, 129.6, 128.8, 128.2, 127.0, 126.9, 124.6, 124.1, 115.9, 115.5, 112.3, 76.9, 71.4, 56.1, 54.4, 46.4, 42.7, 42.6, 39.4, 36.5, 35.3, 24.5, 22.8, 22.7, 22.6, 21.1, 17.2; IR (CHCl₃) 3423, 2965, 2935, 2874, 1747, 1711, 1681, 1605, 1527, 1503, 1487, 1457, 1441, 1370, 1340, 1321, 1280, 1259, 1151, 1093, 1067, 1009, 970 cm⁻¹; Anal. (C₃₆H₄₄ClFN₂O₇) C, H, N.

### Example 85

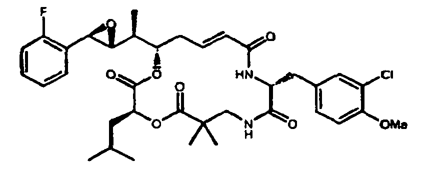

To a solution of the styrene **(shown above)** (0.26 g, 0.387 mmol) in 1.3 mL CH₂Cl₂ at 0 °C, was added 3-chloroperoxybenzoic acid (0.07 g, 0.41 mmol) and toluene (0.65 mL) and stirring continued at 0 °C for 30 minutes. The ice-bath was removed and the reaction allowed to stir at room temperature for 24 hours. After concentration, the residue was purified by reverse-phase HPLC (CH₃CN/H₂O) to give the b-epoxide **(shown above)** as a white foam (0.058 g, 24% corrected for recovered styrene: [α]²⁰_{D} + 18.98 ° (c 1.41, CHCl₃); ¹H NMR (300 MHz, CDCl₃) δ 7.30-7.00 (m, 6 H), 6.86-6.83 (d, 1 H, *J* = 8.5 Hz), 6.80-6.75 (m, 1 H), 5.76-5.71 (d, 1 H, *J* = 15.1 Hz), 5.47-5.45 (d, 1 H, *J* = 7.8 Hz), 5.23-5.20 (m, 1 H), 4.86-4.82 (dd, 1 H, *J* = 10.3, 3.5 Hz), 4.78-4.70 (m, 1 H), 4.01 (s, 1 H), 3.88 (s, 3 H), 3.45-3.38 (dd, 1 H, *J* = 13.4, 8.6 Hz), 3.12-3.08 (m, 3 H), 2.91-2.88 (d, 1 H, *J* = 7.8 Hz), 2.59-2.53 (m, 2 H), 1.80-1.71 (m, 3 H), 1.46-1.25 (m, 1 H), 1.23 (s, 3 H), 1.16 (s, 3 H), 1.16-1.14(d, 3 H, *J* = 7.2 Hz), 0.87-0.83 (m, 6 H); ¹³C NMR (63 MHz, CDCl₃) δ 178.1, 170.5, 170.3, 165.0, 142.0, 131.0, 129.7, 129.6, 128.3, 126.1 126.08, 124.65,124.5, 122.6, 115.5, 115.2, 112.4, 76.0, 71.2, 62.6, 56.2, 54.4, 53.5, 46.5, 42.8, 40.6, 39.4, 36.9, 35.3, 24.7, 22.9, 22.8, 21.3, 13.8; IR (CHCl₃) 3417, 2962, 2948, 1754, 1721, 1681, 1653, 1534, 1504, 1473, 1459, 1441,1303, 1282, 1258,1191, 1148, 1127, 1066 cm⁻¹; FAB HRMS [M + H] for (C₃₆H₄₅ClFN₂O₈) cald 687.2848, found 687.2857.

### Example 86

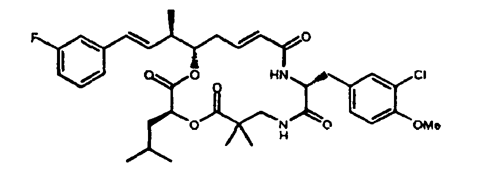

The styrene **(shown above)** (0.85g), as an E/Z mixture, was prepared from aldehyde **18** (2.0 g, 3.45 mmol) and 3-fluorobenzyl triphenylphosphonium bromide (1.92 g, 4.25 mmol) in 37% yield according to the procedure described above for styrene **20**. The mixture of isomers was dissolved in benzene (25 mL) and heated to reflux in the presense of 1.1'-azobis(cyclohexanecarbonitrile) (VAZO) (0.04 g, 0.16 mmol) and thiophenol (0.06 mL, 0.58 mmol) for 20 hours. After concentration the residue was purified by radial PLC (20-100% EtOAc/hexanes) to give the E isomer (0.652 g, 77%) as a white foam: [α]²⁰_{D}+ 30.55 ° (*c* 0.98, CHCl₃); ¹H NMR (300 MHz, CDCl₃) δ 7.30-7.01 (m, 8 H), 6.95-6.88 (m, 1 H), 6.86-6.83 (d, 1 H, *J* = 8.5 Hz), 6.81-6.72 (m, 1 H), 6.40-6.35 (d, 1 H, *J* = 15.9 Hz), 6.07-5.99 (dd, 1 H, *J* = 8.8, 15.9 Hz), 5.78-5.73 (d, 1 H, *J =* 15.1 Hz), 5.50-5.47 (d, 1 H, *J =* 7.9 Hz), 5.08-5.02 (dd, 1 H, *J =* 9.7, 6.7 Hz), 4.87-4.82 (dd, 1 H, *J =* 9.7, 3.0 Hz), 4.78-4.71 (m, 1 H), 3.88 (s, 3 H), 3.45-3.37 (dd, 1 H, *J* = 13.5, 8.6 Hz), 3.15-3.08 (m, 3 H), 2.59-2.52 (m, 2 H), 2.43-2.34 (m, 1 H), 1.72-1.63 (m, 2 H), 1.36-1.26 (m, 1 H), 1.22 (s, 3 H), 1.16 (s, 3 H), 1.15-1.12 (d, 3 H, *J* = 6.9 Hz), 0.80-0.73 (m, 6 H); ¹³C NMR (63 MHz, CDCl₃) δ 177.8, 170.5, 170.4, 165.1, 165.0, 153.9, 141.9, 137.3, 131.7, 130.8, 130.6, 130.0, 129.6, 128.1, 124.6, 122.4, 114.4, 114.1, 112.6, 112.2, 76.8, 71.3, 56.0, 54.4, 46.4, 42.7, 42.1, 39.5, 36.5, 35.2, 24.5, 22.8, 22.64, 22.6, 21.2, 17.2; IR (CHCl₃) 3423, 3008, 2965, 2936, 2874, 1747, 1712, 1680,1652,1585, 1528, 1503, 1486, 1464, 1442, 1320, 1303, 1259, 1193, 1147, 1127, 1067, 973 cm⁻¹; Anal. (C₃₆H₄₄ClFN₂O₇) C, H, N.

### Example 87

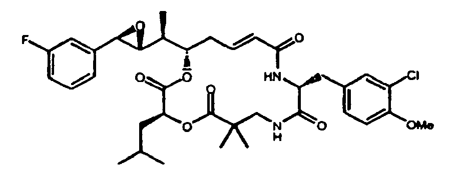

To a solution of the above styrene (0.622 g, 0.927 mmol) in 3.0 mL CH₂Cl₂ at 0 °C, was added 3-chloroperoxybenzoic acid (0.170 g, 0.985 mmol) and toluene (1.5 mL) and stirring continued at 0 °C for 30 minutes. The ice-bath was removed and the reaction allowed to stir at room temperature for 22 hours. After concentration, the residue was purified by reverse-phase HPLC (CH₃CN/H₂O) to give the b-epoxide **(shown above)** as a yellow foam (0.067g, 11% ): [α] ²⁰_{D} + 26.23 ° (*c* 1.54, CHCl₃ ) ; ¹H NMR (300 MHz, CDCl₃) δ 7.37-6.92 (m, 7 H), 6.86-6.83 (d, 1 H, *J =* 8.4 Hz), 6.82-6.73 (m, 1 H), 5.75-5.70 (d, 1 H, *J* = 15.5 Hz), 5.48-5.45 (d, 1 H, *J* = 7.8 Hz), 5.22-5.17 (m, 1 H), 4.85-4.81 (dd, 1 H, *J =* 3.1, 9.8 Hz), 4.76-4.70 (m, 1 H), 3.88 (s, 3 H), 3.68-3.67 (d, 1 H, *J =* 0.89 Hz), 3.46-3.38 (dd, 1 H, *J* = 8.6, 13.5 Hz), 3.13-3.07 (m, 3 H), 2.90-2.87 (dd, 1 H, *J* = 1.5, 7.4 Hz), 2.60-2.37 (m, 2 H), 1.82-1.64 (m, 3 H), 1.36-1.25 (m, 1 H), 1.22 (s, 3 H), 1.16 (s, 3 H), 1.15-1.12 (d, 3 H, *J* = 6.9 Hz), 0.88-0.83 (m, 6 H); ¹³C NMR (63 MHz, CDCl₃) δ 177.9, 170.4, 165.0, 154.0, 141.6, 139.5, 130.8, 130.4, 130.2, 129.6, 128.2, 124.7, 122.4, 121.3, 115.6, 115.3, 112.3, 112.2, 75.8, 71.1, 63.2, 58.2, 56.1, 54.5, 46.4, 42.8, 40.4, 39.4, 36.8, 35.3, 24.5, 22.85, 22.82, 22.7, 21.2, 13.4; IR (KBr) 3416, 3034, 2963, 2934, 2874, 1751, 1721, 1680, 1658, 1539, 1504, 1473, 1442, 1304, 1280, 1258, 1192, 1144, 1066 cm⁻¹; Anal. (C₃₆H₄₄ClFN₂O₈) C, H, N.

### Example 88

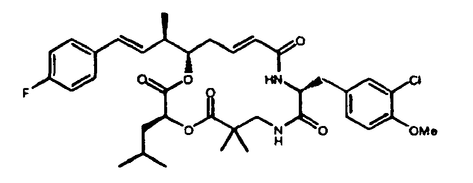

The styrene **(shown above)** (1.24 g), as an E/Z mixture, was prepared from aldehyde **18** (1.5 g, 2.6 mmol) and 4-fluorobenzyl triphenylphosphonium bromide (1.4 g, 3.1 mmol) in 71% yield according to the procedure described above for styrene **20**. The mixture of isomers was dissolved in benzene (40 mL) and heated to reflux in the presense of 1,1'-azobis(cyclohexanecarbonitrile) (VAZO) (0.050 g, 0.20 mmol) and thiophenol (0.076 mL, 0.74 mmol) for 24 hours. After concentration the residue was purified by radial PLC (20-100% EtOAc/hexanes) to give the E isomer (1.06g) as a white foam containing triphenylphosphine oxide by NMR. A 0.150 g sample was purified by reverse-phase HPLC (60:40) CH₃CN:H₂O to give 0.092 g of pure solid: [α]²⁰_{D}+ 27.49 ° (*c* 1.05, CHCl₃ ) ; ¹H NMR (300 MHz, CDCl₃) δ 7.31-6.96 (m, 7 H), 6.85-6.83 (d, 1 H, *J* = 8.5 Hz), 6.81-6.74 (m, 1 H), 6.39-6.34 (d, 1 H, *J* = 15.8 Hz), 5.96-5.88 (dd, 1 H, *J =* 8.8, 15.8 Hz), 5.77-5.72 (d, 1 H, *J* = 15.2 Hz), 5.49-5.47 (d, 1 H, *J* = 7.7 Hz), 5.07-5.02 (m, 1 H), 4.86-4.83 (dd, 1 H, *J* = 9.2, 2.5 Hz), 4.82-4.73 (m, 1 H), 3.87 (s, 3 H), 3.45-3.38 (dd, 1 H, *J* = 13.4, 8.5 Hz), 3.14-3.08 (m, 3 H), 2.57-2.52 (m, 2 H), 2.43-2.34 (m, 1 H), 1.71-1.58 (m, 2 H), 1.36-1.29 (m, 1 H), 1.22 (s, 3 H), 1.16 (s, 3 H), 1.14-1.11 (d, 3 H, J = 6.9 Hz), 0.78-0.73 (m, 6 H); ¹³C NMR (63 MHz, CDCL₃) δ 177.8, 170.5, 170.3, 165.1, 160.2, 154.0, 142.0, 137.6, 132.9, 132.8, 130.8, 130.4, 130.0, 129.6, 128.2, 127.6, 127.5, 124.6, 122.4, 115.6, 115.2, 112.2, 71.3, 56.0, 54.4, 46.4, 42.7, 42.1, 39.5, 36.4, 35.2, 24.5, 22.8, 22.6, 21.2, 17.2; IR (KBr) 3421, 3289, 2862, 2933, 1751, 1722, 1678, 1604, 1534, 1509, 1259, 1228, 1149, 1066, 1024, 1011, 971, 815 cm⁻¹; Anal. (C₃₆H₄₄ClFN₂O₇) C, H, N.

### Example 89

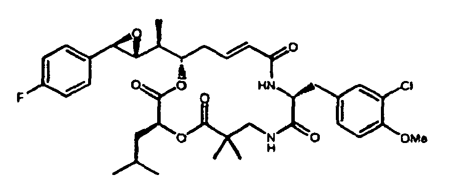

To a solution of the styrene **(shown above)** (0.906 g, 1.35 mmol) in 4.5 mL CH₂Cl₂ at 0 °C was added 3-chloroperoxybenzoic acid (0.25 g, 1.45 mmol) and toluene (2.2 mL) and stirring continued at 0 °C for 30 minutes. The ice-bath was removed and the reaction allowed to stir at room temperature for 23 hours. After diluting with 20 mL of CH₂Cl₂, the reaction mixture was washed with 10% Na₂S₂O₅ (1 x 10mL), water (1 x 10mL), saturated NaHCO₃ (1 x 10mL) and brine (1 x 10mL) and finally was dried over Na₂SO₄. Filtration and concentration gave 0.814 g of the product as a mixture of the b/a epoxides. A 0.23 g portion was purified by reverse-phase HPLC (CH₃CN/H₂O) to give 0.073 g of the b-epoxide **(shown above)** as a white foam: [α]²⁰_{D} + 25.6 (*c* 0.626, CHCl₃); ¹H NMR (300 MHz, CDCl₃) δ 7.26-7.03 (m, 7 H), 6.85-6.83 (d, 1 H, *J =* 8.4 Hz), 6.82-6.72 (m, 1 H), 5.74-5.69 (d, 1 H, *J =* 15.2 Hz), 5.44-5.42 (d, 1 H, *J =* 7.9 Hz), 5.23-5.18 (m, 1 H), 4.85-4.81 (dd, 1 H, *J =* 9.7, 2.9 Hz), 4.77-4.73 (m, 1 H), 3.88 (s, 3 H), 3.66 (s, 1 H), 3.46-3.39 (dd, 1 H, *J =* 13.5, 8.8 Hz), 3.12-3.07 (m, 3 H), 2.89-2.87 (dd, 1 H, *J =* 1.5, 7.7 Hz), 2.60-2.54 (m, 1 H), 2.49-2.41 (m, 1 H), 1.81-1.65 (m, 3 H), 1.34-1.25 (m, 1 H), 1.22 (s, 3 H), 1.16 (s, 3 H), 1.15-1.13 (d, 3 H, *J =* 7.0 Hz), 0.87-0.82 (m, 6 H); ¹³C NMR (63 MHz, CDCl₃) δ 177.8, 170.3, 164.9, 164.7, 154.0, 141.6, 137.8, 132.4, 130.7, 129.6, 128.1, 127.3, 127.2, 124.6, 122.4, 115.8, 115.5, 112.2, 75.8, 71.0, 63.0, 58.2, 56.0, 54.4, 46.3, 42.7, 40.4, 39.3, 36.7, 35.2, 23.5, 22.8, 22.76, 22.6, 21.1, 13.3; IR (CHCl₃) 3426, 3030, 3006, 2964, 2936, 1752, 1711, 1683, 1608, 1514, 1485, 1442, 1303, 1281, 1259, 1188, 1155, 1067, 838 cm⁻¹; Anal. (C₃₆H₄₄ClFN₂O₈) C, H, N.

### Example 90

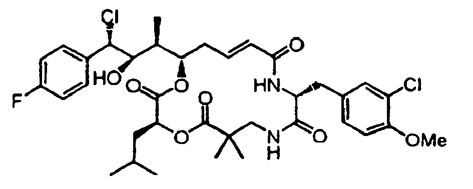

A 4 M solution of HCl in dioxane (0.4 mL, 1.6 mmol) was added dropwise over 5 minutes to a -70 °C solution of b-epoxide **(shown above)** (0.44 g, 0.64 mmol) in 30 mL CH₂Cl₂. Following 2 additional hrs of stirring at -70 °C, the solution was concentrated in vacuo. The crude product was purified by radial PLC (silica gel, 30-50-100% EtOAc/ CH₂Cl₂ ) followed by reverse phase HPLC (50:50) CH₃CN:H₂O to give 0.152 g (33%) of the desired chlorohydrin **(shown above)** as a white foam: [α]²⁰_{D}+ 60.0 ° (c 2.62, CHCl₃); ¹H NMR (300 MHz, CDCl₃) δ 7.41-7.05 (m, 7H), 6.87-6.84 (d, 1H, J = 8.4 Hz), 6.83-6.77 (m, 1H), 5.80-5.75 (d, 1H, J = 15.4 Hz), 5.52-5.49 (d, 1H, J = 7.8 Hz), 5.13-5.21 (m, 1H), 4.94-4.90 (dd, 1H, J = 9.7,3.2 Hz), 4.75-4.72 (m, 1H), 4.67-4.63 (d, 1H, J = 9.5 Hz), 4.00-3.95 (m, 1H), 3.89 (s, 3H), 3.42-3.35 (dd, 1H, J = 8.3, 13.5 Hz), 3.20-3.02 (m, 3H), 2.71-2.65 (m, 1H), 2.49-2.37 (m, 2H), 1.82-1.63 (m, 2H), 1.51-1.38 (m, 2H), 1.23 (s, 3H), 1.17 (s, 3H), 1.04-1.02 (d, 3H, J = 7.0 Hz). 0.97-0.85 (m, 6H); ¹³C NMR (63 MHz, CDCl₃) δ 177.6, 170.5, 170.3, 165.3, 160.7, 153.9, 142.2, 137.5, 134.5, 130.8, 129.8, 129.7, 128.2, 124.6, 122.2, 76.1, 74.0, 71.1, 61.4, 56.1, 54.5, 46.4, 42.7, 39.6, 38.4, 36.3, 35.1, 24.8, 23.0, 22.9, 22.7, 21.5, 8.6; IR (CHCl₃) 3423, 2965, 2935, 2873, 1751, 1715, 1679, 1607, 1528, 1504, 1485, 1464, 1442, 1302, 1281, 1193, 1159, 1152, 1127, 1067 cm⁻¹; Anal. (C₃₆H₄₅Cl₂N₂O₈) C, H, N.

### Example 91

Cryptophycins-151, -152, -153, -154, 155, -156, -159, 160,-161, -166, -167, -172, -181, -188, 234, 236, 238, 247, 251 and 255.

**Table 1**

| | | | |
|---|---|---|---|
| 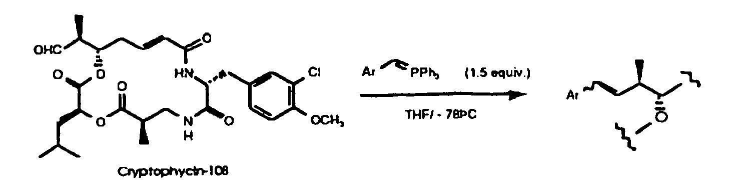 | | | |

| Cryptophycin # | Ar or alkyl group | yield (E+Z) % | % of *trans* isomer (approximately) |
|---|---|---|---|
| 151 | 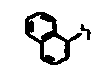 | 72 | 90 |
| 152 | 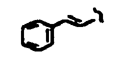 | 80 | 70 |
| 155 | 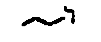 | 60 | <10 |
| 156 | 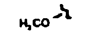 | 51 | 70 |
| 160 | 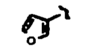 | 61 | 67 |
| 172 | 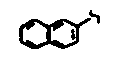 | 78 | 90 |
| 255 | 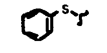 | 85 | 60 |

**Table 2.**

| | | | |
|---|---|---|---|
| 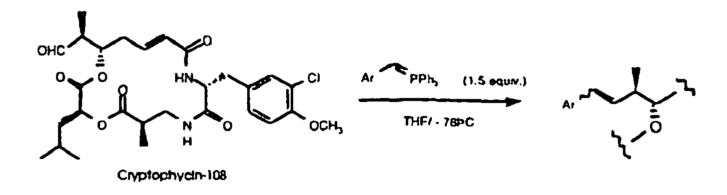 | | | |

| Cryptophycin # | Ar | yield (E+Z) % | % of *E* isomer (approximately) |
|---|---|---|---|
| 153 | 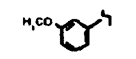 | 80 | 90 |
| 154 | 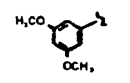 | 63 | 90 |
| 159 | 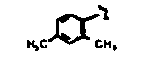 | 75 | 90 |
| 161 | 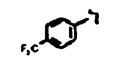 | 61 | 80 |
| 166 | 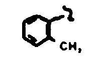 | 38 | 90 |
| 167 | 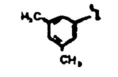 | 48 | 90 |
| 181 | 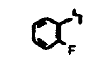 | 73 | 75 |
| 188 | 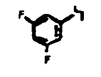 | 85 | 80 |
| 234 | 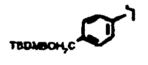 | 81 | 85 |
| 236 | 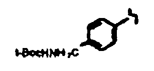 | 59 | 90 |
| 247 | 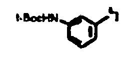 | 51 | >90 |
| 251 | 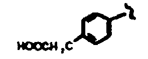 | 43 | >90 |

The typical procedure adapted for the coupling of cryptophycin-108 with a variety of triphenylphosphoranes is described below using the preparation of cryptophycin-152 as an illustrative example.

**Cryptophycin-152:** Cinnamyltriphenylphosphorane was generated by the treatment of cinnamyl triphenylphosphonium chloride (0.311 gm, 0.750 mmol) in THF (5.7 mL) with butyllithium (300 µL of 2.5 M solution in hexane, 0.750 mmol) at -78_C and allowing the contents slowly warm up to the room temperature. Cinnamyltriphenylphosphorane from this reaction mixture (1.46 mL, 0.182 mmol) was slowly added to the aldehyde (68.6 mg, 0.122 mmol) in THF (3 mL) at -78_C and continued the stirring for 2 hr. The reaction mixture was brought to the ambient temperature, treated with saturated NH₄Cl (5mL) followed by water (15 mL) and extracted with ethyl acetate (40 mL). The organic layer was washed with water, dried over MgSO₄ and evaporated. The residue was applied to a small ODS column and eluted with 1:1 H₂O/CH₃CN and 1:3 H₂O/CH₃CN. The latter fraction was evaporated to obtain a mixture of cryptophycin-152 and its Z isomer (E/Z 7:3, 64.3 mg, 80%).

Using the same experimental procedure the aldehyde (cryptophycin-108) was reacted with 1-naphthylmethyl triphenylphosphorane, 3-methoxybenzyl triphenylphosphorane, 3,5-dimethoxybenzyl triphenylphosphorane, methoxymethyl triphenylphosphorane, 2,4-dimethylbenzyl triphenylphosphorane, 3-furanmethyl triphenylphosphorane, p-trifluomethylbenzyl triphenylphosphorane, 2-methylbenzyl triphenylphosphorane, 3,5-dimethylbenzyl triphenylphosphorane, 2-naphthylmethyl triphenylphosphorane, 2-fluorobenzyl triphenylphosphorane, 3, 5 - difluorobenzyl triphenylphosphorane, 4-hydroxymethylbenzyltriphenylphosphorane, 4-(t-Boc-aminomethyl)benzyltriphenylphospho-rane, 3-(N-t-Boc-amino)benzyltriphenylphosphorane and phenylthiomethyltriphenylphos-phonium to obatin cryptophycins-151, -153, -154, -156, -159, -160, -161, -166, -167, -172, -181, -188, -234, -236, -247 and 255 respectively.

Butyllithium was used as a base in the generation of ylides from their corresponding triphenylphosphonium chlorides or bromide salts, except phenyllithium was used in the preparation of methoxymethyltriphenylphosphorane, 3-furanmethyl triphenylphosphorane and 4-hydroxymethylbenzyltriphenylphosphorane.

A similar reaction involving hydroxymehyltriphenylphosphomiumbromide, n-butyllithium and cryptophycin-108 yielded an unexpected analog cryptophycin-155.

A slightly modified procedure was adapted for the preparation of carboxy methyl analog cryptophycin-251. A THF (5 mL) solution of 4-(carboxymethyl)benzyltriphenylphosphoniumbromide (0.289 g, 0.59 mmol) was treated with phenyllithium (1.8 M, 653 µL; 1.18 mmol) at -78_C for 5 min and transferred the flask to an ice water bath. After 30 min, 0.9 mL of this reaction mixture was added slowly to a flask containing cryptophycin-108 (35 mg) in 3 mL of THF at-78_C and allowed the contents to stir for 2 h. The reaction mixture was acidified with 1N HCl (1mL), added water (30 mL) and extracted with ethylacetate (30 mL x 2). The organic layer was dried over MgSO₄ and evaporated. The residue was subjected to a flash chromatography on ODS silica eluting with 1:1 H₂O/CH₃CN and 35:65 H₂O/CH₃CN. The later fraction was evaporated and purified on a reversed phase HPLC column (Econosil C18, 10 µ, 250 x 22 mm, 6 mL/min, 35:65 H₂O/CH₃CN) to obtain an impure sample of cryptophycin-251, which was finally purified by another reversed phase chromatography (Econosil C18, 10 µ, 250 x 10 mm, 3 mL/min, 0.5% solution of acetic acid in 2:3 H₂O/CH₃CN) to obtain cryptophycin-251 (10 mg).

The key experimental data involved in the generation of styrene analogs and cytotoxicity data are summarized in tables 1 and 2.

**Triphenylphosphonium salts:** Cinnamyl triphenylphosphonium chloride, 1-naphthylmethyl triphenylphosphonium chloride, methoxymethyltriphenylphosphonium chloride and 2-methybenzyl triphenylphosphonium bromide are available commercially, where as 3-methoxybenzyl triphenylphosphonium chloride, 3,5-dimethoxybenzyl triphenylphosphonium chloride, 2,4-dimethylbenzyl triphenylphosphonium chloride were prepared by refluxing triphenylphosphine with a slight excess of the corresponding chloride in toluene for 4h. 4-Trifluomethylbenzyl triphenylphosphonium bromide, 3-furonmethyltriphenylphosphonium bromide, 2-naphthylmethyltriphenylphosphonium bromide, 3,5 - difluorobenzyl triphenylphosphonium bromide, 4-(t-butyldimethylsilyloxymethyl)benzyltriphenylphosphonium bromide, 4-(N-t-Boc-aminomethyl)benzyltriphenylphosphonium bromide and 3-(N-t-Boc-amino)benzyltriphenyl-phosphonium bromide were prepared by treating the corresponding bromides with triphenylphosphine in toluene at room temperature for 12 h. 9-(Carboxymethyl)benzyltriphenylphosphoniumbromide was prepared by treating the bromide with triphenylphosphine in 5:1 toluene/THF solution at room temperature for 48 h. 3-furonmethylbromide, 2-naphthalenelmethylbromide and 3-(N-t-Boc-amino)benzylbromide were produced respectively from 3-furonmethanol, 2-naphthalenemethanol and 3-(N-t-Boc-amino)benzylalcohol upon treatment with PBr₃ in THF at - 78_C.^{ref} 3-furonmethanol and 2-naphthalenemethanol are available commercially. 3-(N-t-Boc-amino)benzylalcohol was prepared from a commercial sample of 3-aminobenzylalkohol. 4-(t-butyldimethylsilyloxymethyl)benzylbromide and 4-(N-t-Boc-aminomethyl)benzylbromide were prepared prepared from commercial samples of 4-hydroxymethylbenzoic acid methylester and 4-aminomethylbenzoic acid respectively using the following experimental procedures.

**4-(t-butyldimethylsilylaxymethyl)benzylbromide:** A mixture of 4-hydroxymethyl-benzoic acid methylester (2 gm) and triethylamine (3.36 mL) in dichloromethane (15 mL) was treated with t-butyldimethylsilyltriflate (4.47 gm) at - 78_C. After 30 min the contents were allowed to warm up to the room temperature and continued the stirring for another 30 min. Water (30 mL) and ethylacetate (60 mL) were added to the reaction mixture and the organic layer was washed with 0.3 M KHSO₄ water and brine, dried over MgSO₄ and filtered. The solvent was evaporated and the residue was subjected to flash chromatography on silica column by eluting with 10% EtOAc/hexane to obtain 4-(t-butyldimethylsilyloxymethyl) benzoic acid methyl ester (3.3 gm, 95% yield).

Lithiumaluminumhydride (0.21 gm) dispersed in diethylether (20 mL) was cooled to -78_C under argon and treated dropwise with 4-(t-butyldimethylsilyloxymethyl)benzoic acid methyl ester (2.05 gm) in 10 mL of diethylether. After 30 minutes ethylacetate (2mL) was added to quench the excess hydride, and then was added saturated ammonium chloride (1.5mL). The precipitate was separated by filtration and washed with ether. The solvent was evaporated to give 4-(t-butyldimethylsilyloxymethyl)benzylalkohol (1.71 gm, 93% yield).

9-(t-butyldimethylsilyloxymethyl)benzylalkohol (1.7 gm) was dissolved in THF (15 mL) and treated with phosphoroustribromide (0.609 gm) at -78_C. After 30 min, the reaction was diluted with diethylether (80 mL) and washed with saturated sodium bicarbonate(30 mL), water and brine, dried over MgSO4 The ether layer was evaporated and the residue (2.0 gm) was subjected to flash chromatography on silica using 5% EtOAc/hexane as an eluant to obatin 4-(t-butyldimethylsilyloxymethyl)benzybromide (1.05 gm, 49% yield).

**4-(N-t-Boc-aminomethyl)benzyl bromide:** To a solution of di-t-butyldicarbonate (2.18 g, 10 mmol) in triethylaminedimethylforamide (1:9, 7.5 mL) was added 4-(aminomethyl) benzoic acid (0.75 g, 5 mmol) at room temp and warmed the reaction mixture at 40-50° C for 10 min. After the aminoacid was dissolved, the stirring continued at room temp for another hour . The solvent was removed under vacuum and the residue was acidified with dil HCl (pH > 2) and immediately extracted with EtOAc and the organic layer was dried over MgSO₄. The residue obtained after removal of the solvent was treated with excess of diazomethane (generated from diazald) in ether for 30 min and the excess diazomethane was decomposed by acetic acid. The residue obtained after the removal of solvent was chromatographed over silica column (20 g) using hexanes/EtOAc (9:1) for elution to give methyl 4-(N-t-Boc-aminomethyl)benzoate (1.07 g, 81% yield).

To a cold suspension of LiAlH₄ (60 mg, 1.5 mmol) in ether (5 mL) at -78° C was added dropwise a solution of methyl 4-(N-t-Boc-aminomethyl)benzoate (0.8 g, 3 mmol) in ether (5 mL) and the contents were allowed to warm-up to ambient temp. After 3h, further amount of LiAlH₄ (100 mg) was added and the reaction was continued at room temp for further 10 min. Excess LiAlH₄ was destroyed with EtOAc followed by saturated ammonium chloride (1.0 mL). The solid precipitated was filtered and washed with ehter. The filtrate was evaporated to give 4-(N-t-Boc-aminomethyl)benzyl alcohol ( 250 mg, 35 %).

A solution of 4-(N-t-Boc-aminomethl)benzyl alcohol (250 mg, 1.1 mmol ) in THF (5 mL) was treated with phosphorustribromide (30 uL, 0.32 mmol) at -78° C for 2 h. After this period, the reaction was quenched with solid NaHCO₃ (50 mg) and filtered to remove the solids and the filtrate was evaporated to give 4-(t-Boc-aminomethyl)benzyl bromide (300 mg, 95%)

The desired *E* isomers were separated from the corresponding *E*/*Z* mixtures by crystallization in ethylacetate/ethylether solutions. In the case of cinnamyl and few other analogs with unimpressive *E*/*Z* selectivity, the crude mixture was subjected to isomerization using the follwing illustrative procedure described for cinnamyl analog, cryptophycin-152.

**Isomerization:** A mixture of cryptophycin-152 and its *cis* isomer (*E*/*Z* 7:3, 62 mg, 0.10 mmol) was dissolved in benzene (3 mL) and refluxed with thiophenol (10 µL, 0.10 mol) and 1,1' - azobis(cyclohexanecarbonitrile) (12 mg, 0.05 mmol). After 16 h, the mixture was brought to the ambient temp and applied to a small silica column. The column was washed with dichloromethane and the compound was eluted with 1:1 ethylaceatet/dichloromethane. The solvent was evaporated to yield cryptophycin-151 (53 mg, 85%), which still contaminated with approximately 5% of its *cis* isomer.

**Cryptophycin-151:** EIMS m/z (relative intensity) 688/690 (3.3/1.5), 412/414 (18/6), 277 (100), 233 (18), 195/197 (16/6), 193 (29), 141 (38); high resolution EIMS m/z 688.2900 (calcd for C₃₉H₄₅ClN₂O₇ Δ 1.5 mmu). ¹H NMR data, see table 3; ¹³C NMR data, see table **4**.

**Cryptophycin-152:** EIMS m/z (relative intensity) 664/666 (24/7), 412/414 (21/7), 253 (100), 91 (85); high resolution EIMS m/z 664.2939 (calcd for C₃₇H₄₅ClN₂O₇, Δ - 2.3 mmu) . ¹H NMR data, see table 3; ¹³C NMR data, see table **4**.

**Cryptophycin-153:** EIMS m/z (relative intensity) 668/670 (3.7/1.4), 412/414 (32/12), 257 (62), 198 (100), 195/197 (36/11); high resolution EIMS m/z 668.2817 (calcd for C₃₆H₄₅ClN₂O₈, Δ 4.7 mmu). ¹H NMR data, see table **3**; ¹³C NMR data, see table **4**.

**Cryptophycin-154:** EIMS m/z (relative intensity) 698/700 (2.5/0.8), 412/414 (18/5), 287 (28), 228 (35), 195/197 (18/6), 139 (100); high resolution EIMS m/z 698.2946 (calcd for C₃₇H₄₇ClN₂O₉, Δ 2.5 mmu). ¹H NMR data, see table **3**; ¹³C NMR data, see table 4.

**Cryptophycin-155:** EIMS m/z (relative intensity) 604/606 (20/6), 412/414 (24/8), 280/282 (24/8), 195/197 (100/33); high resolution EIMS m/z 604.2894 (calcd for C₃₂H₄₅ClN₂O₇, Δ 2.1 mmu). ¹H NMR (CDCl₃) amino or *hydroxy acid unit* δ (carbon position, multiplicity; J in Hz) A 5.76 (2, d; 15.5), 6.66 (3, ddd; 15.5, 9.5 and 5.9), 2.32 - 2.44 (4-H₂, m), 4.92 (5, m), 2.66 - 2.76 (6, m), 0.98 (6-Me, d; 6.8), 5.21 (7, t; 10.9), 5.96 (8, dt; 10.9 and 7.4), 2.00 (9- H₂, m), 1.38 (10-H₂, m), 0.91 (10-Me, t; 7.5); B 4.81 (2, m), 5.70 (2-NH, d; 8.5), 3.04 (3, dd; -14.5 and 7.2), 3.14 (3, dd; -14.5 and 5.6), 7.22 (5, d; 2.2), 3.87 (7-OMe, s), 6.84 (8, d; 8.5), 7.08 (9, dd; 8.5 and 2.2); *C* 2.66 - 2.76 (2, m), 1.22 (2-Me, d; 7.4), 3.27 (3, dt; 13.5 and 6.8), 3.52 (3, m), 6.93 (3-NH, br t; 6.4); *D* 4.86 (2, dd; 9.8 and 3.5), 1.49 (3, m), 1.71 - 1.80 (3/4, m), 0.90 (4-Me, d; 6.0), 0.94 (5, d; 6.5); ¹³C NMR (CDCl₃) *unit* δ (carbon position) *A* 165.5 (1), 125.0 (2), 141.6 (3), 36.5 (4), 77.9 (5), 36.2 (6), 17.7 (6-Me), 130.0 (7), 131.6 (8), 29.6 (9-H₂), 22.8 (10-H₂), 13.8 (11-H₃); ***B*** 171.0^{a} (1), 53.5 (2), 35.1 (3), 129.9 (4), 131.0 (5), 122.4 (6), 153.9 (7), 56.1 (7-OCH₃), 112.2 (8), 128.4 (9); *C* 175.6 (1), 38.3 (2), 14.0 (2-Me), 41.2 (3); *D* 170.9^{a}(1), 71.6 (2), 39.5 (3), 24.7 (4), 21.4 (4-Me), 23.1 (5). ^{a} signals are interchangeable.

**Cryptophycin-156:** EIMS m/z (relative intensity) 412/414 (23/6), 381 (11), 280/282 (22/6), 195/197 (100/33); high resolution EIMS m/z 592.2568 (calcd for C₃₀H₄₁ClN₂O₈, Δ -1.6 mmu). ¹H NMR data, see table 3; ¹³C NMR data, see table 4.

**Cryptophycin-159:** EIMS m/z (relative intensity) 666 (3), 412/414 (16/4), 396 (52), 255 (100), 195/197 (30/8), 91 (54); high resolution EIMS m/z 666.3060 (calcd for C₃₇H₄₇ClN₂O₇, Δ 1.1 mmu). ¹H NMR data, see table 3; ¹³C NMR data, see table **4**.

**Cryptophycin-160:** EIMS m/z (relative intensity) 628/630 (9/4), 412/414 (63/23), 280 (25), 217 (89), 195/197 (89/30); high resolution EIMS m/z 628.2532 (calcd for C₃₃H₄₁ClN₂O₈, Δ 2.0 mmu). ¹H NMR data, see table 3; ¹³C NMR data, see table **4**.

**Cryptophycin-161:** EIMS m/z (relative intensity) 706/708 (6/2), 412/414 (49/18), 295 (15), 280/282 (25/7), 195/197 (100/34); high resolution EIMS m/z 706.2623 (calcd for C₃₆H₄₂ClF₃N₂O₇, Δ 1.0 mmu). ¹H NMR data, see table 3; ¹³C NMR data, see table **4**.

**Cryptophycin-166:** EIMS m/z (relative intensity) 652/654 (1.1/0.4), 412/414 (18/5), 241 (37), 195/197 (64/20); high resolution EIMS m/z 652.2918 (calcd for C₃₆H₄₅ClN₂O₇, Δ - 0.2 mmu). ¹H NMR data, see table 3; ¹³C NMR data, see table **4**.

**Cryptophycin-167:** EIMS m/z (relative intensity) 666/668 (3.4/1.1), 412/414 (26/9), 280 (11), 255 (67), 195/197 (46/15); high resolution EIMS m/z 666.3058 (calcd for C₃₇H₄₇ClN₂O₇, Δ 1.4 mmu). ¹H NMR data, see table 3; ¹³C NMR data, see table **4**.

**Cryptophycin-172:** EIMS m/z (relative intensity) 688/690 (3/2), 412/414 (12/4), 277 (67), 218 (100), 195/197 (29/10), 141 (63); high resolution EIMS m/z 688.2916 (calcd for C₃₉H₄₅ClN₂O₇, Δ - 0.1 mmu) . ¹H NMR data, see table 3; ¹³C NMR data, see table **4**.

**Cryptophycin-181:** EIMS m/z (relative intensity) 656/658 (9.0/3.3), 412/414 (84/34), 245 (71), 195/197 (47/7); high resolution EIMS m/z 656.2674 (calcd for C₃₅H₄₂ClFN₂O₇, Δ - 0.9 mmu). ¹H NMR data, see table **3**; ¹³C NMR data, see table **4**.

**Cryptophycin-188:** EIMS m/z (relative intensity) 674/676 (20/4), 412/414 (57/20), 280/282 (20/7), 263 (13), 195/197 (89/27); high resolution EIMS m/z 674.2551 (calcd for C₃₅H₄₁ClF₂N₂O₇, Δ 1.9 mmu) . ¹H NMR data, see table **3**; ¹³C NMR data, see table **4**.

**Cryptophycin-234:** The mixture containing cryptophycin-234 and 15% of its z isomer (58.3 mg) was repeatedly crystallized in EtOAc/ethyl ether solutions to obtain cryptophycin-234 (47 mg) and its z isomer (11 mg).¹H NMR (CDCl₃) *amino or hydroxy acid unit* δ (carbon position, multiplicity; J in Hz) A 5.77 (2, d; 15.2), 6.68 (3, ddd; 15.2, 9.7 and 5.4), 2.37 (4, dt; 14.2 and 10.5), 2.52 (4, m), 4.99 (5, m), 2.55 (6, m), 1.13 (6-Me, d; 6.8), 5.99 (7, dd; 15.8 and 8.8), 6.40 (8, d; 15.8), 7.25^{a} (2'/6', d; 8.2), 7.29^{a} (3'/5', d; 8.2), 4.71 (4'-CH₂OTBDMS, brs), 0.08 (6H) and 0.93 (9H) (4'-CH₂OTBDMS); *B* 4.81 (2, m), 5.77 (2-NH, obscured by other signal), 3.03 (3, dd; -14.4 and 7.3), 3.14 (3, dd; - 14.4 and 5.4), 7.22 (5, d; 2.0), 3.86 (7-OMe, s), 6.83 (8, d; 8.4), 7.07 (9, dd; 8.4 and 2.0); C 2.71 (2, m), 1.22 (2-Me, d; 7.2), 3.29 (3, m), 3.49 (3, m), 6.98 (3-NH, br t; 5.6); *D* 4.84 (2, m), 1.36 (3, m), 1.59 - 1.71 (3/4, m), 0.73 (4-Me, d, 6.4), 0.77 (5, d, 6.2). ^{a} Interchangeable; ¹³C NMR (CDCl₃) unit δ (carbon position) ***A*** 165.5 (1), 125.2 (2), 141.4 (3), 36.4 (4), 77.4 (5), 42.2 (6), 17.3 (6-Me), 129.6 (7), 131.6 (8), 136.4 (1'), 126.0^{a} (2'/6'), 126.3^{a} (3'/5'), 140.9 (4'), 64.7 (4'-CH₂O-), 25.9 and - 5.3 (4'-CH₂OTBDMS); ***B*** 171.0^{b} (1), 53.6 (2), 35.1 (3), 129.9 (4), 131.0 (5), 122.4 (6), 153.9 (7), 56.1 (7-OCH₃), 112.2 (8), 128.4 (9); *C* 175.6 (1), 38.3 (2), 14.0 (2-Me), 41.1 (3); ***D*** 170.9^{b}(1), 71.5 (2), 39.5 (3), 24.5 (4), 22.7 (4-Me), 21.2 (5). ^{a and b}signals are interchangeable.

**Cryptophycin-236:** ¹H NMR (CDCl₃) *amino or hydroxy acid unit* δ (carbon position, multiplicity; *J* in Hz) A 5.76 (2, d; 15.2), 6.68 (3, ddd; 15.2, 9.6 and 5.6), 2.37 (4, m), 2.51 (4, m), 5.00 (5, m), 2.54 (6, m), 1.13 (6-Me, d; 6.9), 6.00 (7, dd; 15.9 and 8.6), 6.39 (8, d; 15.9), 7.20 - 7.30 (2' /3' /5' /6' , br m), 4.28 (4'-CH₂NH-t-Boc, d; 4.9), 4.80 (4' - CH₂NH-t-Boc, d; 4.9), 1.46 (4'-CH₂NH-t-Boc; s); *B* 4.80 (2, m), 5.65 (2-NH, d; 8.5), 3.04 (3, dd; -14.4 and 6.9), 3.13 (3, dd; -14.4 and 5.5), 7.22 (5, d; 2.2), 3.87 (7-OMe, s), 6.83 (8, d; 8.4), 7.08 (9, dd; 8.4 and 2.2); C 2.71 (2, m), 1.22 (2-Me, d; 7.3), 3.27 (3, m), 3.51 (3, m), 6.93 (3-NH, br t; 5.6) ; D 4.84 (2, dd; 9.9 and 3.5), 1.36 (3, m), 1.58 - 1.70 (3/4, m), 0.74 (4-Me, d, 6.4), 0.78 (5, d, 6.4); ¹³C NMR (CDCl₃) unit δ (carbon position) ***A*** 165.4 (1), 125.2 (2), 141.4 (3), 36.5 (4), * (5), 42.2 (6), 17.3 (6-Me), 130.1 (7), 131.4 (8), 135.9 (1'), 126.4 (2'/6'), 127.8 (3'/5'), 138.4 (4'), 44.4 (4'-CH₂NH-t-Boc), 28.4 (4'-CH₂NH-t-Boc); ***B*** 170.9 (1), 53.5 (2), 35.1 (3), 129.9 (4), 131.1 (5), (6), 154.0 (7), 56.2 (7-OCH₃), 112.3 (8), 128.4 (9); C 175.6 (1), 38.3 (2), 14.0 (2-Me), 41.2 (3); D 170.9(1), 71.6 (2), 39.6 (3), 24.5 (4), 22.7 (4-Me), 21.3 (5). * Hidden under solvent signal.

**Cryptophycin-238:** Cryptophycin-234 (14 mg) in THF (1.5 mL) was treated with tetrabutylammonium fluoride solution (25 µL, 1M in THF) at 0_C. After 1 h, saturated NH₄Cl (5 mL) was to the reaction mixture followed by water (15 mL) and extracted with EtOAc (50 mL). The EtoAc layer was dried over MgSO₄ and evaporated. The residue was purified on a small silica column using CH₂Cl₂ and EtOAc as eluants. The latter fraction was evaporated to obtain cryptophycin-238 (11.8 mg). EIMS m/z (relative intensity) 668/670 (3/0.5), 412/414 (38/14), 257 (29), 195/197 (25/10); high resolution EIMS m/z 668.2898 (calcd for C₃₆H₄₅ClN₂O₈, Δ - 3.4 mmu). ¹H NMR (CDCl₃) *amino or hydroxy acid unit δ* (carbon position, multiplicity; *J* in Hz) A 5.76 (2, d; 15.6), 6.68 (3, ddd; 15.6, 9.8 and 5.5), 2.36 (4, dt; 14.4 and 10.4), 2.51 (4, br dd; 14.4 and S.1), 5.00 (5, m), 2.55 (6, m), 1.13 (6-Me, d; 6.8), 6.01 (7, dd; 15.9 and 8.8), 6.40 (8, d; 15.9), 7.31 (2'/3'/5'/6', br s), 4.47 (4'-CH₂OH, brs); *B* 4.80 (2, m), 5.78 (2-NH, d; 8.4), 3.01 (3, dd; -14.4 and 7.4), 3.13 (3, dd; -14.4 and 5.5), 7.21 (5, d; 2.0), 3.86 (7-OMe, s), 6.83 (8, d; 8.5), 7.07 (9, dd; 8.5 and 2.0); C 2.71 (2, m), 1.21 (2-Me, d; 7.1), 3.28 (3, m), 3.49 (3, m), 6.98 (3-NH, br t; 6.0); D 4.84 (2, dd; 9.9 and 3.2), 1.35 (3, m), 1.58 - 1.71 (3/4, m), 0.74 (4-Me, d; 6.4), 0.77 (5, d; 6.3); ¹³C NMR (CDCl₃) unit δ (carbon position) *A* 165.5 (1), 125.2 (2), 141.4 (3), 36.4 (4), 77.4 (5), 42.2 (6), 17.3 (6-Me), 130.1 (7), 131.9 (8), 136.1 (1'), 126.3^{a} (2'/6'), 127.2^{a} (3'/5'), 140.4 (4'), 64.9 (4'-CH₂OH); ***B*** 171.O^{b} (1), 53.7 (2), 35.0 (3), 129.9 (4), 131.0 (5), 122.3 (6), 153.9 (7), 56.1 (7-OCH₃), 112.2 (8), 128.4 (9); C 1.75.6 (1), 38.2 (2), 14.0 (2-Me), 41.1 (3); D 170.8^{b}(1), 71.5 (2), 39.5 (3), 24.5 (4), 22.7 (4-Me), 21.3 (5). ^{a and b}signals are interchangeable.

**Cryptophycin-246:** Cryptophycin-236 (9 mg, 0.012 mmol) in CH₂Cl₂ (50 µL) was treated with 4N HCl in dioxane (20 µL, 0.08 mmol) at room temp. After 1 h, the solvent was evaporated and the residue was subjected to flash chromatography on a small C18 silica column (Alltech, 500 mg) using methanol/water (1:1) for elution. The first fraction (3 mL) after evaporation of the solvent gave cryptophycin-246 (7 mg, 85%). ¹H NMR (CD₃OD) *amino or hydroxy acid unit* δ (carbon position, multiplicity; *J* in Hz) *A* 5.92 (2, dd; 15.2 and 1.7), 6.69 (3, ddd; 15.2, 11.1 and 3.8), 2.35 (4, m), 2.68 (4, m), 5.06 (5, m), 2.63 (6, m), 1.15 (6-Me, d; 7.4), 6.18 (7, dd; 15.9 and 8.9), 6.50 (8, d; 15.9), 7.40 (2' /6' , br d; 8.2), 7.46 (3'/5', br d; 8.2), 4.08 (4'-CH₂NH₂HCl, s); B 4.51 (2, dd; 11.1 and 3.4), 2.75 (3, m), 3.17 (3, dd; -14.5 and 3.9), 7.27 (5, d; 2.0), 3.83 (7-OMe, s), 6.97 (8, d; 8.S), 7.16 (9, dd; 8.5 and 2.0); C 2.73 (2, m), 1.17 (2-Me, d; 8.0), 3.26 (3, m), 3.56 (3, m); D 4.92 (2, dd; 9.8 and 3.9), 1.35 (3, m), 1.54 - 1.65 (3/4, m), 0.71 (4-Me, d; 6.4), 0.75 (5, d; 6.4); ¹³C NMR (CD₃OD) unit δ (carbon position) *A* 168.4 (1), 125.7 (2), 143.5 (3), 37.7 (4), 78.6 (5), 43.5 (6), 17.5 (6-Me), 133.6 (7), 131.9 (8), 139.5^{a} (1'), 128.0 (2'/6'), 130.3 (3'/5'), 133.3^{a} (4'), 44.0 (4'-CH₂NH₂HCl); ***B*** 174.1 (1), 57.4 (2), 36.3 (3), 132.2 (4), 131.5 (5), 123.3 (6), 155.4 (7), 56.6 (7-OCH₃), 113.5 (8), 129.3 (9); C 177.5 (1), 39.0 (2), 15.1 (2-Me), 41.2 (3); D 172.3 (1), 72.8 (2), 41.0 (3), 25.7 (4), 21.7 (4-Me), 23.2 (5). ^{a} signals are interchangeable.

**Cryptophycin-250:** A solution of cryptophycin-247 (5.1 mg) in dichloromethane (80 µL) was treated with hydrochloric acid (40 µL, 4N in dioxane). After 2 h, the reaction mixture was concentrated, diluted with water and passed through a short ODS column. The column was washed with water (5 mL) followed by CH₃CN (3mL). The latter fraction was evaporated to obtain cryptophycin-250 (5 mg). It was further purified on a reversed phase HPLC (Econosil C18, 25 cm x 10 mm, 10 µ, 35% H₂O/CH₃CN, 4 mL/min) to obtain a pure sample (4 mg, t_{R} 18 min). EIMS m/z (relative intensity) 653/655 (15/10), 533 (33), 242 (48), 195/197 (36/13); high resolution EIMS m/z 653.2865 (calcd for C₃₅H₄₄ClN₃O₇, Δ 0.3 mmu). ¹H NMR (CDCl₃) *amino or hydroxy acid unit* δ (carbon position, multiplicity; *J* in Hz) A 5.78 (2, d; 15.1), 6.68 (3, ddd; 15.1, 9.6 and 5.9), 2.35 (4, dt; 14.4 and 10.7), 2.98 (4, br dd; 14.4 and 5.1), 5.01 (5, m), 2.53 (6, m), 1.12 (6-Me, d; 6.8), 6.01 (7, dd; 15.9 and 8.7), 6.33 (8, d; 15.9), 6.81 m, 6.89 d, 6.94 s and 6.99 m (2'/3'-NH₃/4'/6'), 7.15 (5', t; 7. 8) ; B 4.79 (2, m), 5.95 (2-NH), 3.02 (3, dd; -14.4 and 7.3), 3.15 (3, dd;-14.4 and 5.5), 7.22 (5, d; 2.0), 3.86 (7-OMe, s), 6.84 (8, d; 8.4), 7.08 (9, dd; 8.4 and 2.0); C 2.70 (2, m), 1.23 (2-Me, d; 7.1), 3.27 (3, m), 3.51 (3, m), 6.99 (3-NH, m); D 4.87 (2, dd; 9.8 and 3.2), 1.39 (3, m), 1.59 - 1.73 (3/4, m), 0.76 (4-Me, d, 5.4), 0.80 (5, d, 5.6); A 165.8 (1), 125.2 (2), 141.5 (3), 36.4 (4), 77.3 (5), 42.0 (6), 17.1 (6-Me), 131.0 (7), 131.3 (8), 138.3 (1'). 116.0 (2'), 121.1 (4'), 130.0 (5'), 118.0 (6'); ***B*** 171.2 (1), 54.0 (2), 35.0 (3), 129.8 (4), 131.0 (5), 122.4 (6), 153.9 (7), 56.1 (7-OCH₃), 112.3 (8), 128.4 (9); C 175.6 (1), 38.2 (2), 14.2 (2-Me), 91.0 (3); *D* 170.9 (1), 71.5 (2), 39.6 (3), 24.6 (4), 22.8 (9-Me), 21.4 (5).

**Cryptophycin-251:** EIMS m/z (relative intensity) 696 (0.7), 652 (M⁺ - CO₂; 0.7), 412/414 (4/2), 285 (6), 241 (5), 195/197 (11/3) high resolution EIMS m/z (calcd for C₃₇H₄₅ClN₂O₉, .△ mmu). ¹H NMR data, see table 3; ¹³C NMR data, see table **4**.

**Cryptophycin-255:** ¹H NMR (CDCl₃) *amino or hydroxy acid unit* δ (carbon position, multiplicity; *J* in Hz) *A* 5.78 (2, d; 15.1), 6.67 (3, ddd; 15.1, 9.5 and 5.9), 2.37 (4, dt; 14.9 and 10.3), 2.45 (4, m), 4.96 (5, m), 2.52 (6, m), 1.09 (6-Me, d; 6.8), 5.74 (7, dd; 15.1 and 8.9), 6.24 (8, d; 15.1), 7.20 - 7.33 (2'/3'/4'/5'/6', m); *B* 4.83 (2, m), 5.64 (2-NH, d; 8.8), 3.05 (3, dd; -14.6 and 7.1), 3.14 (3, dd; -14.6 and 5.4), 7.22 (5, d; 2.0), 3.88 (7-OMe, s), 6.85 (8, d; 8.5), 7.08 (9, dd; 8.5 and 2.0); *C* 2.72 (2, m), 1.23 (2-Me, d; 7.3), 3.29 (3, m), 3.50 (3, m), 6.95 (3-NH, m); *D* 4.86 (2, dd; 10.0 and 3.4), 1.49 (3, m), 1.63 - 1.85 (3/4, m), 0.89 (4-Me, d, 6.4), 0.94 (5, d, 6.4).

**Table 3. 500 MHz ¹H NMR Data for Cryptophycins-3, -151,-152, -153, -154, -156, -159, -160, -161, -166, -167, -172,-181, -188 and 251 P=position**

| **P** | **3** | **151** | **152** | **153** | **154** | **156** | **159** | **160** | **161** | **166** | **167** | **172** | **181** | **188** | **251** |
|---|---|---|---|---|---|---|---|---|---|---|---|---|---|---|---|
| A2 | 5.77 d | 5.78 | 5.77 | 5.77 | 5.77 | 5.76 | 5.76 | 5.77 | 5.78 | 5.77 | 5.77 | 5.78 | 5.78 | 5.78 | 5.76 |
| 3 | 6.68 ddd | 6.71 | 6.67 | 6.68 | 6.68 | 6.66 | 6.68 | 6.68 | 6.68 | 6.68 | 6.68 | 6.70 | 6.68 | 6.68 | 6.68 |
| 4*R* | 2.37 dt | 2.45 | 2.37 | 2.37 | 2.37 | 2.34 | 2.38 | 2.35 | 2.36 | 2.39 | 2.37 | 2.41 | 2.36 | 2.35 | 2.35 |
| 4*S* | 2.54 brdd | 2.58 | 2.47 | 2.52 | 2.52 | 2.41 | 2.51 | 2.49 | 2.53 | 2.52 | 2.51 | 2.55 | 2.54 | 2.52 | 2.51 |
| 5 | 5.01 ddd | 5.10 | 4.97 | 5.00 | 5.00 | 4.88 | 5.02 | 4.96 | 5.02 | 5.03 | 4.99 | 5.04 | 5.00 | 5.01 | 5.00 |
| 6 | 2.56 m | 2.71 | 2.47 | 2.54 | 2.54 | 2.27 | 2.56 | 2.49 | 2.59 | 2.59 | 2.54 | 2.61 | 2.57 | 2.58 | 2.55 |
| 6-Me | 1.14 d | 1.22 | 1.09 | 1.13 | 1.13 | 1.04 | 1.13 | 1.10 | 1.15 | 1.15 | 1.12 | 1.17 | 1.15 | 1.14 | 1.13 |
| 7 | 6.01 dd | 6.09 | 5.63 dd | 6.00 | 5.99 | 4.53 dd | 5.87 | 5.70 | 6.13 | 5.92 | 5.97 | 6.14 | 6.09 | 6.05 | 6.00 |
| 8 | 6.41 d | 7.19 | 6.23 dd | 6.38 | 6.34 | 6.31 d | 6.59 | 6.26 | 6.45 | 6.63 | 6.34 | 6.57 | 6.58 | 6.34 | 6.39 |
| 9 | | | 6.71 dd | | | | | | | | | | | | |
| 10 | | | 6.49 dd | | | | | | | | | | | | |
| 8/10-Ar-1' | | | | | | | | | | | | 7.67 br s | | | |
| 2' | 7.28 to | 7.41 to | 7.36 br d | 6.85 t | 6.68 d | | 2.31 s | 7.37 br s | 7.42 d | 2.33 s | 6.94 br s | | | 6.83 m | 7.30 d |
| 3' | 7.34 m | 7.58 m | 7.30 br t | 3.80 s | 3.79 s | | 6.95 brs | | 7.55 d | 7.11 to | 2.29 s | 7.54 m | 7.09 m | | 7.23 d |
| 4' | 7.23 m | 7.77 d | 7.22 m | 6.78 br d | 6.36 d | | 2.31 s | 6.47 br s | | 7.39 m | 6.87 br s | 7.78 m | 7.40 m | 6.68 m | 3.63 s |
| 5' | 7.28 to | 7.94 d | 7.30 br t | 7.21 t | 3.79 s | | 6.95 d | 7.35 br s | 7.55 d | " | 2.29 s | 7.78 m | 7.02 m | | 7.23 d |
| 6' | 7.34 m | 7.41 to | 7.36 br d | 6.92 d | 6.68 d | | 7.28 d | | 7.42 d | " | 6.94 br s | 7.44 m | 7.19 m | 6.83 m | 7.30 d |
| 7' | | 7.58 d | | | | | | | | | | 7.44 m | | | |
| 8' | | 8.07 d | | | | | | | | | | 7.78 m | | | |
| D2 | 4.84 dd | 4.85 | 4.87 | 4.84 | 4.84 | 4.87 | 4.84 | 4.83 | 4.84 | 4.85 | 4.85 | 4.86 | 4.85 | 4.84 | 4.84 |
| 3 | 1.62 m | 1.64 | 1.72 | 1.65 | 1.66 | 1.75 | 1.68 | 1.67 | 1.65 | 1.69 | 1.66 | 1.67 | 1.66 | 1.67 | 1.36 |
| 3' | 1.36 m | 1.35 | 1.47 | 1.37 | 1.38 | 1.48 | 1.38 | 1.36 | 1.31 | 1.38 | 1.38 | 1.38 | 1.36 | 1.32 | 1.58 to |
| 4 | 1.62 m | 1.64 | 1.72 | 1.65 | 1.66 | 1.75 | 1.68 | 1.67 | 1.65 | 1.69 | 1.66 | 1.64 | 1.66 | 1.67 | 1.70 m |
| 4-Me | 0.77 | d 7.00. | 0.87 | 0.77 | 0.79 | 0.94 | 0.79 | 0.80 | 0.76 | 0.79 | 0.78 | 0.74 | 0.78 | 0.79 | 0.77 |
| 5 | 0.73d | 0.64 | 0.86 | 0.74 | 0.77 | 0.91 | 0.77 | 0.79 | 0.72 | 0.76 | 0.75 | 0.65 | 0.74 | 0.77 | 0.73 |

Spectra recorded in CDC13; The chemical shifts are for the protons or methyl or methoxyl or hydroxy methyl function positioned on the carbon indicated in the table. The chemical shifts for the proptons in units B and C are within ± 0.2 ppm and coupling constants within ± 0.5 Hz of the corresponding values in cryptophycin-3. J (H,H) in Hz for 151: 3' , 4' =8.3; 5',6' = 8.0; 7',8' = 8.1; J (H,H) in Hz for152: 6,7 = 8.7; 7,8 = 15.3; 8,9 = 10.5; 9, 10 = 15.7; 2',3' = 3', 4'= 4',5' = 5',6' = 7.4; J (H,H) in Hz for 153: 2',4' = 2',6' = 2.0; 4',5' = 5',6' = 7.9; J (H,H) in Hz for 154: 2',4' = 4',6' = 2.2; δ for 8-OCH₃ of the unit A in 156 is 3.51: J (H,H) in Hz for 156: 6,7 =9.3; 7,8 = 12.6; J (H,H) in Hz for 159: 5',6' = 8.3; J (H,H) in Hz for 161: 2',3' = 4',5' = 8.1; J (H,H) in Hz for 251: 2' 3' - 5',6' = 8.2; The observable coupling constants for the rest of the protons in the table are with in ± 0.5 Hz of the corresponding values in 3.

**Table 4. 125 MHz ¹³C NMR Data for Cryptophycins-3, -151,-152, -153, -154, -156, -159, -160, -161, -166, -167, -172,-181, -188 and 251. P=position**

| **P** | **3** | **151** | **152** | **153** | **154** | **156** | **159** | **160** | **161** | **166** | **167** | **172** | **181** | **188** | **251** |
|---|---|---|---|---|---|---|---|---|---|---|---|---|---|---|---|
| A 1 | 165 .4 | 165 .5 | 165 .4 | 165 .4 | 165 .4 | 165 .5 | 165 .5 | 165 .6 | 165. 4 | 165 .4 | 165 .5 | 165 .4 | 165 .4 | 165 .3 | 16 5. 5 |
| 2 | 125 .2 | 125 .3 | 125 .1 | 125 .2 | 125 .2 | 125 .0 | 125 .3 | 125 .0 | 125. 3 | 125 .2 | 125 .1 | 125 .1 | 125 .3 | 125 .3 | 12 5. 2 |
| 3 | 141 .4 | 141 .4 | 141 .4 | 141 .4 | 141 .4 | 141 .7 | 141 .5 | 141 .7 | 141. 2 | 141 .5 | 141 .5 | 141 .4 | 141 .3 | 141 .1 | 14 1. 4 |
| 4 | 36. 5 | 36. 6 | 36. 4 | 36. 4 | 36. 5 | 36. 3 | 36. 5 | 36. 4 | 36.5 | 36. 5 | 36. 5 | 36. 5 | 36. 5 | 36. 5 | 35 .0 |
| 5 | 77. 1 | 77. 5 | 77. 3 | 77. 4 | 77. 3 | 77. 9 | 77. 5 | * | 77.1 | 77. 4 | 77. 5 | 77. 4 | * | 77. 1 | 77 .2 |
| 6 | 42. 3 | 38. 3 | 41. 9 | 42. 2 | 42. 2 | 37. 7 | 42. 3 | 42. 2 | 42.3 | 42. 3 | 42. 3 | 42. 3 | 42. 7 | 42. 1 | 42 .2 |
| 6-Me | 17. 3 | 17. 4 | 17. 2 | 17. 3 | 17. 3 | 18. 5 | 17. 4 | 17. 3 | 17.2 | 17. 4 | 17. 4 | 17. 4 | 17. 3 | 17. 2 | 17 .3 |
| 7 | 130 .0 | 133 .3 | 134 .4 | 130 .4 | 130 .6 | 103 .0 | 129 .4 | 129 .8 | 130. 5 | 130 .4 | 129 .3 | 130 .5 | 127 .1 | 133 .1 | 13 0. 2 |
| 8 | 130 .1 | 128 .9 | 132 .3 | 131 .7 | 132 .0 | 148 .6 | 130 .3 | 121 .5 | 133. 0 | 131 .3 | 132 .0 | 131 .8 | 132 .8 | 130 .0 | 13 1. 3 |
| 9 | | | 128 .4 | | | | | | | | | | | | |
| 10 | | | 132 .0 | | | | | | | | | | | | |
| 8/ 10-Ar- 1' | 136 .7 | 134 .4 | 137 .1 | 138 .2 | 138 .7 | 55. 9† | 134 .8 | | 140. 2 | 135 .8 | 136 .6 | 126 .0 | ** | ** | 13 5. 9 9 |
| 2' | 126 .1 | 123 .6 | 126 .2 | 111 .7 | 104 .4 | | 132 .9 | 143 .6 | 125. 6 | 135 .0 | 124 .0 | 134 .1 | ** | 108 .8 d | 12 6. 4 |
| 3' | 128 .6 | 125 .5 | 128 .6 | 159 .9 | 161 .0 | | 131 .8 | 123 .1 | 126. 3 | 127 .5 | 138 .1 | 128 .2 | 115 .8 | ** | 12 9. 6 |
| 4' | 128 .4 | 128 .0 | 127 .5 | 118 .8 | 99. 7 | | 137 .2 | 107 .3 | 141. 2 | 126 .1 | 129 .6 | 126 .3 | 128 .8 | 102 .8 t | 13 2. 8 |
| 5' | 128 .6 | 123 .5 | 128 .6 | 129 .6 | 161 .0 | | 126 .8 | 140 .1 | 126. 3 | 129 .5 | 138 .1 | 127 .8 | 124 .2 | ** | 12 9. 6 |
| 6' | 126 1 | 125 8^{a} | 126 .2 | 113 .1 | 104 .4 | | 125 .2 | | 125. 6 | 125 .4 | 124 .0 | 123 .3 | 124 .2 | 108 .8 | 12 6. 4 |
| 7' | | 126 .1^{a} | | | | | | | | | | 125 .8 | | | |
| 8' | | 128 .6 | | | | | | | | | | 127 .6 | | | |
| 9' | | 131 .0 | | | | | | | | | | 133 .5 | | | |
| 10 | | 133 .7 | | | | | | | | | | 132 .9 | | | |
| D 1 | 170 .1 | 171 .0 | 170 .8 | 170 .9 | 170 .9 | 170 .9 | 170 .9 | 170 .9 | 170. 8 | 170 .9 | 171 .0 | 170 .9 | 170 .8 | 170 .9 | 17 0. 9 |
| 2 | 71. 6 | 71. 5 | 71. 5 | 71. 5 | 71. 6 | 71. 6 | 71. 5 | 71. 6 | 71.5 | 71. 5 | 71. 6 | 71. 5 | 71. 5 | 71. 5 | 71 .5 |
| 3 | 39. 5 | 39. 5 | 39. 6 | 39. 5 | 39. 5 | 39. 6 | 39. 6 | 39. 5 | 39.6 | 39. 6 | 39. 5 | 39. 5 | 39. 5 | 39. 6 | 39 .6 |
| 4 | 24. 5 | 24. 5 | 24. 6 | 24. 5 | 24. 5 | 24. 7 | 24. 5 | 24. 5 | 24.5 | 24. 5 | 24. 5 | 24. 5 | 24. 5 | 24. 5 | 24 .5 |
| 4-Me | 21. 2 | 21. 1 | 21. 4 | 21. 2 | 21. 2 | 21. 4 | 21. 3 | 21. 3 | 21.2 | 21. 2 | 21. 2 | 21. 2 | 21. 2 | 21. 3 | 21 2 |
| 5 | 22. 6 | 22. 7 | 22. 9 | 22. 7 | 22. 7 | 23. 0 | 22. 7 | 22. 8 | 22.7 | 22. 8 | 22. 7 | 22. 6 | 22. 7 | 22. 8 | 22 .7 |

Spectra recorded in CDCl₃ The chemical shifts for carbons in units B and C are within ± 0.5 ppm of the values in cryptophycin-3. * signals submerged under the CDCl₃. signal; ** Signals could not be found. † signal for 8-OCH₃ of the unit A; a signals in a column could be interchangeable. The 3'-OCH₃ carbon of the unit A in cryptophycin-153 was resonated at δ 55.1 and the 3' and 5'-OCH₃ carbons of 154 were resonated at δ 55.3. The 2' and 4' -CH₃ ocarbon signals of 159 were observed at δ 19.7 and 20.9 respectively. The 2'-CH₃ carbon signal of 166 was observed at δ 19.8. The 3' and 5'-CH₃ carbon signas of 167 were observed at δ 21.2. The methylene and carboxyl carbons of 4'-CH₂COOH were observed at δ 40.4 and 174.5 respectively.

### Example 92

### Epoxide Analogs

Cryptophycins-157, 158, 164, 165, 168, 169, 170, 171, 173, 174, 177, 178, 179, 180, 182, 183, 200, 242 and 269.

**Table 5.**

| | | | | |
|---|---|---|---|---|
| 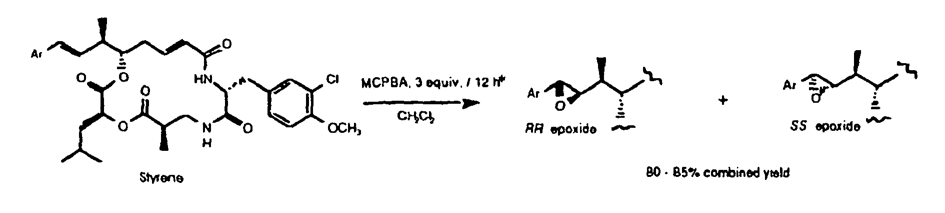 | | | | |

| Styrene Cryptophyc in # | Ar | Epoxide # | Stereo- chemistry | *RR*/*SS* ratio |
|---|---|---|---|---|
| **151** | 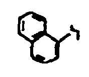 | **157** | *RR* | 2.4: = 1 |
| | | **158** | *SS* | |
| **153** | 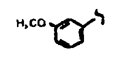 | **168** | *RR* | 2.4 : 1 |
| | | **169** | *SS* | |
| **159** | 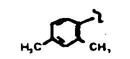 | **170** | *RR* | 2.8 : 1 |
| **166** | 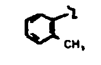 | **177** | *RR* | 2.0 : 1 |
| | | **178** | *SS* | |
| **167** | 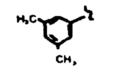 | **179** | *RR* | 2.7 : 1 |
| | | **180** | *SS* | |
| **172** | 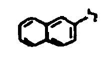 | **173** | *RR* | 1.5 : 1 |
| | | **174** | *SS* | |
| **181** | 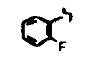 | **182** | *RR* | 2.0 : 1 |
| | | **183** | *SS* | |
| **188** | 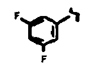 | **200** | *RR* | 1.4 :1 |
| | | | *SS* | |
| **236** | 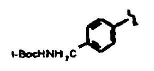 | **242** | *RR* | 2 : 1 |
| | | | *SS* | |
| **238** | 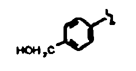 | **269** | *RR* | 2 : 1** |
| | | | *SS* | |

| | | | | |
|---|---|---|---|---|
| * 188 requires 6 equivalents of MCPBA and 36 h for 70% conversion to the epoxides. | | | | |
| ** Inseparable mixture. | | | | |
| * 188 requires 6 equivalents of MCPBA and 36 h for 70% conversion to the epoxides. | | | | |
| ** Inseparable mixture. | | | | |

**General procedure for the epoxidation of styrene analogs:** A solution of styrene analog (0.1 mmol) and m-chloroperoxybenzoic acid (0.3 mmol) in 3 mL of dichloromethane was allowed to stir at room temperature. After 16 h, the reaction mixture was diluted with dichloromethane (3 mL) and washed with phosphate buffer (0.1M, pH 8, 5 mL) to remove the 3-chlorobenzoic acid generated during the reaction. The organic layer was separated, treated with dimethyl sulfide (20 µL) to quench excess peracid and subjected to the buffer wash for the second time. The dichloromethane layer was separated, dried over MgSO₄, evaporated and kept under vacuum for 24 hours. The residue was subjected to HPLC on reversed phase column (Econosil C18, 10 µ, 250 mm x 22 mm, 35% H₂O/CH₃CN, 6 mL/min) to obtain *RR* and *SS* -epoxides.

The experimental details and cytotoxicity data are summarized in table 3.

**Cryptophycins-157 and -158:** Cryptophycin-151 was treated with m-CPBA and the products isolated using reversed phase HPLC to obtain cryptophycins-157 and -158.
Cryptophycin-157: EIMS m/z (relative intensity) 704/706 (1/0.3), 195/197 (14/4), 141 (100), 115 (27); high resolution EIMS m/z 704.2865 (calcd for C₃₉H₄₅ClN₂O₈, - 0.1 mmu error); ¹H NMR data, see table **6;** ¹³C NMR data see table 7.
Cryptophycin-158: EIMS m/z (relative intensity) 704/706 (2.2/1.9), 195/197 (25/9), 141 (100); high resolution EIMS m/z 704.2862 (calcd for C₃₉H₄₅ClN₂O₈, 0.3 mmu error); ¹H NMR data see, table **6**; ¹³C NMR data see table 7.

**Cryptophycins-164 and -165:** A solution of cryptophycin-152 (53 mg, 0.08 mmol) and m-chloroperoxybenzoic acid (15 mg, 0.087 mmol) in 3 mL of dichloromethane was allowed to stir at room temperature for 16 h. The reaction mixture was subjected to the same workup and purification procedure to obtain compounds 164/165A and 164/165B (5 mg each), starting material (10 mg), and other decomposition products. The gross structures were analyzed to be cryptophycins-164 and - 165 but the stereochemistry of the epoxide moiety in the compounds could not be assigned.
Cryptophycin-164/165A: ¹H NMR (CDCl₃) *amino or hydroxy acid unit δ* (carbon position, multiplicity; *J* in Hz) *A* 5.77 (2, d; 15.1), 6.67 (3, ddd; 15.1, 9.7 and 5.4.), 2.37 (4, m), 2.44 (4, m), 4.98 (5, m), 2.48 (6, m), 1.06 (6-Me, d; 6.7), 5.83 (7, dd; 15.6 and 8.5), 5.40 (8, dd; 15.6 and 7.8), 3.32 (9, dd; 7.9 and 2.0), 3.75 (10, d; 2.0), 7.26 (2'/6', m), 7.31-7.37 (3'/4'/5', m); *B* 4.82 (2, m), 5.75 (2-NH, d; 9.2), 3.04 (3, dd; -14.5 and 7.3), 3.14 (3, dd; -14.5 and 5.5), 7.23 (5, d; 2.2), 3.87 (7-OMe, s), 6.84 (8, d; 8.2), 7.09 (9, dd; 8.2 and 2.2); *C* 2.73 (2, m), 1.23 (2-Me, d; 7.2), 3.29 (3, m), 3.51 (3, m), 6.97 (3-NH, br t; 6.3); *D* 4.88 (2, dd; 10.0 and 3.7), 1.52 (3, m), 1.79 (3/4, m), 0.92 (4-Me, d; 6.5), 0.95 (5, d; 6.5). ¹³C NMR (CDCl₃) unit δ (carbon position) *A* 165.4 (1), 125.3 (2), 141.2 (3), 36.5 (4), 77.0 (5), 41.3 (6), 17.0 (6-Me), 136.2 (7), 129.4 (8), 62.5 (9), 60.1 (10), 136.9 (1'). 125.9 (2'/6'), 128.7 (3'/5'), 128.4 (4'); *B* 171.0 (1), 53.6 (2), 35.0(3), 129.9 (4), 131.0 (5), 122.4 (6), 154.0 (7), 56.1 (7-OMe), 112.3 (8), 128.3 (9); *C* 175.6 (1), 38.3 (2), 14.1 (2-Me), 41.2 (3); *D* 170.8 (1), 71.5 (2), 39.6 (3), 29.7 (4), 21.4 (4-Me), 22.9 (5).
Cryptophycin-164/165B: ¹H NMR (CDCl₃) *amino or hydroxy acid unit* δ (carbon position, multiplicity; J in Hz) A 5.76 (2, d; 15.3), 6.66 (3, ddd; 15.3, 9.6 and 5.7.), 2.36 (4, m), 2.40 (4, m), 5.00 (5, m), 2.47 (6, m), 1.08 (6-Me, d; 6.8), 5.85 (7, dd; 15.5 and 8.5), 5.43 (8, ddd; 15.5, 7.6 and 0.6), 3.35 (9, dd; 7.6 and 1.9), 3.75 (10, d; 1.9), 7.27 -7.37 (2'/3'/4'/5'/6' , m); *B* 4.82 (2, m), 5.65 (2-NH, d; 8.5), 3.04 (3, dd; -14.4 and 7.2), 3.14 (3, dd; -14.4 and 5.5), 7.22 (5, d; 2.1), 3.87 (7-OMe, s), 6.84 (8, d; 8.5), 7.08 (9, dd; 8.5 and 2.1); *C* 2.72 (2, m), 1.22 (2-Me, d; 7.2), 3.27 (3, m), 3.52 (3, m), 6.90 (3-NH, br t; 6.3); *D* 4.87 (2, dd; 9.8 and 3.8), 1.48 (3, m), 1.77 (3, m), 1.73 (4, m), 0.89 (4-Me, d; 6.4), 0.90 (5, d; 6.6). ¹³C NMR (CDCl₃) *unit* δ (carbon position) *A* 165.4 (1), 125.2 (2), 191.2 (3), 36.4 (4), 41.3 (6), 16.9 (6-Me), 135.9 (7), 129.1 (8), 62.3 (9), 60.4 (10), 136.8 (1'), 125.5 (2'/6'), 128.6 (3'/5'), 128.4 (4'); *B* 170.9 (1), 53.6 (2), 35.1 (3), 129.8 (4), 131.0 (5), 122.4 4 (6), 154.0 (7), 56.1 (7-OMe), 112.2 (8), 128.4 4 (9); *C* 175.5 (1), 38.3 (2), 19.0 (2-Me), 41.2 (3); *D* 170.8 (1), 71.4 (2), 39.7 (3), 24.7 (4), 21.5 (4-Me), 22.9 (5).

**Cryptophycins-168 and 169:** Cryptophycin-153 was treated with m-CPBA and the products isolated using reversed phase HPLC to obtain cryptophycins-168 and -169.
Cryptophycin-168: EIMS m/z (relative intensity) 684/686 (7/2), 412/414 (17/6), 280/282 (12/6), 257 (20), 212/214 (14/4), 195/197 (69/24), 121 (100); high resolution EIMS m/z 684.2789 (calcd for C₃₆H₄₅ClN₂O₉, 2.6 mmu error); ¹H NMR data, see table 6; ¹³C NMR data, see table 7.
Cryptophycin-169: ¹H NMR data, see table 6; ¹³C NMR data, see table 7.

**Cryptophycins-170 and 171:** Cryptophycin-159 was treated with m-CPBA and the products isolated using reversed phase HPLC to obtain cryptophycins-170 and -171.
Cryptophycin-170: EIMS m/z (relative intensity) 682/684 (4/3), 412/414 (11/4), 280/282 (15/4), 255 (25), 195/197 (52/17); high resolution EIMS m/z 682.3043 (calcd for C₃₇H₄₇ClN₂O₈, - 2.2 mmu error); ¹H NMR data, see table 6; ¹³C NMR data see table 7.
Cryptophycin-171: EIMS m/z (relative intensity) 682/684 (2.0/0.7), 412/414 (7/4), 280/282 (14/4), 255 (19), 195/197 (50/17); high resolution EIMS m/z 682.3006 (calcd for C₃₇H₄₇ClN₂O₈, 1.5 mmu error); ¹H NMR data, see table **6**; ¹³C NMR data, see table **7**.

**Cryptophycins-173 and 174:** Cryptophycin-171 was treated with m-CPBA and the products isolated using reversed phase HPLC to obtain cryptophycins-173 and -174.
Cryptophycin-173: EIMS m/z (relative intensity) 704/706 (1.3/0.4), 412/414 (5.1/1.9), 277 (6.3), 141 (100); high resolution EIMS m/z 704.2838 (calcd for C₃₉H₄₅ClN₂O₈, 2.7 mmu error); ¹H NMR data, see table 6; ¹³C NMR data, see table 7. Cryptophycin-174: ¹H NMR data, see table **6**; ¹³C NMR data, see table 7.

**Cryptophycins-177 and 178:** Cryptophycin-166 was treated with m-CPBA and the products isolated using reversed phase HPLC to obtain cryptophycins-177 and -178.
Cryptophycin-177: EIMS m/z (relative intensity) 668(2.7), 412/414 (9/3), 280/282 (11/4), 241 (16), 195/197 (45/17), 105 (100); high resolution EIMS m/z 668.2835 (calcd for C₃₆H₄₅ClN₂O₈, 2.9 mmu error); ¹H NMR data, see table **6**; ¹³C NMR data, see table 7.
Cryptophycin-178: EIMS m/z (relative intensity) 668/670 (4/1.4), 412/414 (17/6), 280/282 (12/4), 241 (24), 195/197 (59/18), 105 (100); high resolution EIMS m/z 668.2836 (calcd for C₃₆H₄₅ClN₂O₈, 2.9 mmu error); ¹H NMR data, see table 6; ¹³C NMR data, see table **7**.

**Cryptophycins-179 and 180:** Cryptophycin-167 was treated with m-CPBA and the products isolated using the same procedure described above, for the epoxidation of cryptophycin-151, to obtain cryptophycins-179 and -180.
Cryptophycin-179: EIMS m/z (relative intensity) 682/684 (25/13), 412/414 (100/43), 280/282 (100/50), 255 (100), 211 (100), 195/197 (100/100), 121 (100); high resolution EIMS m/z 682.2992 (calcd for C₃₇H₄₇ClN₂O₈, 2.9 mmu error); ¹H NMR data, see table **6**; ¹³C NMR data, see table **7**.
Cryptophycin-180: EIMS m/z (relative intensity) 682/684 (4/3), 412/414 (14/7), 280/282 (16/11), 255 (10), 195/197 (58/21), 119 (100); high resolution EIMS m/z 682.3021 (calcd for C₃₇H₄₇ClN₂O₈, 0 mmu error); ¹H NMR data, see table **6**; ¹³C NMR data, see table **7**.

**Cryptophycins-182 and 183:** Cryptophycin-181 was treated with m-CPBA and the products isolated using reversed phase HPLC to obtain cryptophycins-182 and -183.
Cryptophycin-182: EIMS m/z (relative intensity) 672 (1), 412/414 (11/4), 280/282 (10/4), 245 (12), 195/197 (40/12), 109 (77); high resolution EIMS m/z 672.2590 (calcd for C₃₅H₄₂ClFN₂O₈, 2.3 mmu error); ¹H NMR data, see table **6**; ¹³C NMR data, see table **7**.
Cryptophycin-183: EIMS m/z (relative intensity) 672/674 (5/2), 412/414 (32/12), 280/282 (13/4), 245 (29), 195/197 (92/30), 109 (100); high resolution EIMS m/z 672.2620 (calcd for C₃₅H₄₂ClFN₂O₈, - 0.7 mmu error); ¹H NMR data, see table 6; ¹³C NMR data, see table 7.

**Cryptophycins-200:** Cryptophycin-188 was treated with m-CPBA and the products isolated using the same general procedure, except an additional quantity of peracid (3 equivalents) was added after 12 h and continued the stirring for 36 h to obtain cryptophycins-200 and the SS-epoxide.
Cryptophycin-200: EIMS m/z (relative intensity) 690/692 (42/16), 412/414 (7/1), 263 (19), 195/197 (27); high resolution EIMS m/z 690.2494 (calcd for C₃₅H₄₁ClF₂N₂O₈, 2.6 mmu error); ¹H NMR data, see table 6; ¹³C NMR data, see table **7**.

**Cryptophycin-242:** Cryptophycin-236 was treated with m-CPBA and the products isolated using the same general procedure to obtain cryptophycins-242 and its *SS-*-epoxide. Cryptophycin-242 was purified by normal phase HPLC (Econosil Si, 250 X 10 mm, 10 u, EtOAc ; Hexanes, 1:1, 3 mL/min, t_{R} : 81 min).

Cryptophycin-242: ¹H NMR (CDCl₃) *amino or hydroxy acid unit δ* (carbon position, multiplicity; *J* in Hz) A 5.73 (2, d; 15.4), 6.64 (3, ddd; 15.4, 10.2 and 5.0), 2.42 (4, m), 2.57 (4, m), 5.14 (5, m), 1.79 (6, m), 1.12 (6-Me, d; 6.7), 2.93 (7, dd; 8.9 and 1.6), 3.69 (8, d; 1.6), 7.26^{a} (2'/6', d; 8.2), 7.22^{a} (3'/5', d; 8.2), 4.28 (4'-CH₂NH-t-Boc, d; 5.9), 4.96 (4'-CH₂NH-t-Boc, brs ), 1.44 (4'-CH₂NH-t-Boc, s); *B* 4.66 (2, m), 5.66 (2-NH, d; 7.8), 2.92 (3, dd; -14.5 and 7.2), 3.14 (3, dd; -14.5 and 5.0), 7.21 (5, d; 2.0), 3.85 (7-OMe, s), 6.88 (8, d; 8.5), 7.09 (9, dd; 8.5 and 2.0); C 2.68 (2, m), 1.17 (2-Me, d; 7.4), 3.33 (3, m), 3.39 (3, m), 6.95 (3-NH, br t; 6.1); D 4.85 (2, dd; 10.0 and 3.3), 1.37 (3, m), 1.67 - 1.73 (3/4, m), 0.84 (4-Me, d; 6.7), 0.86 (5, d; 6.7); ¹³C NMR (CDCl₃) unit δ (carbon position) *A* 165.7 (1), 125.4 (2), 141.7 (3), 37.2 (4), 76.6 (5), 40.9 (6), 13.7 (6-Me), 63.4 (7), 59.0 (8), 136.4 (1'), 126.2^{b} (2'/6'), 127.9^{b} (3'/5'), 140.2 (4'),94.5 ((4'-CH₂NH-t-Boc), 28.5 ((4'-CH₂NH-t-Boc); ***B*** 171.2 (1), 54.6 (2), 35.5 (3), 130.5 (4), 131.2 (5), 122.5 (6), 154.4 (7), 56.5 (7-OCH₃), 112.7 (8), 128.8 (9); *C* * (1), 38.6 (2), 14.4 (2-Me), 41.1 (3); *D* 171.2 (1), 71.6 (2), 39.9 (3), 24.9 (4), 23.1 (4-Me), 21.4 (5). ^{a and b}signals with identical superscript are interchangeable.

**Cryptophycin-269:** Cryptophycin-243 (6 mg) in acetone (1.2 mL) was treated with solid K₂CO₃ under vigorous stirring in a reaction vial at room temperature. After 12 h, the reaction mixture was filtered and the solvent evaporated. The residue was purified on a short silica column using CH₂Cl₂ and EtoAc mixtures to obtain cryptophycin-269 (5.1 mg). ¹H NMR (CDCl₃) *amino or hydroxy acid unit* δ (carbon position, multiplicity; *J* in Hz) A 5.70 (2, d; 15.4), 6.67 (3, m), 2.42 (4, dt; 14.4 and 10.4), 2.53 (4, m), 5.11 - 5.19 (5, m), 1.63 - 1.83 (6, m), 1.14 (6-Me, d; 6.8), 2.92 (7, d; 7.6), 3.69 (8, br s), 7.37^{a} (2' /6' , d; 7.9), 7.24^{a} (3' /5' , d; 7.9), 4.71 (4'-CH₂OH, s); *B* 4.74 - 4.85 (2, m), 5.76 (2-NH, d; 8.3), 2.98 (3, dd; -14.5 and 7.7), 3.13 (3, dd; -14.5 and 5.3), 7.20 (5, br s), 3.86 (7-OMe, s), 6.82 (8, d; 8.3), 7.05 (9, br d; 8.3); *C* 2.68 (2, m), 1.14 (2-Me, d; 7.3), 3.32 (3, m), 3.45 (3, m), 6.98 (3-NH, br m); *D* 4.74 - 4.85 (2, m), 1.35 (3, m), 1.63 - 1.83 (3/4, m), 0.85 (4-Me, d; 6.1), 0.87 (5, d; 6.1).
^{a} signals with identical superscript are interchangeable.

**Table 6. ¹H NMR Data for Cryptophycins-1, -157, -158, -168, -169, -170, -173, -174, -177, -178, -179, -180, -182, -183 and 200. P=position**

| **P** | **1** | **157** | **158** | **168** | **169** | **170** | **173** | **174** | **177** | **178** | **179** | **180** | **182** | **183** | **200** |
|---|---|---|---|---|---|---|---|---|---|---|---|---|---|---|---|
| A 2 | 5.74 d | 5.72 | 5.82 | 5.73 | 5.82 | 5.75 | 5.73 | 5.83 | 5.76 | 5.82 | 5.74 | 5.82 | 5.76 | 5.81 | 5.75 |
| 3 | 6.68 ddd | 6.70 | 6.72 | 6.67 | 6.70 | 6.68 | 6.68 | 6.72 | 6.69 | 6.70 | 6.66 | 6.71 | 6.69 | 6.70 | 6.67 |
| 4*R* | 2.45 dt | 2.56 | 2.72 m | 2.45 | 2.67 m | 2.49 | 2.46 | 2.70 m | 2.51 | 2.58 m | 2.45 | 2.57 m | 2.55 | 2.57 | 2.42 |
| 4*S* | 2.55 brdd | 2.63 | 2.62 m | 2.54 | 2.56 m | 2.54 | 2.59 | 2.60 m | 2.54 | 2.69 m | 2.54 | 2.69 m | 2.56 | 2.68 | 2.53 |
| 5 | 5.16 ddd | 5.24 | 5.21 | 5.15 | 5.14 m | 5.17 | 5.18 | 5.17 | 5.18 | 5.15 m | 5.14 | 5.13 m | 5.17 | 5.15 | 5.14 |
| 6 | 1.80 m | 2.04 | 1.93 m | 1.78 | 1.78 | 1.86 | 1.85 | 1.83 | 2.87 | 1.77 | 1.76 | 1.75 | 1.82 | 1.79 | 1.81 |
| 6-Me | 1.14d | 1.20 | 1.20 | 1.14 | 1.05 | 1.14 | 1.18 | 1.08 | 1.14 | 1.10 | 1.14 | 1.14 | 1.14 | 1.08 | 1.11 |
| 7 | 2.92d d | 2.99 | 2.92 | 2.90 | 2.87 | 2.86 | 3.03 | 3.01 | 2.87 | 2.80 | 2.92 | 2.89 | 2.90 | 2.87 | 2.85 |
| 8 | 3.69d | 4.41 | 4.26 | 3.66 | 3.58 | 3.85 | 3.85 | 3.77 | 3.88 | 3.74 | 3.61 | 3.53 | 4.01 | 3.88 | 3.66 |
| 9 | | | | | | | | | | | | | | | |
| 10 | | | | | | | | | | | | | | | |
| 8/10-Ar-1' | | | | | | | 7.77 br s | 7.75 br s | | | | | | | |
| 2' | 7.25 m | 7.41 d | 7.42 br d | 6.77 br s | 6.76 br s | 2.38 s | | | 2.42 s | 2.40 s | 6.85 br s | 6.86 br s | | | 6.77 m |
| 3' | 7.34 to | 7.46 t | 7.47 dd | 3.81 s | 3.81 s | 6.99 brs | 7.29 dd | 7.30 d | 7.11 to | 7.15 to | 2.31 s | 2.31 | 7.05 to | 7.03 to | |
| 4' | 7.39 m | 7.82 d | 7.82 br d | 6.87 br d | 6.85 d | 2.30 s | 7.82 d | 7.83 to | 7.24 in | 7.22 m | 6.96 br s | 6.95 br s | 7.33 m | 7.31 m | 6.77 m |
| 5' | " | 7.91 d | 7.91 d | 7.27 t | 7.26 t | 7.00 s | 7.84 d | 7.83 m | " | " | 2.31 s | 2.31 s | " | " | |
| 6' | 7.25 m | 7.53 td | 7.53 ddd | 6.85 d | 6.85 d | 7.00 s | 7.50 m | 7.46 to | " | " | 6.85 br s | 6.86 br s | " | " | 6.77 in |
| 7' | | 7.57 td | 7.57 ddd | | | | 7.50 m | 7.52 m | | | | | | | |
| 8' | | 8.08 d | 8.07 d | | | | 7.84 d | 7.83 m | | | | | | | |
| D 2 | 4.83 dd | 4.81 | 4.91 | 4.82 | 4.91 | 4.83 | 4.81 | 4.92 | 4.84 | 4.90 | 4.82 | 4.92 | 4.84 | 4.91 | 4.84 |
| 3 | 1.36 m | 1.34 | 1.48 | 1.36 | 1.50 | 1.36 | 1.31 | 1.49 | 1.39 | 1.50 | 1.33 | 1.51 | 1.39 | 1.56 | 1.36 |
| 3' | 1.70 m | 1.68 | 1.71 | 1.70 | 1.74 | 1.70 | 1.66 | 1.72 | 1.73 | 1.68 | 1.71 | 1.75 | 1.72 | 1.76 | 1.67 to |
| 4 | 1.70 m | 1.63 | 1.71 | 1.70 | 1.74 | 1.70 | 1.64 | 1.72 | 1.73 | 1.68 | 1.71 | 1.75 | 1.72 | 1.76 | 1.80 m |
| 4-Me | 0.86 d | 0.78 | 0.87 | 0.87 | 0.91 | 0.85 | 0.79 | 0.87 | 0.86 | 0.91 | 0.85 | 0.92 | 0.87 | 0.92 | 0.89 |
| 5 | 0.85 d | 0.74 | 0.85 | 0.85 | 0.89 | 0.84 | 0.75 | 0.87 | 0.85 | 0.89 | 0.84 | 0.89 | 0.85 | 0.90 | 0.87 |

Spectra recorded in CDCl₃; The chemical shifts are for the proton or methyl or methoxyl function positioned on the carbon indicated in the table. The chemical shifts for the proptons in units B and C are within ± 0.2 ppm and coupling constants within ± 0.5 Hz of the values for those in cryptophycin-1. J (H,H) in Hz for 157: 2',3' - 3', 4' = 7.7; 5',6' = 6',7' = 7',8' = 7.9; 5',7' = 1.5; 6',8' = 1.1; J (H,H) in Hz for 168: 4',5' = 5',6' = 8.0; J (H,H) in Hz for 173: 3',4' = 8.5; 1',3'= 1.4; 5',6'= 7',8' = 8.3; The observable couplings for protons on the aryl segment of 158, 169 and 174 are within ± 0.5 Hz of the values of 157, 168 and 173 respectively. The observable coupling constants for the rest of the protons in the table are with in ± 0.5 Hz of the values in cryptophycin-1.

**Table 7. ¹³C NMR Data for Cryptophycins-157, -158, -168, - 169, -170, -173, -174, -177, -178, -179, -180, -182, -183 and 200. P=position**

| **P** | **157** | **158** | **168** | **169** | **170** | **173** | **174** | **177** | **178** | **179** | **180** | **182** | **183** | **200** |
|---|---|---|---|---|---|---|---|---|---|---|---|---|---|---|
| A 1 | 165. 3 | 165 .5 | 165 .3 | 165 .5 | 165 .3 | 165 .3 | 165 .4 | 165 .4 | 165 .5 | 165. 3 | 165 .5 | 165 .3 | 165. 4 | 165 .3 |
| 2 | 125. 3 | 125 .2 | 125 .3 | 125 .2 | 125 .3 | 125 .3 | 125 .3 | 125 .3 | 125 .3 | 125. 2 | 125 .2 | 125 .2 | 125. 3 | 125 .4 |
| 3 | 141. 0 | 141 .4 | 141 .0 | 141 .4 | 141 .0 | 141 .0 | 141 .4 | 141 .1 | 141 .4 | 141. 2 | 141 .5 | 141 .2 | 141. 3 | 140 .8 |
| 4 | 36.6 | 36.8 | 36. 7 | 36. 7 | 36. 6 | 36. 8 | 36. 8 | 36. 5 | 36. 8 | 36.8 | 36.7 | 36. 7 | 36.7 | 36.7 |
| 5 | 76.1 | * | 76.1 | * | 76.0 | 76.2 | * | 76. 0 | * | 76.2 | * | 76. 2 | 76.8 | 75.9 |
| 6 | 40.1 | 41. 0 | 40. 6 | 41. 0 | 40. 3 | 40. 7 | 41. 0 | 40. 2 | 41. 0 | 40.8 | 41. 0 | 40. 5 | 40.9 | 40. 2 |
| 6-Me | 13.1 | 13. 8 | 13.6 | 13.5 | 13.3 | 13.6 | 13.5 | 13.1 | 13.8 | 13.7 | 13. 6 | 13. 7 | 13.3 | 13. 3 |
| 7 | 62.6 | 62.7 | 63.0 | 63.1 | 62.4 | 63.2 | 64.3 | 62.4 | 62.7 | 62.9 | 63. 1 | 62. 5 | 62.6 | 63. 3 |
| 8 | 56.3 | 54.7 | 58.9 | 56.3 | 56.3 | 59.3 | 56.5 | 56.2 | 54.5 | 59.2 | 56.4 | 53.4 | 51.1 | 57. 6 |
| 8/ 10-Ar-1' | 132.6 | 133.0 | 138.4 | 138.8 | 137.8 | 125.3 | 124.9 | 134.9 | 135.3 | 136.6 | 137.0 | 124.3 | ** | 141.1 m |
| 2' | 122. 4 | 122 .4^{†} | 110 .9 | 110 .5 | 135 .8 | 133 .5 | 133 .3 | 135 .9 | 135 .6 | 123. 4 | 123.1 | 161.2 | ** | 108 .4 d |
| 3' | 125. 5 | 125 .5 | 160 .0 | 160 .0 | 131 .0 | 122 .6 | 122 .7 | 130 .2 | 129 .9 | 138. 4 | 138 .3 | 115 .3 | 115. 2 | 163 .4 dd |
| 4' | 128. 5 | 128 .3 | 114 .0 | 111 4.0 | 131 .8 | 128 .7 | 128 .5 | 128 .0 | 128 .8 | 130. 3 | 128 .4 | 129 .7 | 129. 5 | 103 .9 t |
| 5' | 129. 0 | 128 .9 | 129 .8 | 129 .7 | 124 .3 | 127 .8 | 127 .7^{a} | 124 .2 | 124 .0 | 138. 4 | 138 .3 | 124 .5 | 129. 5 | 163 .4 dd |
| 6' | 126. 0 | 126 .0 | 118 .0 | 117 .9 | 127 .0 | 126 .3^{a} | 126 .2^{b} | 126 .4 | 126 .3 | 123. 4 | 123 .1 | 126 .1 | 124. 4 | 108 .4 |
| 7' | 126. 6 | 126 .5 | | | | 126 .6^{a} | 126 .5^{b} | | | | | | | |
| 8' | 122. 3 | 122 .3^{†} | | | | 127 .8 | 127 .8^{a} | | | | | | | |
| 9' | 131. 4 | 131 .2 | | | | 133 .2 | 133 .2 | | | | | | | |
| 10 | 133. 4 | 133 .3 | | | | 134 .2 | 134 .5 | | | | | | | |
| D 1 | 170. 7 | 170 .8 | 170 .7 | 170 .9 | 170 .7 | 170 .8 | 170 .9 | 170 .7 | 170 .9 | 170. 8 | 171 .9 | 170 .7 | 170. 8 | 170 .7 |
| 2 | 71.4 | 71. 5 | 71. 3 | 71. 5 | 71.3 | 71. 3 | 71. 5 | 71.4 | 71.5 | 71.3 | 71. 5 | | 71.5 | 71. 3 |
| 3 | 39.4 | 39.3 | 39.4 | 39.3 | 39.4 | 39.4 | 39.3 | 39.5 | 39.3 | 39.4 | 39.3 | 39.4 | 39.3 | 39.5 |
| 4 | 24.5 | 24. 6 | 24. 5 | 24. 7 | 24. 5 | 24. 5 | 24. 7 | 24. 6 | 24. 7 | 24.5 | 24. 7 | 24. 6 | 24.7 | 24. 6 |
| 4-Me | 22.8 | 23.0 | 21. 3 | 21. 4 | 21. 3 | 21. 2 | 21. 4 | 21. 4 | 21. 4 | 21.2 | 21.4 | 21. 3 | 21.4 | 21.3 |
| 5 | 21.3 | 21. 3 | 22. 9 | 23. 1 | 22. 8 | 22. 8 | 23. 0 | 22. 9 | 23. 1 | 22.9 | 23. 1 | 22. 9 | 23.1 | 22. 9 |

| | | | | | | | | | | | | | | |
|---|---|---|---|---|---|---|---|---|---|---|---|---|---|---|
| Spectra recorded in CDCl₃, The chemical shifts for carbons in units B and C are within ± 0.5 ppm of the values in cryptophycin-1. | | | | | | | | | | | | | | |
| *Signals submerged under the CDCl₃ signal. | | | | | | | | | | | | | | |
| ** Signals could not be found. | | | | | | | | | | | | | | |
| ^{†}, a and b signals with identical super scripts in a column are interchangeable. | | | | | | | | | | | | | | |

The 3'-OCH₃ signal in both 168 and 169 was observed at d 55.3. The 2' and 4'- CH₃ carbon signals of 170 were ovserved respectively at d 18.9 and 21.0. The 2'-CH₃ carbon of 177 and 178 resonated at d 19.0 and 18.9 respectively. 3' and 5'-CH₃ signals of 179 were observed at d 21.3 and those of 180 were observed at 21.2.

### Example 93

### Chlorohydrin Analogs

Cryptophycins-163, 184, 185, 186, 187, 191, 192, 193, 194, 195, 212, 216, 217, 222, 223, 224, 243, 252, 253, 263, 264, 265, 272 and 273.

**Table 8.**

| | | | | |
|---|---|---|---|---|
| 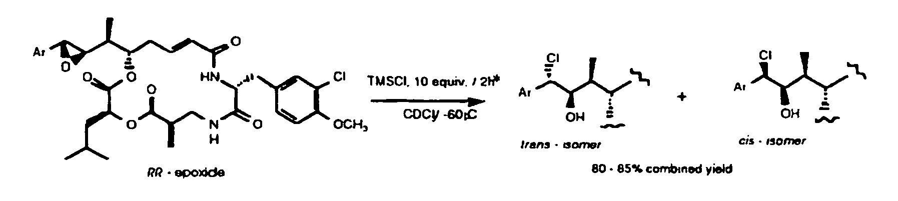 | | | | |

| epoxide Cryptophyc in # | Ar | chlorohydrin # | Stereo- chemistry | *trans*/*cis* ratio |
|---|---|---|---|---|
| 157 | 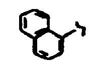 | **191** | *trans* | 1.1 : 1 |
| | | **192** | *cis* | |
| 168 | 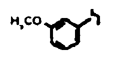 | **195** | *trans* | 1 : < |
| | | | *cis* | 0.1 |
| 170 | 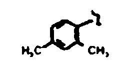 | **216** | *trans* | 1 : 2.6 |
| | | **217** | *cis* | |
| 179 | 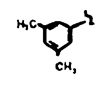 | **222** | *trans cis* | < 0.1 : |
| | | | | 1 |
| 172 | 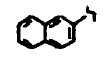 | **193** | *trans* | 1 : 2 |
| | | **194** | *cis* | |
| 181 | 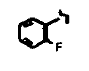 | **223** | *trans* | 1 : < |
| | | | *cis* | 0.1 |
| 200 | 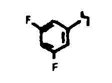 | **212** | *trans* | 1 : < |
| | | | *cis* | 0.1 |
| ****** | 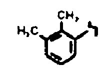 | 184 | *trans* | |
| ** | 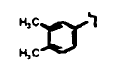 | **224** | *trans* | 1 : 2 |
| | | **186** | *cis* | |
| | 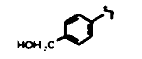 | **243** | *trans* | 1 : < |
| *** | | | *cis* | 0.1 |

| | | | | |
|---|---|---|---|---|
| * Cryptophycin-200 undergo only 30% conversion under these conditions. | | | | |
| ** A mixture of *RR* epoxides of 3,4 - and 2,3 - dimethylphenyl analogs. | | | | |
| *** A mixture of *RR* and *SS* epoxides. | | | | |
| * Cryptophycin-200 undergo only 30% conversion under these conditions. | | | | |
| ** A mixture of *RR* epoxides of 3,4 - and 2,3 - dimethylphenyl analogs. | | | | |
| *** A mixture of *RR* and *SS* epoxides. | | | | |

**Cryptophycin-163:** To a solution of cryptophycin-81 (12 mg, 0.018 mmol) in CH₂Cl₂ (1.5 mL) at 0_C was added m-CPBA (5 mg, 0.029 mmol) follwed by HCl in acetic acid (1.0 M solution, 25 µL) and the temperature was allowed to rise to rt and continued the stirring for 6h. Evaporation of the solvent follwed by flash chromatography of the residue over C18 silica column (12 cmx 1 cm) using H₂O and CH₃CN mixtures yielded a mixture of chlorohydrins (12 mg, 93%). Attempted purification on reversed phase HPLC led to the partial hydrolysis into diols. However, purification by normal phase HPLC (Econosil silica, 250 mm x 10 mm, 1:1 EtoAc/hexane) gave cryptophycin-163 (t_{R} 37.2 min, 3.0 mg). The proton and carbon NMR data was given in the tables **9** and **10** respectively.

**Cryptophycins-184, -185, -186, 187 and 224:** A mixture of 3, 4 - dimethylbenzyl triphenyl -phosphonium chloride and 2, 3 - dimethylbenzyl triphenylphosphonium chloride (7:3) was converted to 3, 4- and 2, 3 - dimethylbenzyl triphenylphosphoranes and then treated with cryptophycin-108 using the same procedure described earlier for the preparation of cryptophycin-152. An inseparable mixture of four styrene isomers was obtained in 68% combined yield. The mixture (45 mg) in CH₂Cl₂ (3 mL) was treated with m-CPBA (25 mg) at rt and kept under stirring for 15 h. The solvent was removed and the residue subjected to HPLC (Econosil C-18, 250 x 22 mm 7:3 H₂O/CH₃CN, 6 mL/min), and collected two fractions I (t_{R} 54.0 min, 20 mg) and II (t_{R} 61.5 min, 12 mg) . Fraction I (9 mg) in CH₂Cl₂ (2.0 mL) was treated with TMSCl (40 µL) at -78_C and allowed the temp gradually rise to room temp After 3h, evaporation of the solvent follwed by chromatography on reversed phase HPLC (Econsil C18, 10 m, 250 x 22 mm, 35:65 H₂O/CH₃CN, 6 mL/min) gave cryptophycin-186 (t_{R} 57.8 min, 4.7 mg), cryptophycin-224 (t_{R} 74.5 min, 2.3 mg) and cryptophycin-184 (t_{R} 78.2 min, 2.6 mg). Using the same procedure, Fraction II was treated with TMSCl and the compounds were separated to obtain cryptophycin-185 (t_{R} 65.0 min, 0.7 mg) and cryptohycin-187 (t_{R} 69.2 min, 8.0 mg).

**Cryptophycin-184:** EIMS m/z (relative intensity) 682/684 (1.1/0.4), 236 (10), 195 (9), 135 (100), 119 (83); high resolution EIMS m/z 682.2999 (M⁺ - HCl, calcd for C₃₇H₄₇ClN₂O₈, 2.2 mmu error); The proton and carbon data are given in the tables 9 and 10 respectively.

**Cryptophycin-185:** EIMS m/z (relative intensity) 682/684 (6/2), 412/414 (14/5), 280/282 (14/5), 255 (10), 195/197 (50/16), 119 (100); high resolution EIMS m/z 682.3015 (M⁺ - HCl, calcd for C₃₇H₄₇ClN₂O₈, 0.6 mmu error); The proton and carbon data were given in the tables 9 and 10 respectively.

**Cryptophycin-186:** EIMS m/z (relative intensity) 682/684 (1.3/0.6), 412/414 (2.5/0.8), 254 (7), 195 (11), 119 (100); high resolution EIMS m/z 682.3015 (M⁺ - HCl, calcd for C₃₇H₄₇ClN₂O₈, 0.6 mmu error); The proton and carbon data were given in the tables **9** and **10** respectively.

**Cryptophycin-187:** EIMS m/z (relative intensity) 682/684 (0.9/0.2), 412/914 (3/1), 255 (3), 259 (7), 195 (11), 135 (100), 119 (94); high resolution EIMS m/z 682.3044 (M⁺ - HCl, calcd for C₃₇H₄₇ClN₂O₈, -2.3 mmu error); The proton and carbon data were given in the tables **9** and **10** respectively.

**Cryptophycin-224:** EIMS m/z (relative intensity) 682/684 (3/1), 412/414 (3/1), 255 (4), 254 (9), 195/197 (28/6), 119 (100); high resolution EIMS m/z 682.3064 (M⁺ - HCl, calcd for C₃₇H₄₇ClN₂O₈, - 4.3 mmu error); The proton and carbon data were given in the tables **9** and **10** respectively.

### Cryptophycins-191, 192, -193, -194, -195, -212, -216, -217, - 222 and -223:

**Genaral procedure for the preparation of chlorohydrins:** A solution of RR-epoxide in chloroform was cooled to - 60_C and treated with excess trimethylsilyl chloride (10 equivalents). After 2 h, the solvent was evaporated and residue was purified on a reversed phase HPLC (Econosil C18, 10µ, 35% H₂O/CH₃CN, 3 mL/min) to obtain *trans* and *cis* chlorohydrins.

The experimental details for the preparation of the cryptophycins-191, 192, -193, -194, -195, -212, -216, -217, - 222, -223 and 224, and cytotoxicity are summarized in table **8.**

**Cryptophycin-195:** EIMS m/z (relative intensity) 684/686 (3/2), 412/414 (12/4), 257 (11), 195/197 (46/15); high resolution EIMS m/z 684.2784 (M⁺-HCl, calcd for C₃₆H₄₅ClN₂O₉, Δ 3.0 mmu); ¹H NMR data, see table **9**; ¹³C NMR data, see table **10**.

**Cryptophycin-212:** EIMS m/z (relative intensity) ; high resolution EIMS m/z (M⁺-HCl, calcd for C₃₅H₄₁ClF₂N₂O₈, Δ mmu); ¹H NMR data, see table **9**; ¹³C NMR data, see table **10**.

**Cryptophycin-216:** EIMS m/z (relative intensity) 682/684 (6/3), 412/414 (6/2), 255 (4), 195/197 (21/7); high resolution EIMS m/z 682.2990 (M⁺-HCl, calcd for C₃₇H₄₇ClN₂O₈, Δ 3.1 mmu) ; ¹H NMR (CDCl₃) *amino or hydroxy acid unit δ* (carbon position, multiplicity; *J* in Hz) A 5.79 (2, d; 15.4), 6.70 (3, ddd; 15.4, 9.5 and 5.9), 2.36 (4, m), 2.67 (4, br dd; 14.9 and 4.9), 5.01 (5, m), 2.52 (6, m), 1.07 (6-Me, d; 7.1), 4.07 (7, br d; 9.8), 5.01 (8, d; 9.8), 2.36 (2'-CH₃, s), 7.01 (2', br s), 2.31 (4'-CH₃, s), 7.06 (5', brd; 8.1), 7.32 (6', d; 8.1) ; *B* 4.81 (2, m), 5.71 (2-NH, d; 8.5), 3.02 (3, dd; - 14.4 and 7.9), 3.16 (3, dd; -14.4 and 5.6), 7.23 (5, d; 2.0), 3.88 (7-OMe, s), 6.85 (8, d; 8.3), 7.09 (9, dd; 8.3 and 2.0); C 2.74 (2, m), 1.23 (2-Me, d; 7.1), 3.25 (3, m), 3.53 (3, m), 6.93 (3-NH, br m); D 4.93 (2, dd; 10.2 and 3.2), 1.49 (3, m), 1.68 - 1.85 (3/4, m), 0.95 (4-Me, d; 6.9), 0.96 (5, d; 6.4).

**Cryptophycin-217:** EIMS m/z (relative intensity) 682/684 (3/1), 412/414 (6/1), 255 (5), 195/197 (23/7); high resolution EIMS m/z 682.2920 (M⁺-HCl, calcd for C₃₇H₄₇ClN₂O₈, Δ 10.1 mmu); ¹H NMR (CDCl₃) *amino or hydroxy acid unit* δ (carbon position, multiplicity; *J* in Hz) A 5.72 (2, d; 15.4), 6.66 (3, ddd; 15.4, 9.7 and 5.4), 2.17 (4, dt; 13.9 and 10.9), 2.53 (4, br dd; 13.9 and 5.0), 5.08 (5, m), 1.61 (6, m), 0.88 (6-Me, d; 6.8), 4.28 (7, br d; 9.6), 5.13 (8, d; 9.6), 2.38 (2'-CH₃, s), 7.03 (2', br s), 2.32 (4'-CH₃, s), 7.03 (5', brd; 8.5), 7.17 (6', d; 8.5); *B* 4.80 (2, m), 5.66 (2-NH, d; 8.1), 3.03 (3, dd; -14.4 and 7.2), 3.14 (3, dd; -14.4 and 5.6), 7.21 (5, d; 1.6), 3.87 (7-OMe, s), 6.83 (8, d; 8.3), 7.08 (9, dd; 8.3 and 1.7); C 2.74 (2, m), 1.24 (2-Me, d; 7.1), 3.29 (3, m), 3.50 (3, m), 6.96 (3-NH, br m); D 9.89 (2, dd; 9.8 and 3.4), 1.52 (3, m), 1.87 (3, m), 1.72 (4, m), 0.94 (4-Me, d; 7.3), 0.97 (5, d; 6.8).

**Cryptophycin-222:** EIMS m/z (relative intensity) 718/720 (0.4/0.2), 682/684 (13/7), 412/414 (15/5), 280/282 (16/5), 255 (12), 195/197 (59/19), 119 (100); high resolution EIMS m/z 718.2767 (calcd for C₃₇H₄₈Cl₂N₂O₈, Δ 2.1 mmu). ¹H NMR data, see table **9**; ¹³C NMR data, see table **10**.

**Cryptophycin-223:** EIMS m/z (relative intensity) 672/674 (1/0.8), 412/414 (3/1), 195/197 (15/3), 199 (100), 109 (35); high resolution EIMS m/z 708.2373 (calcd for C₃₅H₄₃FCl₂N₂O₈, Δ 0.7 mmu). ¹H NMR data, see table 9; ¹³C NMR data, see table 10.

### Cryptophycins-243:

Epoxidation of cryptophycin-238: Cryptophycin-238 (11.8 mg) was dissolved in dichloromethane (1.5 mL) and treated with MCPBA (9.6 mg). After 15 h, the reaction mixture was diluted with dichloromethane (3 mL) and washed with 0.1 M phosphate buffer at pH 8 (3 mL). The organic layer was treated with dimethylsulfide (10 µL) and repeated the buffer wash. The organic layer was evaporated and the residue (12 mg) was unsuccessfully purified on a reversed phase HPLC (Econosil C18, 25 cm x 22 mm, 10 µ, 35% H₂O/CH₃CN, 6 mL/min) to give a mixture of *RR* and *SS* epoxides (6.2 mg).

Treatment with TMSCl: The mixture was dissolved in chloroform (1 mL) and treated with TMSCl (15 µL) at -60_C. After 2 h, the solvent was evaporated the residue (7 mg) was subjected to a normal phase HPLC (Econosil Si, 25 cm x 10 mm, 10 µ, 85% EtOAc/hexane, 4 mL/min). The fraction collected at 30 min was further purified on a reversed phase HPLC (Econosil C18, 25 cm x 10 mm, 10 µ, 45% H₂O/CH₃CN, 3 mL/min) to obtain cryptophycin-243 (2.4 mg).

**Cryptophycin-243:** ¹H NMR (CDCl₃) *amino or hydroxy acid unit* δ (carbon position, multiplicity; J in Hz) A 5.76 (2, d; 15.0), 6.67 (3, ddd; 15.0, 9.6 and 5.4), 2.35 (4, dt; 14.4 and 10.4), 2.64 (4, br dd; 14.4 and 5.1), 5.07 (5, m), 2.50 (6, m), 1.04 (6-Me, d; 6.8), 4.02 (7, br d; 9.8), 4.68 (8, d; 9.8), 7.39 (2' /3' /5' /6' , br s), 4.70 (4'-CH₂OH, brs); *B* 4.74 (2, m), 5.72 (2-NH, d; 7.8), 2.99 (3, dd; -14.4 and 7.3), 3.14 (3, dd; -14.4 and 5.6), 7.22 (5, d; 2.0), 3.88 (7-OMe, s), 6.84 (8, d; 8.4), 7.08 (9, dd; 8.4 and 2.0); C 2.73 (2, m), 1.21 (2-Me, d; 7.1),3.23 (3, dt; 13.7 and 7.1),3.50 (3, dt; 13.7 and 4.5), 6.93 (3-NH, br dd; 7.1 and 4.5); D 4.91 (2, dd; 10.0 and 3.4), 1.97 (3, m), 1.64 - 1.84 (3/4, m), 0.94 (4-Me, d; 6.4), 0.95 (5, d; 6.3); ¹³C NMR (CDCl₃) *unit* δ (carbon position) *A* 165.6 (1), 125.0 (2), 141.9 (3), 36.3 (4), 76.4 (5), 38.3 (6), 8.7 (6-Me), 74.0 (7), 62.0 (8), 137.7 (1'), 127.4^{a} (2'/6'), 128.2^{a} (3'/5'), 142.2 (4'), 64.6 (4'-CH₂OH); ***B*** 171.1^{b} (1), 53.6 (2), 35.1 (3), 129.9 (4), 131.0 (5), 122.4 (6), 154.0 (7), 56.2 (7-OCH₃), 112.3 (8), 128.4 (9); *C* 175.4 (1), 38.5 (2), 14.1 (2-Me), 41.2 (3); *D* 170.5^{b}(1), 71.4 (2), 39.7 (3), 24.8 (4), 23.2 (4-Me), 21.6 (5). ^{a and b}signals with identical superscript are interchangeable.

### Cryptophycins-252 and 253:

Epoxidation of cryptophycin-247: Cryptophycin-247 (12 mg) was dissolved in dichloromethane (1 mL) and treated with MCPBA (9.8 mg). After 15 h, the reaction mixture was diluted with dichloromethane (3 mL) and washed with 0.1 M phosphate buffer at pH 8 (3 mL). The organic layer was separated and repeated again the buffer wash after adding dimethylsulfide (15 mL). The organic layer was evaporated and the residue (11.5 mg) was purified on a reversed phase HPLC (Econosil C18, 25 cm x 10 mm, 10 µ, 35% H₂O/CH₃CN, 3 mL/min) to give a major *RR*-epoxide (5 mg) and a minor *SS*- epoxide (3 mg).

***RR*-epoxide:** ¹H NMR (CDCl₃) *amino or hydroxy acid unit* δ (carbon position, multiplicity; *J* in Hz) A 5.74 (2, d; 15.6), 6.67 (3, ddd; 15.6, 9.5 and 5.6), 2.44 (4, dt; 14.7 and 10.5), 2.50 - 2.58 (4, m), 5.15 (5, m), 1.62 - 1.81 (6, m), 1.12 (6-Me, d; 6.8), 2.91 (7, dd; 7.3 and 1.5), 3.66 (8, d; 1.5), 7.38 (2', br s), 6.54 (3'-NH, s), 1.51 (3'-NH-t-Boc), 7.21 - 7.30 (4'/5', m), 6.91 (6', br d; 6.8); *B* 4.79 (2, m), 5.69 (2-NH, d; 8.5), 3.00 (3, dd; 14.5 and 7.3), 3.13 (3, dd; 14.5 and 5.5), 7.20 (5, d; 2.0), 3.86 (7-OMe, s), 6.83 (8, d; 8.3), 7.06 (9, dd; 8.3 and 2.0); *C* 2.70 (2, m), 1.22 (2-Me, d; 7.1), 3.30 (3, m), 3.46 (3, m), 6.97 (3-NH, m); *D* 4.83 (2, dd; 10.0 and 3.4), 1.36 (3, m), 1.62 - 1.81 (3/4, m), 0.85 (4-Me, d; 5.9), 0.87 (5, d; 5.6).

Deprotection of the amino group and preparation of chlorohydrins: A solution of RR-epoxide (8.0 mg) in dichloromethane (100 µL) was treated with hydrochloric acid (50 µL, 4N in dioxane). After 30 min, the solvent was evaporated and the residue was purified on a reversed phase HPLC (Econosil C18, 25 cm x 10 mm, 10 µ, 40% H₂O/CH₃CN, 3.5 mL/min) to obtain cryptophycin-252 (5 mg, t_{R} min) and cryptophycin-253 (2 mg, t_{R} min).

### Cryptophycin-252 cytotoxicity data: Cell line (# of folds potent than cryptophycin-1) KB (1.20) and LoVo (1.25)

**Cryptophycin-252:** EIMS m/z (relative intensity) 670/672 (7.2/2.2), 669/671 (19.2/7.8), 280/282 (6.8/2.4), 242 (4.7), 195/197 (43/12); high resolution EIMS m/z 669.2856 (M⁺ -2HCl-H) calcd for C₃₅H₄₄ClN₃O₈, -3.9 mmu error); ¹H NMR (MeOH) *amino or hydroxy acid unit* δ (carbon position, multiplicity; J in Hz) *A* 5.93 (2, d; 15.1), 6.61 - 6.78 (3, m), 2.33 (4, dt; 14.7 and 9.8), 2.69 - 2.80 (4, m), 5.11 (5, m), 2.46 (6, m), 0.95 - 1.04 (6-Me, m), 3.99 (7, brd; 9.4), 4.62 (8, d; 9.4), 7.28 (2', br s), 7.17 (4', brd; 8.1), 7.07 (5', t; 8.1), 6.98 (6', d; 8.1); *B* 4.50 (2, dd; 10.5 and 3.2), 2.69 - 2.80 (3, m), 3.18 (3, brd; 13.7), 6.61 - 6.78 (5, 8 and 9, m), 3.86 (7-OMe, s); *C* 2.69 - 2.80 (2, m), 1.19 (2-Me, d; 7.3), 3.28 (3, brd; 13.4), 3.58 (3, br d; 13.4); *D* 5.01 (2, dd; 9.8 and 2.7), 1.53 (3, m), 1.70 - 1.84 (3/4, m), 0.95 - 1.04 (4/5-Me, m).

**Cryptophycin-253:** ¹H NMR (MeOH) *amino or hydroxy acid unit* δ (carbon position, multiplicity; J in Hz) A 5.84 (2, dd; 15.3 and 1.6), 6.60 - 6.74 (3, m), 2.10 (4, dt; 14.9 and 11.2), 2.64 (4, m), 5.05 (5, m), 1.50 (6, m), 0.90 (6-Me, d; 6.8), 9.01 (7, dd; 9.5 and 1.2), 4.75 (8, d; 9.5), 6.60 - 6.74 (2' /4' /6' , m), 7.09 (5', t; 7.8); *B* 4.48 (2, dd; 11.4 and 3.9), 2.71 (3, dd; 14.4 and 11.4), 3.16 (3, dd; 14.4 and 3.7), 7.26 (5, d; 2.2), 3.86 (7-OMe, s), 6.96 (8, d; 8.4), 7.15 (9, dd; 8.4 and 2.2); *C* 2.76 (2, m), 1.18 (2-Me, d; 7.3), 3.26 (3, m), 3.58 (3, dd; 13.5 and 3.1); *D* 4.94 (2, dd; 9.4 and 3.8), 1.58 (3, m), 1.80 (3, m), 1.70 (4, m), 0.97 (4-Me, d; 6.1), 0.98 (5, d; 6.3).

### Cryptophycins-263, 264 and 265:

A mixture of cryptophycin-242 and its ss-epoxide (26 mg) was dissolved in CH₂Cl₂ (5 mL) and treated with TMSC1 (1.0 Msolution in CH₂Cl₂, 120 µL) at -78_C. After 3 h, the solvent was evaporated and the residue (27 mg) was subjected to reversed phase HPLC (Econosil C18, 250 mm x 22 mm, 10 µ, 65:35 CH₃CN/H₂O, 6 mL/min) to give cryptophycin-264 (6.0 mg, t_{R} 43 min), and a mixture of cryptophycins-263 and 265 (15.8 mg, t_{R} 59 min). Cryptophycins-263 and 265 were separated by normal phase HPLC (Econosil silica, 250 mm x 10 mm, 10 µ, 55 : 45 EtoAc/hexane, 3 mL/min) to give cryptophycin-265 (7.1 mg, 51 min) and cryptophycin-263 (7.8 mg, 79.0 min).

**Cytotoxicity data**

| cryptophycin # | cytotoxicity, # of folds potent than crypto-1 KB LoVo | |
|---|---|---|
| 263 | 1.67 | 0.56 |
| 264 | 0.044 | 0.023 |
| 265 | 0.0033 | 0.021 |

**Cryptophycin-263:** ¹H NMR (CDCl₃) *amino or hydroxy acid unit* δ (carbon position, multiplicity; *J* in Hz) *A* 5.79 (2, d; 15.6), 6.68 (3, m), 2.37 (4, m), 2.66 (4, br dd; 14.2 and 4.2), 5.10 (5, m), 2.49 (6, m), 1.03 (6-Me, d; 6.8), 4.00 (7, br d; 9.4), 4.64 (8, d; 9.4), 7.35 (2'/6', br d; 7.9), 7.29 (3'/5', brd; 7.9), 4.31 (4'-CH₂NH-t-BOC, d; 4.8), 4.92 (4'-CH₂NH-t-BOC, brs), 1.45 (4'-CH₂NH-t-BOC, s) ; *B* 4.79 (2, m), 5.76 (2-NH, d; 8.6), 3.01 (3, dd; -14.4 and 7.3), 3.15 (3, dd; -14.4 and 9.9), 7.23 (5, d; 1.3), 3.87 (7-OMe, s), 6.84 (8, d; 8.4), 7.09 (9, br d; 8.4); C 2.73 (2, m), 1.22 (2-Me, d; 7.2), 3.24 (3, m), 3.53 (3, m), 6.93 (3-NH, br t; 5.5); *D* 4.92 (2, dd; 9.7 and 3.1), 1.48 (3, m), 1.78 (3, m), 1.73 (4, m), 0. 93 (4-Me, d; 6.4), 0.94 (5, d; 6.4); ¹³C NMR (CDCl₃) unit δ (carbon position) *A* 165.6 (1), 125.3 (2), 141.6 (3), 36.4 (4), 76.5 (5), 38.3 (6), 8.7 (6-Me), 74.1 (7), 61.8 (8), 137.5^{a} (1'), 128.3 (2'/6'), 128.0 (3'/5'), 140.3^{a} (4'), 44.2 (4'-CH₂NH-t-BOC), 28.4 & 155.9 (4'-CH₂NH-t-BOC); *B* 171.1^{b} (1), 53.7 (2), 35.1 (3), 130.0 (4), 131.1 (5), 122.4 (6), 154.0 (7), 56.2 (7-OCH₃), 112.3 (8), 128.5 (9); *C* 175.4 (1), 38.4 (2), 14.1 (2-Me), 41.3 (3); *D* 170.6^{b}(1), 71.4 (2), 39.8 (3), 24.8 (4), 23.2 (4-Me), 21.6 (5). ^{a and b} signals with identical superscript are interchangeable.

**Cryptophycin-264:** ¹H NMR (CDCl₃) *amino or hydroxy acid unit* δ (carbon position, multiplicity; J in Hz) A 5.71 (2, d; 15.4), 6.63 (3, ddd; 15.4, 9.9 and 5.5), 2.12 (4, m), 2.51 (4, br dd; 14.2 and 5.0), 5.05 (5, m), 1.47 (6, m), 0.92 (6-Me, d; 6.8), 4.06 (7, dd; 9.6 and 1.5), 4.87 (8, d; 9.6), 7.29 (2'/6', br d; 8.4), 7.27 (3'/5', brd; 8.4), 4.33 (4'-CH₂NH-t-BOC, brs), 1.47 (4'-CH₂NH-t-BOC, s) ; *B* 4.80 (2, m), 5.69 (2-NH, brs), 3.01 (3, dd; -14.5 and 7.2), 3.13 (3, dd; - 14.5 and 5.4), 7.21 (5, d; 2.0), 3.86 (7-OMe, s), 6. 83 (8, d; 8.4), 7.07 (9, dd; 8.4 and 2.0); *C* 2.73 (2, m), 1.23 (2-Me, d; 7.3), 3.28 (3, m), 3.52 (3, m), 6.93 (3-NH, br t; 5.7); *D* 4.86 (2, m), 1.50 (3, m), 1.86 (3, m), 1.73 (4, m), 0.95 (4-Me, d; 6.6), 0.98 (5, d; 6.8); ¹³C NMR (CDCl₃) unit δ (carbon position) *A* 165.4 (1), 125.1 (2), 141.4 (3), 36.2 (4), 76.0 (5), 38.3 (6), 8.8 (6-Me), 74.3 (7), 68.4 (8), 136.6^{a} (1'), 128.0 (2'/6'), 127.7 (3'/5'), 140.3^{a} (4'), 44.2 (4'-CH₂NH-t-BOC), 28.4 (4'-CH₂NH-t-BOC); ***B*** 171.0^{b} (1), 53.6 (2), 35.0 (3), 129.9 (4), 131.0 (5), 122.4 (6), 153.9 (7), 56.1 (7-OCH₃), 112.2 (8), 128.4 (9); *C* 175.4 (1), 38.5 (2), 14.1 (2-Me), 41.2 (3); *D* 170.4^{b} (1), 71.4 (2), 39.8 (3), 24.8 (4), 23.2 (4-Me), 21.7 (5). ^{a and b} signals with identical superscript are interchangeable.

**Cryptophycin-265:** ¹H NMR (CDCl₃) *amino or hydroxy acid unit* δ (carbon position, multiplicity; *J* in Hz) *A* 5.74 (2, d; 15.5), 6.68 (3, ddd; 15.5, 9.9 and 5.3), 2.36 (4, m), 2.54 (4, br dd; 14.2 and 4.9), 5.40 (5, m), 1.87 (6, m), 1.05 (6-Me, d; 7.0), 3.71 (7, br t; 5.8), 5.07 (8, d; 5.3), 7.36 (2'/6', br d; 8.2), 7.28 (3'/5', brd; 8.2), 4.30 (4'-CH₂NH-t-BOC, brs), 4.91 (4'-CH₂NH-t-BOC, brs), 1.46 (4'-CH₂NH-t-BOC, s) ; *B* 4.78 (2, m), 5.70 (2-NH, brs), 3.03 (3, dd; -14.5 and 7.1), 3.12 (3, dd; -14.5 and 5.5). 7.21 (5, d; 1.8), 3.87 (7-OMe, s), 6.84 (8, d; 8.4), 7.07 (9, dd; 8.4 and 1.8); *C* 2.72 (2, m), 1.23 (2-Me, d; 7.1), 3.33 (3, m), 3.48 (3, m), 6.97 (3-NH, br t; 5.7) ; *D* 4.83 (2, dd; 9.7 and 4.3), 1.43 (3, m), 1.70 (3, m), 1.63 (4, m), 0.93 (4-Me, d; 6.4), 0.88 (5, d; 6.4); ¹³C NMR (CDCl₃) unit δ (carbon position) *A* 165.5 (1), 125.0 (2), 142.0 (3), 34.6 (4), -74.4 (5), 39.8 (6), 12.9 (6-Me), 77.5 (7), 66.7 (8), 137.3^{a} (1'), 127.9 (2'/6'), 127.8 (3'/5'), 139.9^{a} (4'), 44.2 (4'-CH₂NH-t-BOC), 28.4 (4'-CH₂NH-t-BOC); ***B*** 171.O^{b} (1), 53.6 (2), 35.1 (3), 129.9 (4), 131.0 (5), 122.4 (6), 154.0 (7), 56.1 (7-OCH₃), 112.3 (8), 128.4 (9); *C* 175.6 (1), 38.4 (2), 14.1 (2-Me), 41.3 (3); *D* 170.3^{b}(1), 71.5 (2), 39.4 (3), 24.7 (4), 22.8 (4-Me), 21.6 (5). ^{a and b} signals with identical superscript are interchangeable.

**Cryptophycin-272:** Cryptophycin-263 (4.9 mg, 0.006 mmol) in CH₂Cl₂ (30 µL) was treated with 4N HCl in dioxane (30 µL, 0.012 mmol) at room temperature. After 3 h, the solvent was removed and the residue was kept under vacuum for 2 d to remove last traces of dioxane, to give cryptophycin-272 (4.4 mg, 98%). ¹H NMR (CD₃OD) *amino or hydroxy acid unit* δ (carbon position, multiplicity; *J* in Hz) A 5.94 (2, dd; 15.0, 1.8), 6.69 (3, ddd; 15.0, 11.1 and 4.9), 2.36 (4, m), 2.72 (4, m), 5.13 (5, m), 2.48 (6, m), 1.01 (6-Me, d; 7.1), 4.02 (7, dd; 9.4 and 2.0), 4.84 (8, d; 9.4), 7.45 (2'/6', d; 8.4), 7.51 (3'/5', d; 8.4), 4.11 (4' -CH₂NH₂HCl, s); *B* 4.51 (2, dd; 11.2 and 3.9), 2.74 (3, m), 3.18 (3, dd; -14.5 and 3.9), 7.28 (5, d; 2.2), 3.84 (7-OMe, s), 6.98 (8, d; 8.4), 7.17 (9, dd; 8.4 and 2.3); C 2.77 (2, m), 1.19 (2-Me, d; 7.5), 3.29 (3, m), 3.58 (3, dd; 13.7 and 3.3); D 5.02 (2, dd; 9.5 and 3.8), 1.56 (3, m), 1.78 (3, m), 1.78 (4, m), 0.97 (4-Me, d; 6.6), 0.99 (5, d; 6.6); ¹³C NMR (CD₃OD) *unit* δ (carbon position) A 168.4 (1), 125.4 (2), 144.0 (3), 37.7 (4), 77.3 (5), 40.4 (6), 8.1 (6-Me), 74.8 (7), 63.3 (8), 142.5^{a} (1'), 130.0 (2'/6'), 130.3^{a} (3'/5'), 134.6^{a} (4'), 44.0 (4'-CH₂NH₂HCl); *B* 174.1 (1), 57.4 (2), 36.3 (3), 132.2 (4), 131.5 (5), 123.3 (6), 155.4 (7), 56.6 (7-OCH₃), 113.5 (8), 129.3 (9); C 177.5 (1), 39.1 (2), 15.1 (2-Me), 41.2 (3); D 172.1 (1), 72.8 (2), 41.2 (3), 26.0 (4), 22.1 (4-Me), 23.6 (5). ^{a} signals are interchangeable.

**Cryptophycins-273:** 3-Chloroperbenzoic acid (128 mg) was added to a solution of cryptophycin-234 (146 mg) in CH₂Cl₂ (8 mL) at 0_C. The solution was allowed to slowly warm-up to the room temperature and continued the stirring for 12 h. The reaction mixture was diluted with CH₂Cl₂ (7 mL) and washed with phosphate buffer (2 x 10 mL, 0.1 M, pH 8). The organic layer was separated and treated with dimethylsulfide (50 mL) to quench excess peracid, and subjected to the buffer wash again. The organic layer was evaporated and the residue was subjected to reversed phase HPLC (250 mm x 22 mm, 10 µ, 15 % H₂O/CH₃CN, 6 mL/min) to obtain the RR-epoxide (74 mg, 50%).

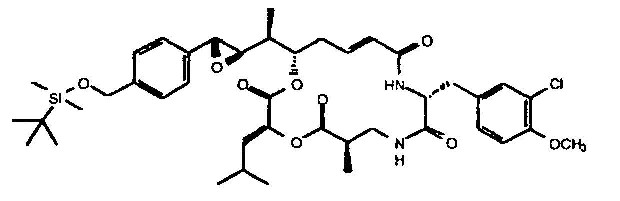

A solution of the epoxide (74 mg) in THF (3 mL) was treated with 115 µL of 1M solution of tetrabutylammonium fluoride in CH₂Cl₂. After 1.5 h, saturated NH₄Cl solution was added to the reaction mixture and extracted with EtoAc (2 x 30 mL). The organic extract was dried over MgSO₄ and evaporated. The residue was purified on a small silica column using 50 - 100% EtoAc/CH₂Cl₂ to obtain cryptophycin-269 (62 mg, 98%).

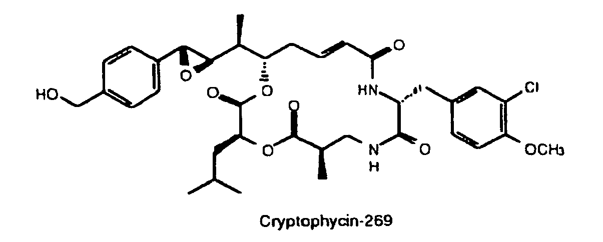

To a solution of cryptophycin-269 (62 mg), N-(t-BOC)glycine (30 mg) and DMAP (8 mg) in CH₂Cl₂ (3 mL) was added DCC (160 µL, 1M solution in CH₂Cl₂ at 0_C. The reaction mixture was stirred at 0_C for 15 min and at room temperature for 2 h. At the end of this period, the mixture was filtered and concentrated in vacuo. The residue was subjected to a flash chromatography on ODS silica using 75 - 30%. H₂O/CH₃CN to obtain the N-t-BOC-glycinate ester of cryptophycin-269 (64.4 mg, 85 %).

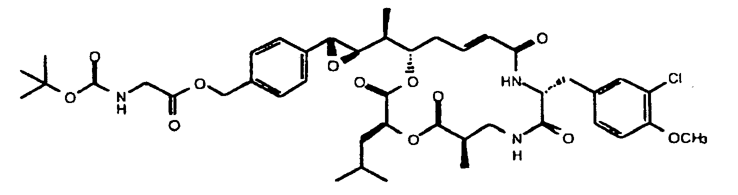

To a solution of glycinate ester (64.4 mg) in chloroform (3 mL) at - 60_C was added 200 µL of 1M solution of TMSCl in CH₂Cl₂. After 1 h, the solvent was evaporated and the residue was purified on a small silica column using 40 - 90% EtOAc/hexanes to obtain the trans chlorohydrin of the glycinate(60 mg, 89%).

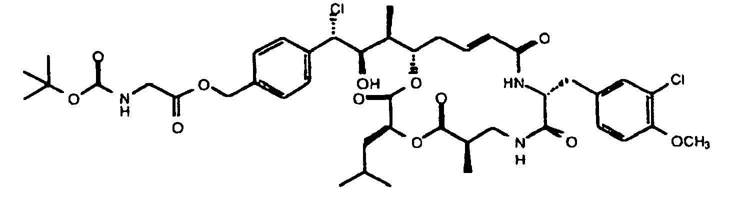

A solution of the chlorohydrin (55 mg) in dichloromethane (0.25 mL) was treated with 4M solution of hydrogen chloride in 1,4-dioxane (0.1 mL). The resulting mixture was stirred at room temperature for 2h, concentrated in vacuo and dried on the freeze dryer for 48 h. Cryptophycin-273 was obtained as a white solid.

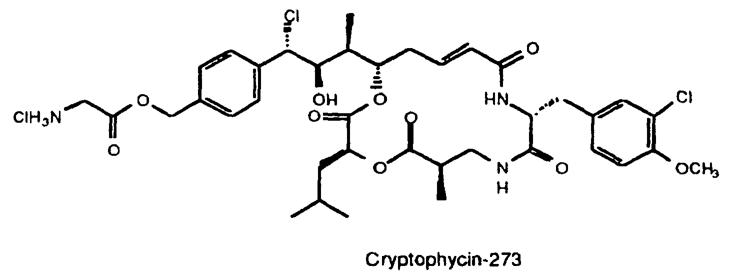

**Table 9. ¹H NMR Data for Cryptophycins-8, 163, 184, 185, 186, 187, 191, 192, 193, 194, 195, 212, 222, 223 and 224. P=position**

| **P** | **8** | **163** | **184** | **185** | **186** | **187** | **191** | **192** | **193** | **194** | **195** | **212** | **222** | **223** | **224** |
|---|---|---|---|---|---|---|---|---|---|---|---|---|---|---|---|
| A 2 | 5.79 d | 5.80 | 5.79 | 5.74 | 5.71 | 5.75 | 5.80 | 5.68 | 5.80 | 5.67 | 5.79 | 5.80 | 5.71 | 5.80 | 5.79 |
| 3 | 6.69 ddd | 6.70 | 6.70 | 6.68 | 6.64 | 6.69 | 6.70 | 6.63 | 6.70 | 6.62 | 6.69 | 6.68 | .6.65 | 6.69 | 6.69 |
| 4*R* | 2.37 m | 2.35 m | 2.38 m | 2.36 m | 2.15 m | 2.37 m | 2.40 | 2.10 | 2.40 | 2.09 | 2.37 | 2.40 | 2.17 | 2.39 | 2.37 |
| 4*S* | 2.67 m | 2.75 m | 2.70 m | 2.55 m | 2.53 m | 2.59 in | 2.70 | 2.47 | 2.69 | 2.51 | 2.68 | 2.64 | 2.54 | 2.60 | 2.68 |
| 5 | 5.10 ddd | 5.00 | 5.12 | 5.32 m | 5.06 m | 5.40 m | 5.14 | 5.07 | 5.13 brt | 5.07 | 5.11 | 5.10 | 5.07 | 5.11 | 5.11 |
| 6 | 2.50 m | 2.85 | 2.54 | 1.91 | 1.50 m | 1.83 m | 2.65 | 1.64 | 2.55 | 1.57 | 2.49 | 2.47 | 1.51 | 2.48 | 2.50 |
| 6-Me | 1.05 d | 1.13 | 1.09 | 1.09 | 0.93 | 1.06 | 1.18 | 0.95 | 1.09 | 0.90 | 1.04 | 1.03 | 0.93 | 1.04 | 1.04 |
| 7 | 4.00 brdt | 4.41 | 4.09 | 3.84 | 4.07 | 3.74 | 4.35 | 4.48 | 4.12 | 4.20 | 3.99 | 3.95 | 4.07 | 4.16 | 3.98 |
| 8 | 4.65 d | 4.80 | 5.12 | 5.46 | 4.83 | 5.02 | 5.66 | 5.72 | 4.84 | 5.06 | 4.61 | 4.59 | 4.81 | 5.03 | 4.60 |
| 9 | | | | | | | | | | | | | | | |
| 10 | | | | | | | | | | | | | | | |
| 8/10-Ar-1' | | | | | | | | | 7.84 m | 7.76 brs | | | | | |
| 2' | 7.36 to | 7.25 d | 2.30 s | 2.28 | 7.06 d | 7.15 br s | 7.72 brs | 7.56 | | | 6.97 brd | 6.94 m | 6.90 br s | | 7.15 br s |
| 3 | 7.40 m | 6.88 d | 2.31 s | 2.30 | 2.27 d | 2.24 s | 7.50 brm | 7.49 | 7.52 m | 7.42 dd | 7.30 t | | 2.32 | 7.06 to | 2.26 |
| 4' | | 3.81 s | 7.13 to | 7.13 to | 2.27 d | 2.26 s | 7.88 d | 7.88 | 7.89 d | 7.88 d | 6.88 dd | 6.80 tt | 6.99 br s | 7.46 m | 2.26 |
| 5' | " | 6.88 d | 7.17 m | 7.16 m | 7.13 d | 7.11 br.s | 7.90 d | 7.92 | 7.84 in | 7.85 m | 3.81 s | | 2.32 | " | 7.12 to |
| 6' | " | 7.25 d | " | " | 7.03 dd | " | 7.54 t | 7.52 to | 7.52 m | 7.51 to | 6.93 t | 6.94 m | 6.90 bs | " | 7.15 m |
| 7' | | | | | | | 7.59 t | 7.63 m | 7.52 m | 7.56 m | | | | | |
| 8' | | | | | | | 8.13 brs | 8.13 brs | 7.84 m | 7.85 m | | | | | |
| D 2 | 4.92 dd | 4.93 | 4.93 | 4.81 m | 4.86 | 4.81 | 4.93 | 4.86 | 4.93 dd | 4.84 dd | 4.92 | 4.91 | 4.87 dd | 4.92 dd | 4.93 m |
| 3 | 1.45 m | 1.41 | 1.50 | 1.43 | 1.53 | 1.42 | 1.47 | 1.51 | 1.46 | 1.46 | 1.45 | 1.43 | 1.54 | 1.50 | 1.46 m |
| 3' | 1.78 m | 1.78 | 1.79 | 1.68 | 1.85 | 1.69 | 1.74 | 1.86 | 1.70 to | 1.84 | 1.70 to | 1.66 to | 1.85 | 1.74 to | 1.72 to |
| 4 | 1.76 m | 1.78 | 1.79 | 1.63 | 1.85 | 1.59 | 1.74 | 1.64 | 1.81 m | 1.58 | 1.82 m | 1.82 m | 1.70 | 1.84 m | 1.82 m |
| 4-Me | 0.94 d | 0.95 | 0.95 | 0.91 | 0.97 | 0.91 | 0.93 brs | 0.93 | 0.95 | 0.97 | 0.93 | 0.94 | 0.97 | 0.95 | 0.98 |
| 5 | 0.93 d | 0.92 | 0.95 | 0.87 | 0.94 | 0.86 | 0.93 brs | 0.90 | 0.93 | 0.96 | 0.93 | 0.92 | 0.94 | 0.93 | 0.98 |

Spectra recorded in CDCl₃; The chemical shifts are for the protons or methyl or methoxyl function positioned on the carbons indicated in the table. The chemical shifts for the proptons on units B and C are within ± 0.2 ppm and coupling constants ± 0.5 Hz of the values for those in cryptophycin-8. J (H,H) in Hz for 163: 2', 3' = 5',6' = 8.7; J (H,H) in Hz for186: 2',6' = 2.0; 5',6' = 7.7; J (H,H) in Hz for193 and 194: 3',4' = 8.5; J (H,H) in Hz for 195: 2',3' = 3', 4' = 7.9; 2', 6' = 4'6' = 2.4; The observable coupling constants for the rest od the protons in the table are with in ± 0.5 Hz of the values for those in cryptophycin-8. J (HH) and J (H,F) in Hz for 212: 3'-F,4-H = 4-H,5-F =8.7; 2',9' = 4',6'= 2.3.

**Table 10. 125 MHz ¹³C NMR Data for Cryptophycins-8, -163, 184, -185, -186, 187, 191, 192, 194, 195, 222, 223 and 224. P=position**

| **P** | **8** | **163** | **184** | **185** | **186** | **187** | **191**^{**†**} | **192**^{**†**} | **194** | **195** | **212** | **222** | **223** | **224** |
|---|---|---|---|---|---|---|---|---|---|---|---|---|---|---|
| A 1 | 165 .5 | 165.4 | 165 .5 | 165 .4 | 165 .4 | 165 .5 | 165.5 | 165.4 | 165. 4 | 165.5 | 165.5 | 165.9 | 165.5 | 165.5 |
| 2 | 125 .1 | 125 .3 | 125 .2 | 124 .9 | 125 .0 | 125 .0 | 125.1 | 125.4 | 125. 1 | 125.1 | 125.4 | 125.1 | 125.3 | 125.2 |
| 3 | 141 .6 | 141 .1 | 141 .7 | 142 .0 | 141 .6 | 142 .1 | 141.7 | 141.6 | 141. 5 | 141.7 | 141.3 | 141.6 | 191.9 | 141.6 |
| 4 | 36. 3 | 36. 9 | 36. 5 | 34. 9 | 36. 2 | 34. 6 | 36.5 | 36.2 | 36.1 | 36.4 | 36.3 | 36.1 | 36.4 | 36.4 |
| 5 | 76. 3 | 75. 8 | 76. 4 | 74. 7 | 76. 2 | 74. 3 | 76.4 | 76.3 | 76.1 | 76.3 | 76.3 | 76.2 | 76.4 | ** |
| 6 | 38. 2 | 39. 5 | 38. 3 | 39. 9 | 38. 3 | 39. 6 | 38.3 | 38.3 | 38.3 | 38.3 | 38.5 | 38.4 | 38.5 | 38.4 |
| 6-Me | 8.7 | 9.6 | 8.7 | 13. 3 | 8.7 | 12. 9 | 8.8 | 9.2 | 8.7 | 8.7 | 8.7 | 8.7 | 8.6 | 8.7 |
| 7 | 74. 0 | 65. 1 | 74.1 | 76.4 | 74.3 | 77. 7 | 74.0 | 73.9 | 74.3 | 74.0 | 74.0 | 74.3 | 73.1 | 74.0 |
| 8 | 61. 9 | 62. 3 | 57. 5 | 63. 6 | 69. 0 | 67. 3 | 57.2 | 62.8 | 67.1 | 61.9 | 60.7 | 69.1 | 55.3 | 62.0 |
| 9 | | | | | | | | | | | | | | |
| 10 | | | | | | | | | | | | | | |
| 8/10-Ar-1' | 138 .3 | 131 .2 | 135 .9 | 133 .6 | 135 .0 | 135 .5 | * | * | 126. 9 | 139.7 | 142.7 | 137.4 | 130.7 | 135.5 |
| 2' | 128 .0 | 128 .8 | 137 .7 | 137 .5 | 128 .6 | 128 .7 | * | * | * | 114.1 a | 111.2 d | 125.1 | 160.6 | 129.1 |
| 3' | 129 .1 | 114 .1 | 137 .7 | 136 .1 | 137 .4 | 137 .3 | 125.2 | 125.0 | 129. 0 | 160.0 | 163.0 dd | 138.7 | 116.1 | 137.5 |
| 4' | 129 .2 | 160 .1 | 130 .5 | 130 .4 | 137 .9 | 137 .5 | 129.2 | 127.1 | 129. 3 | 114.2 a | 104.5 t | 130.9 | 129.3 | 138.0 |
| 5' | 129 .1 | 114 .1 | 126 .4 | 126 .1 | 130 .3 | 130 .1 | 129.2 | 129.4 | 127. 8 | 130.2 | 163.0 | 138.7 | 124.8 | 130.3 |
| 6' | 128 .0 | 128 .8 | 124 .5 | 125 .7 | 124 .7 | 124 .9 | 127.0 | 126.3 | 127. 0 | 120.0 | 111.2 d | 125.1 | 130.7 | 125.3 |
| 7' | | | | | | | 126.2 | 126.3 | 127. 1 | | | | | |
| 8' | | | | | | | * | * | 128. 0 | | | | | |
| 9' | | | | | | | * | 130.5 | * | | | | | |
| 10 | | | | | | | 134.5 | * | 134. 7 | | | | | |
| D 1 | 170 .6 | 170 .2 | 170 .6 | 170 .2 | 170 .4 | 170 .3 | 170.6 | 170.4 | 170. 9 | 170.6 | 170.7 | 170.4 | 170.7 | 170.6 |
| 2 | 71. 3 | 71. 3 | 71. 3 | 71. 5 | 71. 4 | 71. 5 | 71.3 | 71.4 | 71.4 | 71.3 | 71.3 | 71.4 | 71.4 | 71.4 |
| 3 | 39. 7 | 39. 6 | 39. 7 | 39. 3 | 39. 7 | 39. 4 | 39.6 | 39.7 | 39.7 | 39.7 | 39.9 | 39.7 | 39.6 | 39.7 |
| 4 | 24. 7 | 24. 8 | 24. 7 | 24. 6 | 24. 8 | 24. 7 | 24.0 | 24.8 | 24.8 | 24.7 | 24.7 | 24.8 | 24.7 | 24.7 |
| 4-Me | 23. 1 | 23. 3 | 23. 2 | 22. 8 | 23. 1 | 22. 7 | 23.1 | 23.1 | 23.1 | 23.0 | 23.0 | 23.1 | 23.1 | 23.1 |
| 5 | 21. 5 | 21. 5 | 21. 5 | 21. 6 | 21. 7 | 21. 6 | 21.5 | 21.7 | 21.7 | 21.4 | 21.5 | 21.8 | 21.4 | 21.5 |

| | | | | | | | | | | | | | | |
|---|---|---|---|---|---|---|---|---|---|---|---|---|---|---|
| Spectra recorded in CDCl3. The chemical shifts for carbons in units B and C are within ± 0.5 ppm of the values in cryptophycin-8. | | | | | | | | | | | | | | |
| * Signals could not be found due to the smaller size of the sample. | | | | | | | | | | | | | | |
| † Assignments are tentative. | | | | | | | | | | | | | | |
| ** Merged with solvent signals. | | | | | | | | | | | | | | |

The 4-OMe carbon of 163 and also that of 195 was resonated at δ 55.3. The 2'-Me and 3'-Me carbons of 185 appeared at δ 15.0 and 20.9 respectively. The 3'-Me and 4'-Me carbons of 186 were observed at δ 19.6 and 19.9 respectively. The same carbons were observed respectively at δ 19.5 and 19.8 in 187 and at δ 19.5 and 19.8 in 224. The 3' and 5'-Me carbons of 223 were observed at 21.3.

### Example 94

The general group R in **2'(below)** is comprised of an ether substituent and several acyl substituents, as depicted in **Table 1 (also below)**. The novel cryptophycin analogs were prepared in moderate to high yield via esterification techniques. See, Mulzer, J., Trost B.M. Fleming, I., Ed. *Comprehensive Organic Synthesis;* 6:323-380, (Pergamon press: Oxford, 1991) ; Benz, G. Trost, B.M., Fleming, I, Ed., *Comprehensive Organic Synthesis,* 6:381-417, (Pergamon press: Oxford, 1991). Amine hydrochloride salts were prepared in high yield from the corresponding t-butyl carbamates upon treatment with hydrochloric acid while di-sodium salt **7'** was derived from **6'** following hydrochloric acid induced *t*-butyl ester cleavage and sodium hydroxide treatment. Pyridinium salt **9'** was prepared from **1'** following the method of Nicolaou . Nicolauo, *et. al. Angew. Chem. Int. Ed. Engl.,* **33** : 1583+ (1994). 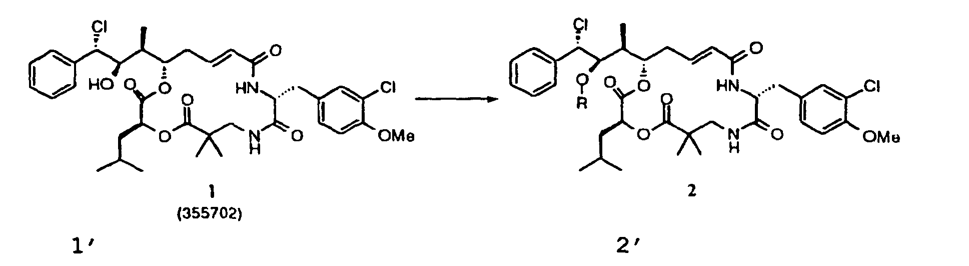 (as used in description of synthesis methods below)

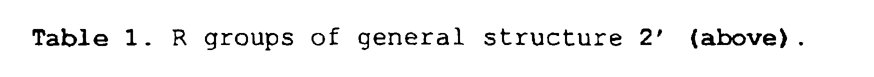

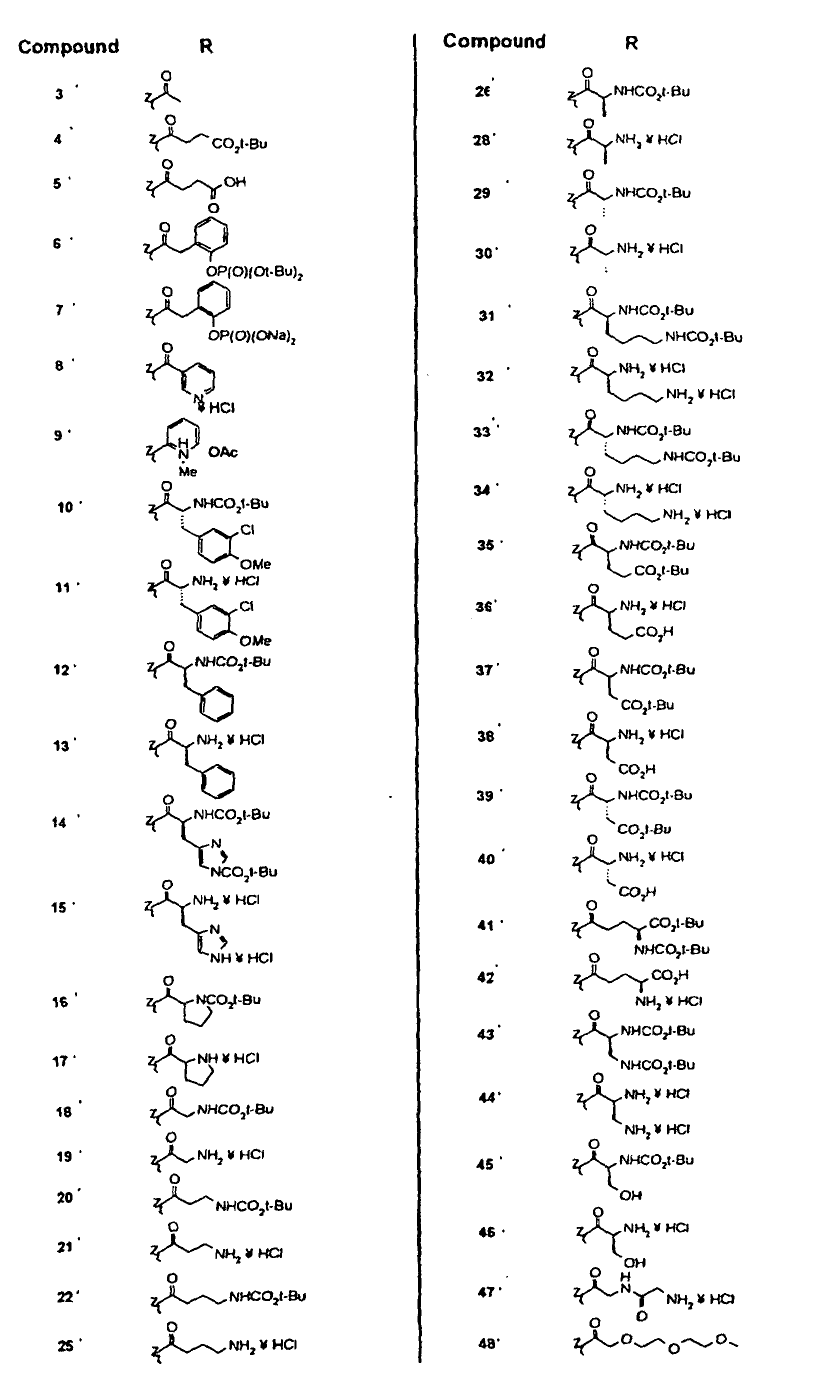

The preparation of the conjugates depicted in Table 1 follows several protocols typically involving activated ester methodology followed by chromatography and acid induced deblocking where necessary. Thus, treatment of **1'** with acetic anhydride in the presence of triethylamine and 4-dimethylamino pyridine provides **3'** in 89% yield after flash chromatography. Similarly, **5'** can be pepared from **1'** *via* the agency of succinic anhydride followed by reverse phase HPLC purification.⁷ Alternatively, **5'** is prepared in high yield upon acid treatment of mono-*t*-butyl ester **4'.** Exposure of a pyridine solution of **1'** to commercially available nicotinoyl chloride hydrochloride in the presence of triethyamine and 4-dimethylamino pyridine followed by chromatography and hydrogen chloride treatment gives rise to **8'** in high yield. Pyridinium salt **9'** is prepared in 47% yield according to the method of Nicolaou (ibid) whereby 1' is treated with commercially available 2-fluoro-1-methylpyridinium *p*-toluenesulfonate followed by reverse phase HPLC purification with concomitant anion exchange (acetate for *p*-toluenesulfonate) and lyophilization. Remaining esters are all prepared in moderate to high yields from 1' and commercially available (except in the case of **4', 6, 10', 43',** and **45')** N-*t*-boc protected amino acids with activation via the agency 1,3-dicyclohexylcarbodiimide in the presence of 4-dimethylamino pyridine. Hydrochloride salts are prepared in high yield upon treatment with a 4.0 M solution of hydrogen chloride in dioxane and removal of solvent *in vacuo.* Di-sodium salt **7'** is derived from **6'** following hydrochloric acid induced t-butyl ester cleavage and sodium hydroxide treatment. The requisite acid **24'** for the preparation of **5'** is synthesized in 63% yield by way of a 5 step sequence featuring the method of Johns for installing the phosphate functionality. Perich, J.W.; Johns R.B. et al. Synthesis **1988:** 142.

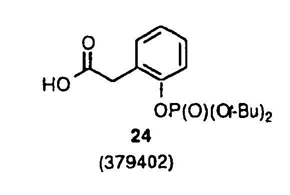

Several of the novel conjugates have been assayed for *in vitro* cytotoxicity in the Gc3 tumor cell model, as described herein *supra.* These results are shown in **Table 2** below:

**Table 2. In vitro cytotoxicity data for cryptophycin derivatives.**

| Compound | Gc3 IC₅₀ (nM) | Compound | Gc3 IC₅₀ (nM) |
|---|---|---|---|
| **1'** | 0.065 | **28'** | 2.6 |
| **3'** | 83 | **30'** | 30 |
| **5'** | 31 | **32'** | 7.2 |
| **7'** | 3.7 | **34'** | 6.5 |
| **8'** | 116 | **36'** | 2.8 |
| **9'** | 2.2 | **38'** | 1.6 |
| **11'** | 6.8 | **40'** | 2.0 |
| **13'** | 7.0 | **42'** | 15 |
| **15'** | 60 | **44'** | 28 |
| **17'** | 3.6 | **46'** | 50 |
| **19'** | 0.10 | **47'** | 2.5 |
| **21'** | 21 | **48'** | 10 |
| **25'** | 230 | | |

Stability studies were conducted at aqueous pHs ranging from 4-8 and percent product remaining intact was determined.

The conversion of *t*-butyl carbamates to the corresponding salts could be effected with any strong acid, namely, mineral acids comprised of hydrogen halides, hydrogen sulfates, hydrogen phosphates, hydrogen nitrates, hydrogen perchlorates, or strong organic acids such as trifluoroacetic, *p*-toluenesulfonic, and methanesulfonic. The same acids could be used to produce salts of type 8' from the corresponding free base. A variety of counterions (cations) could comprise salts of type 7' including any of the alkali and alkaline earth metals. A variety of counterions (anions) could comprise salts of type 9', namely, any conjugate base of an acid (organic or mineral).

**Preparation of Cryptophycin 55 acetate (3') (LSN 362376).** To a solution of 1' (93 mg, 0.13 mmol) in 659 µl of methylene chloride at 0 °C was added triethylamine (55 µl, 0.40 mmol), 4-dimethylamino pyridine (1.6 mg, 0.013 mmol), and acetic anhydride (19 µl, 0.20 mmol). After stirring at 0 °C for 1h the reaction was quenched with 19 µl of methanol, concentrated to 0.5 volume, and applied directly to a flash chromatography column (19 g of flash silica gel). Elution with ethyl acetate-hexanes (3:1) provided 88 mg (89%) of the title compound as a white foam: 500 MHz ¹H NMR (CDCl₃) δ 7.38-7.31 (m, 5H), 7.24 (d, 1H, J = 2.1 Hz), 7.22-7.18 (m, 1H), 7.10 (dd, 1H, J = 8.5, 2.1 Hz), 6.88 (d, 1H, J = 8.5 Hz), 6.75 (ddd, 1H, J = 15, 13, 4.6 Hz), 5.78 (dd, 1H, J = 15, 1.0 Hz), 5.55 (d, 1H, J = 7.9 Hz), 5.46 (dd, 1H, J = 9.8, 1.2 Hz), 9.95 (dd, 1H, J = 11, 2.9 Hz), 4.89 (ddd, 1H, J = 9.9, 9.9, 1.7 Hz), 4.81 (d, 1H, J = 9.8 Hz), 4.79-4.74 (m, 1H), 3.91 (s, 3H), 3.39 (dd, 1H, J = 13, 8.1 Hz), 3.22 (dd, 1H, J = 13, 4.1 Hz), 3.16 (dd, 1H, J = 14, 5.1 Hz), 3.07 (dd, 1H, J = 14, 7.6 Hz), 2.65-2.55 (m, 2H), 2.47-2.39 (m, 1H), 1.95 (ddd, 1H, J = 14, 13, 4.6 Hz), 1.86-1.77 (m, 1H), 1.73-1.66 (m, 1H), 1.68 (s, 3H), 1.27 (s, 3H), 1.19 (s, 3H), 1.09 (d, 3H, J = 7.1 Hz), 1.03 (d, 3H, J = 6.7 Hz), 0.97 (d, 3H, J = 6.6 Hz).

**Preparation of Cryptophycin 55 succinatetert-butyl ester (4') (LSN 384665).** To a solution of **1'** (133 mg, 0.188 mmol), succinic acid mono-tert-butyl ester¹⁰ (66 mg, 0.377 mmol) and 4-dimethylamino pyridine (2.3 mg, 0.019 mmol) in 850 µl of anhydrous methylene chloride at room temperature was added a solution of 1,3-dicyclohexylcarbodiimide (78 mg, 0.377 mmol) in 92 µl of methylene chloride. After stirring at room temperature for 1 h the reaction was treated with 150 mg of celite and diluted with 1 ml of ethyl acetate-hexanes (3:1) and filtered through a plug of celite, washing with ethyl acetate-hexanes (3:1). The filtrate and washings were concentrated *in vacuo* to an off-white oil. Chromatography (25 g of flash silica gel), eluting with ethyl acetate-hexanes (2:1) provided 157 mg (97%) of the title compound as a white foam: 500 MHz ¹H NMR (CDCl₃) δ 7.38-7.30 (m, 5H), 7.24 (d, 1H, J = 2.0 Hz), 7.22-7.18 (m, 1H), 7.09 (dd, 1H, J = 8.5, 2.0Hz), 6.88 (d, 1H, J = 8.5 Hz), 6.74 (ddd, 1H, J = 15, 10, 4.6 Hz), 5.76 (d, 1H, J = 15 Hz), 5.51 (d, 1H, J = 8.0 Hz), 5.47 (d, 1H, J = 9.6 Hz), 4.97 (dd, 1H, J = 11, 3.1 Hz), 4.90 (t, 1H, J = 9.3 Hz), 4.83 (d, 1H, J = 9.6 Hz), 4.79-4.73 (m, 1H), 3.91 (s, 3H), 3.39 (dd, 1H, J = 13, 8.0 Hz), 3.22 (dd, 1H, J = 13, 4.0 Hz), 3.16 (dd, 1H, J = 14, 5.1 Hz), 3.08 (dd, 1H, J = 14, 7.6 Hz), 2.63-2.55 (m, 2H), 2.44-2.37 (m, 1H), 2.32-2.21 (m, 2H), 2.19-2.06 (m, 2H), 1.99-1.92 (m, 1H), 1.85-1.68 (m, 2H), 1.42 (s, 9H), 1.27 (s, 3H), 1.19 (s, 3H), 1.08 (d, 3H, J = 7.0 Hz), 1.03 (d, 3H, J = 6.6 Hz), 0.98 (d, 3H, J = 6.5 Hz).

**Preparation of Cryptophycin 55 succinate (5') (LSN 377092) from cryptophycin 55 succinate tert-butyl ester.** To a solution of **4'** (127 mg, 0.147 mmol) in 491 µl of methylene chloride at room temperature was added a 4.0 M solution of hydrogen chloride in 1,4-dioxane (184 µl, 0.737 mmol). The solution was allowed to stir at room temperature for 5 h. Concentration *in vacuo* provided 119 mg (100%) of the title compound as a white foam.

**Preparation of Cryptophycin 55 succinate (5') (LSN 377092) from succinic anhydride.** To a solution of **1'** (27 mg, 0.038 mmol) and succinic anhydride (5.7 mg, 0.057 mmol) in 383 µl of methylene chloride at room temperature was added triethylamine (16 µl, 0.115 mmol) and 4-dimethylamino pyridine (4.7 mg, 0.038 mmol). After stirring for 19 h, another 5.7 mg (0.057 mmol) of succinic anhydride and 4.7 mg (0.038 mmol) of 4-dimethylamino pyridine were added followed by stirring an additional 29 h. The reaction was treated with 0.5 ml of 1 N aqueous hydrochloric acid and washed with methylene chloride (3 X 0.5 ml). The combined organic extracts were dried (Na₂SO₄), filtered, and concentrated *in vacuo* to white foam. Reverse phase HPLC⁷ purification provided 10 mg (32%) of the title compound as a white foam: 500 MHz ¹H NMR (CDCl₃) δ 7.36-7. 31 (m, 6H), 7.22 (br s, 1H), 7.08 (d, 1H, J = 8.4 Hz), 7.02 (br s, 1H), 6.87 (d, 1H, J = 8.4 Hz), 6.61 (m, 1H), 5.94-5.87 (m, 2H), 5.51 (d, 1H, J = 9.8 Hz), 4.95 (dd, 1H, J = 10, 2.8 Hz), 4.87-4.76 (m, 3H), 3.90 (s, 3H), 3.39 (dd, 1H, J = 16, 5.6 Hz), 3.28 (dd, 1H, J = 16, 8.2 Hz), 3.17 (dd, 1H, J = 16, 5.6 Hz), 3.05 (dd, 1H, J = 16, 8.2 Hz), 2.68-2.62 (m, 1H), 2.60-2.46 (m, 2H), 2.45-2.28 (m, 3H), 2.01-1.93 (m, 2H), 1.88-1.70 (m, 2H), 1.26 (s, 3H), 1.18 (s, 3H), 1.12 (d, 3H, J = 7.0 Hz), 1.04 (d, 3H, J = 6.6 Hz), 1.00 (d, 3H, J = 6.5 Hz).

**Preparation of Cryptophycin 55 (2'-di-*t-*butylphosphatyl)phenylacetate (6') (LSN 379407).** To a solution of 1' (0.102 mmol), **24'** (46 mg, 0.134 mmol), and 4-dimethylamino pyridine (12 mg, 0.102 mmol) in 250 µl of anhydrous methylene chloride at room temperature was added a solution of 1,3-dicyclohexylcarbodiimide (27 mg, 0.134 mmol) in 50 µl of methylene chloride. After stirring at room temperature for 6 h the reaction was diluted with 1 ml of ethyl acetate-hexanes (3:1) and filtered through a plug of celite, washing with ethyl acetate-hexanes (3:1). The filtrate and washings were concentrated *in vacuo* to a purple foam. Chromatography (15 g of flash silica gel), eluting with ethyl acetate-hexanes (4:1) provided 86 mg (82%) of the title compound as a white foam: 500 MHz ¹H NMR (CDCl₃) δ 7.35 (d, 1H, J = 8.3 Hz), 7.30-7.19 (m, 8H), 7.11 (dd, 1H, J = 8.4, 2.0 Hz), 7.02 (t, 1H, J = 7.5 Hz), 6.87 (d, 1H, J = 8.4 Hz), 6.84 (d, 1H, J = 7.5 Hz), 6.73 (ddd, 1H, J = 15, 13, 4.7 Hz), 5.92 (d, 1H, J = 7.9 Hz), 5.79 (dd, 1H, J = 15, 1.0 Hz), 5.43 (dd, 1H, J = 9.4, 1.8 Hz), 4.98 (dd, 1H, J = 12, 3.1 Hz), 4.81 (ddd, 1H, J = 9. 9, 9.9, 1.8 Hz), 4.75 (d, 1H, J = 9.4 Hz), 4.73-4.67 (m, 1H), 3.90 (s, 3H), 3.49 (d, 1H, J = 16 Hz), 3.44-3.38 (m, 1H), 3.38 (d, 1H, J = 16 Hz), 3.27-3.17 (m, 2H), 3.10 (dd, 1H, J = 14, 8.2 Hz), 2.55-2.46 (m, 2H), 2.37-2.27 (m, 1H), 1.95 (ddd, 1H, J = 14, 12, 4.5 Hz), 1.83-1.70 (m, 2H), 1.49 (s, 18H), 1.27 (s, 3H), 1.20 (s, 3H), 1.03 (d, 3H, J = 6.5 Hz), 0.97 (d, 3H, J = 6.4 Hz), 0.92 (d, 3H, J = 7.0 Hz).

**Preparation of 2'-(di-t-Butylphosphatyl)phenylacetic acid (24') (LSN 379402).** To a solution of 2'-hydroxyphenethyl alcohol (1.05 g, 7.60 mmol) in 15.2 ml of N, N-dimethylformamide at 0 °C was added imidazole (621 mg, 9.11 mmol) and *tert*-butyldimethylsilyl chloride (1.26 g, 8.34 mmol). After stirring at 0 °C for 40 min and at room temperature for 45 min, another 155 mg (2.28 mmol) of imidazole and 229 mg (1.52 mmol) of tert-butyldimethylsilyl chloride were added. The reaction was allowed to stir for an additional 15 min at which time 150 ml of tert-butyl methy ether was added. The mixture was washed with cold 1N aqueous hydrochloric acid (1 X 15 ml) followed by water (1 X 15 ml). The organic layer was dried (Na₂SO₄), filtered, and concentrated *in vacuo* to a yellow oil. Chromatography (70 g of flash silica gel), eluting with hexanes-ethyl acetate (5:1) provided 1.81 g (94%) of the primary silyl ether as a faintly off-white oil. To a solution of the silyl ether (506 mg, 2.00 mmol) and di-tert-butyl diethylphosphoramidite (600 µl of 93%, 2.00 mmol) in 2 ml of tetrahydrofuran at room temperature was added 1-H-tetrazole (421 mg, 6.01 mmol). After stirring for 45 min the reaction mixture was cooled to -10 °C and rapidly treated with a solution of m-chloroperbenzoic acid (450 mg of 99%, 2.61 mmol) in 3.6 ml of methylene chloride. The cloudy white reaction was allowed to warm to room temperature and stir for 15 min. The reaction was quenched with 4 ml of 10% aqueous sodium bisulfite, stirred vigorously for 10 min, diluted with 15 ml of tert-butyl methy ether, and washed with 10% aqueous sodium bisulfite (2 X 10 ml) followed by 0.5 N aqueous sodium hydroxide (2 X 10 ml). The organic layer was dried (Na₂SO₄), filtered, and concentrated *in vacuo* to a colorless oil (941 mg) which was used directly in the next step. The crude phosphate was dissolved in 10 ml of tetrahydrofuran, cooled to 0 °C, and treated with a 1 M solution of tetra-n-butylammonium fluoride in tetrahydrofuran (2.4 ml, 2.4 mmol). After stirring at 0 °C for 20 min and at room temperature for 1.5 h, the reaction was diluted 60 ml of tert-butyl methy ether and washed with water (1 X 10 ml) followed by brine (1 X 10 ml). The organic layer was dried (Na₂SO₄), filtered, and concentrated in *vacuo* to a yellow oil. Chromatography (50 g of flash silica gel), eluting with ethyl acetate-hexanes (3:1) provided 525 mg (79%) of the 1° alcohol as an off-white oil. To a solution of the alcohol (123 mg, 0.372 mmol) in acetonitrile-carbon tetrachloride (1:1, 1.49 ml) at room temperature was added water (1.1 ml) followed by sodium periodate (239 mg, 1.12 mmol) and ruthenium (III) chloride hydrate (1.8 mg, 0.0082 mmol). The brown mixture was allowed to stir rapidly at room temperature for 55 min. Upon concentration *in vacuo* and chromatography (8 g of flash silica gel, eluting with 10% methanol-ethyl acetate) 109 mg (85%) of the title compound was obtained as a purple oil: 500 MHz ¹H NMR (CDCl₃) δ 7.49 (d, 1H, J = 7.53 Hz), 7.30-7.15 (m, 3H), 3.77 (s, 2H), 1.51 (s, 18H).

**Preparation of Cryptophycin 55 (2'-phosphatyl)phenylacetate di-sodium salt (7') (LSN 374122).** To a solution of 6' (84 mg, 0.081 mmol) in 400 µl of methylene chloride at room temperature was added a 4.0 M solution of hydrogen chloride in 1,4-dioxane (81 µl, 0.33 mmol). The faint yellow solution was allowed to stir at room temperature for 2 h. After concentration *in vacuo* to an off-white foam, the crude dihydrogen phosphate was dissolved in 614 µl of tetrahydrofuran and treated with a 5.00 N aqueous solution of sodium hydroxide (33 µl, 0.163 mmol). After stirring for 10 min the mixture was concentrated *in vacuo* to a tan foam. The crude salt was taken up in 1 ml of hot acetonitrile and 0.1 ml of hot water. The insolubles were filtered off and the filtrate was concentrated *in vacuo* to provide 69 mg (87%) of the title compound as a white solid: 500 MHz ¹H NMR (MeOH-d₄) 57.58 (d, 1H, J = 8.2 Hz), 7.38-7.32 (m, 2H), 7.32-7.28 (m, 3H), 7.28 (d, 1H, J = 2.1 Hz), 7.17 (dd, 1H, J = 8.5, 2.1 Hz), 7.09 (ddd, 1H, J = 7.8, 7.8, 1.7 Hz), 6.98 (d, 1H, J = 8.5 Hz), 6.79-6.70 (m, 2H), 6.67 (d, 1H, J = 7.4 Hz), 5.91 (dd, 1H, J = 15, 1.7 Hz), 5.45 (dd, 1H, J = 9.4, 1.6 Hz), 5.06 (dd, 1H, J = 10, 2.7 Hz), 5.01 (d, 1H, J = 9.4 Hz), 4.89-4.80 (m, 1H), 4.47 (dd, 1H, J = 11, 3.8 Hz), 3.84 (s, 3H), 3.67 (d, 1H, J = 16 Hz), 3.45 (d, 1H, J = 14 Hz), 3.42 (d, 1H, J = 16 Hz), 3.18 (dd, 1H, J = 14, 3.8 Hz), 3.12 (d, 1H, J = 14 Hz), 2.77 (dd, 1H, J = 14, 11 Hz), 2.67-2.60 (m, 1H), 2.56-2.48 (m, 1H), 2.31-2.22 (m, 1H), 1.96-1.88 (m, 1H), 1.85-1.77 (m, 2H), 1.22 (s, 3H), 1.20 (s, 3H), 1.03 (d, 3H, J = 6.2 Hz), 0.98 (d, 3H, J = 6.1 Hz), 0.93 (d, 3H, J = 7.1 Hz) .

**Preparation of Cryptophycin 55 nicotinoate hydrochloride salt (8') (LSN 368265).** To a solution of 1' (50 mg, 0.071 mmol) in 354 µl of pyridine at room temperature was added nicotinoyl chloride hydrochloride (15 mg, 0.085 mmol) followed by triethylamine (23 µl, 0.170 mmol). After stirring for 1.5 h, 4-dimethylamino pyridine (8.6 mg, 0.071 mmol) was added. After stirring 5 h, additional triethylamine (23 µl, 0.170 mmol), 4-dimethylamino pyridine (8.6 mg, 0.071 mmol), and nicotinoyl chloride hydrochloride (15 mg, 0.085 mmol) was added along with a 50 µl pyridine rinse. After stirring 18 h the reaction was treated with 0.5 ml of saturated aqueous sodium bicarbonate and washed with methylene chloride (4 X 1 ml). The combined organic extracts were dried (Na₂SO₄), filtered, and concentrated in *vacuo* to a light brown oil. Chromatography (14 g of flash silica gel), eluting with ethyl acetate-hexanes (10:1) provided 49 mg (85%) of the free base as a white foam. The nicotinoate was dissolved in 1 ml of methylene chloride and treated with a 1.0 M solution of hydrogen chloride in diethyl ether (90 µl, 0.090 mmol). The clear, colorless soution was allowed to stand at room temperature for 5 min. Removal of the solvent *in vacuo* produced 51 mg of the title compound as a white foam: 500 MHz ¹H NMR (CDCl₃) δ 8.94 (s, 1H), 8.78 (br s, 1H), 8.29 (d, 1H, J = 7.0 Hz), 7.57 (br s, 1H), 7.38 (d, 2H, J = 7.1 Hz), 7.30-7.16 (m, 5H), 7.10 (dd, 1H, J = 8.4, 1.7 Hz), 6.88 (d, 1H, J = 8.4 Hz), 6.71 (m, 1H), 5.80 (d, 1H, J = 15 Hz), 5.74 (d, 1H, J = 9.6 Hz), 5.56 (br s, 1H), 5.00 (d, 1H, J = 9.6 Hz), 4.95 (t, 1H, J = 8.9 Hz), 4.84 (d, 1H, J = 9.8 Hz), 4.77-4.72 (m, 1H), 3.91 (s, 3H), 3.39 (dd, 1H, J = 13, 8.2 Hz), 3.23-3.14 (m, 2H), 3.06 (dd, 1H, J = 14, 7.6 Hz), 2.81-2.74 (m, 1H), 2.62-2.45 (m, 2H), 1.93 (ddd, 1H, J = 14, 12, 4.8 Hz), 1.78-1.70 (m, 1H), 1.66-1.59 (m, 1H), 1.25 (s, 3H), 1.20 (d, 3H, J = 7.0 Hz), 1.19 (s, 3H), 0.98 (d, 3H, J = 6.7 Hz), 0.84 (d, 3H, J = 6.5 Hz).

**Preparation of Cryptophycin 55 N-methylpyridinium acetate salt (9') (LSN 366550).** To a solution of **1'** (53 mg, 0.075 mmol) in 751 µl of methylene chloride at 0 °C was added triethylamine (13 µl, 0.090 mmol) followed by 2-fluoro-l-methylpyridinium p-toluenesulfonate (23 mg, 0.083 mmol). The heterogeneous reaction mixture was warmed to room temperature and stirred for 3.5 h at which time another 11 mg (0.039 mmol) of 2-fluoro-1-methylpyridinium *p-*toluenesulfonate was added. After stirring for 14.5 h another 11 mg (0.039 mmol) of 2-fluoro-1-methylpyridinium *p-*toluenesulfonate was added followed by another 11 mg (0.039) of 2-fluoro-1-methylpyridinium *p*-toluenesulfonate and 13 µl (0.090) of triethylamine after 2.5 h. After stirring an additional 1h the reaction was concentrated *in vacuo* to an orange foam. Purification by reverse phase HPLC⁸ with concomitant anion exchange (acetate for p-toluenesulfonate) followed by lyophilization yielded 30 mg (47%) of the title compound as a white solid: 500 MHz ¹H NMR (DMSO-d₆) δ 8.65-8.58 (m, 2H), 8.36 (t, 1H, J = 7.8 Hz), 7.68 (d, 1H, J = 8.9 Hz), 7.60 (d, 1H, J = 6.6 Hz), 7.48 (t, 1H, J = 6.6 Hz), 7.35-7.21 (m, 6H), 7. 19 (dd, 1H, J = 8.5, 1.9 Hz), 7.05 (d, 1H, J = 8.5 Hz), 6.49 (ddd, 1H, J = 16, 13, 4.0 Hz), 5.91 (d, 1H, J = 16 Hz), 5.72 (d, 1H, J = 8.0 Hz), 5.66 (dd, 1H, J = 8.0, 1.9 Hz), 5.32-5.27 (m, 1H), 4.73 (dd, 1H, J = 9.7, 4.3 Hz), 4.24 (ddd, 1H, J = 11, 9.8, 3.7 Hz), 3.93 (s, 3H), 3.81 (s, 3H), 3.32 (dd, 1H, J = 13, 9.3 Hz), 3.05-2.97 (m, 2H), 2.77-2.57 (m, 3H), 2.54-2.47 (m, 1H), 1.76 (s, 3H), 1.68-1.62 (m, 1H), 1.55-1.46 (m, 1H), 1.37-1.30 (m, 1H), 1.15 (d, 3H, J = 7.0 Hz), 1.13 (s, 3H), 1.00 (s, 3H), 0.88 (d, 3H, J = 6.7 Hz), 0.73 (d, 3H, J = 6.5 Hz) .

**Preparation of Cryptophycin 55 N-*t*-Boc-3-(3-chloro-4-methoxyphenyl)-(D)-alaninate (10') (LSN 382049).** To a solution of 1' (23 mg, 0.033 mmol), N-t-Boc-3-(3-chloro-4-methoxyphenyl)-(*D*)-alanine^{4c} (16 mg, 0.049 mmol), and 4-dimethylamino pyridine (few crystals) in 143 µl of anhydrous methylene chloride at room temperature was added a solution of 1,3-dicyclohexylcarbodiimide (10 mg, 0.049 mmol) in 20 µl of methylene chloride. After stirring for 2 h, the cloudy white reaction mixture was diluted with ethyl acetate-hexanes (2:1, 1 ml), stirred for 10 min, and filtered through a plug of celite, washing with ethyl acetate:hexanes (2:1). The filtrate and washings were concentrated *in vacuo* to a colorless oil. Chromatography (14 g of flash silica gel, 2:1 ethyl acetate-hexanes) afforded 29 mg (88%) of the title compound as a white foam: 500 MHz ¹H NMR (CDCl₃) δ 7.42-7.27 (m, 5H), 7.24 (d, 1H, J = 2.0 Hz), 7.25-7.18 (m, 1H), 7.09 (dd, 1H, J = 8.4, 1.9 Hz), 6.91-6.86 (m, 2H), 6.84-6.70 (m, 3H), 5. 75 (d, 1H, J = 15 Hz), 5.53 (d, 1H, J = 9.6 Hz), 5.47 (d, 1H, J = 7.6 Hz), 5.00 (dd, 1H, J = 10, 2.9 Hz), 4.90-9.80 (m, 2H), 9.78-4.71 (m, 1H), 4.63 (d, 1H, J = 8.3 Hz), 9.19-4.12 (m, 1H), 3.91 (s, 3H), 3.88 (s, 3H), 3.40 (dd, 1H, J = 13, 8.1 Hz), 3.25-3.12 (m, 2H), 3.07 (dd, 1H, J = 14, 7.6 Hz), 2.67-2.57 (m, 2H), 2.39-2.27 (m, 2H), 2.15 (dd, 1H, J = 14, 8.0 Hz), 2.01 (ddd, 1H, J = 14, 12, 4.2 Hz), 1.87-1.76 (m, 2H), 1.39 (s, 9H), 1.27 (s, 3H), 1.20 (s, 3H), 1.09-1.04 (m, 6H), 1.01 (d, 3H, J = 6.3 Hz).

**Preparation of Cryptophycin 55 3-(3-chloro-4-methoxyphenyl)-(D)-alaninate hydrochloride salt (11') (LSN 382048).** To a solution of **10'** (27 mg, 0.027 mmol) in 265 µl of methylene chloride at room temperature was added a 4.0 M solution of hydrogen chloride in 1,4-dioxane (33 µl, 0.133 mmol). After stirring for 3 h, the clear, colorless reaction mixture was concentrated *in vacuo* to provide 26 mg (96%, corrected for 5 wt% dioxane) of the title compound as a white foam: 500 MHz ¹H NMR (MeOH-d₄) δ 7.79 (d, 1H, J = 7.3 Hz), 7.49-7.45 (m, 2H), 7.43-7.48 (m, 3H), 7.31 (d, 1H, J = 2.0 Hz), 7.20 (dd, 1H, J = 8.9, 2.1 Hz), 7.13 (d, 1H, J = 2.2 Hz), 7.07-6.95 (m, 3H), 6.71 (ddd, 1H, 15, 13, 3.8 Hz), 5.98 (dd, 1H, J = 15, 1.8 Hz), 5.69 (d, 1H, J = 10 Hz), 5.22 (d, 1H, J = 10 Hz), 5.18 (dd, 1H, J = 10, 2.5 Hz), 9.89-9.80 (m, 1H), 9.53 (dd, 1H, J = 11, 3.7 Hz), 4.16 (dd, 1H, J = 10, 4.4 Hz), 3.88 (s, 3H), 3.87 (s, 3H), 3.51 (dd, 1H, J = 13, 9.9 Hz), 3.20 (dd, 1H, J = 14, 3.7 Hz), 3.14 (dd, 1H, J = 13, 2.3 Hz), 2.82-2.75 (m, 3H), 2.45 (dd, 1H, J = 15, 4.5 Hz), 2.42-2.34 (m, 1H), 2.08-2.00 (m, 1H), 1.97-1.86 (m, 3H), 1.27 (s, 3H), 1.21 (s, 3H), 1.16 (d, 3H, J = 7.1 Hz), 1.10 (d, 3H, J = 6.1 Hz), 1.06 (d, 3H, J = 6.0 Hz).

**Preparation of Cryptophycin 55 N-t-Boc-(L)-phenylalaninate (12') (LSN 382235).** To a solution of 1' (29 mg, 0.041 mmol), N-*t*-Boc-*L*)-phenylalanine (16 mg, 0.062 mmol), and 4-dimethylamino pyridine (0.5 mg, 0.0041 mmol) in 165 µl of anhydrous methylene chloride at room temperature was added a solution of 1,3-dicyclohexylcarbodiimide (13 mg, 0.062 mmol) in 42 µl of methylene chloride. After stirring for 40 min, the cloudy white reaction mixture was diluted with ethyl acetate-hexanes (2:1, 0.5 ml), stirred for 10 min, and filtered through a plug of celite, washing with ethyl acetate:hexanes (2:1). The filtrate and washings were concentrated *in vacuo* to a colorless oil. Chromatography (12 g of flash silica gel, 2:1/ethyl acetate-hexanes) afforded 20 mg (50%) of the title compound as a white foam: 500 MHz ¹H NMR (MeOH-d₄) δ 7.40-7.47 (m, 2H), 7.26-7.37 (m, 6H), 7.12-7.22 (m, 4H), 7.00 (d, 1H, J = 8.4 Hz), 6.78-6.71 (m, 1H), 5.94 (d, 1H, J = 15 Hz), 5.48 (d, 1H, J = 9.5 Hz), 5.15-5.10 (m, 1H), 5.06 (d, 1H, J = 9.5 Hz), 4.68 (t, 1H, J = 9.7 Hz), 4.52 (dd, 1H, J = 11, 3.7 Hz), 4.14-4.10 (m, 1H), 3.87 (s, 3H), 3.49 (d, 1H, J = 13 Hz), 3.20 (dd, 1H, J = 14, 3.7 Hz), 3.14 (d, 1H, J = 13 Hz), 2.77 (dd, 1H, J = 14, 11 Hz), 2.67 (dd, 1H, J = 14, 6.2 Hz), 2.62-2.57 (m, 2H), 2.51 (dd, 1H, J = 14, 9.1 Hz), 2.31-2.23 (m, 1H), 2.00-1.92 (m, 1H), 1.91-1.82 (m, 2H), 1.38 (s, 9H), 1.26 (s, 3H), 1.24 (s, 3H), 1.08 (d, 3H, J = 5.9 Hz), 1.03 (d, 3H, J = 5.8 Hz), 0.87 (d, 3H, J = 7.0 Hz).

**Preparation of Cryptophycin 55 (*L*)-phenylalaninate hydrochloride salt (13') (LSN 382236).** To a solution of 12' (18 mg, 0.019 mmol) in 189 µl of methylene chloride at room temperature was added a 4.0 M solution of hydrogen chloride in 1,4-dioxane (24 µl, 0.094 mmol). After stirring for 4 h, the clear, colorless reaction mixture was concentrated in *vacuo* to provide 15 mg (88%, corrected for 2 wt% dioxane) of the title compound as a white foam: 500 MHz ¹H NMR (MeOH-d₄) δ 7.79 (d, 1H, J = 7.6 Hz), 7.48-7.23 (m, 11H), 7.20 (dd, 1H, J = 8.6, 1.6 Hz), 6.75-6.68 (m, 1H), 5.97 (d, 1H, J = 16 Hz), 5.55 (d, 1H, J = 9.2 Hz), 5.15-5.11 (m, 2H), 4.71 (t, 1H, J = 10 Hz), 4.55-4.51 (m, 1H), 3.87 (s, 3H), 3.21 (dd, 1H, J = 14, 3.8 Hz), 3.17-3.10 (m, 2H), 2.94 (dd, 1H, J = 15, 8.1 Hz), 2.78 (dd, 1H, J = 14, 12 Hz), 2.69-2.63 (m, 2H), 2.34-2.27 (m, 1H). 1.98-1.92 (m, 1H), 1.89-1.82 (m, 2H), 1.25 (s, 3H), 1.22 (s, 3H), 1.06 (d, 3H, J = 6.2 Hz), 1.03 (d, 3H, J = 6.1 Hz), 0.89 (d, 3H, J = 7 .0 Hz) .

**Preparation of Cryptophycin 55 (L)-histidinate dihydrochloride salt (15') (LSN 384046).** To a solution of **1'** (19 mg, 0.027 mmol), N,N'-di-*t*-Boc-(*L*)-histidine benzene complex (18 mg, 0.040 mmol), and 4-dimethylamino pyridine (0.3 mg, 0.0027 mmol) in 100 µl of anhydrous methylene chloride at room temperature was added a solution of 1,3-dicyclohexylcarbodiimide (8.3 mg, 0.040 mmol) in 35 µl of methylene chloride. After stirring for 60 min, another 18 mg of N,N'-di-*t*-Boc-(L)-histidine benzene complex (0.040 mmol) and 8.3 mg of 1,3-dicyclohexylcarbodiimide ( 0.040 mmol) was added. The cloudy white reaction mixture was stirred another 4 h, diluted with ethyl acetate-hexanes (3:1, 0.5 ml), stirred for 10 min, and filtered through a plug of celite, washing with ethyl acetate:hexanes (3:1). The filtrate and washings were concentrated *in vacuo* to a colorless oil. Chromatography (13 g of flash silica gel, 3:1/ethyl acetate-hexanes) afforded 17 mg (61%) of the N,N'-di-t-Boc compound (**14'**) as a white foam. To a solution of 14 (17 mg, 0.016 mmol) in 160 µl of methylene chloride at room temperature was added a 4.0 M solution of hydrogen chloride in 1,4-dioxane (24 µl, 0.098 mmol). After stirring for 5 h, the cloudy white reaction mixture was concentrated *in vacuo* to provide 15.7 mg (100%, corrected for 4 wt% dioxane) of the title compound as a white foam: 500 MHz ¹H NMR (MeOH-d₄) δ 8.91 (s, 1H), 7.77 (d, 1H, J = 9.5 Hz), 7.51-7.37 (m, 6H), 7.31 (d, 1H, J = 2.1 Hz), 7.20 (dd, 1H, J = 8.4, 2.1 Hz), 6.66-6.58 (m, 1H), 5.99 (d, 1H, J = 15 Hz), 5.57 (d, 1H, J = 9.6 Hz), 5.20 (d, 1H, J = 9.6 Hz), 5.15 (dd, 1H, J = 10, 3.0 Hz), 4.58 (t, 1H, J = 11 Hz), 4.53 (dd, 1H, 11, 3.8 Hz), 4.14 (t, 1H, J = 6.4 Hz), 3.87 (s, 3H), 3.55-3.47 (m, 1H), 3.27-3.12 (m, 3H), 2.81-2.68 (m, 3H), 2.40-2.31 (m, 1H), 1.98-1.81 (m, 3H), 1.25 (s, 3H), 1.21 (s, 3H),1.10-1.04 (m, 6H), 1.04 (d, 3H, J = 6.1Hz).

**Preparation of Cryptophycin 55 N-t-Boc-(L)-prolinate (16') (LSN 382926).** To a solution of 1' (19 mg, 0.027 mmol), N-*t*-Boc-(*L*)-proline (8.7 mg, 0.040 mmol), and 4-dimethylamino pyridine (0.3 mg, 0.0027 mmol) in 100 µl of anhydrous methylene chloride at room temperature was added a solution of 1,3-dicyclohexylcarbodiimide (8.3 mg, 0.040 mmol) in 35 µl of methylene chloride. After stirring for 45 min, the cloudy white reaction mixture was diluted with ethyl acetate-hexanes (3:1, 0.5 ml), stirred for 10 min, and filtered through a plug of celite, washing with ethyl acetate:hexanes (3:1). The filtrate and washings were concentrated *in vacuo* to a colorless oil. Chromatography (15 g of flash silica gel, 3:1/ethyl acetate-hexanes) afforded 11 mg (46%) of the title compound as a white foam: 500 MHz ¹H NMR (CDCl₃) δ 7.39-7.30 (m, 5H), 7.24 (d, 1H, J = 2.0 Hz), 7.21-7.18 (m, 1H), 7.09 (dd, 1H, J = 8.4, 2.0 Hz), 6.87 (d, 1H, J = 8.4 Hz), 6.80-6.68 (m, 1H), 5.77 (d, 1H, J = 15 Hz), 5.61 (br s, 1H), 5.56 (t, 1H, J = 9.5 Hz), 5.04-4.68 (m, 4H), 4.19-4.15 (m, 1H), 3.90 (s, 3H), 3.41-3.34 (m, 2H), 3.27-3.02 (m, 4H), 2.64-2.50 (m, 2H), 2.35-2.25 (m, 1H), 2.10-2.00 (m, 1H), 1.90-1.72 (m, 3H), 1.70-1.50 (m, 3H), 1.44 (s, 9H), 1.28 (s, 3H), 1.20 (s, 3H), 1.08-0.90 (m, 9H) .

**Preparation of Cryptophycin 55 (L)-prolinate hydrochloride salt (17') (LSN 382927).** To a solution of 16' (11 mg, 0.012 mmol) in 122 µl of methylene chloride at room temperature was added a 4.0 M solution of hydrogen chloride in 1,4-dioxane (15 µl, 0.061 mmol). After stirring for 5 h, the clear, colorless reaction mixture was concentrated *in vacuo* to provide 10 mg (100%) of the title compound as a white solid: 500 MHz ¹H NMR (MeOH-d₄) δ 7.77 (dd, 1H, J = 9.4, 2.3 Hz), 7.48-7.42 (m, 2H), 7.39-7.35 (m, 3H), 7.31 (d, 1H, J = 2.2 Hz), 7.20 (dd, 1H, J = 8.4, 2.2 Hz), 7.01 (d, 1H, J = 8.4 Hz), 6.68 (ddd, 1H, J = 15, 13, 3.8 Hz), 5.98 (dd, 1H, J = 15, 1.4 Hz), 5.52 (d, 1H, J = 9.8 Hz), 5.18 (d, 1H, J = 9.8 Hz), 5.18-5.14 (m, 1H), 4.78 (t, 1H, J = 10 Hz), 4.52 (dd, 1H, J = 11, 3.9 Hz), 3.99 (t, 1H, J = 7.3 Hz), 3.87 (s, 3H), 3.48 (dd, 1H, J = 14, 9.7 Hz), 3.28-3.12 (m, 4H), 2.82-2.73 (m, 3H), 2.42-2.34 (m, 2H), 2.09-1.79 (m, 6H), 1.24 (s, 3H), 1.88 (s, 3H), 1.16 (d, 3H, J = 7.0 Hz), 1.07 (d, 3H, J = 6.3 Hz), 1.04 (d, 3H, J = 6.2 Hz).

**Preparation of Cryptophycin 55 N-t-Boc-glycinate (18') (LSN 379403).** To a solution of 1' ( 118 mg, 0.167 mmol), N-*t-*Boc-glycine (44 mg, 0.251 mmol), and 4-dimethylamino pyridine (2.0 mg, 0.0167 mmol) in 490 µl of anhydrous methylene chloride at room temperature was added a solution of 1,3-dicyclohexylcarbodiimide (52 mg, 0.251 mmol) in 67 µl of methylene chloride. After stirring for 50 min, the cloudy white reaction mixture was diluted with ethyl acetate-hexanes (3:1, 1 ml), stirred for 10 min, and filtered through a plug of celite, washing with ethyl acetate-hexanes (3:1). The filtrate and washings were concentrated *in vacuo* to a colorless oil. Chromatography (19 g of flash silica gel, 3:1/ethyl acetate-hexanes) afforded 138 mg (96%) of the title compound as a white foam: 500 MHz ¹H NMR (CDCl₃) δ 7.34 (s, 5H), 7.24 (d, 1H, J = 2.0 Hz), 7.23-7.19 (m, 1H), 7.10 (dd, 1H, J = 8.4, 2.0 Hz), 6.88 (d, 1H, J = 8.4 Hz), 6.79-6.70 (m, 1H), 5.77 (d, 1H, J = 13 Hz), 5.50 (d, 1H, J = 8.0 Hz), 5.47 (d, 1H, J = 9.8 Hz), 4.97 (dd, 1H, J = 11, 2.7 Hz), 4.89 (t, 1H, J = 10 Hz), 4.83 (d, 1H, J = 9.8 Hz), 4.79-4.72 (m, 1H), 4.68 (br s, 1H), 3.91 (s, 3H), 3.66 (dd, 1H, J = 18, 5.3 Hz), 3.42-3.35 (m, 2H), 3.21 (dd, 1H, J = 13, 4.0 Hz), 3.17 (dd, 1H, J = 15, 5.1 Hz), 3.08 (dd, 1H, J = 15, 7.6 Hz), 2.66-2.57 (m, 2H), 2.47-2.38 (m, 1H), 1.95 (ddd. 1H, J = 14, 12, 9.7 Hz), 1.85-1.77 (m, 1H), 1.75-1.67 (m, 1H), 1.43 (s, 9H), 1.27 (s, 3H), 1.20 (s, 3H), 1.08 (d, 3H, J = 7.0 Hz), 1.03 (d, 3H, J = 6.7 Hz), 0.98 (d, 3H, J = 6.5 Hz).

**Preparation of Cryptophycin 55 glycinate hydrochloride salt (19') (LSN 368422).** To a solution of 18' (122 mg, 0.141 mmol) in 471 µl of methylene chloride at room temperature was added a 4.0 M solution of hydrogen chloride in 1,4-dioxane (178 µl, 0.707 mmol). After stirring for 1h 20 min, the clear, colorless reaction mixture was concentrated in *vacuo* to provide 120 mg (99%, corrected for 7 wt% dioxane) of the title compound as a white foam: 500 MHz ¹H NMR (MeOH-d₄) δ 7.81 (dd, 1H, J = 8.5, 2.2 Hz), 7.46-7.41 (m, 2H), 7.40-7.36 (m, 3H), 7.31 (d, 1H, J = 2.1 Hz), 7.20 (dd, 1H, J = 8.4, 2.1 Hz), 7.01 (d, 1H, J = 8.4 Hz), 6.70 (ddd, 1H, J = 15, 13, 3.7 Hz), 5.97 (dd, 1H, J = 15, 1.7 Hz), 5.55 (d, 1H, J = 9.9 Hz), 5.18 (d, 1H, J = 9.9 Hz), 5. 14 (dd, 1H, J = 10, 2.8 Hz), 4.84 (t, 1H, J = 10 Hz), 4.52 (dd, 1H, J = 11, 3.7 Hz), 3.87 (s, 3H), 3.78 (d, 1H, J = 18 Hz), 3.50 (dd, 1H, J = 13, 9.8 Hz), 3.23 (d, 1H, J = 18 Hz), 3.20 (dd, 1H, J = 14, 3.6 Hz), 3.13 (dd, 1H, J = 13, 2.4 Hz), 2.80-2.69 (m, 3H), 2.41-2.32 (m, 1H), 1.99-1.92 (m, 1H), 1.91-1.81 (m, 2H), 1.25 (s, 3H), 1.20 (s, 3H), 1.12 (d, 3H, J = 7.0 Hz), 1.06 (d, 3H, J = 6.2 Hz), 1.04 (d, 3H, 6.2 Hz).

**Preparation of Cryptophycin 55 N-t-Boc-β-alaninate (20') (LSN 379404).** To a solution of 1' (102 mg, 0.145 mmol), N-*t*-Boc-β-alanine (41 mg, 0.217 mmol), and 4-dimethylamino pyridine (18 mg, 0.145 mmol) in 400 µl of anhydrous methylene chloride at room temperature was added a solution of 1,3-dicyclohexylcarbodiimide (45 mg, 0.217 mmol) in 82 µl of methylene chloride. After stirring for 3.5 h, the cloudy white reaction mixture was diluted with ethyl acetate-hexanes (3:1, 1 ml), stirred for 10 min, and filtered through a plug of celite, washing with ethylacetate-hexanes (3:1). The filtrate and washings were concentrated in *vacuo* to a colorless oil. Chromatography (21 g of flash silica gel, 2:1 then 4:1/ethyl acetate-hexanes) afforded 121 mg (95%) of the title compound as a white foam: 500 MHz ¹H NMR (CDCl₃) δ-7.44-7.39 (m, 2H), 7.37-7.31 (m, 3H), 7.32 (d, 1H, J = 2.1 Hz), 7.20 (dd, 1H, J = 8.4, 2.1 Hz), 7.01 (d, 1H, J = 8.4 Hz), 6.72 (ddd, 1H, J = 15, 13, 3.7 Hz), 5.96 (dd, 1H, J = 15, 1.6 Hz), 5.51 (d, 1H, J = 9.8 Hz), 5.11-5.06 (m, 1H), 5.08 (d, 1H, J = 9.8 Hz), 4.90-4.83 (m, 1H), 4.50 (dd, 1H, J = 11, 3.6 Hz), 3.86 (s, 3H), 3.52-3.46 (m, 1H), 3.20 (dd, 1H, J = 14, 3.6 Hz), 3.13 (br d, 1H, J = 14 Hz), 3.05-2.92 (m, 2H), 2.79-2.63 (m, 3H), 2.45-2.37 (m, 1H), 2.24 (dt, 1H, J = 16, 7.0 Hz), 2.08-1.99 (m, 1H), 1.96-1.79 (m, 3H), 1.43 (s, 9H), 1.25 (s, 3H), 1.21 (s, 3H), 1.12 (d, 3H, J = 7.0 Hz), 1.06 (d, 3H, J = 6.2 Hz), 1.02 (d, 3H, J = 6.1 Hz).

**Preparation of Cryptophycin 55 β-alaninate hydrochloride salt (21') (LSN 377718).** To a solution of 20' (119 mg, 0.136 mmol) in 452 µl of methylene chloride at room temperature was added a 4.0 M solution of hydrogen chloride in 1,4-dioxane (170 µl, 0.679 mmol). After stirring for 2 h 15 min, the cloudy, white reaction mixture was concentrated *in vacuo* to provide 110 mg (96%, corrected for 4 wt% dioxane) of the title compound as a white foam: 500 MHz ¹H NMR (MeOH-d₄) δ 7.80 (dd, 1H, J = 9.7, 2.3 Hz), 7.45-7.40 (m, 2H), 7.39-7.32 (m, 3H), 7.20 (dd, 1H, J = 8.4, 2.1 Hz), 7.01 (d, 1H, J = 8.4 Hz), 6.68 (ddd, 1H, J = 15, 13, 3.8 Hz), 5.98 (dd, 1H, J = 15, 1.7 Hz), 5.48 (dd, 1H, J = 9.4 1.0 Hz), 5.15-5.11 (m, 1H), 5.13 (d, 1H, J = 9.4 Hz), 4.82 (t, 1H, J = 10 Hz), 4.51 (dd, 1H, J = 11, 3.7 Hz), 3.90 (s, 3H), 3.50 (dd, 1H, J = 14, 9.8 Hz), 3.20 (dd, 1H, J = 14, 3.7 Hz), 3.14 (dd, 1H, J = 14, 2.4 Hz), 2.85 (t, 2H, J = 7.0 Hz), 2.80-2.65 (m, 5H), 2.54 (dt, 1H, J = 17, 7.4 Hz), 2.42-2.33 (m, 1H), 2.22 (dt, 1H, J = 17, 6.7 Hz), 1.90-1.81 (m, 3H), 1.25 (s, 3H), 1.20 (s, 3H), 1.13 (d, 3H, J = 7.1 Hz), 1.08 (d, 3H, J = 6.3 Hz), 1.04 (d, 3H, J = 6.2 Hz.

**Preparation of Cryptophycin 55 N-t-Boc-γ-aminobutyrate (22') (LSN 379401).** To a solution of **1'** (48 mg, 0.068 mmol), N-*t*-Boc-4-aminobutyric acid (18 mg, 0.088 mmol), and 4-dimethylamino pyridine (8 mg, 0.068 mmol.) in 150 µl of anhydrous methylene chloride at room temperature was added a solution of 1,3-dicyclohexylcarbodiimide (18 mg, 0.088 mmol) in 50 µl of methylene chloride. After stirring for 45 min, the cloudy white reaction mixture was diluted with ethyl acetate-hexanes (3:1, 0.5 ml), stirred for 5 min, and filtered through a plug of celite, washing with ethyl acetate-hexanes (3:1). The filtrate and washings were concentrated *in vacuo* to a colorless oil. Chromatography (15 g of flash silica gel, 3:1/ethyl acetate-hexanes) afforded 55 mg (90%) of the title compound as a white foam: 500 MHz ¹H NMR (CDCl₃) δ 7.38-7.32 (m, 5H), 7.24 (d, 1H, J = 1.9 Hz), 7.22-7.19 (m, 1H), 7.10 (dd, 1H, J = 8.4, 1.9 Hz), 6.88 (d, 1H, J = 8.4 Hz), 6.75 (ddd, 1H, J = 15, 13, 3.9 Hz), 5.78 (d, 1H, J = 15 Hz), 5.60-5.55 (m, 1H), 5.49 (dd, 1H, J = 9.8, 1.4 Hz), 4.96 (dd, 1H, J = 11, 3.0 Hz), 4.89 (t, 1H, J = 9.2 Hz), 4.81 (d, 1H, J = 9.8 Hz), 4.78-4.70 (m, 1H), 4.44 (br s, 1H), 3.91 (s, 3H), 3.40 (dd, 1H, J = 14, 8.1 Hz), 3.22 (dd, 1H, J = 14, 4.1 Hz), 3.22-3.15 (m, 1H), 3.08 (dd, 1H, J = 14, 7.8 Hz), 2.89-2.82 (m, 2H), 2.67-2.56 (m, 2H), 2.47-2.38 (m, 1H), 2.11-2.04 (m, 1H), 2.00-1.77 (m, 3H), 1.75-1.67 (m, 1H), 1.45 (s, 9H), 1.50-1.40 (m, 2H), 1.27 (s, 3H), 1.20 (s, 3H), 1.09 (d, 3H, J = 7.0 Hz), 1.04 (d, 3H, J = 6.6 Hz), 0.98 (d, 3H, J = 6.6 Hz).

**Preparation of Cryptophycin 55 γ-aminobutyrate hydrochloride salt (25') (LSN 368513).** To a solution of 22' (53 mg, 0.059 mmol) in 297 µl of methylene chloride at room temperature was added a 1.0 M solution of hydrogen chloride in diethyl ether (297 µl, 0.297 mmol). The starting material precipitated as a white paste which was redissolved with an additional 150 µl of methylene chloride. After stirring for 4 h, another 59 µl (0.059 mmol) of hydrogen chloride solution was added. Stirring was continued for another 14 h and the reaction mixture was concentrated in *vacuo* to provide 49 mg (100%) of the title compound as a white foam: 500 MHz ¹H NMR (DMSO-d₆) δ 8.49 (d, 1H, J = 8.0 Hz), 7.72 (br s, 3H), 7.44-7.33 (m, 5H), 7.32 (d, 1H, J = 1.9 Hz), 7.29 (dd, 1H, J = 9.4, 2.6 Hz), 7.20 (dd, 1H, J = 8.5, 1.9 Hz), 7.06 (d, 1H, J = 8.5 Hz), 6.48 (ddd, 1H, J = 15, 13, 3.9 Hz), 5.87 (d, 1H, J = 15 Hz), 5.37 (d, 1H, J = 9.7 Hz), 5.33 (d, 1H, J = 9.7 Hz), 5.04-5.01 (m, 1H), 4.73 (t, 1H, J = 11 Hz), 4.25 (ddd, 1H, J = 12, 9.8, 3.5 Hz), 3.82 (s, 3H), 3.40-3.30 (m, 1H), 3.07-3.01 (m, 2H), 2.72 (dd, 1H, J = 14, 12 Hz), 2.65-2.47 (m, 4H), 2.38-2.28 (m, 1H), 2.21 (dt, 1H, J = 17, 7.5 Hz), 1.97 (dt, 1H, J = 17, 7.5 Hz), 1.80-1.70 (m, 3H), 1.54-1.46 (m, 2H), 1.17 (s, 3H), 1.03 (s, 3H), 1.01 (d, 3H, J = 7.0 Hz), 0.99 (d, 3H, J = 5.8 Hz), 0.95 (d, 3H, J = 5.8 Hz).

**Preparation of Cryptophycin 55 N-t-Boc-(L)-alaninate (26') (LSN 379405).** To a solution of 1' (103 mg, 0.146 mmol), N-*t*-Boc-(*L*)-alanine (41 mg, 0.219 mmol), and 4-dimethylamino pyridine (18 mg, 0.146 mmol) in 400 µl of anhydrous methylene chloride at room temperature was added a solution of 1,3-dicyclohexylcarbodiimide (45 mg, 0.219 mmol) in 87 µl of methylene chloride. After stirring for 5 h 50 min, the cloudy white reaction mixture was treated with another 5.5 mg (0.029 mmol) of N-*t*-Boc-(*L*)-alanine, 6.0 mg (0.029 mmol) of 1,3-dicyclohexylcarbodiimide, and a few crystals of 4-dimethylamino pyridine. After stirring an additional 1 h, the reaction was diluted with ethyl acetate-hexanes (3:1, 1 ml), stirred for 10 min, and filtered through a plug of celite, washing with ethyl acetate:hexanes (3:1). The filtrate and washings were concentrated *in vacuo* to a colorless oil. Chromatography (22 g of flash silica gel, 1.5:1 then 2:1 then 4:1/ethyl acetate-hexanes) afforded 96 mg (75%) of the title compound as a white foam: 500 MHz ¹H NMR (CDCl₃) δ 7.35-7.30 (m, 5H), 7.26-7.21 (m, 2H), 7.10 (dd, 1H, J = 8.4, 1.9 Hz), 6.88 (d, 1H, J = 8.4 Hz), 6.76 (ddd, 1H, J = 15, 13, 4.2 Hz), 5.77 (d, 1H, J = 15 Hz), 5.52 (d, 1H, 7.6 Hz), 5.44 (d, 1H, J = 9.7 Hz), 4.98 (dd, 1H, J = 11, 2.5 Hz), 4.85-4.81 (m, 2H), 4.75 (q, 1H, J = 6.8 Hz), 4.56 (d, 1H, J = 7.8 Hz), 4.01-3. 96 (m, 1H), 3.91 (s, 3H), 3.41 (dd, 1H, J = 13, 8.3 Hz), 3.20 (dd, 1H, J = 13, 4.0 Hz), 3.16 (dd, 1H, J = 15, 5.9 Hz), 3.08 (dd, 1H, J = 15, 7.6 Hz), 2. 65-2. 57 (m, 2H), 2.40-2.31 (m, 1H), 2.02-1.96 (m, 1H), 1.87-1.73 (m, 2H), 1.43 (s, 9H), 1.27 (s, 3H), 1.20 (s, 3H), 1.11-1.02 (m, 9H), 0.99 (d, 3H, J = 6.3 Hz).

**Preparation of Cryptophycin 55 (*L*)-alaninate hydrochloride salt (28') (LSN 377719).** To a solution of **26'** (95 mg, 0.108 mmol) in 361 µl of methylene chloride at room temperature was added a 4.0 M solution of hydrogen chloride in 1,4-dioxane (135 µl, 0.542 mmol) . After stirring for 2.5 h, the cloudy, white reaction mixture was concentrated *in vacuo* to provide 90 mg (96%, corrected for 6 wt% dioxane) of the title compound as a white foam: 500 MHz ¹H NMR (MeOH-d₄) δ 8.54 (d, 1H, 7.6 Hz), 7.81 (br d, 1H, J = 9.7 Hz), 7.46-7.44 (m, 2H), 7.39-7.37 (m, 3H), 7.32 (d, 1H, J = 2.0 Hz), 7.20 (dd, 1H, J = 8.4, 2.0 Hz), 7.01 (d, 1H, J = 2.0 Hz), 6.69 (ddd, 1H, J = 15, 11, 3.7 Hz), 5.99 (d, 1H, 15 Hz), 5.55 (d, 1H, J = 9.8 Hz), 5.20 (d, 1H, J = 9.8 Hz), 5.15 (dd, 1H, J = 11, 2.7 Hz), 4.78 (t, 1H, J = 11 Hz), 4.53-4.50 (m, 1H), 3.87 (s, 3H), 3.65 (q, 1H, J = 7.3 Hz), 3.50 (dd, 1H, J = 13, 9.8 Hz), 3.20 (dd, 1H, J = 14, 3.5 Hz), 3.14 (br d, 1H, J = 13 Hz), 2.81-2.71 (m, 3H), 2.41-2.34 (m, 1H), 1.98-1.93 (m, 1H), 1.88-1.82 (m, 2H), 1.41 (d, 3H, J = 7.3 Hz), 1.25 (s, 3H), 1.20 (s, 3H), 1.13 (d, 3H, J = 7.0 Hz), 1.06 (d, 3H, J = 6.2 Hz), 1.04 (d, 3H, 6.0 Hz).

**Preparation of Cryptophycin 55 N-*t*-Boc-(*D*)-alaninate (29') (LSN 382426).** To a solution of 1' (25 mg, 0.035 mmol), N-*t*-Boc-(*D*)-alanine (10 mg, 0.053 mmol), and 4-dimethylamino pyridine (0.4 mg, 0.0035 mmol) in 130 µl of anhydrous methylene chloride at room temperature was added a solution of 1,3-dicyclohexylcarbodiimide (11 mg, 0.053 mmol) in 47 µl of methylene chloride. After stirring for 5.5 h, the cloudy white reaction mixture was diluted with ethyl acetate-hexanes (3:1, 0.5 ml), stirred for 10 min, and filtered through a plug of celite, washing with ethyl acetate:hexanes (3:1). The filtrate and washings were concentrated in *vacuo* to a colorless oil. Chromatography (15 g of flash silica gel, 2:1/ethyl acetate-hexanes) afforded 26 mg (83%) of the title compound as a white foam: 500 MHz ¹H NMR (CDCl₃) δ 7.49-7.29 (m, 5H), 7.24 (d, 1H, J = 2.0 Hz), 7.22-7.18 (m, 1H), 7.09 (dd, 1H, 8.4, 2.0 Hz), 6.87 (d, 1H, J = 8.4 Hz), 6.76 (ddd, 1H, J = 15, 13, 4.4, Hz), 5.77 (d, 1H, 15 Hz), 5.56 (d, 1H, J = 9.9 Hz), 5.48 (d, 1H, J = 7.7 Hz), 5.01 (dd, 1H, J = 10, 2.6 Hz), 4.91 (t, 1H, J = 9.4 Hz), 4.84 (d, 1H, J = 9.9 Hz), 4.81-4.73 (m, 2H), 3.99-3.93 (m, 1H), 3.91 (s, 3H), 3.39 (dd, 1H, J = 13, 8.0 Hz), 3.22 (dd, 1H, J = 13, 3. 6 Hz), 3.17 (dd, 1H, J = 14, 5.0 Hz), 3.08 (dd, 1H, J = 14 Hz), 2.68-2.58 (m, 2H), 2.42-2.35 (m, 1H), 2.04-1.94 (m, 1H), 1 .87-1.50 (m, 2H), 1.42 (s, 9H), 1.27 (s, 3H), 1.20 (s, 3H), 1.09 (d, 3H, J = 7.1 Hz), 1.04 (d, 3H, J = 6.4 Hz), 0.99 (d, 3H, J = 6.3 Hz), 0.65 (d, 3H, J = 6.8 Hz).

**Preparation of Cryptophycin 55 (D)-alaninate hydrochloride salt (30') (LSN 382425).** To a solution of **29'** (24 mg, 0.027 mmol) in 274 µl of methylene chloride at room temperature was added a 4.0 M solution of hydrogen chloride in 1,4-dioxane (34 µl, 0.137 mmol). After stirring for 3.5 h, the clear, colorless reaction mixture was concentrated in *vacuo* to provide 24 mg (100%, corrected for 8 wt% dioxane) of the title compound as a white foam: 500 MHz ¹H NMR (MeOH-d₄) δ 7.79 (d, 1H, J = 9.5 Hz), 7.47-7.40 (m, 2H), 7.40-7.36 (m, 3H), 7.31 (d, 1H, J = 2.0 Hz), 7.20 (dd, 1H, J = 8.4, 2.0 Hz), 7.01 (d, 1H, J = 8.4 Hz), 6.71 (ddd, 1H, J = 15, 13, 3.7 Hz), 5.98 (dd, 1H, J = 15, 1.6 Hz), 5.65 (d, 1H, J = 10 Hz), 5.20 (d, 1H, J = 10 Hz), 5.17 (dd, 1H, J = 11, 2.5 Hz), 4.88-4.78 (m, 1H), 4.53 (dd, 1H, J = 11, 3.7 Hz), 3.95 (q, 1H, J = 7.2 Hz), 3.87 (s, 3H), 3.51 (dd, 1H, J = 13, 9.8 Hz), 3.20 (dd, 1H, J = 14, 3. 6 Hz), 3.14 (dd, 1H, J = 13, 2.3 Hz), 2.81-2.74 (m, 3H), 2.41-2.34 (m, 1H), 2.07-1.99 (m, 1H), 1.96-1.84 (m 2H), 1.26 (s, 3H), 1.21 (s, 3H), 1.15 (d, 3H, J = 7.1 Hz), 1.09 (d, 3H, J = 6.0 Hz), 1.05 (d, 3H, J = 6.0 Hz), 0.80 (d, 3H, J = 7.4 Hz).

**Preparation of Cryptophycin 55 Nα-Nε-di-*t*-Boc-(*L*)-lysinate (31') (LSN 379406).** To a solution of **1'** (105 mg, 0.149 mmol), Nα-Nε-di-*t*-Boc-(*L*)-lysine (67 mg, 0.193 mmol), and 4-dimethylamino pyridine (18 mg, 0.149 mmol) in 400 µl of anhydrous methylene chloride at room temperature was added a solution of 1,3-dicyclohexylcarbodiimide (40 mg, 0.193 mmol) in 96 µl of methylene chloride. After stirring for 4 h, the cloudy white reaction mixture was treated with another 10 mg (0.030 mmol) of Nα-Nε-di-*t*-Boc-*(L)*-lysine and 6.1 mg (0.030 mmol) of 1,3-dicyclohexylcarbodiimide as a soln in 100 µl of methylene chloride. After stirring an additional 1 h, the cloudy white reaction mixture was diluted with ethyl acetate-hexanes (3:1, 1 ml), stirred for 10 min, and filtered through a plug of celite, washing with ethyl acetate:hexanes (3:1). The filtrate and washings were concentrated *in vacuo* to a white foam which was resubmitted to the above conditions using 34 mg (0.097 mmol) of Nα-Nε-di-t-Boc-(L)-lysine, 20 mg (0.097 mmol) of 1,3-dicyclohexylcarbodiimide, and 9.1 mg (0.075 mmol) of 4-dimethylamino pyridine. After stirring for 1.5 h, the reaction was processed as above to provide a crude white foam. Chromatography (21 g of flash silica gel, 1:1 then 4:1/ethyl acetate-hexanes) afforded 112 mg (73%) of the title compound as a white foam: 500 MHz ¹H NMR (MeOH -d₄) δ 7.42-7.37 (m, 2H), 7.36-7.29 (m, 3H), 7.27 (br s, 1H), 7.16 (br d, 1H, J = 8.5 Hz), 6.97 (d, 1H, J = 8.5 Hz), 6.72 (ddd, 1H, J = 15, 13, 3.5 Hz), 5.92 (d, 1H, J = 15 Hz), 5.50 (d, 1H, J = 11 Hz), 5.11-5.04 (m, 2H), 4.84 (t, 1H, J = 10 Hz), 4.48 (dd, 1H, J = 11, 3.6 Hz), 3. 84 (s, 3H), 3.75 (br s, 1H), 3.50-3.43 (m, 1H), 3.17 (dd, 1H, J = 14, 3.6 Hz), 3.11 (d, 1H, J = 14 Hz), 2.97-2.91 (m, 2H), 2 . 76-2 . 58 (m, 3H), 2.36-2.27 (m, 1H), 1.98-1.80 (m, 3H), 1.48-1.38 (m, 2H), 1.43 (s, 9H), 1.40 (s, 9H), 1.35-1.25 (m, 2H), 1.23 (s, 3H), 1.20 (s, 3H), 1.15-1.09 (m, 2H), 1.07 (d, 3H, J = 6.8 Hz), 1.06 (d, 3H, J = 6.0 Hz), 1.01 (d, 3H, J = 6.1 Hz).

**Preparation of Cryptophycin 55 (L)-lysinate di-hydrochloride salt (32') (LSN 377562).** To a solution of **31'** (107 mg, 0.103 mmol) in 345 µl of methylene chloride at room temperature was added a 4.0 M solution of hydrogen chloride in 1,4-dioxane (155 µl, 0.621 mmol). After stirring for 4 h, the cloudy white reaction mixture was filtered. The collected white solid was washed with methylene chloride (2 x 1 ml) and dried *in vacuo* at room temperature to provide 87 mg (93%) of the title compound: 500 MHz ¹H NMR (MeOH-d₄) δ 8.61 (d, 1H, J = 7.7 Hz), 7.81 (d, 1H, J = 7.7 Hz), 7.47-7.44 (m, 2H), 7.40-7.38 (m, 3H), 7.31 (d, 1H, J = 2.2 Hz), 7.20 (dd, 1H, J = 8.4, 2.2 Hz), 7.00 (d, 1H, J = 8.4 Hz), 6.63 (ddd, 1H, J = 15, 13, 4.0 Hz), 6.00 (dd, 1H, J = 15, 1.6 Hz), 5.55 (d, 1H, J = 9.8 Hz), 5.20 (d, 1H, J = 9.8 Hz), 5.15 (dd, 1H, J = 10, 2.9 Hz), 4.68 (t, 1H, J = 11 Hz), 9.55-9 . 99 (m, 1H), 3.87 (s, 3H), 3.79 (t, 1H, J = 5.6 Hz), 3.52 (dd, 1H, J = 14, 9.9 Hz), 3.20 (dd, 1H, J = 14, 3.6 Hz), 3.13 (dd, 1H, J = 13, 2.4 Hz), 3.06-2.98 (m, 1H), 2.94-2.87 (m, 1H), 2.85-2.74 (m, 3H), 2.45-2.38 (m, 1H), 1.98-1.76 (m, 5H), 1.71-1.64 (m, 2H), 1.39-1.30 (m, 2H), 1.25 (s, 3H), 1.18 (d, 3H, J = 8.2 Hz), 1.17 (s, 3H), 1.08 (d, 3H, *J* = 6.2 Hz), 1.05 (d, 3H, J = 6.1 Hz).

**Preparation of Cryptophycin 55 Nα-Nε-di-*t*-Boc-(*D*)-lysinate (33') (LSN 382504).** To a solution of 1' (24 mg, 0.035 mmol), Nα-Nε-di-*t*-Boc-*(D)*-lysine 247 mg, 0.069 mmol), and 4-dimethylamino pyridine (0.4 mg, 0.0035 mmol) in 140 µl of anhydrous methylene chloride at room temperature was added a solution of 1,3-dicyclohexylcarbodiimide (14 mg, 0.069 mmol) in 30 µl of methylene chloride. After stirring for 70 min, the cloudy white reaction mixture was diluted with ethyl acetate-hexanes (3:1, 0.5 ml), stirred for 10 min, and filtered through a plug of celite, washing with ethyl acetate:hexanes (3:1). The filtrate and washings were concentrated *in vacuo* to an off-white oil. Chromatography (15 g of flash silica gel, 2:1/ethyl acetate-hexanes) afforded 30 mg (87%) of the title compound as a white foam: 500 MHz ¹H NMR (MeOH -d₄) δ 7.46-7.40 (m, 2H), 7.39-7.31 (m, 3H), 7.31 (d, 1H, J = 1.8 Hz), 7.20 (dd, 1H, J = 8.4, 1.8 Hz), 7.01 (d, 1H, J = 8.4 Hz), 6.75 (ddd, 1H, J = 16, 11, 3.7 Hz), 5.93 (d, 1H, J = 16 Hz), 5.59 (d, 1H, J = 10 Hz), 5.13-5.09 (m, 2H), 4.92 (t, 1H, J = 9.8 Hz), 4.52 (dd, 1H, J = 11, 3.6 Hz), 3.87 (s, 3H), 3.79-3.75 (m, 1H), 3.48 (d, 1H, J = 13 Hz), 3.21 (dd, 1H, J = 14, 3.7 Hz), 3.12 (d, 1H, J = 13 Hz), 2.97-2.91 (m, 2H), 2.80-2.62 (m, 3H), 2.41-2.32 (m, 1H), 1.98-1.82 (m, 3H), 1.48-1.38 (m, 2H), 1.46 (s, 9H), 1.42 (s, 9H), 1.35-1.20 (m, 2H), 1.25 (s, 3H), 1.21 (s, 3H), 1.15-0.09 (m, 2H), 1.14 (d, 3H, J = 7.0 Hz), 1.07 (d, 3H, J = 6.0 Hz), 1.03 (d, 3H, J = 6.0 Hz) .

**Preparation of Cryptophycin 55 (*D*)-lysinate di-hydrochloride salt (34') (LSN 377503).** To a solution of 33' (28 mg, 0.027 mmol) in 181 µl of methylene chloride at room temperature was added a 4.0 M solution of hydrogen chloride in 1,4-dioxane (41 µl, 0.162 mmol). After stirring for 5.5 h, the cloudy white reaction mixture was concentrated *in vacuo* to provide 25 mg (96%, corrected for 4.5 wt% dioxane) of the title compound as a white solid: 500 MHz ¹H NMR (MeOH-d₄) δ 8.47 (d, 1H, J = 7.6 Hz), 7.76 (dd, 1H, J = 9.6, 2.1 Hz), 7.47-7.36 (m, 5H), 7.28 (d, 1H, J = 2.1 Hz), 7.17 (dd, 1H, J = 8.4, 2.1 Hz), 6.98 (d, 1H, J = 8.4 Hz), 6.68 (ddd, 1H, J = 15, 11, 3.7 Hz), 5.95 (dd, 1H, J = 15, 1.1 Hz), 5.67 (d, 1H, J = 10 Hz), 5.21 (d, 1H, J = 10 Hz), 5.14 (dd, 1H, J = 10, 2.5 Hz), 4.83-4.77 (m, 1H), 4.52-4.49 (m, 1H), 3.92 (t, 1H, J = 6.1 Hz), 3.84 (s, 3H), 3.47 (dd, 1H, J = 14, 9.8 Hz), 3.18 (dd, 1H, J = 14, 3.6 Hz), 3.12 (dd, 1H, J = 14, 2.2 Hz), 2.83 (t, 2H, J = 7.6 Hz), 2.78-2.70 (m, 3H), 2.38-2.30 (m, 1H), 2.02-1.82 (m, 3H), 1.52-1.43 (m, 2H), 1.23 (s, 3H), 1.22-1.00 (m, 4H), 1.18 (s, 3H), 1.13 (d, 3H, J = 8.2 Hz), 1.06 (d, 3H, J = 6.2 Hz), 1.02 (d, 3H, J = 6.1 Hz).

**Preparation of Cryptophycin 55 N-*t*-Boc-γ-*t*-butyl ester-(*L*)-glutamate (35') (LSN 382366).** To a solution of 1' (27 mg, 0.038 mmol), N-*t*-Boc-(*L*)-glutamic acid γ-*t*-butyl ester (17 mg, 0.057 mmol), and 4-dimethylamino pyridine (0.5 mg, 0.0038 mmol) in 150 µl of anhydrous methylene chloride at room temperature was added a solution of 1,3-dicyclohexylcarbodiimide (12 mg, 0.057 mmol) in 41 µl of methylene chloride. After stirring for 60 min, the cloudy white reaction mixture diluted with ethyl acetate-hexanes (2:1, 2 ml), stirred for 10 min, and filtered through a plug of celite, washing with ethyl acetate:hexanes (2:1). The filtrate and washings were concentrated in *vacuo* to an off-white oil. Chromatography (15 g of flash silica gel, 1:1 ethyl acetate-hexanes) afforded 28 mg (74%) of the title compound as a white foam: 500 MHz ¹H NMR (CDCl₃) δ 7.39-7.29 (m, 5H), 7 .24 (d, 1H, J = 1 . 9 Hz), 7.22-7.18 (m, 1H), 7.10 (dd, 1H, J = 8.5, 1.9 Hz), 6.87 (d, 1H, J = 8.5 Hz), 6.76 (ddd, 1H, J = 15, 13, 4.5 Hz), 5.78 (d, 1H, J = 15 Hz), 5.65 (d, 1H, J = 7.6 Hz), 5.43 (d, 1H, J = 9.7 Hz), 4.99 (dd, 1H, J = 10, 2.7 Hz), 4.90-4.80 (m, 1H), 4.83 (d, 1H, J = 9.7 Hz), 4.69-4.61 (m, 1H), 4.55 (d, 1H, J = 8.4 Hz), 4.01-3.96 (m, 1H), 3.90 (s, 3H), 3.40 (dd, 1H, J = 13, 8.0 Hz), 3.24 (dd, 1H, J = 13, 4.1 Hz), 3.18 (dd, 1H, J = 15, 5.0 Hz), 3.11 (dd, 1H, J = 15, 7.9 Hz), 2.65-2.57 (m, 2H), 2.39-2.30 (m, 1H), 2.14-2.09 (m, 2H), 2.00-1.92 (m, 1H), 1.87-1.73 (m, 3H), 1.60-1.50 (m, 1H), 1.44 (s, 18H), 1.27 (s, 3H), 1.21 (s, 3H), 1.08 (d, 3H, J = 7.0 Hz), 1.05 (d, 3H, J = 6.4 Hz), 1.00 (d, 3H, J = 6.3 Hz) .

**Preparation of Cryptophycin 55 (L)-α-glutamate hydrochloride salt (36') (LSN 382367).** To a solution of 35' (23 mg, 0.023 mmol) in 232 µl of methylene chloride at room temperature was added a 4.0 M solution of hydrogen chloride in 1,4-dioxane (29 µl, 0.116 mmol). After stirring for 8.5 h, the clear, colorless reaction mixture was concentrated in *vacuo* to provide 20 mg (97%, corrected for 3 wt% dioxane) of the title compound as a white foam: 500 MHz ¹H NMR (MeOH-d₄) δ 7.79 (d, 1H, J = 7.6 Hz), 7.50-7.36 (m, 5H), 7.31 (d, 1H, J = 2.0 Hz), 7.20 (dd, 1H, J = 8.6, 2.0 Hz), 7.01 (d, 1H, J = 8.6 Hz), 6.75-6.65 (m, 1H), 5.98 (d, 1H, 15 Hz), 5.57 (d, 1H, J = 9.7 Hz), 5.20 (d, 1H, J = 9.7 Hz), 5.15 (dd, 1H, J = 11, 3.0 Hz), 4.90-4.80 (m, 1H), 4.52 (dd, 1H, J = 11, 3.7 Hz), 3.87 (s, 3H), 3.80-3.66 (m, 1H), 3.49 (dd, 1H, J = 13, 10 Hz), 3.21 (dd, 1H, J = 14, 3.6 Hz), 3.15 (dd, 1H, J = 14, 2.3 Hz), 2.82-2.72 (m, 3H), 2.41-2.30 (m, 3H), 2.05-1.81 (m, 5H), 1.25 (s, 3H), 1.20 (s, 3H), 1.15 (d, 3H, J = 7.1 Hz), 1.07 (d, 3H, J = 6.2 Hz), 1.05 (d, 3H, J = 6.1 Hz).

**Preparation of Cryptophycin 55 N-*t*-Boc-β-*t*-butyl ester-(*L*)-aspartate (37') (LSN 382501) and cryptophycin 55 N-*t*-Boc-β-t-butyl ester-(*D*)-aspartate (39') (LSN 387040).** To a solution of **1'** (176 mg, 0.249 mmol), N-t-Boc-(L)-aspartic acid β-*t*-butyl ester (144 mg, 0.499 mmol), and 4-dimethylamino pyridine (2.0 mg, 0.066 mmol) in 1.0 ml of anhydrous methylene chloride at room temperature was added a solution of 1,3-dicyclohexylcarbodiimide (103 mg, 0.499 mmol) in 200 µl of methylene chloride. After stirring for 45 min, the cloudy white reaction mixture was treated with 88 mg of celite and diluted with ethyl acetate-hexanes (3:1, 2 ml), stirred for 10 min, and filtered through a plug of celite, washing with ethyl acetate:hexanes (3:1). The filtrate and washings were concentrated *in vacuo* to an off-white oil. Chromatography (30 g of flash silica gel, 2:1 ethyl acetate-hexanes) afforded pure **37'** (lower Rf) along with mixed fractions. The mixed fractions were chromatographed (25 g of flash silica gel, 1:1 then 2:1 then 3:1 ethyl acetate-hexanes) affording more pure 37' along with pure **39'** (higher Rf). All fractions containing pure **37'** were combined to yield 149 mg (61%) as a white foam while all fractions containing pure **39'** were combined to yield 56 mg (23%) as a white foam. 500 MHz ¹H NMR (CDCl₃) data for **37'**: δ 7.39-7.30 (m, 5H), 7.24 (d, 1H, J = 2.0 Hz), 7.24-7.18 (m, 1H), 7.09 (dd, 1H, *J* = 8.4, 2.0 Hz), 6.88 (d, 1H, J = 8.4 Hz), 6.76 (ddd, 1H, J = 15, 13, 4.3 Hz), 5.75 (d, 1H, 15 Hz), 5.50 (d, 1H, J = 7.9 Hz), 5.46 (d, 1H, J = 9.2 Hz), 5.01-4.98 (m, 2H), 4.93 (t, 1H, J = 9.9 Hz), 4.85 (d, 1H, J = 9.2 Hz), 4.78-4.71 (m, 1H), 4.17-4.10 (m, 1H), 3.91 (s, 3H), 3.41 (dd, 1H, J = 13, 8.2 Hz), 3.22 (dd, 1H, *J* = 13, 3.9 Hz), 3.17 (dd, 1H, J = 14, 5.1 Hz), 3.09 (dd, 1H, J = 14, 7.7 Hz), 2.61-2.50 (m, 4H), 2.36-2.28 (m, 1H), 2.02-1.97 (m, 1H), 1.83-1.76 (m, 2H), 1.45 (s, 9H), 1.41 (s, 9H), 1.28 (s, 3H), 1.20 (s, 3H), 1.06-1.01 (m, 6H), 0.99 (d, 3H, J = 6.2 Hz). 500 MHz ¹H NMR (CDCl₃) data for **39'**: δ 7.39-7.30 (m, 5H), 7.24 (d, 1H, J = 2.0 Hz), 7.24-7.18 (m, 1H), 7.09 (dd, 1H, J = 8.4, 2.0 Hz), 6.88 (d, 1H, J = 8.4 Hz), 6.73 (ddd, 1H, J = 15, 13, 4.3 Hz), 5.74 (d, 1H, 15 Hz), 5.47 (d, 1H, J = 7.9 Hz), 5.42 (d, 1H, J = 9.1 Hz), 5.29 (d, 1H, J = 9.0 Hz), 4.98 (dd, 1H, J = 10, 3.0 Hz), 4.92 (t, 1H, J = 9.6 Hz), 4.85 (d, 1H, J = 9.1 Hz), 4.77-4.71 (m, 1H), 4.18-4.12 (m, 1H), 3.91 (s, 3H), 3.39 (dd, 1H, J = 13, 8.1 Hz), 3.21 (dd, 1H, J = 13, 9.0 Hz), 3.16 (dd, 1H, J = 14, 5.2 Hz), 3.07 (dd, 1H, J = 14, 7.6 Hz), 2.63-2.52 (m, 2H), 2.45 (dd, 1H, J = 17, 5.8 Hz), 2.36-2.26 (m, 1H), 2.22 (dd, 1H, J = 17, 4.5 Hz), 1.99-1.90 (m, 1H), 1.86-1.69 (m, 2H), 1.47 (s, 9H), 1.43 (s, 9H), 1.25 (s, 3H), 1.19 (s, 3H), 1.11 (d, 3H, J = 7.0 Hz), 1.03 (d, 3H, J = 6.5 Hz), 0.99 (d, 3H, J = 6.4 Hz).

**Preparation of Cryptophycin 55 (L)-aspartate hydrochloride salt (38') (LSN 382502).** To a solution of **37'** (146 mg, 0.149 mmol) in 498 µl of methylene chloride at room temperature was added a 4.0 M solution of hydrogen chloride in 1,4-dioxane (374 µl, 1.99 mmol). After stirring for 19 h, the clear, colorless reaction mixture was concentrated in *vacuo* to provide 131 mg (100%, corrected for 2 wt% dioxane) of the title compound as a white foam: 500 MHz ¹H NMR (MeOH-d₄) δ 7.79 (d, 1H, J = 7.6 Hz), 7.45-7.36 (m, 5H), 7.31 (d, 1H, J = 2.0 Hz), 7.20 (dd, 1H, J = 8.6, 2.0 Hz), 7.01 (d, 1H, J = 8.6 Hz), 6.75 (ddd, 1H, J = 15, 13, 3.7 Hz), 5.96 (dd, 1H, 15, 1.8 Hz), 5. 59 (d, 1H, J = 10 Hz), 5.17 (d, 1H, J = 10 Hz), 5.14 (dd, 1H, J = 11, 2.1 Hz), 9.89 (t, 1H, J = 11Hz), 4.52 (dd, 1H, J = 11, 3.7 Hz), 3.87 (s, 3H), 3.55 (t, 1H, J = 4.4 Hz), 3.52-3.47 (m, 1H), 3.21 (dd, 1H, J = 14, 3.6 Hz), 3.13 (d, 1H, J = 14 Hz), 2.99 (dd, 1H, J = 18, 5.0 Hz), 2.83 (dd, 1H, J = 18, 3.9 Hz), 2.81-2.68 (m, 3H), 2.35-2.28 (m, 1H), 2.02-1.84 (m, 3H), 1.26 (s, 3H), 1.21 (s, 3H), 1.08-1.02 (m, 9H).

**Preparation of Cryptophycin 55 (D)-aspartate hydrochloride salt (40') (LSN 387039).** To a solution of 39' (53 mg, 0.054 mmol) in 271 µl of methylene chloride at room temperature was added a 4.0 M solution of hydrogen chloride in 1,4-dioxane (136 µl, 0.542 mmol). After stirring for 14 h, the clear, colorless reaction mixture was concentrated *in vacuo* to provide 47 mg (94%, corrected for 6 wt% dioxane) of the title compound as a white foam: 500 MHz ¹H NMR (MeOH-d₄) δ 7.79 (d, 1H, J = 7.6 Hz), 7.45-7.36 (m, 5H), 7.31 (d, 1H, J = 2.0 Hz), 7.20 (dd, 1H, J = 8.6, 2.0 Hz), 7.01 (d, 1H, J = 8.6 Hz), 6.71 (ddd, 1H, J = 15, 13, 3.7 Hz), 5.96 (dd, 1H, 15, 1.8 Hz), 5.63 (d, 1H, J = 10 Hz), 5.19 (d, 1H, J = 10 Hz), 5.15 (dd, 1H, J = 11, 2.1 Hz), 4.86-4.80 (m, 1H), 4.55-4.50 (m, 1H), 4.24 (dd, 1H, J = 8.4, 4.0 Hz), 3.87 (s, 3H), 3.50 (dd, 1H, J = 13, 9.7 Hz), 3.21 (dd, 1H, J = 14, 3.6 Hz), 3.14 (dd, 1H, J = 13, 2.5 Hz), 2.80-2.70 (m, 3H), 2.40-2.32 (m, 1H), 2.21 (dd, 1H, J = 18, 4.0 Hz), 2.09-1.97 (m, 2H), 1.92-1.89 (m, 2H), 1.26 (s, 3H), 1.21 (s, 3H), 1.15 (d, 3H, J = 7.1 Hz), 1.08 (d, 3H, J = 6.1 Hz), 1.04 (d, 3H, J = 6.0 Hz).

**Preparation of Cryptophycin 55 N-*t*-Boc-α-*t*-butyl ester-(L)-glutamate (41') (LSN 362572).** To a solution of 1' (23 mg, 0.033 mmol), N-*t*-Boc-(*L*)-glutamic acid α-t-butyl ester (15 mg, 0.049 mmol), and 4-dimethylamino pyridine (0.4 mg, 0.0033 mmol) in 120 µl of anhydrous methylene chloride at room temperature was added a solution of 1,3-dicyclohexylcarbodiimide (10 mg, 0.049 mmol) in 40 µl of methylene chloride. After stirring for 45 min, the cloudy white reaction mixture diluted with ethyl acetate-hexanes (3:1, 0.5 ml), stirred for 10 min, and filtered through a plug of celite, washing with ethyl acetate:hexanes (3:1). The filtrate and washings were concentrated *in vacuo* to an off-white oil. Chromatography (15 g of flash silica gel, 2:1 ethyl acetate-hexanes) afforded 24 mg (75%) of the title compound as a white foam: 500 MHz ¹H NMR (CDCl₃) δ 7.39-7.30 (m, 5H), 7.24 (d, 1H, J = 2.1 Hz), 7.20-7.16 (m, 1H), 7.09 (dd, 1H, J = 8.4, 2.1 Hz), 6.87 (d, 1H, J = 8.4 Hz), 6.74 (ddd, 1H, J = 15, 13, 4. 6 Hz), 5.78 (d, 1H, J = 15 Hz), 5.55 (br d, 1H, J = 6.4 Hz), 5.46 (dd, 1H, J = 9.6, 1.0 Hz), 4.96 (dd, 1H, J = 11, 3.0 Hz), 9.91-4.80 (m, 2H), 9.83 (d, 1H, J = 9.6 Hz), 4.72 (br s, 1H), 3.99 (br s, 1H), 3.91 (s, 3H), 3.39 (dd, 1H, J = 13, 7.9 Hz), 3.24 (dd, 1H, J = 13, 3.9 Hz), 3.18 (dd, 1H, J = 14, 4.5 Hz), 3.09 (dd, 1H, J = 14, 7.8 Hz), 2.63-2.55 (m, 2H), 2.44-2.37 (m, 1H), 2.10-1.92 (m, 3H), 1.85-1.78 (m, 1H), 1.76-1.61 (m, 3H), 1.46 (s, 9H), 1.45 (s, 9H), 1.27 (s, 3H), 1.20 (s, 3H), 1.08 (d, 3H, J = 7.0 Hz), 1.04 (d, 3H, J = 6.6 Hz), 0.98 (d, 3H, J = 6.4 Hz) .

**Preparation of Cryptophycin 55 (L) -γ-glutamate hydrochloride salt (42') (LSN 382514).** To a solution of 41' (21 mg, 0.021 mmol) in 212 µl of methylene chloride at room temperature was added a 4.0 M solution of hydrogen chloride in 1,4-dioxane (53 µl, 0.212 mmol). After stirring for 23.5 h, the reaction mixture was diluted with methanol and passed through a small plug of celite. Concentration in *vacuo* to provide 20 mg (100%, corrected for 6 wt% dioxane) of the title compound as a white foam: 500 MHz ¹H NMR (MeOH-d₄) δ 7.79 (d, 1H, J = 7.6 Hz), 7.50-7.29 (m, 6H), 7.20 (dd, 1H, J = 8.4, 2.0 Hz), 7.01 (d, 1H, J = 8.4 Hz), 6.66 (ddd, 1H, J = 15, 13, 3.7 Hz), 5.97 (dd, 1H, 15, 1.5 Hz), 5.52 (dd, 1H, J = 10, 0.8 Hz), 5.14 (dd, 1H, J = 11, 3.0 Hz), 5.10 (d, 1H, J = 10 Hz), 4.90-4.80 (m, 1H), 4.51 (dd, 1H, J = 11, 3.7 Hz), 3.90-3.80 (m, 1H), 3.87 (s, 3H), 3.50 (dd, 1H, J = 13, 10 Hz), 3.19 (dd, 1H, J = 14, 3.7 Hz), 3.13 (dd, 1H, J = 14, 2.3 Hz), 2.81-2.67 (m, 3H), 2.41-2.32 (m, 2H), 2.15-2.06 (m, 1H), 1.97-1.79 (m, 5H), 1.25 (s, 3H), 1.19 (s, 3H), 1.13 (d, 3H, J = 7.1 Hz), 1.08 (d, 3H, J = 5.9 Hz), 1.04 (d, 3H, J = 5.8 Hz).

**Preparation of Cryptophycin 55 N,N'-di-t-Boc-(S)-2,3-diaminopropionate (43') (LSN 382765).** To a solution of 1' (21 mg, 0.030 mmol), N,N'-di-*t*-Boc-(*S*)-2,3-diaminopropionic acid (18 mg, 0.060 mmol), and 4-dimethylamino pyridine (0.3 mg, 0.0030 mmol) in 110 µl of anhydrous methylene chloride at room temperature was added a solution of 1,3-dicyclohexylcarbodiimide (12 mg, 0.060 mmol) in 39 µl of methylene chloride. After stirring for 70 min, the cloudy white reaction mixture diluted with ethyl acetate-hexanes (3:1, 0.5 ml), stirred for 10 min, and filtered through a plug of celite, washing with ethyl acetate:hexanes (3:1). The filtrate and washings were concentrated in *vacuo* to an off-white oil. Chromatography (15 g of flash silica gel, 2:1 ethyl acetate-hexanes) afforded 24 mg (80%) of the title compound as a white foam: 500 MHz ¹H NMR (MeOH-d₄) 87.49-7.40 (m, 2H), 7.38-7.30 (m, 4H), 7.19 (dd, 1H, J = 8.5, 1.9 Hz), 7.00 (d, 1H, J = 8.5 Hz), 6.74 (ddd, 1H, J = 16, 11, 3.5 Hz), 5.95 (d, 1H, J = 16 Hz), 5.49 (d, 1H, J = 10 Hz), 5.16-5.08 (m, 2H), 4.90-4.80 (m, 1H), 4.51 (dd, 1H, J = 11, 3.7 Hz), 4.06-4.10 (m, 1H), 3. 87 (s, 3H), 3. 98 (d, 1H, J = 13 Hz), 3.19 (dd, 1H, J = 14, 3.7 Hz), 3.14 (d, 1H, J = 13 Hz), 3.03 (dd, 1H, J = 14, 4.2 Hz), 2.94-2.87 (m, 1H), 2.80-2.59 (m, 3H), 2.39-2.30 (m, 1H), 1.98-1.81 (m, 3H), 1.43 (s, 18H), 1.25 (s, 3H), 1.21 (s, 3H), 1.10 (d, 3H, J = 7.0 Hz), 1.07 (d, 3H, J = 6.0 Hz), 1.03 (d, 3H, J = 5.8 Hz).

**Preparation of Cryptophycin 55 (S)-2,3-diaminopropionate dihydrochloride salt (44') (LSN 382764).** To a solution of **43'** (21 mg, 0.021 mmol) in 211 µl of methylene chloride at room temperature was added a 4.0 M solution of hydrogen chloride in 1,4-dioxane (42 µl, 0.169 mmol). After stirring for 5 h, the reaction mixture was concentrated *in vacuo* to to provide 18.5 mg (100%, corrected for 3 wt% dioxane) of the title compound as a white solid: 500 MHz ¹H NMR (MeOH-d₄) δ 7.50-7.39 (m, 5H), 7.31 (d, 1H, J = 2.0 Hz), 7.20 (dd, 1H, J = 8.4, 2.0 Hz), 7.01 (d, 1H, J = 8.4 Hz), 6.68 (ddd, 1H, J = 15, 11, 3.8 Hz), 5.99 (dd, 1H, 15, 1.7 Hz), 5.56 (d, 1H, J = 9.6 Hz), 5.24 (d, 1H, J = 9.6 Hz) 5.16 (dd, 1H, J = 10, 3.1 Hz), 4.90-4.80 (m, 1H), 4.51 (dd, 1H, J = 11, 3.8 Hz), 4.04-4.00 (m, 1H), 3.87 (s, 3H), 3.50-3.45 (m, 1H), 3.26 (dd, 1H, J = 14, 4.0 Hz), 3.22-3.12 (m, 3H), 2.81-2.75 (m, 3H), 2.42-2.32 (m, 1H), 1.98-1.78 (m, 3H), 1.25 (s, 3H), 1.19 (s, 3H), 1.22-1.16 (m, 3H), 1.07 (d, 3H, J = 6.4 Hz), 1.04 (d, 3H, J = 6.3 Hz) .

**Preparation of Cryptophycin 55 N-t-Boc-(L)-serinate (45') (LSN 384340).** To a solution of **1'** (38 mg, 0.053 mmol), N-*t*-Boc-O-*tert*-butyldimethylsilyl(L)-serine¹¹ (51 mg, 0.160 mmol), and 4-dimethylamino pyridine (0.6 mg, 0.0053 mmol) in 200 µl of anhydrous methylene chloride at room temperature was added a solution of 1,3-dicyclohexylcarbodiimide (33 mg, 0.160 mmol) in 69 µl of methylene chloride. After stirring for 4 h, the cloudy white reaction mixture was diluted with ethyl acetate-hexanes (2:1, 1 ml), stirred for 10 min, and filtered through a plug of celite, washing with ethyl acetate:hexanes (2:1). The filtrate and washings were concentrated *in vacuo* to a colorless oil which was used directly in the next step. To a solution of the crude silyl ether (54 mg, 0.054 mmol) in 268 µl of tetrahydrofuran at room temperature was added 268 µl of a stock solution of hydrogen fluoride-pyridine (prepared from 0.5 g of HF•pyridine (Aldrich), 4 ml of tetrahydrofuran, and 1 ml of pyridine). After stirring for 4 h, another 67 µl of stock HF-pyridine solution was added. After stirring for 30 min, the reaction was treated with 0.6 ml of saturated aqueous sodium bicarbonate and washed with ethyl acetate (1 ml X 3). The combined organics were dried (Na₂SO₄), filtered, and concentrated in vacuo to a faint yellow foam. Chromatography (16 g of flash SiO₂ eluting with 3:1 then 6:1 ethyl acetate-hexanes) provided 26 mg (44%) of the title compound as a white foam: 500 MHz ¹H NMR (MeOH-d₄) δ 7.79 (dd, 1H, J = 9.5, 2.4 Hz), 7.45-7.38 (m, 2H), 7.37-7.29 (m, 3H), 7.29 (d, 1H, J = 2.0 Hz), 7.18 (dd, 1H, J = 8.4, 2.0 Hz), 6.99 (d, 1H, J = 8.4 Hz), 6.72 (ddd, 1H, J = 15, 13, 3.7 Hz), 5.95 (dd, 1H, J = 15, 1.6 Hz), 5.53 (d, 1H, J = 9.5 Hz), 5.12-5.08 (m, 2H), 4.94 (t, 1H, J = 10 Hz), 4.52 (dd, 1H, J = 12, 3.6 Hz), 3.92 (t, 1H, J = 4.6 Hz), 3.85 (s, 3H), 3.52-3.46 (m, 3H), 3.20 (dd, 1H, J = 14, 3.5 Hz), 3.14 (dd, 1H, J = 13, 3.0 Hz), 2.75 (dd, 1H, J = 14, 11 Hz), 2.73-2.66 (m, 1H), 2.65-2.59 (m, 1H), 2.38-2.29 (m, 1H), 2.00-1.82 (m, 3H), 1.43 (s, 9H), 1.25 (s, 3H), 1.22 (s, 3H), 1.10-1.06 (m, 6H), 1.03 (d, 3H, 6.0Hz).

**Preparation of Cryptophycin 55 (*L*)-serinate hydrochloride salt (46') (LSN 384339).** To a solution of 45' (26 mg, 0.029 mmol) in 291 µl of methylene chloride at room temperature was added a 4.0 M solution of hydrogen chloride in 1,4-dioxane (36 µl, 0.146 mmol) . After stirring for 2.5 h, the clear, colorless reaction mixture was concentrated *in vacuo* to provide 23 mg (94%, corrected for 2 wt% dioxane) of the title compound as a white foam: 500 MHz ¹H NMR (MeOH-d₄) δ 7.48-7.40 (m, 2H), 7.39-7.31 (m, 3H), 7.31 (d, 1H, J = 2.2 Hz), 7.20 (dd, 1H, J = 8.5, 2.0 Hz), 7.01 (d, 1H, J = 8.5 Hz), 6.70 (ddd, 1H, J = 15, 13, 3.7 Hz), 5.95 (dd, 1H, 15, 1.8 Hz), 5.59 (d, 1H, J = 10 Hz), 5.20 (d, 1H, J = 10 Hz), 5.14 (dd, 1H, J = 11, 2.1 Hz), 4.94 (t, 1H, J = 10 Hz), 4.52 (dd, 1H, J = 3.5 Hz), 3.96 (dd, 1H, J = 11, 3. 9 Hz), 3.83 (dd, 1H, J = 11, 2.8 Hz), 3.66 (t, 1H, J = 3.2 Hz), 3.50 (d, 1H, J = 14 Hz), 3.20 (dd, 1H, J = 14, 3.5 Hz), 3.13 (d, 1H, J = 14 Hz), 2.80-2.71 (m, 3H), 2.38-2.28 (m, 1H), 2.01-1.82 (m, 3H), 1.26 (s, 3H), 1.21 (s, 3H), 1.13 (d, 3H, J = 7.0 Hz), 1.07 (d, 3H, J = 6. 1 Hz), 1.05 (d, 3H, J = 6.1 Hz).

**Preparation of Cryptophycin 55 glycidylglycinate hydrochloride salt (47') (LSN 387750).** To a solution of 1' (18 mg, 0.026 mmol), N-t-Boc-glycidylglycine (12 mg, 0.051 mmol), and 4-dimethylamino pyridine (0.3 mg, 0.0026 mmol) in 128 µl of anhydrous methylene chloride and 28 µl of N,N-dimethylformamide at room temperature was added a solution of 1,3-dicyclohexylcarbodiimide (11 mg, 0.051 mmol) in 28 µl of methylene chloride. After stirring for 3 h, another 24 mg (0.102 mmol) of N-t-Boc-glycidylglycine and 22 mg (0.102 mmol) of 1,3-dicyclohexylcarbodiimide in 30 µl of N,N-dimethylformamide was added. After stirring for 1.5 h, the cloudy white reaction mixture diluted with ethyl acetate-hexanes (3:1, 0.5 ml), stirred for 10 min, and filtered through a plug of celite, washing with ethyl acetate:hexanes (3:1). The filtrate and washings were concentrated *in* vacuo to a colorless oil. Chromatography (12 g of flash silica gel, 1% methanol in ethyl acetate) afforded 12 mg of a white foam which was used as is in the next experiment. To a solution of the N-Boc glycidylglycinate (12 mg, 0.013 mmol) from above in 130 µl of methylene chloride at room temperature was added a 4.0 M solution of hydrogen chloride in 1,4-dioxane (16 µl, 0.065 mmol). After stirring for 3.5 h, the reaction mixture was concentrated *in vacuo* to provide 11 mg (100%) of the title compound as a white foam: 500 MHz ¹H NMR (MeOH-d₄) δ 7.45-7.29 (m, 5H), 7.31 (d, 1H, J = 2.1 Hz), 7.20 (dd, 1H, J = 8.4, 2.1 Hz), 7.01 (d, 1H, J = 8.4 Hz), 6.72 (ddd, 1H, J = 15, 11, 3.8 Hz), 5.96 (dd, 1H, 15, 1.4 Hz), 5.49 (dd, 1H, J = 10, 0.8 Hz), 5.14-5.09 (m, 2H), 4.90-4.80 (m, 1H), 4.51 (dd, 1H, J = 11, 3.9 Hz), 3.91-3.84 (m, 1H), 3.87 (s, 3H), 3.70-3.65 (m, 2H), 3.50 (d, 1H, J = 14Hz), 3.38 (d, 1H, J = 18 Hz), 3.19 (dd, 1H, J = 14, 3.6 Hz), 3.12 (d, 1H, J = 14 Hz), 2.82-2.63 (m, 3H), 2.40-2.30 (m, 1H), 1.97-1.80 (m, 3H), 1.25 (s, 3H), 1.20 (s, 3H), 1.12 (d, 3H, J = 7.0 Hz), 1.06 (d, 3H, J = 6.0 Hz), 1.03 (d, 3H, J = 5.9 Hz).

**Preparation of Cryptophycin 55 3,6,9-tioxadecanoate (48') (LSN 387414).** To a solution of **1'** (18 mg, 0.026 mmol), 3,6,9-trioxadecanoic acid (7.8 µl, 0.051 mmol), and 4-dimethylamino pyridine (0.3 mg, 0.0026 mmol) in 100 µl of anhydrous methylene chloride at room temperature was added a solution of 1,3-dicyclohexylcarbodiimide (11 mg, 0.051 mmol) in 28 µl of methylene chloride. After stirring for 30 min, the cloudy white reaction mixture diluted with ethyl acetate-hexanes (3:1, 0.5 ml), stirred for 10 min, and filtered through a plug of celite, washing with ethyl acetate:hexanes (3:1). The filtrate and washings were concentrated *in vacuo* to an off-white oil. Chromatography (12 g of flash silica gel, 2% methanol in ethyl acetate) afforded 19 mg (86%) of the title compound as a white foam: 500 MHz ¹H NMR (CDCl₃) δ 7.49-7.29 (m, 5H), 7.24 (d, 1H, J = 1.8 Hz), 7.22-7.19 (m, 1H), 7.10 (dd, 1H, J = 8.4, 1.8 Hz), 6.87 (d, 1H, J = 8.4 Hz), 6.74 (ddd, 1H, J = 15, 10, 4.5 Hz), 5.32-5.28 (m, 2H), 5.56 (d, 1H, J = 9.7 Hz), 4.98-4.90 (m, 2H), 4.83 (d, 1H, J = 9.7 Hz), 4.77-4.71 (m, 1H), 3.90 (s, 3H), 3.83 (d, 1H, J = 17 Hz), 3.65-3.55 (m, 7H), 3.42-3.33 (m, 6H), 3.24-3.18 (m, 2H), 3.03 (dd, 1H, J = 15, 8.1 Hz), 2.69-2.61 (m, 1H), 2.61-2.56 (m, 1H), 2.54-2.46 (m, 1H), 2.00-1.93 (m, 1H), 1.85-1.77 (m, 1H), 1.72-1.66 (m, 1H), 1.26 (s, 3H), 1.19 (s, 3H), 1.09 (d, 3H, J = 7.0 Hz), 1.02 (d, 3H, J = 6.6 Hz), 0.97 (d, 3H, J = 6.5 Hz).

## Claims

1. A compound of formula I 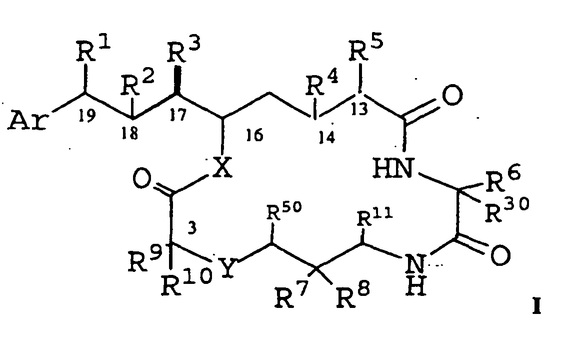 wherein:
Ar is selected from phenyl, an unsubstituted aromatic group, an aromatic group substituted with a halogen atom or a C₁-C₇ alkyl group, an unsubstituted 3- to 8-membered heteroaromatic group, C₁-C₁₂ alkyl, C₂-C₁₂ alkenyl, C₂-C₁₂ alkynyl, NR⁵¹R⁵², OR⁵³, and a group of formula Ar'; 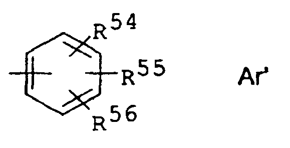
R¹ is selected from halogen and OH;
R² is halogen or OR³¹, provided that one, but not both, of R¹ and R² is halogen;
R³ is a C₁-C₆ alkyl group;
R⁴ is H or OH;
R⁵ is H or OH; or R⁴ and R⁵ may be taken together to form a second bond between C₁₃ and C₁₄;
R⁶ is selected from benzyl, hydroxybenzyl, (C₁-C₃ alkoxy)benzyl, halohydroxybenzyl, dihalohydroxybenzyl, halo(C₁-C₃ alkoxy)benzyl and dihalo(C₁-C₃ alkoxy)benzyl groups;
R⁷ is H or a C₁-C₆ lower alkyl group;
R⁸ is H or a C₁-C₆ lower alkyl group;
R⁹ is H or a C₁-C₆ lower alkyl group;
R¹⁰ is H or a C₁-C₆ lower alkyl group;
R¹¹ is hydrogen;
R³¹ is R³²;
R³² is selected from naturally occurring amino acids, C₁₂H₂₂O₁₁ and C₆H₁₀O₅ carbohydrates, C₁₂H₂₂O₁₁ and C₆H₁₀O₅ carbohydrates which carry from 1 to 3 amino substituents, (saccharide)_{q} wherein saccharide is lactose, maltose, sucrose, fructose or starch and C(O)R³³;
R³³ is R³⁷R³⁸;
R³⁷ is (C₁-C₆)alkyl;
R³⁸ is COOR³⁹, 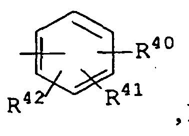 NH₂ or a naturally occurring amino acid;
R³⁹ is H or (C₁-C₆)alkyl;
R⁴⁰, R⁴¹, and R⁴² are each independently selected from hydrogen, OR⁴³, halo, NH₂, NO₂, OPO(OR⁴⁶)₂, OR⁴⁴phenyl, and R⁴⁵;
R⁴³ is C₁-C₆ alkyl;
R⁴⁴ is C₁-C₆ alkylene;
R⁴⁵ is selected from an aromatic group and an aromatic group substituted with a hydrogen atom or a C₁-C₇ alkyl group;
R⁴⁶ is selected from H, Na, and -C(CH₃)₃;
R⁵¹ is selected from hydrogen and C₁-C₃ alkyl;
R⁵² is selected from hydrogen and C₁-C₃ alkyl;
R⁵³ is selected from C₁-C₁₂ alkyl;
R⁵⁴ is selected from hydrogen, C₁-C₆ alkyl, an aromatic group, phenyl, COOR⁵⁷, PO₃H, SO₃H, SO₂R⁵⁸, NR⁵⁹R⁶⁰, NHOR⁶¹, NHCH₂R^{61'}, CN, NO₂, halogen, OR⁶², and SR⁶³;
R⁵⁵ is selected from hydrogen, C₁-C₆ alkyl, an aromatic group, phenyl, COOR⁵⁷, PO₃H, SO₃H, SO₂R⁵⁸, NR⁵⁹R⁶⁰, NHOR⁶¹, NHCH₂R^{61'}, CN, NO₂, halogen, OR⁶², and SR⁶³;
R⁵⁶ is selected from hydrogen, C₁-C₆ alkyl, an aromatic group, phenyl, COOR⁵⁷, PO₃H, SO₃H, SO₂R⁵⁸, NR⁵⁹R⁶⁰, NHOR⁶¹, NHCH₂R^{61'}, CN, NO₂, halogen, OR⁶², and SR⁶³;
R⁵⁷ is selected from hydrogen and C₁-C₁₂ alkyl;
R⁵⁸ is selected from hydrogen and C₁-C₁₂ alkyl;
R⁵⁹ is selected from hydrogen, (C₁-C₆) alkyl and fluorenylmethoxycarbonyl (FMOC);
R⁶⁰ is selected from hydrogen and (C₁-C₆) alkyl;
R⁶¹ is selected from hydrogen, OR⁶⁴, CH₂NHR⁶⁵, NHR⁶⁵ and fluorenylmethoxycarbonyl (FMOC);
R^{61'} is selected from hydrogen, OR⁶⁴, CH₂NHR⁶⁵, NHR⁶⁵ and fluorenyhnethoxycarbonyl (FMOC);
R⁶² is selected from hydrogen and C₁-C₆ alkyl;
R⁶³ is selected from hydrogen and C₁-C₆ alkyl;
R⁶⁴ is selected from hydrogen, (C₁-C₆) alkyl, and CH₂NR⁶⁶R⁶⁷;
R⁶⁵ is selected from hydrogen, C₁-C₆ alkyl, NH₂, and fluorenylmethoxycarbonyl (FMOC);
R⁶⁶ is selected from hydrogen, C₁-C₆ alkyl and fluorenylmethoxycarbonyl (FMOC);
R⁶⁷ is selected from hydrogen and C₁-C₆ alkyl;
q is 2, 3, or 4;
X is O, NH or (C₁-C₇ alkyl)amino; and
Y is C, O, NH, S, SO, SO₂ or (C₁-C₇ alkyl)amino;
or a pharmaceutically acceptable salt or solvate thereof.

2. A compound according to claim 1 wherein Y is O.

3. A compound according to claim 2, wherein X is O.

4. A compound according to claim 3, wherein R⁶ is benzyl, hydroxybenzyl, halohydroxybenzyl, dihalohydroxybenzyl, haloalkoxybenzyl or dihaloalkoxybenzyl, R⁹ is isobutyl and R¹⁰ is hydrogen.

5. A compound selected from Cryptophycin 55 acetate, Cryptophycin 55 succinate, Cryptophycin 55 (2'-di-t-butylphosphatyl) phenylacetate, Cryptophycin 55 (2'-phosphatyl) phenylacetate, Cryptophycin 55 nicotinoate, Cryptophycin 55 N-methylpyridinium, Cryptophycin 55 N-t-Boc-3-(3-chloro-4-methoxyphenyl)-(D)-alaninate, Cryptophycin 55 3-(3-chloro-4-methoxyphenyl)-(D)-alaninate, Cryptophycin 55 N-t-Boc-glycinate, Cryptophycin 55 N-t-Boc-β-alaninate, Cryptophycin 55 N-t-Boc-γ-aminobutyrate, Cryptophycin 55 N-t-Boc-(L)-alaninate, Cryptophycin 55 N-t-Boc-(D)-alaninate, and Cryptophycin 55 Nα-Nε-di-t-Boc-(L)-lysinate; or a pharmaceutically acceptable salt thereof, wherein Cryptophycin 55 is an alcohol of the formula

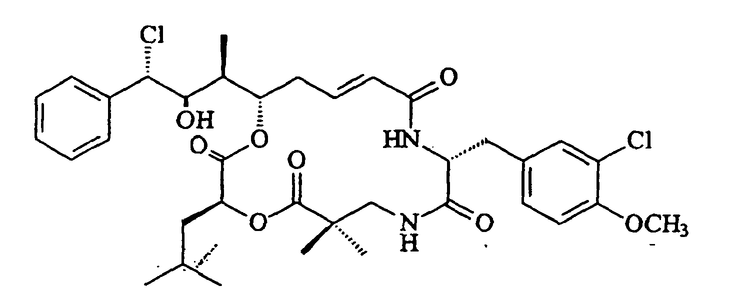

6. A compound selected from 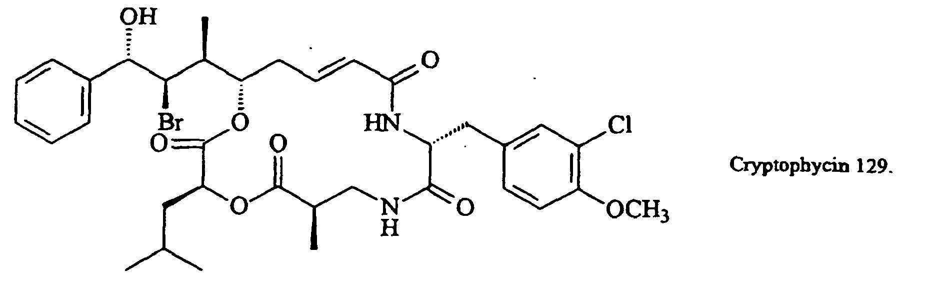 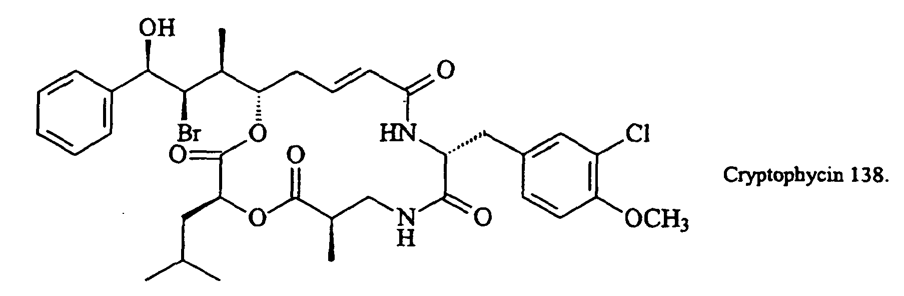 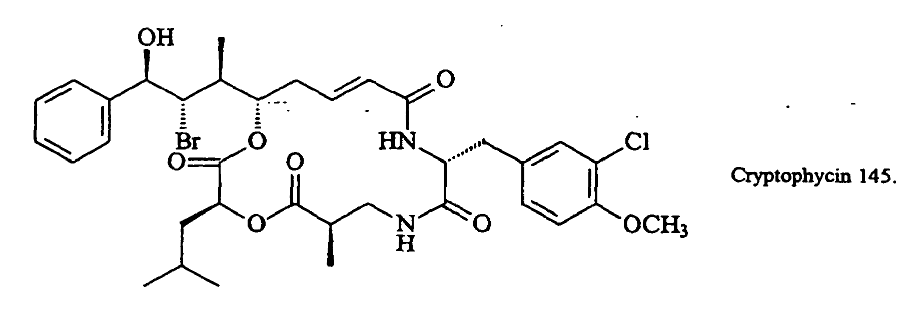 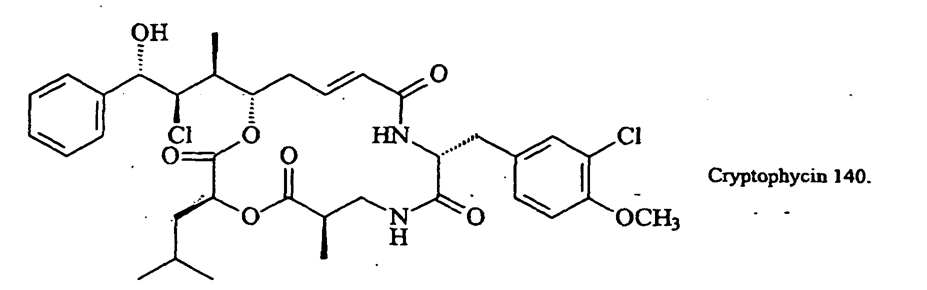 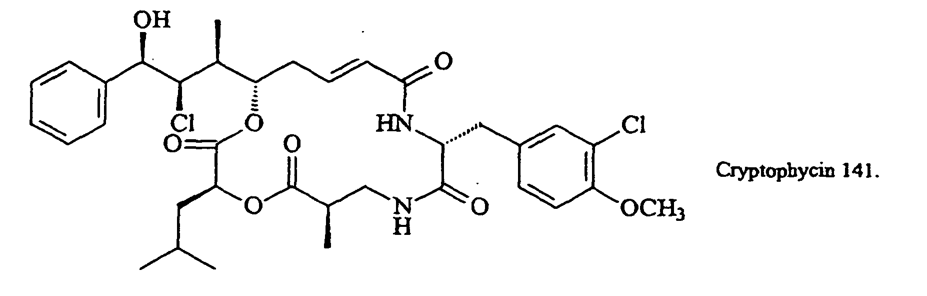 or a pharmaceutically acceptable salt thereof.

7. A compound selected from 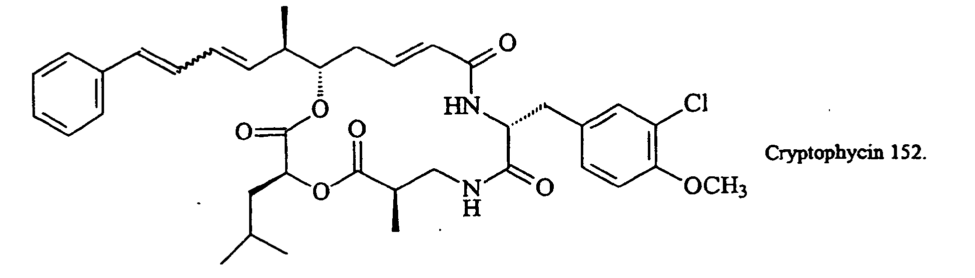 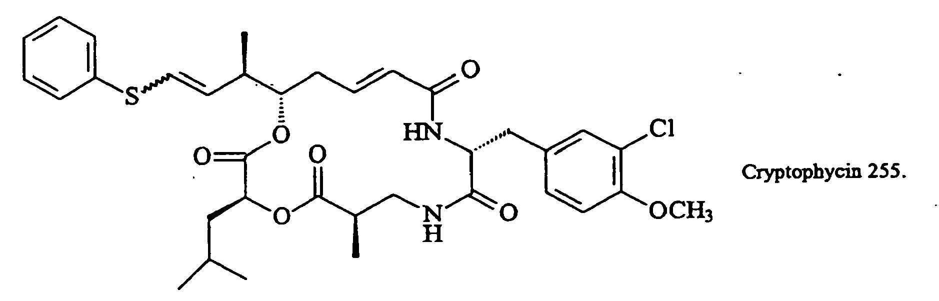 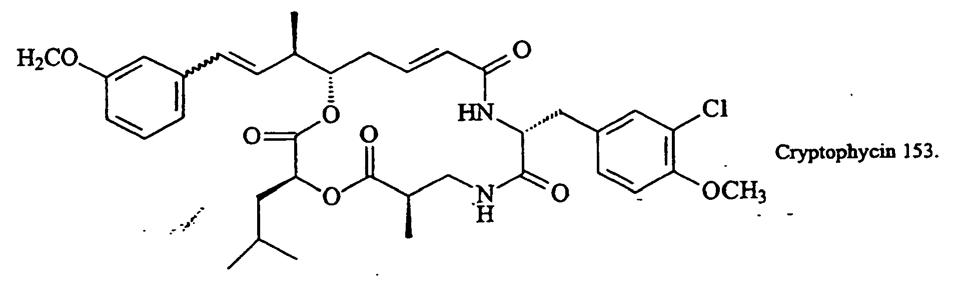 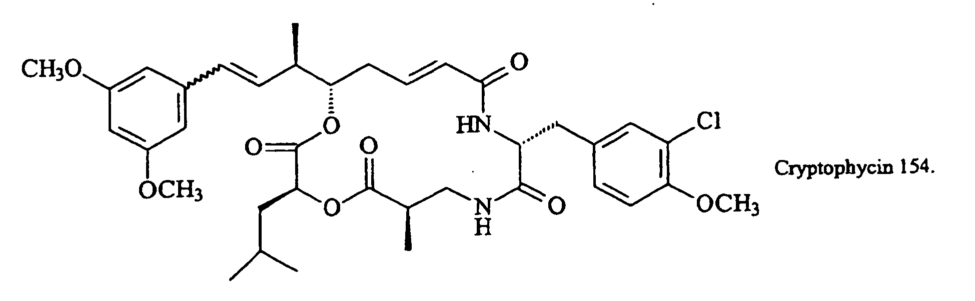 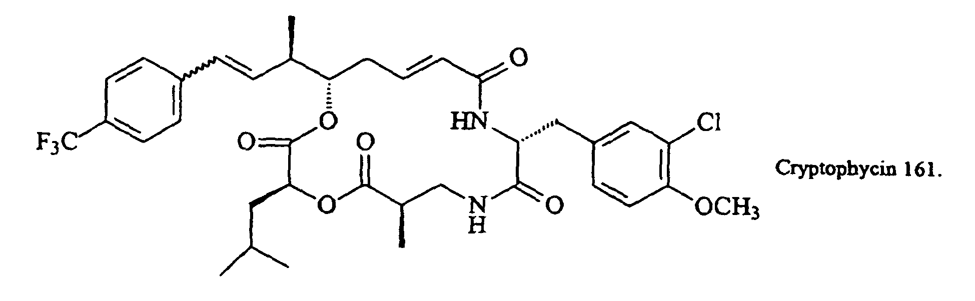 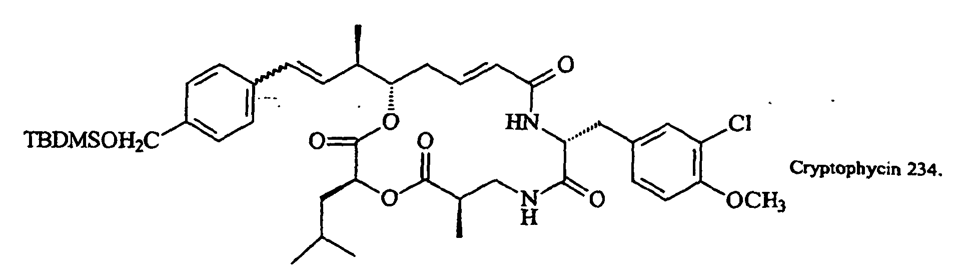 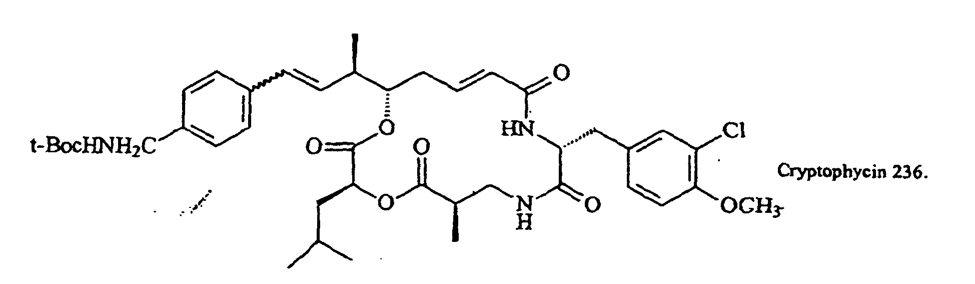 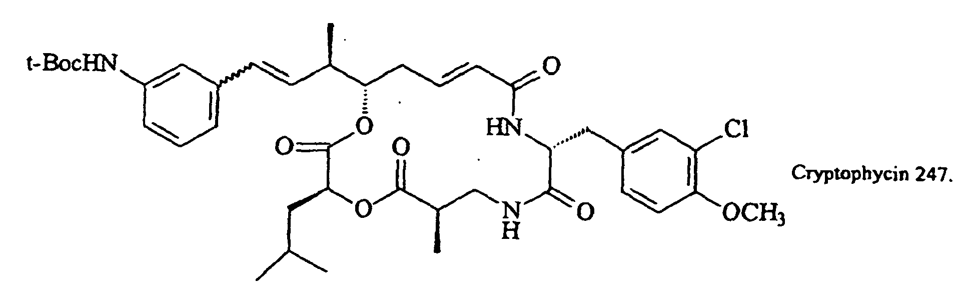 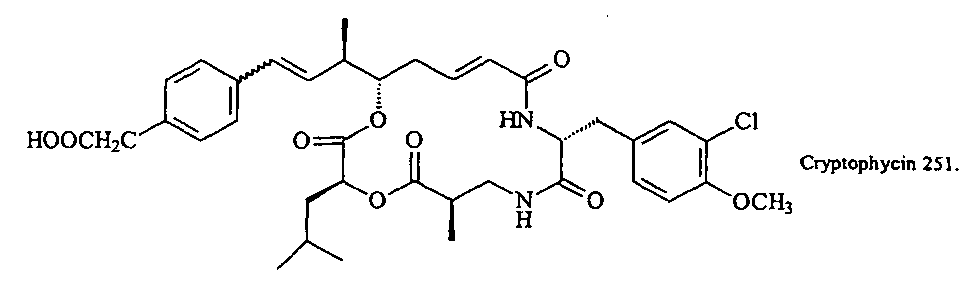 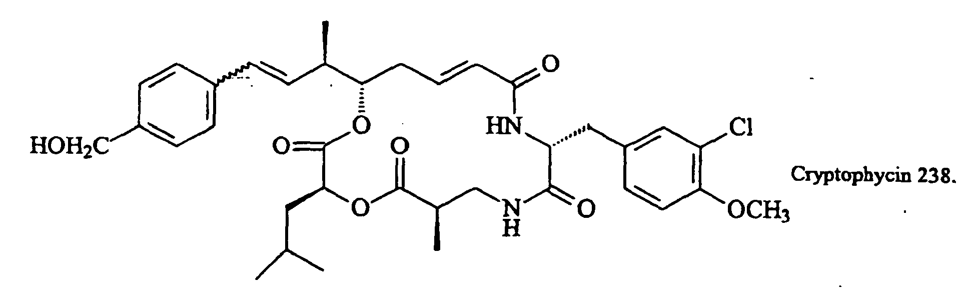 or a pharmaceutically acceptable salt thereof.

8. A compound selected from Cryptophycin 55 succinate tert-butyl ester, Cryptophycin 55 (2'-di-t-butylphosphatyl) phenylacetate, Cryptophycin 55 (2'-phosphatyl) phenylacetate, Cryptophycin 55 nicotinoate, Cryptophycin 55 N-methylpyridinium, Cryptophycin 55 N-t-Boc-3-(3-chloro-4-methoxyphenyl)-(D)-alaninate, Cryptophycin 55 N-t-Boc-(L)-phenylalaninate, Cryptophycin 55 (L)-phenylalaninate, Cryptophycin 55 (L)-histidinate, Cryptophycin 55 N-t-Boc-(L)-prolinate, Cryptophycin 55 (L)-prolinate, Cryptophycin 55 N-t-Boc glycinate, Cryptophycin 55 glycinate, Cryptophycin 55 N-t-Boc-β-alaninate, Cryptophycin 55 β-alaninate, Cryptophycin 55 N-t-Boc-γ-aminobutyrate, Cryptophycin 55 γ-aminobutyrate, Cryptophycin 55 N-t-Boc-(L)-alaninate, Cryptophycin 55 (L)-alaninate, Cryptophycin 55 N-t-Boc-(D)-alaninate, Cryptophycin 55 (D)-alaninate, Cryptophycin 55 Nα-Nε-di-t-Boc-(L)-lysinate, Cryptophycin 55 (L)-lysinate, Cryptophycin 55 Nα-Nε-di-t-Boc-(D)-lysinate, Cryptophycin 55 (D)-lysinate, Cryptophycin 55 N-t-Boc-γ-t-butyl ester-(L)-glutamate, Cryptophycin 55 (L)-α-glutamate, Cryptophycin 55 N-t-Boc-β-t-butyl ester-aspartate, Cryptophycin 55 (D)-aspartate, Cryptophycin 55 N-t-Boc-α-t-butyl ester-(L)-glutamate, Cryptophycin 55 (L)-γ-glutamate, Cryptophycin 55 N,N'-dit-Boc-(S)-2,3-diaminopropionate, Cryptophycin 55 (S)-2,3-diaminopropionate, Cryptophycin 55 N-t-Boc-(L)-serinate, Cryptophycin 55 (L)-serinate, and Cryptophycin 55 succinate; or a pharmaceutically acceptable salt thereof, wherein Cryptophycin 55 is an alcohol as defined in claim 5.

9. A compound according to any one of claims 1 to 4, wherein R⁷ and R⁸ are each methyl.

10. A compound according to any one of claims 1 to 4 and 9, wherein R² is a glycinate.

11. A compound of the formula 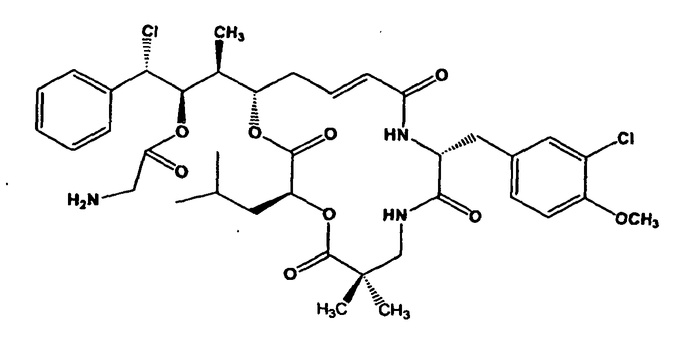 which compound is Cryptophycin 55 glycinate, or a pharmaceutically acceptable salt thereof.

12. A compound according to claim 11 which is the hydrochloride salt of Cryptophycin 55 glycinate.

13. A compound according to any one of the preceding claims for use in treating the human or animal body.

14. A compound according to claim 13, for use in treating a neoplasm in a mammal.

15. A compound according to claim 13 for use in treating a fungal infection in a mammal.

16. Use of a compound according to any one of claims 1 to 12 in the manufacture of a medicament for use in treating a neoplasm in a mammal.

17. Use of a compound according to any one of claims 1 to 12 in the manufacture of a medicament for use in treating a fungal infection in a mammal.

18. A formulation comprising a compound according to any one of claims 1 to 12 and one or more pharmaceutically acceptable carrier or diluent.

## Patentansprüche

1. Verbindung mit der folgenden Formel I 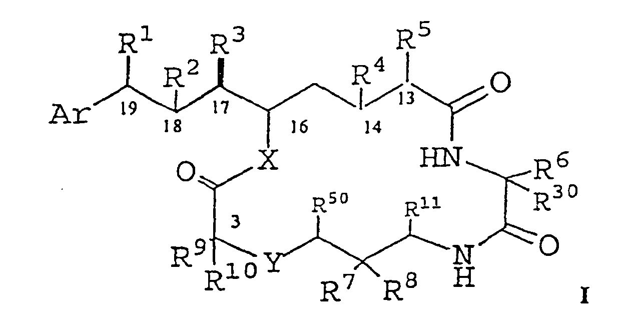 wobei:
Ar ausgewählt ist aus Phenyl, einer unsubstituierten aromatischen Gruppe, einer aromatischen Gruppe, die mit einem Halogenatom oder einer C₁-C₇-Alkylgruppe substituiert ist, einer unsubstituierten heteroaromatischen Gruppe mit 3 bis 8 Elementen, C₁-C₁₂-Alkyl, C₂-C₁₂-Alkenyl, C₂-C₁₂-Alkynyl, NR⁵¹R⁵², OR⁵³ und einer Gruppe der Formel Ar'; 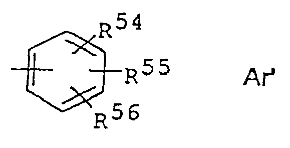
R¹ aus Halogen und OH ausgewählt ist;
R² Halogen oder OR³¹ ist,mit der Maßgabe, daß eines, jedoch nicht beide, von R¹ und R² Halogen ist;
R³ eine C₁-C₆-Alkylgruppe ist;
R⁴ H oder OH ist;
R⁵ H oder OH ist; oder R⁴ und R⁵ zusammen verwendet werden können, um eine zweite Bindung zwischen C₁₃ und C₁₄ zu bilden;
R⁶ ausgewählt ist aus Benzyl-, Hydroxybenzyl-, (C₁-C₃-Alkoxy)benzyl-, Halogenhydroxybenzyl-, Dihalogenhydroxybenzyl-, Halogen(C₁-C₃-Alkoxy)benzyl- und Dihalogen(C₁-C₃-Alkoxy)benzylgruppen;
R⁷ H oder eine niedere C₁-C₆-Alkylgruppe ist;
R⁸ H oder eine niedere C₁-C₆-Alkylgruppe ist;
R⁹ H oder eine niedere C₁-C₆-Alkylgruppe ist;
R¹⁰ H oder eine niedere C₁-C₆-Alkylgruppe ist;
R¹¹ Wasserstoff ist;
R³¹ R³² ist;
R³² ausgewählt ist aus natürlich vorkommenden Aminosäuren, C₁₂H₂₂O₁₁- und C₆H₁₀O₅-Kohlenwasserstoffen, C₁₂H₂₂O₁₁- und C₆H₁₀O₅-Kohlenwasserstoffen, die 1 bis 3 Aminosubstituenten tragen, (Saccharid)_{q}, wobei Saccharid Lactose, Maltose, Sucrose, Fructose oder Stärke und C(O)R³³ ist;
R³³ R³⁷R³⁸ ist;
R³⁷ (C₁-C₆)Alkyl ist;
R³⁸COOR³⁹, 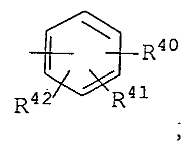 NH₂ oder eine natürlich vorkommende Aminosäure ist;
R³⁹ H oder (C₁-C₆)Alkyl ist;
R⁴⁰, R⁴¹ und R⁴² jeweils unabhängig ausgewählt sind aus Wasserstoff, OR⁴³, Halogen, NH₂, NO₂, OPO(OR⁴⁶)₂, OR⁴⁴-Phenyl und R⁴⁵;
R⁴³ C₁-C₆-Alkyl ist;
R⁴⁴ C₁-C₆-Alkylen ist;
R⁴⁵ ausgewählt ist aus einer aromatischen Gruppe und einer mit einem Wasserstoffatom oder einer C₁-C₇-Alkylgruppe substituierten aromatischen Gruppe;
R⁴⁶ ausgewählt ist aus H, Na und -C(CH₃)₃;
R⁵¹ ausgewählt ist aus Wasserstoff und C₁-C₃-Alkyl;
R⁵² ausgewählt ist aus Wasserstoff und C₁-C₃-Alkyl;
R⁵³ ausgewählt ist aus C₁-C₁₂-Alkyl;
R⁵⁴ ausgewählt ist aus Wasserstoff, C₁-C₆-Alkyl, einer aromatischen Gruppe, Phenyl, COOR⁵⁷, PO₃H, SO₃H, SO₂R⁵⁸, NR⁵⁹R⁶⁰, NHOR⁶¹, NHCH₂R^{61'}, CN, NO₂, Halogen, OR⁶² und SR⁶³;
R⁵⁵ ausgewählt ist aus Wasserstoff, C₁-C₆-Alkyl, einer aromatischen Gruppe, Phenyl, COOR⁵⁷, PO₃H, SO₃H, SO₂R⁵⁸, NR⁵⁹R⁶⁰, NHOR⁶¹, NHCH₂R^{61'}, CN, NO₂, Halogen, OR⁶² und SR⁶³;
R⁵⁶ ausgewählt ist aus Wasserstoff, C₁-C₆-Alkyl, einer aromatischen Gruppe, Phenyl, COOR⁵⁷, PO₃H, SO₃H, SO₂R⁵⁸, NR⁵⁹R⁶⁰, NHOR⁶¹, NHCH₂R^{61'}, CN, NO₂, Halogen, OR⁶² und SR⁶³;
R⁵⁷ ausgewählt ist aus Wasserstoff und C₁-C₁₂-Alkyl;
R⁵⁸ ausgewählt ist aus Wasserstoff und C₁-C₁₂-Alkyl;
R⁵⁹ ausgewählt ist aus Wasserstoff, (C₁-C₆)-Alkyl und Fluorenylmethoxycarbonyl (FMOC);
R⁶⁰ ausgewählt ist aus Wasserstoff und (C₁-C₆)-Alkyl;
R⁶¹ ausgewählt ist aus Wasserstoff, OR⁶⁴, CH₂NHR⁶⁵, NHR⁶⁵ und Fluorenylmethoxycarbonyl (FMOC);
R^{61'} ausgewählt ist aus Wasserstoff, OR⁶⁴, CH₂NHR⁶⁵, NHR⁶⁵ und Fluorenylmethoxycarbonyl (FMOC);
R⁶² ausgewählt ist aus Wasserstoff und C₁-C₆-Alkyl;
R⁶³ ausgewählt ist aus Wasserstoff und C₁-C₆-Alkyl;
R⁶⁴ ausgewählt ist aus Wasserstoff, (C₁-C₆)-Alkyl und CH₂NR⁶⁶R⁶⁷;
R⁶⁵ ausgewählt ist aus Wasserstoff, C₁-C₆-Alkyl, NH₂ und Fluorenylmethoxycarbonyl (FMOC);
R⁶⁶ ausgewählt ist aus Wasserstoff, C₁-C₆-Alkyl und Fluorenylmethoxycarbonyl (FMOC);
R⁶⁷ ausgewählt ist aus Wasserstoff und C₁-C₆-Alkyl;
q 2, 3 oder 4 ist;
X O, NH oder (C₁-C₇-Alkyl)amino ist; und
Y C, O, NH, S,SO, SO, oder (C₁-C₇-Alkyl)amino ist;
oder ein pharmazeutisch akzeptables Salz oder Solvat davon ist.

2. Verbindung nach Anspruch 1, wobei Y O ist.

3. Verbindung nach Anspruch 2, wobei X O ist.

4. Verbindung nach Anspruch 3, wobei R⁶ Benzyl, Hydroxybenzyl, Halogenhydroxybenzyl, Dihalogenhydroxybenzyl, Halogenalkoxybenzyl oder Dihalogenalkoxybenzyl ist, R⁹ Isobutyl ist und R¹⁰ Wasserstoff ist.

5. Verbindung, die ausgewählt ist aus Cryptophycin-55-Acetat, Cryptophycin-55-Succinat, Cryptophycin-55(2'-di-t-Butylphosphatyl)phenylacetat, Cryptophycin-55(2'-Phosphatyl)phenylacetat, Cryptophycin-55-Nicotinoat, Cryptophycin-55-N-Methylpyridinium, Cryptophycin-55-N-t-Boc-3-(3-chloro-4-methoxyphenyl)-(D)-Alaninat, Cryptophycin-55-3(3-chloro-4-methoxyphenyl)-(D)-Alaninat, Cryptophycin-55-N-t-Boc-glycinat, Cryptophycin-55-N-t-Boc-β-Alaninat, Cryptophycin-55-N-t-Boc-γ-Aminobutyrat, Cryptophycin-55-N-t-Boc-(L)-Alaninat, Cryptophycin-55-N-t-Boc-(D)-Alaninat und Cryptophycin-55-Nα-Nε-di-t-Boc-(L)-Lysinat; oder einem pharmazeutisch akzeptablen Salz davon, wobei Cryptophycin 55 ein Alkohol der folgenden Formel ist:

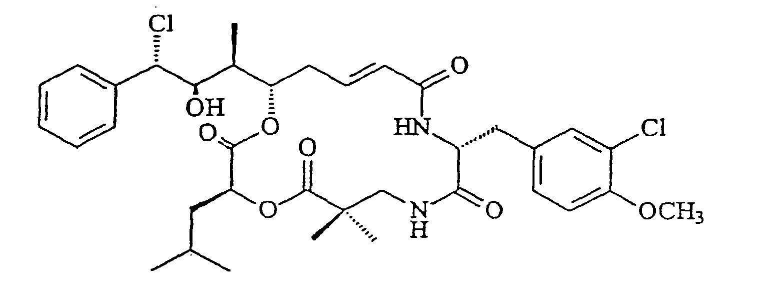

6. Verbindung, die ausgewählt ist aus 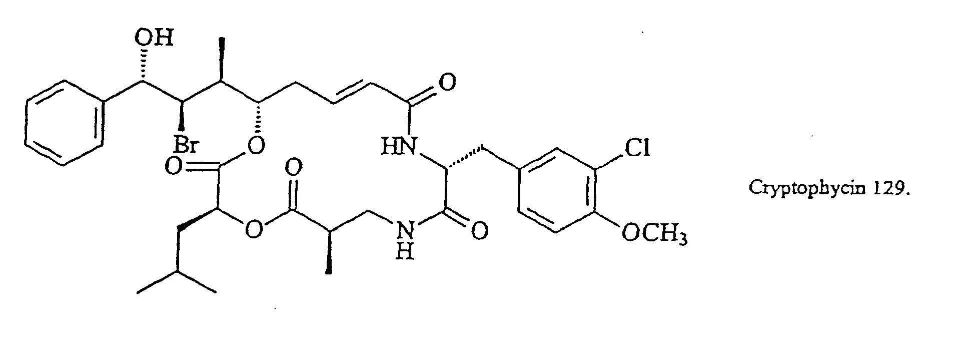 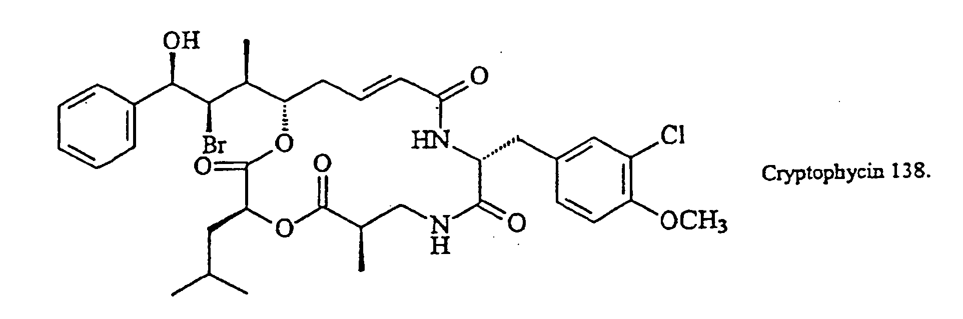 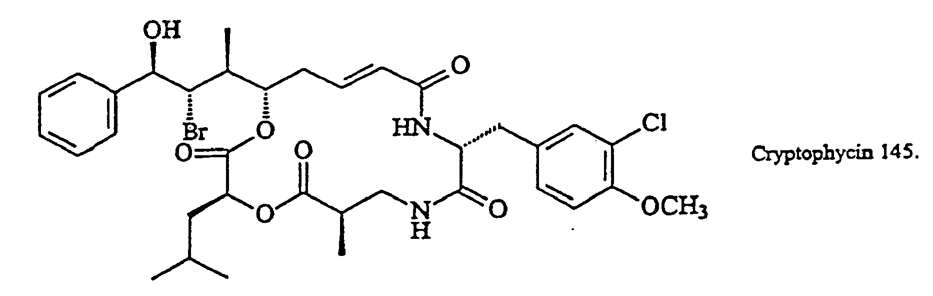 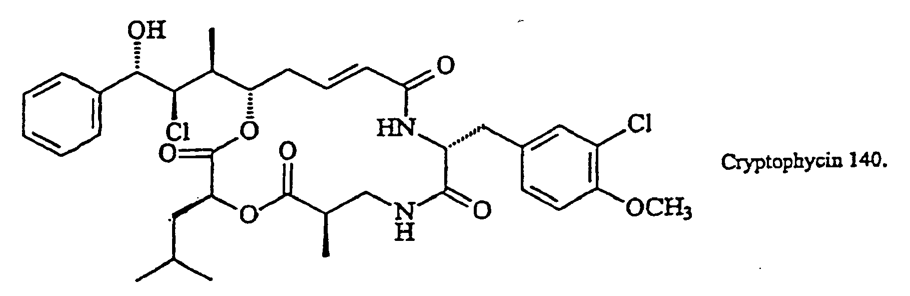 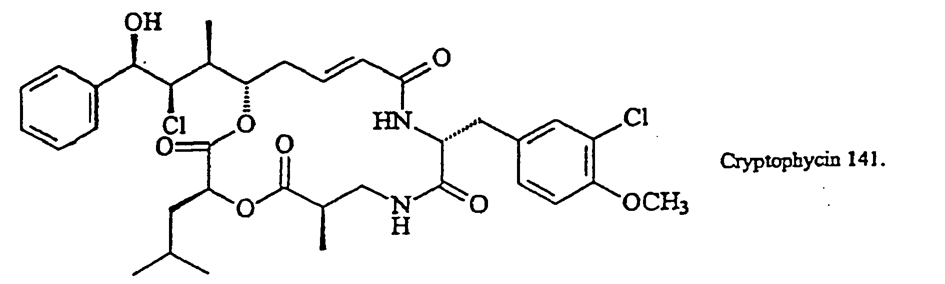 oder einem pharmazeutisch akzeptablen Salz davon.

7. Verbindung, die ausgewählt ist aus 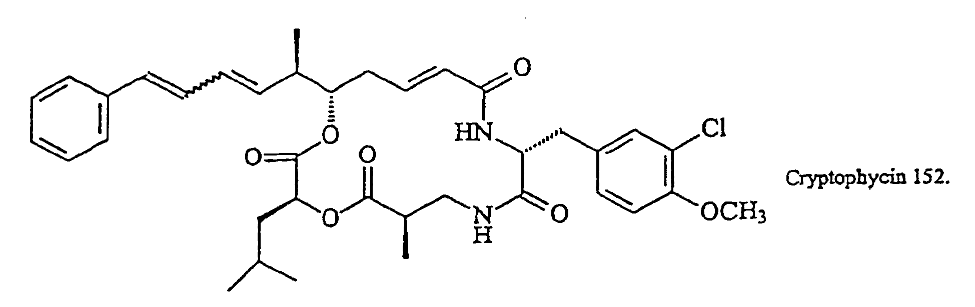 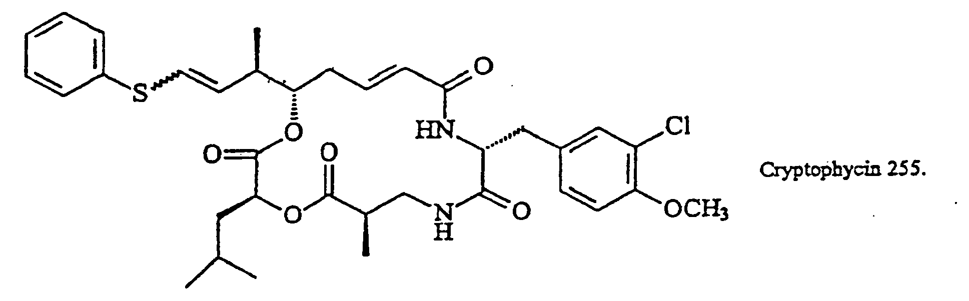 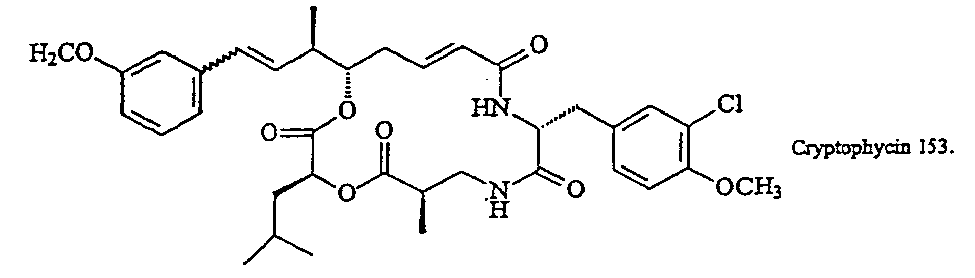 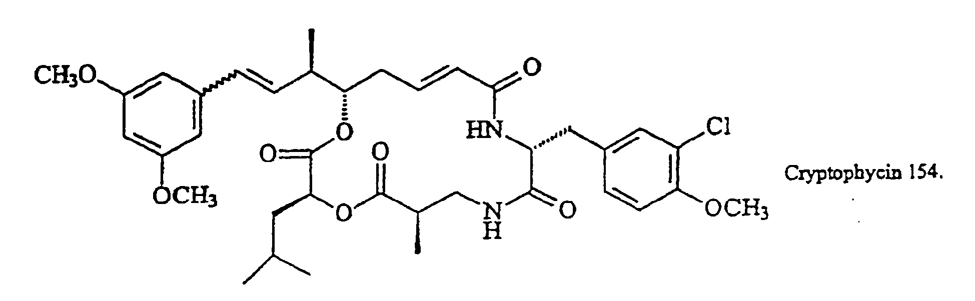 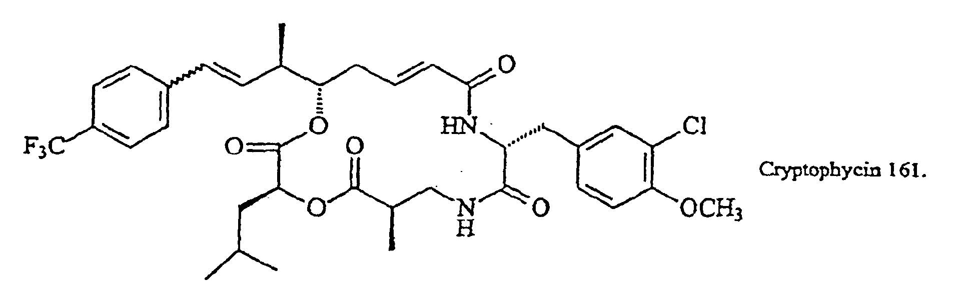 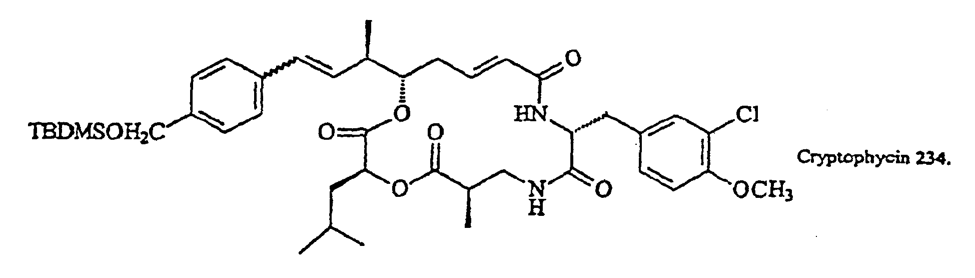 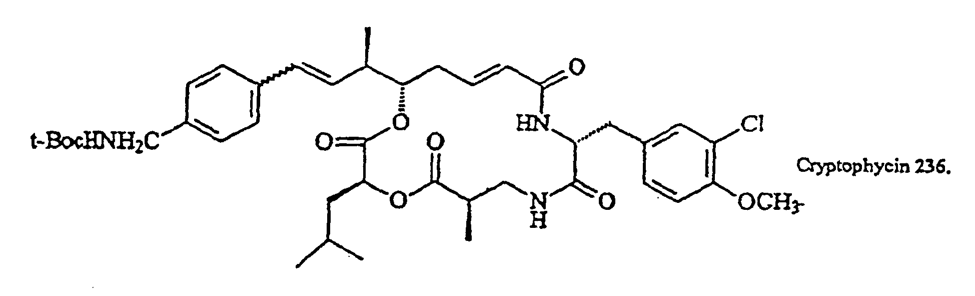 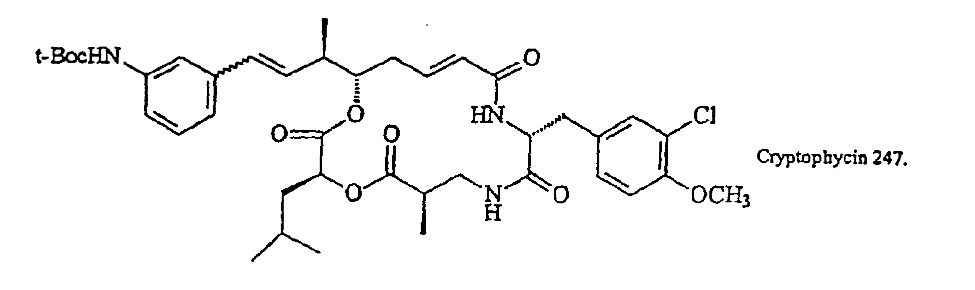 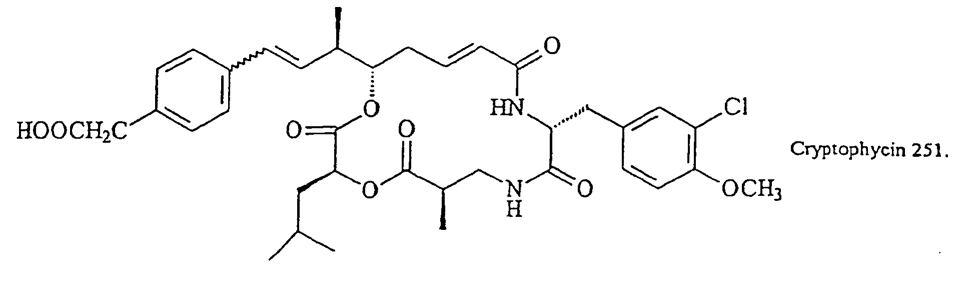 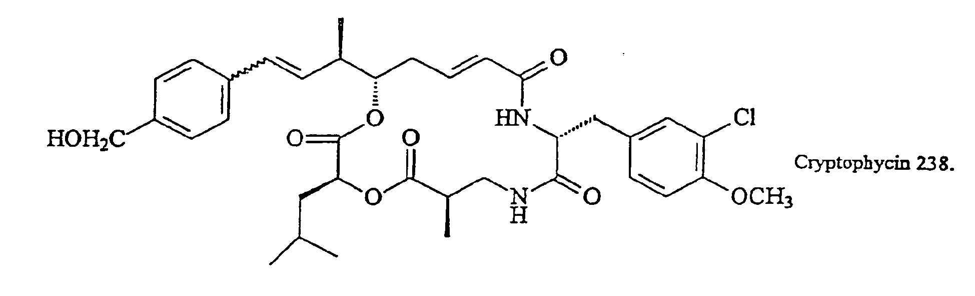 oder einem pharmazeutisch akzeptablen Salz davon.

8. Verbindung, die ausgewählt ist aus Cryptophycin-55-Succinat-tert.-Butylester, Cryptophycin-55-(2'-Di-t-butylphosphatyl)phenylacetat, Cryptophycin-55-(2'-Phosphatyl)phenylacetat, Cryptophycin-55-Nicotinoat, Cryptophycin-55-N-Methylpyridinium, Cryptophycin-55-N-t-Boc-3-(3-chloro-4-methoxyphenyl)-(D)-Alaninat, Cryptophycin-55-N-t-Boc-(L)-Phenylalaninat, Cryptophycin-55-(L)-Phenylalaninat, Cryptophycin-55-(L)-Histidinat, Cryptophycin-55-N-t-Boc-(L)-Prolinat, Cryptophycin-55-(L)-Prolinat, Cryptophycin-55-N-t-Boc-Glycinat, Cryptophycin-55-Glycinat, Cryptophycin-55-N-t-Boc-β-Alaninat, Cryptophycin-55-β-Alaninat, Cryptophycin-55-N-t-Boc-γ-Aminobutyrat, Cryptophycin-55-γ-Aminobutyrat, Cryptophycin-55-N-t-Boc-(L)-Alaninat, Cryptophycin-55-(L)-Alaninat, Cryptophycin-55-N-t-Boc-(D)-Alaninat, Cryptophycin-55-(D)-Alaninat, Cryptophycin-55-Nα-Nε-di-t-Boc-(L)-Lysinat, Cryptophycin-55-(L)-Lysinat, Cryptophycin-55-Nα-Nε-di-t-Boc-(D)-Lysinat, Cryptophycin-55-(D)-Lysinat, Cryptophycin-55-N-t-Boc-γ-t-Butylester-(L)-Glutamat, Cryptophycin-55-(L)-α-Glutamat, Cryptophycin-55-N-t-Boc-β-t-Butylester-Aspartat, Cryptophycin-55-(D)-Aspartat, Cryptophycin-55-N-t-Boc-α-t-Butylester-(L)-Glutamat, Cryptophycin-55-(L)-γ-Glutamat, Cryptophycin-55-N,N'-Di-t-Boc-(S)-2,3-Diaminopropionat, Cryptophycin-55-(S)-2,3-Diaminopropionat, Cryptophycin55-N-t-Boc-(L)-Serinat, Cryptophycin-55-(L)-Serinat und Cryptophycin-55-Succinat; oder einem pharmazeutisch akzeptablen Salz davon, wobei Cryptophycin 55 ein Alkohol wie in Anspruch 5 definiert ist.

9. Verbindung nach einem der Ansprüche 1 bis 4, wobei R⁷ und R⁸ jeweils Methyl ist.

10. Verbindung nach einem der Ansprüche 1 bis 4 und 9, wobei R² ein Glycinat ist.

11. Verbindung der Formel 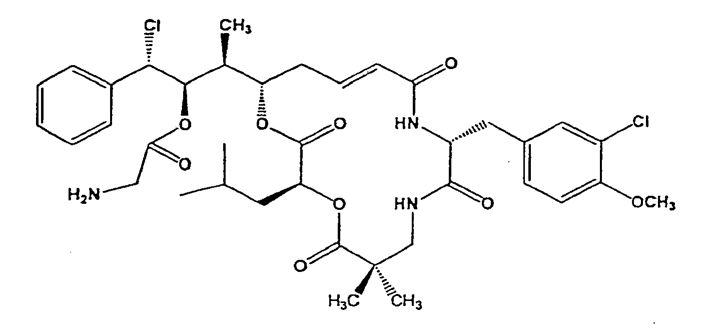 wobei die Verbindung Cryptophycin-55-Glycinat oder ein pharmazeutisch akzeptables Salz davon ist.

12. Verbindung nach Anspruch 11, welche das Hydrochloridsalz von Cryptophycin-55-Glycinat ist.

13. Verbindung nach einem der vorhergehenden Ansprüche zum Gebrauch bei der Behandlung des menschlichen oder tierischen Körpers.

14. Verbindung nach Anspruch 13 zum Gebrauch bei der Behandlung eines Neoplasmas bei einem Säuger.

15. Verbindung nach Anspruch 13 zum Gebrauch bei der Behandlung einer Pilzinfektion bei einem Säuger.

16. Verwendung einer Verbindung nach einem der Ansprüche 1 bis 12 bei der Herstellung eines Medikaments zum Gebrauch bei der Behandlung eines Neoplasmas bei einem Säuger.

17. Verwendung einer Verbindung nach einem der Ansprüche 1 bis 12 bei der Herstellung eines Medikaments zum Gebrauch bei der Behandlung einer Pilzinfektion bei einem Säuger.

18. Formulierung, die eine Verbindung nach einem der Ansprüche 1 bis 12 und einen oder mehrere pharmazeutisch akzeptable Träger oder Verdünner aufweist.

## Revendications

1. Composé de formule I 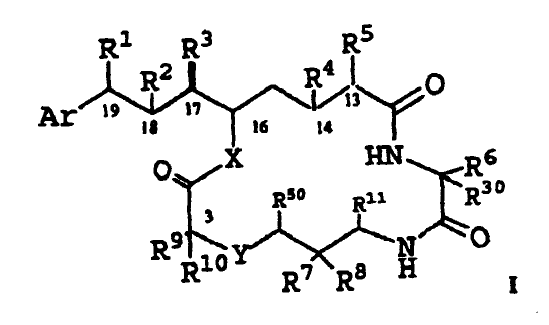 dans lequel :
Ar est choisi parmi un phényle, un groupe aromatique non substitué, un groupe aromatique substitué par un atome d'halogène ou un groupe alkyle en C₁ à C₇, un groupe hétéroatomique de 3 à 8 éléments non substitué, un alkyle en C₁ à C₁₂, un alcényle en C₂ à C₁₂, un alcynyle en C₂ à C₁₂, NR⁵¹R⁵², OR⁵³, et un groupe de formule Ar' : 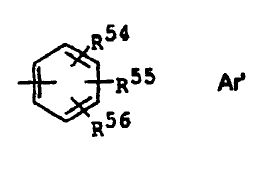
R¹ est choisi parmi un halogène et OH ;
R² est un halogène ou OR³¹, à condition que l'un des R¹ et R², mais pas les deux, soit un halogène ;
R³ est un groupe alkyle en C₁ à C₆;
R⁴ est H ou OH ;
R⁵ est H ou OH ; ou R⁴ ou R⁵ peuvent être pris ensemble pour former une seconde liaison entre C₁₃ et C₁₄ ;
R⁶ est choisi parmi les groupes benzyle, hydroxybenzyle, (alcoxy en C₁-C₃)benzyle, halohydroxybenzyle, dihalo-hydroxybenzyle, halo(alcoxy en C₁-C₃)benzyle, et dihalo (alcoxy en C₁-C₃) benzyle ;
R⁷ est H ou un groupe alkyle inférieur en C₁ à C₆ ;
R⁸ est H ou un groupe alkyle inférieur en C₁ à C₆ ;
R⁹ est H ou un groupe alkyle inférieur en C₁ à C₆ ;
R¹⁰ est H ou un groupe alkyle inférieur en C₁ à C₆ ;
R¹¹ est l'hydrogène ;
R³¹ est R³²;
R³² est choisi parmi les acides aminés présents dans la nature, les glucides C₁₂H₂₂O₁₁ et C₆H₁₀O₅ ; les glucides C₁₂H₂₂O₁₁ et C₆H₁₀O₅ qui portent 1 à 3 substituants amines ; (saccharide)_{q} dans lequel le saccharide est le lactose, le maltose, le sucrose, le fructose ou l'amidon et C(O)R³³ ;
R³³ est R³⁷R³⁸ ;
R³⁷ est un alkyle en C₁ à C₆ ;
R³⁸ est COOR³⁹, 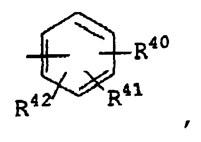 NH₂, ou un acide aminé présent dans la nature ;
R³⁹ est H ou un alkyle en C₁ à C₆;
R⁴⁰, R⁴¹, et R⁴² sont chacun choisis indépendamment parmi l' hydrogène, OR⁴³, halo, NH₂, NO₂, OPO (OR⁴⁶)₂, OR⁴⁴phényl, et R⁴⁵ ;
R⁴³ est un alkyle en C₁ à C₆ ;
R⁴⁴ est un alkylène en C₁ à C₆ ;
R⁴⁵ est choisi parmi un groupe aromatique et un groupe aromatique substitué par un atome d'hydrogène ou un groupe alkyle en C₁-C₇;
R⁴⁶ est choisi parmi H, Na, et -C(CH₃)₃ ;
R⁵¹ est choisi parmi l'hydrogène et un alkyle en C₁ à C₃ ;
R⁵² est choisi parmi l'hydrogène et un alkyle en C₁ à C₃ ;
R⁵³ est choisi parmi un alkyle en C₁ à C₁₂ ;
R⁵⁴ est choisi parmi l'hydrogène, un alkyle en C₁ à C₆, un groupe aromatique, un phényle, COOR⁵⁷, PO₃H, SO₃H, SO₂R⁵⁸, NR⁵⁹R⁶⁰, NHOR⁶¹, NHCH₂R^{61'}, CN, NO₂, un halogène, OR⁶², et SR⁶³;
R⁵⁵ est choisi parmi l'hydrogène, un alkyle en C₁ à C₆, un groupe aromatique, un phényle, COOR⁵⁷, PO₃H, SO₃H, SO₂R⁵⁸, NR⁵⁹R⁶⁰, NHOR⁶¹, NHCH₂R^{61'}, CN, NO₂, un halogène, OR⁶², et SR⁶³ ;
R⁵⁶ est choisi parmi l'hydrogène, un alkyle en C₁ à C₆, un groupe aromatique, un phényle, COOR⁵⁷, PO₃H, SO₃H, SO₂R⁵⁸, NR⁵⁹R⁶⁰, NHOR⁶¹, NHCH₂R^{61'}, CN, NO₂, un halogène, OR⁶², et SR⁶³ ;
R⁵⁷ est choisi parmi l'hydrogène et un alkyle en C₁ à C₁₂ ;
R⁵⁸ est choisi parmi l'hydrogène et un alkyle en C₁ à C₁₂ ;
R⁵⁹ est choisi parmi l'hydrogène, un alkyle en C₁ à C₆, et un fluorénylméthoxycarbonyle (FMOC) ;
R⁶⁰ est choisi parmi l'hydrogène et un alkyle en C₁ à C₆ ;
R⁶¹ est choisi parmi l'hydrogène, OR⁶⁴, CH₂NHR⁶⁵, NHR⁶⁵ et un fluorénylméthoxycarbonyle (FMOC) ;
R^{61'} est choisi parmi l'hydrogène, OR⁶⁴, CH₂NHR⁶⁵, NHR⁶⁵ et un fluorénylméthoxycarbonyle (FMOC);
R⁶² est choisi parmi l'hydrogène et un alkyle en C₁ à C₆;
_{R}⁶³ est choisi parmi l'hydrogène et un alkyle en C₁ à C₆;
R⁶⁴ est choisi parmi l'hydrogène, un alkyle en C₁ à C₆ ; et CH₂NR⁶⁶R⁶⁷;
R⁶⁵ est choisi parmi l'hydrogène, un alkyle en C₁ à C₆, NH₂, et un fluorénylméthoxycarbonyle (FMOC) ;
R⁶⁶ est choisi parmi l'hydrogène, un alkyle en C₁ à C₆, et un fluorénylméthoxycarbonyle (FMOC) ;
R⁶⁷ est choisi parmi l'hydrogène et un alkyle en C₁ à C₆ ;
q vaut 2, 3 ou 4 ;
X est O, NH ou un (alkyle en C₁ à C₇)amino ; et
Y est C, O, NH, S, SO, SO₂ ou un (alkyle en C₁ à C₇)amino ;
ou un sel ou un solvate de ceux-ci, pharmaceutiquement acceptable.

2. Composé selon la revendication 1, dans lequel Y est O.

3. Composé selon la revendication 2, dans lequel X est O.

4. Composé selon la revendication 3, dans lequel R⁶ est le benzyle, l'hydroxybenzyle, l'halohydroxybenzyle, le dihalohydroxybenzyle, l'haloalcoxybenzyle ou le dihaloalcoxybenzyle, R⁹ est l'isobutyle et R¹⁰ est l'hydrogène.

5. Composé choisi parmi l'acétate de cryptophycine 55, le succinate de cryptophycine 55, le (2'-di-tert-butylphosphatyl)phénylacétate de cryptophycine 55, le (2'-phosphatyl)phénylacétate de cryptophycine 55, le nicotinoate de cryptophycine 55, le N-méthylpyridinium de cryptophycine 55, le N-tert-Boc-3-(3-chloro-4-méthoxyphényl)-(D)-alaninate de cryptophycine 55, le 3-(3-chloro-4-méthoxyphényl)-(D)-alaninate de cryptophycine 55, le N-tert-Boc-glycinate de cryptophycine 55, le N-tert-Boc-β-alaninate de cryptophycine 55, le N-tert-Boc-γ-aminobutyrate de cryptophycine 55, le N-tert-Boc-(L)-alaninate de cryptophycine 55, le N-tert-Boc-(D)-alaninate de cryptophycine 55, et le Nα-Nε-di-tert-Boc-(L)-lysinate de cryptophycine 55 ; ou un sel pharmaceutiquement acceptable de ceux-ci, dans lequel la cryptophycine 55 est un alcool de formule :

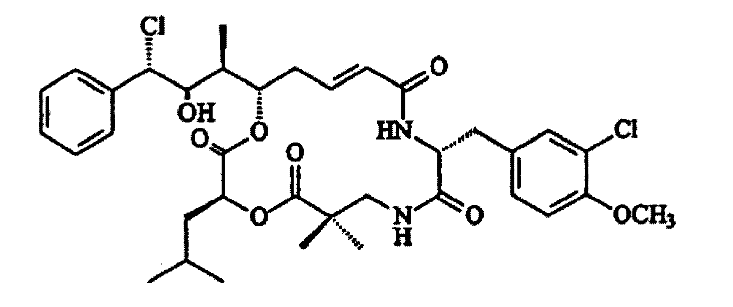

6. Composé choisi parmi
la cryptophycine 129, 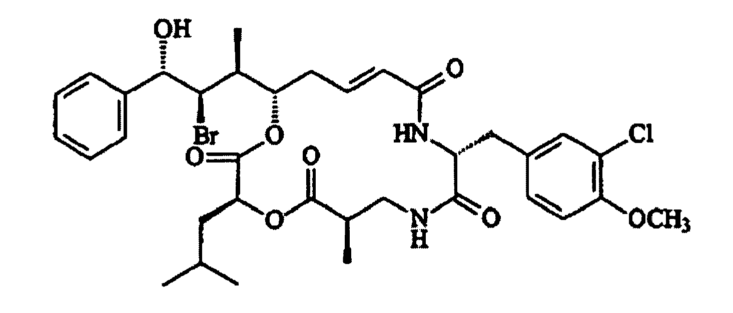 la cryptophycine 138, 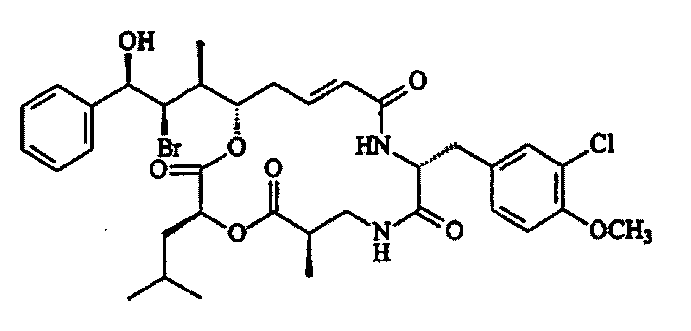 la cryptophycine 145, 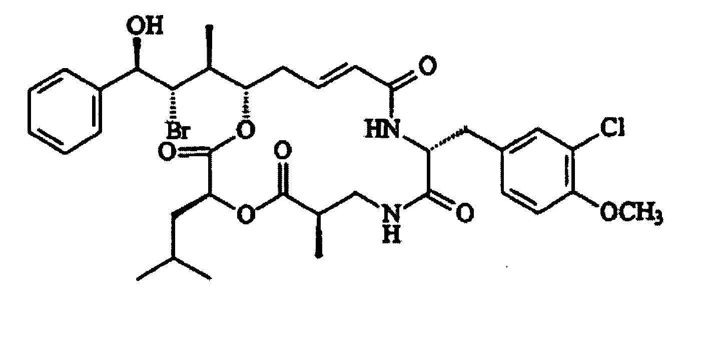 la cryptophycine 140, 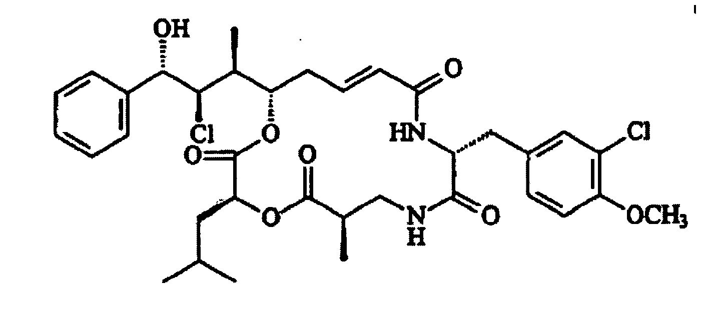 la cryptophycine 141, 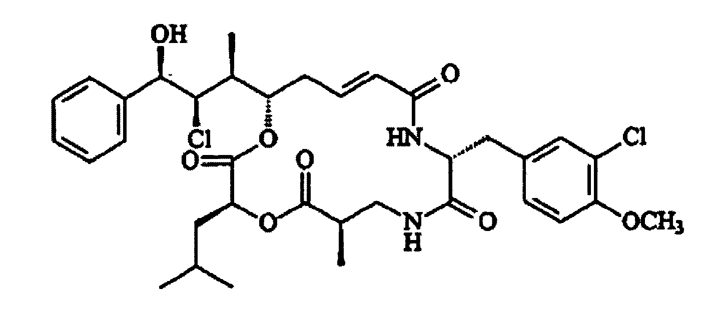 ou un sel pharmaceutiquement acceptable de ceux-ci.

7. Composé choisi parmi
la cryptophycine 152, 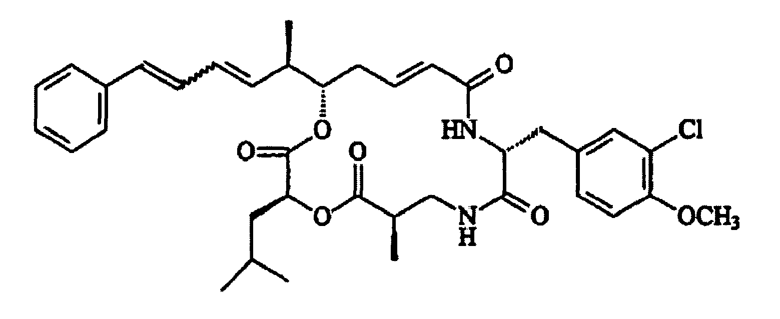 la cryptophycine 255, 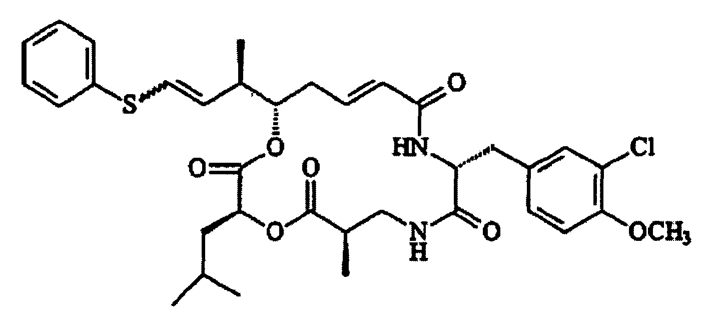 la cryptophycine 153, 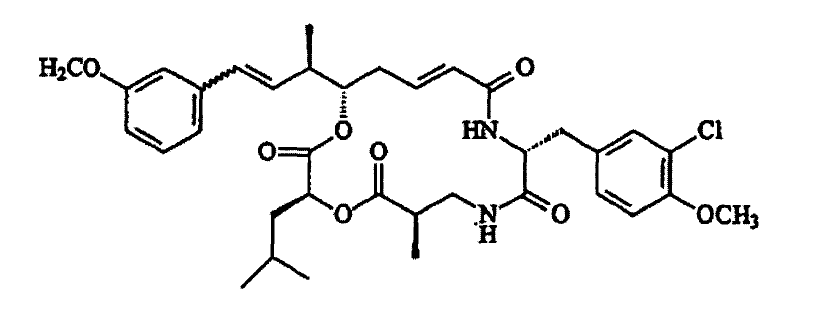 la cryptophycine 154, 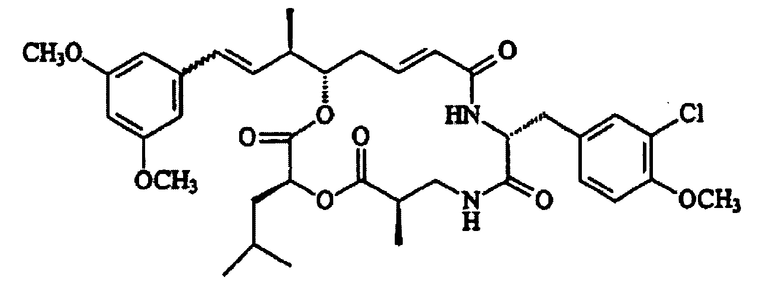 la cryptophycine 161, 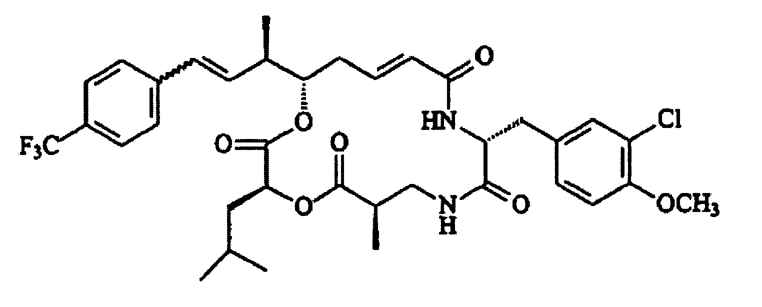 la cryptophycine 234, 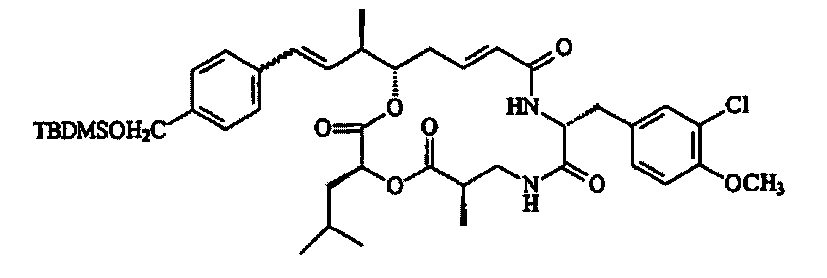 la cryptophycine 236, 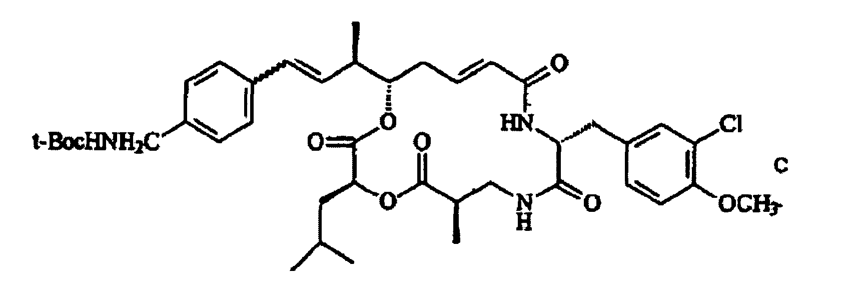 la cryptophycine 247, 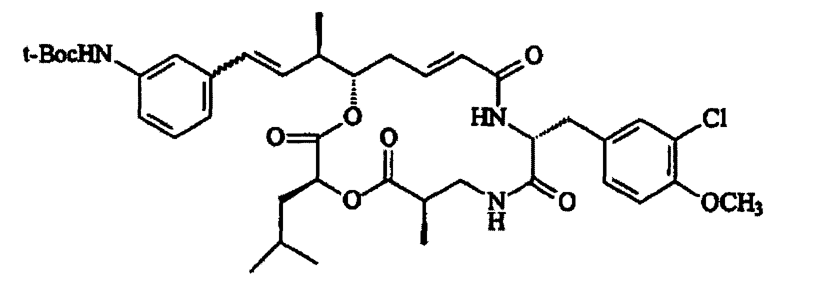 la cryptophycine 251, 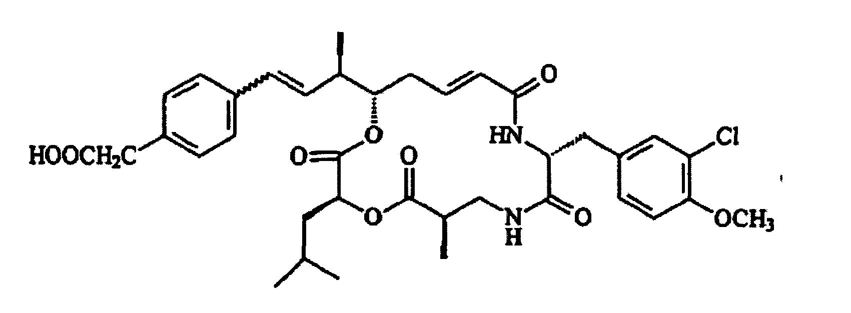 la cryptophycine 238, 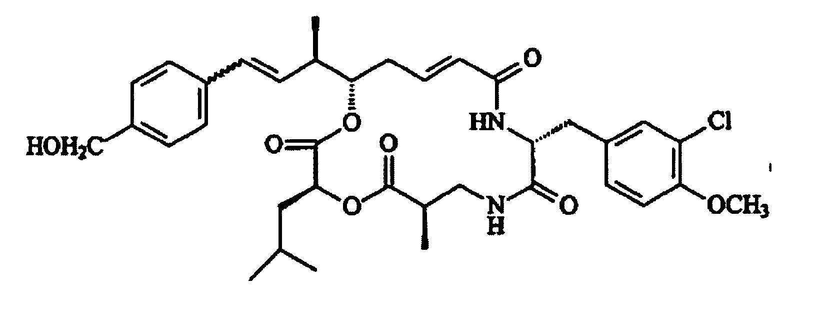 ou un sel pharmaceutiquement acceptable de ceux-ci.

8. Composé choisi parmi le succinate tert-butyl ester de cryptophycine 55, le (2'-di-tert-butylphosphatyl)phénylacétate de cryptophycine 55, le (2'-phosphatyl)phénylacétate de cryptophycine 55, le nicotinoate de cryptophycine 55, le N-méthylpyridinium de cryptophycine 55, le N-tert-Boc-3-(3-chloro-4-méthoxyphényl)-(D)-alaninate de cryptophycine 55, le N-tert-Boc-(L)-phénylalaninate de cryptophycine 55, le (L)-phénylalaninate de cryptophycine 55, le (L)-histidinate de cryptophycine 55, le N-tert-Boc-(L)-prolinate de cryptophycine 55, le (L)-prolinate de cryptophycine 55, le N-tert-Boc-glycinate de cryptophycine 55, le glycinate de cryptophycine 55, le N-tert-Boc-β-alaninate de cryptophycine 55, le β-alaninate de cryptophycine 55, le N-tert-Boc-γ-aminobutyrate de cryptophycine 55,1e γ-aminobutyrate de cryptophycine 55, le N-tert-Boc-(L)-alaninate de cryptophycine 55, le (L)-alaninate de cryptophycine 55, le N-tert-Boc-(D)-alaninate de cryptophycine 55, le (D)-alaninate de cryptophycine 55, le Nα-Nε-di-tert-Boc-(L)-lysinate de cryptophycine 55 ; le (L)-lysinate de cryptophycine 55, le Nα-Nε-di-tert-Boc-(D)-lysinate de cryptophycine 55, le(D)-lysinate de cryptophycine 55, le N-tert-Boc-γ-tert-butyl ester-(L)-glutamate de cryptophycine 55, le (L)-α-glutamate de cryptophycine 55, le N-tert-Boc-β-tert-butyl ester aspartate de cryptophycine 55, le (D)-aspartate de cryptophycine 55, le N-tert-Boc-α-tert-butyl ester-(L)-glutamate de cryptophycine 55, le (L)-γ-glutamate de cryptophycine 55, le N,N'-di-tert-Boc-(S)-2,3-diaminopropionate de cryptophycine 55, le (S)-2,3-diaminopropionate de cryptophycine 55, le N-tert-Boc-(L)-serinate de cryptophycine 55, le (L)-serinate de cryptophycine 55 et le succinate de cryptophycine 55 ; ou un sel pharmaceutiquement acceptable de ceux-ci, dans lequel la cryptophycine 55 est un alcool tel que défini dans la revendication 5.

9. Composé selon l'une quelconque des revendications 1 à 4, dans lequel R⁷ et R⁸ sont chacun le méthyle.

10. Composé selon l'une quelconque des revendications 1 à 4 et la revendication 9, dans lequel R² est un glycinate.

11. Composé de formule 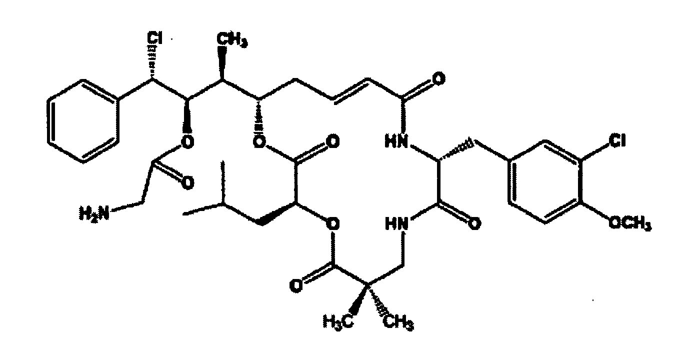 lequel composé est le glycinate de cryptophycine 55, ou un sel pharmaceutiquement acceptable de celui-ci.

12. Composé selon la revendication 11 lequel est le sel hydrochlorure du glycinate de cryptophycine 55.

13. Composé selon l'une quelconque des revendications précédentes pour une utilisation dans le traitement du corps humain ou animal.

14. Composé selon la revendication 13, pour une utilisation dans le traitement d'un néoplasme chez un mammifère.

15. Composé selon la revendication 13, pour une utilisation dans le traitement d'une infection mycosique chez un mammifère.

16. Utilisation d'un composé selon l'une quelconque des revendications 1 à 12 dans la fabrication d'un médicament pour une utilisation dans le traitement d'un néoplasme chez un mammifère.

17. Utilisation d'un composé selon l'une quelconque des revendications 1 à 12 dans la fabrication d'un médicament pour une utilisation dans le traitement d'une infection mycosique chez un mammifère.

18. Formulation comprenant un composé selon l'une quelconque des revendications 1 à 12 et un ou plusieurs supports ou diluants pharmaceutiquement acceptables.
